(19) Europäisches Patentamt / European Patent Office / Office européen des brevets

(11) **EP 2 509 600 B1**

(12) **EUROPEAN PATENT SPECIFICATION**

(45) Date of publication and mention of the grant of the patent:
**02.08.2017 Bulletin 2017/31**

(21) Application number: **10796233.4**

(22) Date of filing: **09.12.2010**

(51) Int Cl.:
***A61K 31/495*** (2006.01) ***A61K 31/496*** (2006.01)
***A61K 31/506*** (2006.01) ***A61K 31/551*** (2006.01)
***C07D 243/08*** (2006.01) ***C07D 405/12*** (2006.01)
***C07D 213/72*** (2006.01) ***C07D 239/42*** (2006.01)
***C07D 471/04*** (2006.01) ***C07D 295/192*** (2006.01)
***A61P 35/00*** (2006.01)

(86) International application number:
**PCT/US2010/059778**

(87) International publication number:
**WO 2011/072174 (16.06.2011 Gazette 2011/24)**

(54) **THERAPEUTICALLY ACTIVE COMPOUNDS FOR USE IN THE TREATMENT OF CANCER CHARACTERIZED AS HAVING AN IDH MUTATION**

THERAPEUTISCH AKTIVE VERBINDUNGEN ZUR VERWENDUNG BEI DER BEHANDLUNG VON KREBS MIT IDH-MUTATION

COMPOSÉS THÉRAPEUTIQUEMENT ACTIFS DESTINÉS AU TRAITEMENT D'UN CANCER CARACTÉRISÉ PAR UNE MUTATION IDH

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.12.2009 US 285122 P**
**12.03.2010 US 313532 P**

(43) Date of publication of application:
**17.10.2012 Bulletin 2012/42**

(73) Proprietor: **Agios Pharmaceuticals, Inc.**
**Cambridge, MA 02139 (US)**

(72) Inventors:
- **SALITURO, Francesco G.**
**Marlborough**
**MA 01752 (US)**
- **SAUNDERS, Jeffrey O.**
**Lincoln**
**Massachusetts 01773 (US)**

(74) Representative: **Bösl, Raphael Konrad**
**Isenbruck Bösl Hörschler LLP**
**Patentanwälte**
**Prinzregentenstraße 68**
**81675 München (DE)**

(56) References cited:
**WO-A1-2006/070198** **WO-A1-2009/013126**
**WO-A1-2010/105243** **WO-A1-2011/002817**
**WO-A2-2004/073619** **WO-A2-2004/074438**
**FR-A1- 2 735 127**

- **DATABASE REGISTRY [Online] CHEMICAL ABSTRACTS SERVICE, COLUMBUS, OHIO, US; 1 April 2005 (2005-04-01), XP002626839, Database accession no. 847757-57-7**
- **DATABASE REGISTRY [Online] CHEMICAL ABSTRACTS SERVICE, COLUMBUS, OHIO, US; 29 May 2008 (2008-05-29), XP002626840, Database accession no. 1023444-33-8**
- **DATABASE REGISTRY [Online] CHEMICAL ABSTRACTS SERVICE, COLUMBUS, OHIO, US; 21 July 2004 (2004-07-21), XP002626841, Database accession no. 713505-78-3**
- **DATABASE REGISTRY [Online] CHEMICAL ABSTRACTS SERVICE, COLUMBUS, OHIO, US; 28 December 2008 (2008-12-28), XP002626842, Database accession no. 1090629-29-0**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 509 600 B1

- YAN HAI ET AL: "IDH1 and IDH2 mutations in gliomas.", THE NEW ENGLAND JOURNAL OF MEDICINE 19 FEB 2009 LNKD-PUBMED:19228619, vol. 360, no. 8, 19 February 2009 (2009-02-19), pages 765-773, XP002626843, ISSN: 1533-4406
- BALSS JOERG ET AL: "Analysis of the IDH1 codon 132 mutation in brain tumors", ACTA NEUROPATHOLOGICA, vol. 116, no. 6, December 2008 (2008-12), pages 597-602, XP002626844, ISSN: 0001-6322

## Description

CLAIM OF PRIORITY

This application claims priority from U.S.S.N. 61/285,122, filed December 9, 2009 and U.S.S.N. 61/313,532, filed March 12, 2010.

BACKGROUND OF INVENTION

Isocitrate dehydrogenase, also known as IDH, is an enzyme which participates in the citric acid cycle. It catalyzes the third step of the cycle: the oxidative decarboxylation of isocitrate, producing alpha-ketoglutarate ($\alpha$-ketoglutarate or $\alpha$-KG) and $CO_2$ while converting NAD+ to NADH. This is a two-step process, which involves oxidation of isocitrate (a secondary alcohol) to oxalosuccinate (a ketone), followed by the decarboxylation of the carboxyl group beta to the ketone, forming alpha-ketoglutarate. Another isoform of the enzyme catalyzes the same reaction; however this reaction is unrelated to the citric acid cycle, is carried out in the cytosol as well as the mitochondrion and peroxisome, and uses NADP+ as a cofactor instead of NAD+.

It has also been discovered that a neoactivity associated with IDH mutants and that the product of the neoactivity can be significantly elevated in cancer cells. While not wishing to be bound by theory it is believed that the balance between the production and elimination of neoactive product, e.g., 2HG, e.g., R-2HG, is important in disease. Neoactive mutants can increase the level of neoactive product, while other processes, e.g., in the case of 2HG, e.g., R-2HG, enzymatic degradation of 2HG, e.g., by 2HG dehydrogenase, reduce the level of neoative product. An incorrect balance is associated with disease. Accordingly, there is an ongoing need for modulators of IDH mutants having alpha hydroxyl neoactivity.

SUMMARY OF INVENTION

Described herein are compounds, compositions (e.g., pharmaceutical compositions) for use in a method of treating cancer. The compounds and compositions can be used to modulate an isocitrate dehydrogenase (IDH) mutant (e.g., IDH1m or IDH2m) having alpha hydroxyl neoactivity. Also described herein are kits comprising a compound or composition of this invention. Note that subject-matter which is not encompassed by the scope of the claims does not form part of the presently claimed invention. The present invention is directed to the use of a compound of formula (I) or a pharmaceutically acceptable salt thereof:

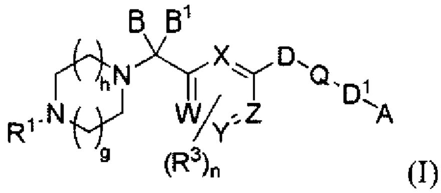

(I)

wherein:

- W, X, Y and Z are each independently selected from CH or N;
- B and $B^1$ are independently selected from hydrogen and alkyl or when taken together with the carbon to which they are attached form a carbonyl group;
- Q is C=O or $SO_2$;
- D and $D^1$ are independently selected from a bond, oxygen and $NR^c$; wherein at least one of D and D1 is $NR^c$;
- A is aryl or heteroaryl each substituted with 0-3 occurrences of $R^2$;
- $R^1$ is independently selected from alkyl, acyl, cycloalkyl, aryl, heteroaryl, heterocyclyl, heterocyclylalkyl, cycloalkylalkyl, aralkyl, and heteroaralkyl; each of which may be optionally substituted with 0-3 occurrences of $R^d$;
- each $R^2$ is independently selected from halo, hydroxy, haloalkyl, aryl, heteroaryl, alkyl, $-NR^cR^{c'}$, alkyl-$NR^cR^{c'}$, $-OR^a$, -C(O)OH, $-C(O)OR^b$, $-C(O)NR^cR^{c'}$, cycloalkyl, heterocyclyl, heterocyclylalkyl, cycloalkylalkyl, aralkyl, and heteroaralkyl;
- each $R^3$ is independently selected from halo, haloalkyl, alkyl, alkenyl, alkynyl, heterocyclyl and $-OR^a$, or two adjacent $R^3$s (when n is 2) taken together with the carbon atoms they are attached to form an optionally substituted heterocyclyl;
- each $R^a$ is independently selected from alkyl, alkoxy, alkylalkoxy, alkylalkoxylalkoxy, alkyl-$C(O)OR^b$, alkyl-$C(O)OR^b$, and haloalkyl;

each $R^b$ is independently alkyl;
each $R^c$ and $R^{c'}$ is independently selected from hydrogen, alkyl, alkyl-C(O)O$R^b$ and alkenyl;
each $R^d$ is independently selected from halo, haloalkyl, alkyl, nitro, cyano, and -O$R^a$, or two $R^d$ taken together with the carbon atoms to which they are attached form an optionally substituted heterocyclyl;
n is 0, 1, or 2;
h is 0, 1, 2; and
g is 0, 1 or 2;

in the manufacture of a medicament for treating a cancer characterized as having an IDH mutation in a subject in need thereof.

Furthermore, the present invention is directed to a compound of formula (I) or a pharmaceutically acceptable salt thereof:

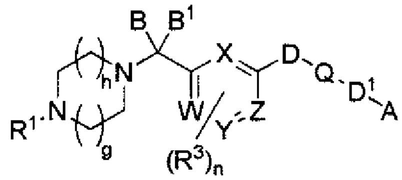

(I)

wherein:

W, X, Y and Z are each independently selected from CH or N;
B and $B^1$ are independently selected from hydrogen and alkyl or when taken together with the carbon to which they are attached form a carbonyl group;
Q is C=O or $SO_2$;
D and $D^1$ are independently selected from a bond, oxygen and N$R^c$; wherein at least one of D and D1 is N$R^c$;
A is aryl or heteroaryl each substituted with 0-3 occurrences of $R^2$;
$R^1$ is independently selected from alkyl, acyl, cycloalkyl, aryl, heteroaryl, heterocyclyl, heterocyclylalkyl, cycloalkylalkyl, aralkyl, and heteroaralkyl; each of which may be optionally substituted with 0-3 occurrences of $R^d$;
each $R^2$ is independently selected from halo, hydroxy, haloalkyl, aryl, heteroaryl, alkyl, -N$R^c$ $R^{c'}$, alkyl-N$R^c$ $R^{c'}$, -O$R^a$, -C(O)OH, -C(O)O$R^b$,-C(O)N$R^c$$R^{c'}$, cycloalkyl, heterocyclyl, heterocyclylalkyl, cycloalkylalkyl, aralkyl, and heteroaralkyl;
each $R^3$ is independently selected from halo, haloalkyl, alkyl, alkenyl, alkynyl, heterocyclyl and -O$R^a$, or two adjacent $R^3$s (when n is 2) taken together with the carbon atoms they are attached to form an optionally substituted heterocyclyl;
each $R^a$ is independently selected from alkyl, alkoxy, alkylalkoxy, alkylalkoxylalkoxy, alkyl-C(O)O$R^b$, alkyl-C(O)O$R^b$, and haloalkyl;
each $R^b$ is independently alkyl;
each $R^c$ and $R^{c'}$ is independently selected from hydrogen, alkyl, alkyl-C(O)O$R^b$ and alkenyl;
each $R^d$ is independently selected from halo, haloalkyl, alkyl, nitro, cyano, and -O$R^a$, or two $R^d$ taken together with the carbon atoms to which they are attached form an optionally substituted heterocyclyl;
n is 0, 1, or 2;
h is 0, 1, 2; and
g is 0, 1 or 2;
for use in a method of treating a cancer characterized as having an IDH mutation in a subject in need thereof.

In one embodiment, disclosed herein is a compound and/or pharmaceutical composition comprising a compound of formula (I) or a pharmaceutically acceptable salt thereof:

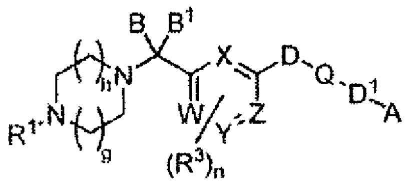

(I)

wherein:

W, X, Y and Z arc each independently selected from CH or N;
B and $B^1$ are independently selected from hydrogen, alkyl or when taken together with the carbon to which they are attached form a carbonyl group;
Q is C=O or $SO_2$;
D and $D^1$ are independently selected from a bond, oxygen or $NR^c$;
A is aryl or heteroaryl each substituted with 0-3 occurrences of $R^2$;
$R^1$ is independently selected from alkyl, acyl, cycloalkyl, aryl, heteroaryl, heterocyclyl, heterocyclylalkyl, cycloalkylalkyl, aralkyl, and heteroaralkyl; each of which may be optionally substituted with 0-3 occurrences of $R^d$;
each $R^2$ is independently selected from halo, hydroxy, haloalkyl, aryl, heteroaryl, alkyl, $-NR^cR^{c'}$, alkyl-$NR^cR^{c'}$, $-OR^a$, -C(O)OH, $-C(O)OR^b$, $-C(O)NR^cR^{c'}$, cycloalkyl, heterocyclyl, heterocyclylalkyl, cycloalkylalkyl, aralkyl, or heteroaralkyl;
each $R^3$ is independently selected from halo, haloalkyl, alkyl, alkenyl, alkynyl, heterocyclyl and $-OR^a$, or two adjacent $R^3$s (when n is 2) taken together with the carbon atoms they are attached to form an optionally substituted heterocyclyl;
each $R^a$ is independently selected from alkyl, alkoxy, alkylalkoxy, alkylalkoxylalkoxy, alkyl-$C(O)OR^b$, alkyl-$C(O)OR^b$, and haloalkyl;
each $R^b$ is independently alkyl;
each $R^c$ and $R^{c'}$ is independently selected from hydrogen, alkyl, alkyl-$C(O)OR^b$ and alkenyl;
each $R^d$ is independently selected from halo, haloalkyl, alkyl, nitro, cyano, and $-OR^a$, or two $R^d$ taken together with the carbon atoms to which they are attached form an optionally substituted heterocyclyl;
n is 0, 1, or 2;
h is 0, 1, 2; and
g is 0, 1 or 2.

In one aspect, included is a method of treating a subject having a cell proliferation-related disorder, *e.g.,* a precancerous disorder, or cancer, the method comprising by administering to the subject a compound or composition described herein (e.g., a compound of formula (I)), for example, a therapeutically effective amount of a compound described herein. In another aspect, included is a method of treating aciduria, e.g., 2-hydroxyglutaric aciduria, in a subject. The cell proliferation-related disorder can be characterized by a somatic allele, e.g., a preselected allele, or mutant allele, of an IDH, *e.g.,* IDH1 or IDH2, which encodes a mutant IDH, *e.g.,* IDH1 or IDH2, enzyme having a neoactivity.

As used herein, neoactivity refers to alpha hydroxy neoactivity. Neoactivity and alpha hydroxyl neoactivity are used interchanagly herein. Alpha hydroxy neoactivity is the ability to convert an alpha ketone to an alpha hydroxy. In embodiments alpha hydroxy neoactivity proceeds with a reductive cofactor, e.g., NADPH or NADH. In embodiments the alpha hydroxy neoactivity is 2HG neoactivity. 2HG neoactivity, as used herein, refers to the ability to convert alpha ketoglutarate to 2-hydroxyglutarate (sometimes referred to herein as 2HG), *e.g.,* R-2-hydroxyglutarate (sometimes referred to herein as R-2HG).

In an embodiment the compound (e.g., a compound of formula (I)) or composition described herein results in lowering the level of a neoactivity product, e.g., 2HG, e.g., R-2HG.

In an embodiment the compound (e.g., a compound of formula (I)) or composition described herein reduces the level a neoactivity of an IDH, *e.g.,* IDH1 or IDH2, *e.g.,* 2HG neoactivity.

In an embodiment the compound (e.g., a compound of formula (I)) or composition described herein reduces the level of the product of a mutant having a neoactivity of an IDH, *e.g.,* IDH1 or IDH2 mutant, *e.g.,* it reduces the level of 2HG, *e.g.,* R-2HG.

In an embodiment the compound described herein (e.g., a compound of formula (I)) inhibits, *e.g.,* specifically, a neoactivity of an IDH, *e.g.,* IDH1 or IDH2, *e.g.,* 2HG neoactivity; or inhibits both the wildtype activity and a neoactivity of an IDH, *e.g.,* IDH1 or IDH2, e.g, 2HG neoactivity.

In an embodiment the IDH is IDH1 and the neoactivity is 2HG neoactivity. Mutations in IDH1 associated with 2HG neoactivity include mutations at residue 132, e.g., R132H or R132C.

Other IDH1 mutations associated with alpha hydroxy neoactivity, e.g., 2HG neoactivity include mutations at residue 71, e.g., a mutation having other than a Val at residue 71, e.g., V71I.

Other IDH1 mutations associated with alpha hydroxy neoactivity, e.g., 2HG neoactivity include mutations at residue 100, e.g., a mutation having other than an Arg at residue 100, and mutations at residue 109, e.g., a mutation having other than an Arg atu residue 109.

In an embodiment the IDH is IDH2 and the neoactivity of the IDH2 mutant is 2HG neoactivity. Mutations in IDH2 associated with 2HG neoactivity include mutations at residue 172. Mutations in IDH2 associated with 2HG neoactivity include mutations at residue 140.

Treatment methods described herein can comprise evaluating a neoactivity genotype or phenotype. Methods

of obtaining and analyzing samples, and the in vivo analysis in subjects, described elsewhere herein, e.g., in the section entitled, "Methods of evaluating samples and/or subjects," can be combined with this method.

In an embodiment, prior to or after treatment, the method includes evaluating the growth, size, weight, invasiveness, stage or other phenotype of the cell proliferation-related disorder.

In an embodiment, prior to or after treatment, the method includes evaluating the IDH, e.g., IDH1 or IDH2, neoactivity genotype, e.g., 2HG genotype, or neoactivity phenotype, e.g., 2HG, e.g., R-2HG, phenotype. Evaluating the 2HG genotype can comprise determining if an IDH1 or IDH2 mutation having neoactivity, e.g., 2HG neoactivity, is present, e.g., a mutation disclosed herein having neoactivity, e.g., 2HG neoactivity. Neoactivity phenotype, e.g., 2HG, e.g., R-2HG, phenotype, as used herein, refers to the level of neoactivity product (i.e., alpha hydroxyl neoactivity product), e.g., 2HG, e.g., R-2HG, level of neoactivity, e.g., 2HG neoactivity, or level of mutant IDH enzyme having neoactivity, e.g., 2HG neoactivity (or corresponding mRNA). The evaluation can be by a method described herein.

In an embodiment the subject can be evaluated, before or after treatment, to determine if the cell proliferation-related disorder is characterized by a neoactivity product, e.g., 2HG, *e.g.,* R-2HG.

In an embodiment a cancer, *e.g.,* a glioma or brain tumor in a subject, can be analyzed, *e.g.,* by imaging and/or spectroscopic analysis, *e.g.,* magnetic resonance-based analysis, *e.g.,* MRI and/or MRS, *e.g.,* before or after treatment, to determine if it is characterized by presence of an alpha hydroxy neoactivity product, e.g., 2HG, *e.g.,* R-2HG.

In an embodiment the method comprises evaluating, *e.g.*, by direct examination or evaluation of the subject, or a sample from the subject, or receiving such information about the subject, the IDH, *e.g.,* IDH1 or IDH2, genotype, or an alpha hydroxy neoactivity product, e.g., 2HG, *e.g.,* R-2HG phenotype of, the subject, *e.g.,* of a cell, *e.g.,* a cancer cell, characterized by the cell proliferation-related disorder. (The evaluation can be, *e.g.,* by DNA sequencing, immuno analysis, evaluation of the presence, distribution or level of an alpha hydroxy neoactivity product, e.g., 2HG, *e.g.,* R-2HG, *e.g.,* from spectroscopic analysis, *e.g.,* magnetic resonance-based analysis, *e.g.*, MRI and/or MRS measurement, sample analysis such as serum or spinal cord fluid analysis, or by analysis of surgical material, *e.g.*, by mass-spectroscopy). In embodiments this information is used to determine or confirm that a proliferation-related disorder, *e.g.,* a cancer, is characterized by an alpha hydroxy neoactivity product, e.g., 2HG, *e.g.,* R-2HG. In embodiments this information is used to determine or confirm that a cell proliferation-related disorder, *e.g.,* a cancer, is characterized by an IDH, *e.g.,* IDH1 or IDH2, allele described herein, *e.g.,* an IDH1 allele having a mutation, *e.g.,* a His or Cys at residue 132, or an IDH2 allele having a mutation at residue 172 or residue 140.

In an embodiment, before and/or after treatment has begun, the subject is evaluated or monitored by a method described herein, *e.g.,* the analysis of the presence, distribution, or level of an alpha hydroxy neoactivity product, e.g., 2HG, *e.g.,* R-2HG, *e.g.,* to select, diagnose or prognose the subject, to select an inhibitor, or to evaluate response to the treatment or progression of disease.

In an embodiment the cell proliferation-related disorder is a tumor of the CNS, *e.g.,* a glioma, a leukemia, *e.g.,* AML or ALL, e.g., B-ALL or T-ALL, prostate cancer, or myelodysplasia or myelodysplastic syndrome and the evaluation is: evaluation of the presence, distribution, or level of an alpha hydroxy neoactivity product, e.g., 2HG, *e.g.,* R-2HG; or evaluation of the presence, distribution, or level of a neoactivity, e.g., 2HG neoactivity, of an IDH1 or IDH2, mutant protein.

In an embodiment, before or after treatment has begun, the genotype of an IDH mutation associated with alpha hydroxy neoactivity, e.g., 2HG neoactivity, other than a mutation at reside 132 of IDH1 or other than a mutation at residue 140 or 172 of IDH2, is determined.

In an embodiment the presence of an IDH1 mutation at residue 100 or 109 of IDHlassociated with alpha hydroxy neoactivity, e.g., 2HG neoactivity, e.g., a mutation having other than an Arg at residue 100 or 109 is determined, e.g., by sequencing genomic DNA or cDNA, from an affected cell.

In an embodiment the disorder is other than a solid tumor. In an embodiment the disorder is a tumor that, at the time of diagnosis or treatment, does not have a necrotic portion. In an embodiment the disorder is a tumor in which at least 30, 40, 50, 60, 70, 80 or 90% of the tumor cells carry an IHD, e.g., IDH1 or IDH2, mutation having 2HG neoactivity, at the time of diagnosis or treatment.

In an embodiment the cell proliferation-related disorder is a cancer, e.g., a cancer described herein, characterized by an IDH1 somatic mutant having alpha hydroxy neoactivity, e.g., 2HG neoactivity, *e.g.*, a mutant described herein. In an embodiment the tumor is characterized by increased levels of an alpha hydroxy neoactivity product, 2HG, e.g., R-2HG, as compared to non-diseased cells of the same type.

In an embodiment the method comprises selecting a subject having a glioma, on the basis of the cancer being characterized by unwanted, i.e.,increased, levels of an alpha hydroxy neoactivity, product, e.g., 2HG, *e.g.,* R-2HG.

In an embodiment the cell proliferation-related disorder is a tumor of the CNS, *e.g.,* a glioma, *e.g.,* wherein the tumor is characterized by an IDH1 somatic mutant having alpha hydroxy neoactivity, e.g., 2HG neoactivity, *e.g.*, a mutant described herein. Gliomas include astrocytic tumors, oligodendroglial tumors, oligoastrocytic tumors, anaplastic astrocytomas, and glioblastomas. In an embodiment the tumor is characterized by increased levels of an alpha hydroxy neoactivity product, e.g., 2HG, e.g., R-2HG, as compared to non-diseased cells of the same type. *E.g.,* in an embodiment, the IDH1 allele encodes an IDH1 having other than an Arg at residue 132. *E.g.,* the allele encodes His, Ser, Cys, Gly,

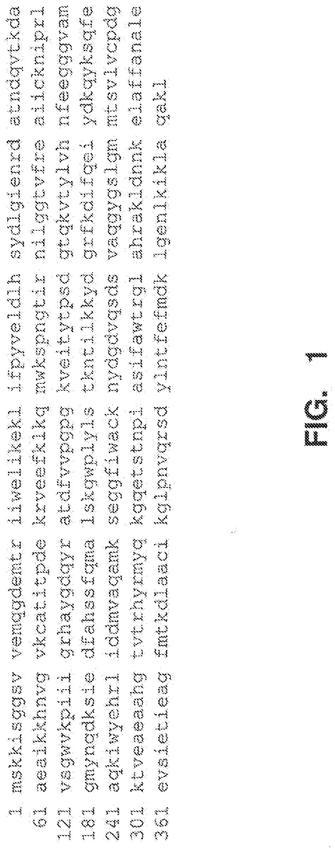

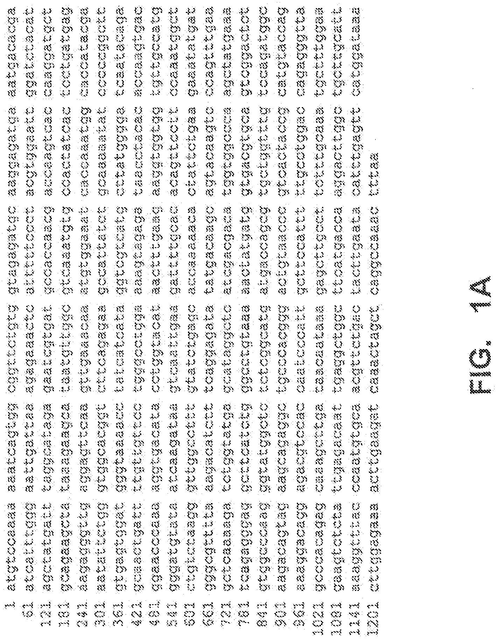

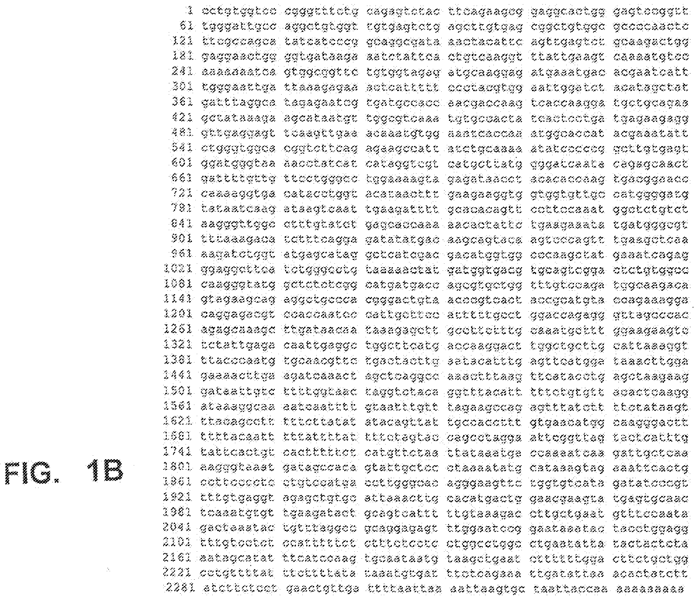

Val, Pro or Leu, or any residue described in Yan *et al*., at residue 132, according to the sequence of SEQ ID NO:1 (see also **Fig. 1**). In an embodiment the allele encodes an IDH1 having His at residue 132. In an embodiment the allele encodes an IDH1 having Ser at residue 132.

In an embodiment the IDH1 allele has an A (or any other nucleotide other than C) at nucleotide position 394, or an A (or any other nucleotide other than G) at nucleotide position 395. In an embodiment the allele is a C394A or a G395A mutation according to the sequence of SEQ ID NO:2.

In an embodiment the method comprises selecting a subject having a glioma, wherein the cancer is characterized by having an IDH1 allele described herein, *e.g.,* an IDH1 allele having His or Cys at residue 132 (SEQ ID NO:1).

In an embodiment the method comprises selecting a subject having a glioma, on the basis of the cancer being characterized by an IDH1 allele described herein, *e.g.,* an IDH1 allele having His or Cys at residue 132 (SEQ ID NO: 1).

In an embodiment, the IDH1 allele encodes an IDH1 having other than a Val at residue 71, e.g., V71I.

In an embodiment the method comprises selecting a subject having a glioma, wherein the cancer is characterized by having an IDH1 allele described herein, *e.g.,* an IDH1 allele having Ile at residue 71 (SEQ ID NO:1).

In an embodiment the method comprises selecting a subject having a glioma, on the basis of the cancer being characterized by an IDH1 allele described herein, *e.g.,* an IDH1 allele having Ile at residue 71 (SEQ ID NO:1).

In an embodiment, the IDH1 allele encodes an IDH1 having other than an Arg at residue 109.

In an embodiment the method comprises selecting a subject having a glioma, wherein the cancer is characterized by having an IDH1 allele described herein, *e.g.,* an IDH1 allele other than an Arg at residue 100 or other than an Arg at residue 109

In an embodiment the method comprises selecting a subject having a glioma, on the basis of the cancer being characterized by an IDH1 allele described herein, *e.g.,* an IDH1 allele having other than an Arg at residue 100 or other than an Arg at residue 109.

In an embodiment the method comprises selecting a subject having a glioma, on the basis of the cancer being characterized by unwanted, i.e., increased, levels of an alpha hydroxy neoactivity, product, e.g., 2HG, *e.g.,* R-2HG.

In an embodiment the cell proliferation-related disorder is localized or metastatic prostate cancer, *e.g.,* prostate adenocarcinoma, *e.g.,* wherein the cancer is characterized by an IDH1 somatic mutant having alpha hydroxy neoactivity, e.g., 2HG neoactivity, *e.g*., a mutant described herein. In an embodiment the cancer is characterized by increased levels of an alpha hydroxy neoactivity product, e.g., 2HG, e.g., R-2HG, as compared to non-diseased cells of the same type.

*E.g.,* in an embodiment, the IDH1 allele encodes an IDH1 having other than an Arg at residue 132. *E.g.,* the allele encodes His, Ser, Cys, Gly, Val, Pro or Leu, or any residue described in Kang et al, 2009, Int. J. Cancer, 125: 353-355 at residue 132, according to the sequence of SEQ ID NO:1 (see also **FIG. 1**). In an embodiment the allele encodes an IDH1 having His or Cys at residue 132.

In an embodiment the IDH1 allele has a T (or any other nucleotide other than C) at nucleotide position 394, or an A (or any other nucleotide other than G) at nucleotide position 395. In an embodiment the allele is a C394T or a G395A mutation according to the sequence of SEQ ID NO:2.

In an embodiment the method comprises selecting a subject having prostate cancer, *e.g*., prostate adenocarcinoma, wherein the cancer is characterized by an IDH1 allele described herein, *e.g.,* an IDH1 allele having His or Cys at residue 132 (SEQ ID NO:1).

In an embodiment the method comprises selecting a subject having prostate cancer, *e.g*., prostate adenocarcinoma, on the basis of the cancer being characterized by an IDH1 allele described herein, *e.g.,* an IDH1 allele having His or Cys at residue 132 (SEQ ID NO:2).

In an embodiment, the IDH1 allele encodes an IDH1 having other than a Val at residue 71, e.g., V71I.

In an embodiment the method comprises selecting a subject having prostate cancer, wherein the cancer is characterized by having an IDH1 allele described herein, *e.g.,* an IDH1 allele having Ile at residue 71 (SEQ ID NO:1).

In an embodiment the method comprises selecting a subject having prostate cancer, on the basis of the cancer being characterized by an IDH1 allele described herein, *e.g.,* an IDH1 allele having Ile at residue 71 (SEQ ID NO:1).

In an embodiment, the IDH1 allele encodes an IDH1 having other than an Arg at residue 100 or other than an Arg at residue 109.

In an embodiment the method comprises selecting a subject having prostate cancer, wherein the cancer is characterized by having an IDH1 allele described herein, *e.g.,* an IDH1 allele other than an Arg at residue 100 or other than an Arg at residue 109.

In an embodiment the method comprises selecting a subject having prostate cancer, on the basis of the cancer being characterized by an IDH1 allele described herein, *e.g.,* an IDH1 allele having other than an Arg at residue 100 or other than an Arg at residue 109.

In an embodiment the method comprises selecting a subject having prostate cancer, on the basis of the cancer being characterized by unwanted, i.e., increased, levels of an alpha hydroxy neoactivity product, e.g., 2HG, *e.g.,* R-2HG.

In an embodiment the cell proliferation-related disorder is a hematological cancer, *e.g.,* a leukemia, *e.g.,* AML, or ALL, wherein the hematological cancer is characterized by an IDH1 somatic mutant having alpha hydroxy neoactivity,

e.g., 2HG neoactivity, *e.g*., a mutant described herein. In an embodiment the cancer is characterized by increased levels of an alpha hydroxy neoactivity product, e.g., 2HG, e.g., R-2HG, as compared to non-diseased cells of the same type.

In an embodiment the cell proliferation-related disorder is acute lymphoblastic leukemia *(e.g.,* an adult or pediatric form), *e.g.,* wherein the acute lymphoblastic leukemia (sometimes referred to herein as ALL) is characterized by an IDH1 somatic mutant having alpha hydroxy neoactivity, e.g., 2HG neoactivity, *e.g*., a mutant described herein. The ALL can be, e.g., B-ALL or T-ALL. In an embodiment the cancer is characterized by increased levels of an alpha hydroxy neoactivity product, e.g., 2HG, e.g., R-2HG, as compared to non-diseased cells of the same type. *Eg*., in an embodiment, the IDH1 allele is an IDH1 having other than an Arg at residue 132 (SEQ ID NO:1). *E.g.,* the allele encodes His, Ser, Cys, Gly, Val, Pro or Leu, or any residue described in Kang *et a.l*, at residue 132, according to the sequence of SEQ ID NO:1 (see also **FIG. 1**). In an embodiment the allele encodes an IDH1 having Cys at residue 132.

In an embodiment the IDH1 allele has a T (or any other nucleotide other than C) at nucleotide position 394. In an embodiment the allele is a C394T mutation according to the sequence of SEQ ID NO:2.

In an embodiment the method comprises selecting a subject having ALL, e.g., B-ALL or T-ALL, characterized by an IDH1 allele described herein, *e.g.,* an IDH1 allele having Cys at residue 132 according to the sequence of SEQ ID NO:1.

In an embodiment the method comprises selecting a subject ALL, e.g., B-ALL or T-ALL, on the basis of cancer being characterized by having an IDH1 allele described herein, *e.g.,* an IDH1 allele having Cys at residue 132 (SEQ ID NO:1).

In an embodiment, the IDH1 allele encodes an IDH1 having other than a Val at residue 71, e.g., V71I.

In an embodiment the method comprises selecting a subject having ALL, e.g., B-ALL or T-ALL, wherein the cancer is characterized by having an IDH1 allele described herein, *e.g.,* an IDH1 allele having Ile at residue 71 (SEQ ID NO: 1).

In an embodiment the method comprises selecting a subject having ALL, e.g., B-ALL or T-ALL, on the basis of the cancer being characterized by an IDH1 allele described herein, *e.g.,* an IDH1 allele having Ile at residue 71 (SEQ ID NO:1).

In an embodiment, the IDH1 allele encodes an IDH1 having other than an Arg at residue 100 or other than an Arg at residue 109.

In an embodiment the method comprises selecting a subject having ALL, e.g., B-ALL or T-ALL, wherein the cancer is characterized by having an IDH1 allele described herein, *e.g.,* an IDH1 allele other than an Arg at residue 100 or other than an Arg at residue 109.

In an embodiment the method comprises selecting a subject having ALL, e.g., B-ALL or T-ALL, on the basis of the cancer being characterized by an IDH1 allele described herein, *e.g.,* an IDH1 allele having other than an Arg at residue 100 or other than an Arg at residue 109.

In an embodiment the method comprises selecting a subject having ALL, e.g., B-ALL or T-ALL, on the basis of the cancer being characterized by unwanted, i.e., increased, levels of an alpha hydroxy neoactivity product, e.g., 2HG, *e.g.,* R-2HG.

In an embodiment the cell proliferation-related disorder is acute myelogenous leukemia (*e.g.,* an adult or pediatric form), *e.g.,* wherein the acute myelogenous leukemia (sometimes referred to herein as AML) is characterized by an IDH1 somatic mutant having alpha hydroxy neoactivity, e.g., 2HG neoactivity, *e.g*., a mutant described herein. In an embodiment the cancer is characterized by increased levels of an alpha hydroxy neoactivity product, e.g., 2HG, e.g., R-2HG, as compared to non-diseased cells of the same type. *E.g.,* in an embodiment, the IDH1 allele is an IDH1 having other than an Arg at residue 132 (SEQ ID NO:1). *E.g.,* the allele encodes His, Ser, Cys, Gly, Val, Pro or Leu, or any residue described in Kang *et al*., at residue 132, according to the sequence of SEQ ID NO:1 (see also **FIG. 1**). In an embodiment the allele encodes an IDH1 having Cys at residue 132.

In an embodiment the IDH1 allele has a T (or any other nucleotide other than C) at nucleotide position 394. In an embodiment the allele is a C394T mutation according to the sequence of SEQ ID NO:2.

In an embodiment the method comprises selecting a subject having acute myelogenous lymphoplastic leukemia (AML) characterized by an IDH1 allele described herein, *e.g.,* an IDH1 allele having Cys at residue 132 according to the sequence of SEQ ID NO:1.

In an embodiment the method comprises selecting a subject having acute myelogenous lymphoplastic leukemia (AML) on the basis of cancer being characterized by having an IDH1 allele described herein, *e.g.,* an IDH1 allele having Cys at residue 132 (SEQ ID NO:1).

In an embodiment the method comprises selecting a subject having acute myelogenous lymphoplastic leukemia (AML), on the basis of the cancer being characterized by unwanted, i.e., increased, levels of an alpha hydroxy neoactivity product, e.g., 2HG, *e.g.,* R-2HG.

In an embodiment, the IDH1 allele encodes an IDH1 having other than a Val at residue 71, e.g., V71I.

In an embodiment the method comprises selecting a subject having AML wherein the cancer is characterized by having an IDH1 allele described herein, *e.g.,* an IDH1 allele having Ile at residue 71 (SEQ ID NO:1).

In an embodiment the method comprises selecting a subject having AML, on the basis of the cancer being characterized by an IDH1 allele described herein, *e.g.,* an IDH1 allele having Ile at residue 71 (SEQ ID NO:1).

In an embodiment, the IDH1 allele encodes an IDH1 having other than an Arg at residue 100 or other than an Arg at residue 109.

In an embodiment the method comprises selecting a subject having AML, wherein the cancer is characterized by having an IDH1 allele described herein, *e.g.,* an IDH1 allele other than an Arg at residue 100 or other than an Arg at residue 109.

In an embodiment the method comprises selecting a subject having AML, on the basis of the cancer being characterized by an IDH1 allele described herein, *e.g.,* an IDH1 allele having other than an Arg at residue 100 or other than an Arg at residue 109.

In an embodiment the method further comprises evaluating the subject for the presence of a mutation in the NRAS or NPMc gene.

In an embodiment the cell proliferation-related disorder is myelodysplasia or myelodysplastic syndrome, *e.g.*, wherein the myelodysplasia or myelodysplastic syndrome is characterized by having an IDH1 somatic mutant having alpha hydroxy neoactivity, e.g., 2HG neoactivity, *e.g.*, a mutant described herein. In an embodiment the disorder is characterized by increased levels of an alpha hydroxy neoactivity product, e.g., 2HG, e.g., R-2HG, as compared to non-diseased cells of the same type. *E.g.,* in an embodiment, the IDH1 allele is an IDH1 having other than an Arg at residue 132 (SEQ ID NO:1). *Eg*., the allele encodes His, Ser, Cys, Gly, Val, Pro or Leu, or any residue described in Kang *et a.l*, according to the sequence of SEQ ID NO:1 (see also **FIG. 1**). In an embodiment the allele encodes an IDH1 having Cys at residue 132.

In an embodiment the IDH1 allele has a T (or any other nucleotide other than C) at nucleotide position 394. In an embodiment the allele is a C394T mutation according to the sequence of SEQ ID NO:2.

In an embodiment the method comprises selecting a subject having myelodysplasia or myelodysplastic syndrome characterized by an IDH1 allele described herein, *e.g.,* an IDH1 allele having Cys at residue 132 according to the sequence of SEQ ID NO:1.

In an embodiment the method comprises selecting a subject having myelodysplasia or myelodysplastic syndrome on the basis of cancer being characterized by having an IDH1 allele described herein, *e.g.,* an IDH1 allele having Cys at residue 132 (SEQ ID NO:1).

In an embodiment, the IDH1 allele encodes an IDH1 having other than a Val at residue 71, e.g., V71I.

In an embodiment the method comprises selecting a subject having myelodysplasia or myelodysplastic syndrome wherein the disorder is characterized by having an IDH1 allele described herein, *e.g.,* an IDH1 allele having Ile at residue 71 (SEQ ID NO:1).

In an embodiment the method comprises selecting a subject having myelodysplasia or myelodysplastic syndrome, on the basis of the disorder being characterized by an IDH1 allele described herein, *e.g.,* an IDH1 allele having Ile at residue 71 (SEQ ID NO:1).

In an embodiment, the IDH1 allele encodes an IDH1 having other than an Arg at residue 100 or other than an Arg at residue 109.

In an embodiment the method comprises selecting a subject having myelodysplasia or myelodysplastic syndrome wherein the disorder is characterized by having an IDH1 allele described herein, *e.g.,* an IDH1 allele other than an Arg at residue 100 or other than an Arg at residue 109.

In an embodiment the method comprises selecting a subject having myelodysplasia or myelodysplastic syndrome on the basis that the disorder is characterized by an IDH1 allele described herein, *e.g.,* an IDH1 allele having other than an Arg at residue 100 or other than an Arg at residue 109.

In an embodiment the method comprises selecting a subject having myelodysplasia or myelodysplastic syndrome, on the basis of the cancer being characterized by unwanted, i.e., increased, levels of an alpha hydroxy neoactivity product, e.g., 2HG, *e.g.,* R-2HG.

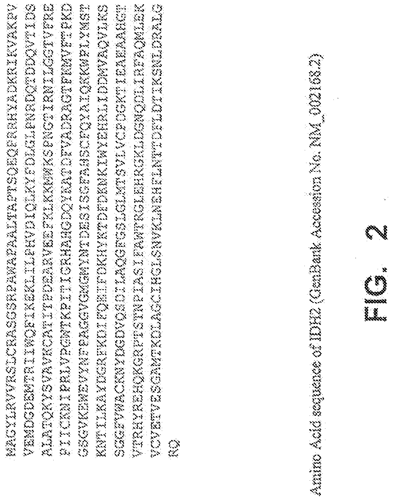

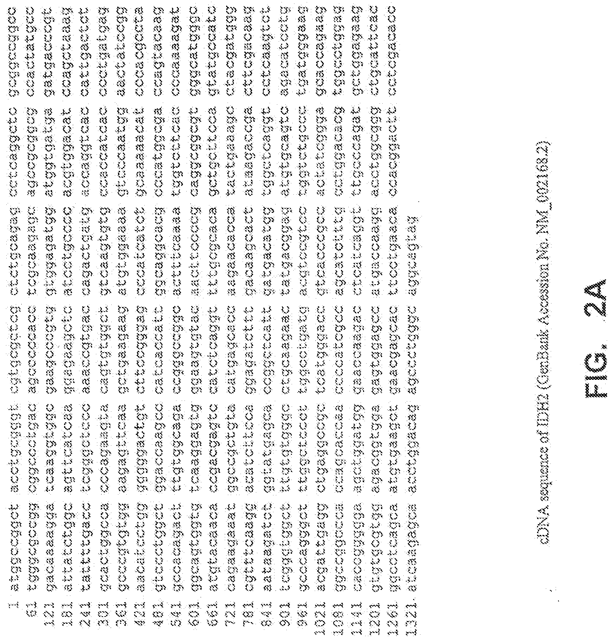

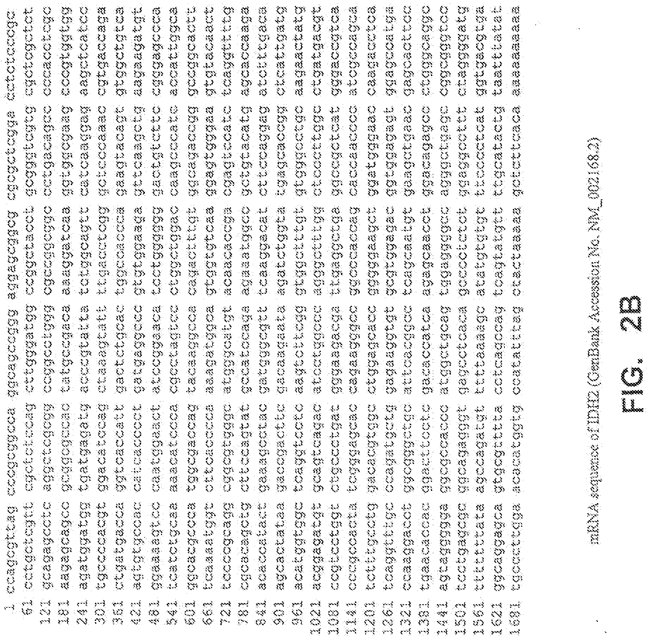

In an embodiment the cell proliferation-related disorder is a glioma, characterized by a mutation, or preselected allele, of IDH2 associated with an alpha hydroxy neoactivity, e.g., 2HG neoactivity. *E.g.*, in an embodiment, the IDH2 allele encodes an IDH2 having other than an Arg at residue 172. *E.g.,* the allele encodes Lys, Gly, Met, Trp, Thr, Ser, or any residue described in described in Yan *et al*., at residue 172, according to the sequence of SEQ ID NO:4(see also **Fig. 2**). In an embodiment the allele encodes an IDH2 having Lys at residue 172. In an embodiment the allele encodes an IDH2 having Met at residue 172.

In an embodiment the method comprises selecting a subject having a glioma, wherein the cancer is characterized by having an IDH2 allele described herein, *e.g.,* an IDH2 allele having Lys or Met at residue 172 (SEQ ID NO:4).

In an embodiment the method comprises selecting a subject having a glioma, on the basis of the cancer being characterized by an IDH2 allele described herein, *e.g.,* an IDH2 allele having Lys or Met at residue 172 (SEQ ID NO:4).

In an embodiment the method comprises selecting a subject having a glioma, on the basis of the cancer being characterized by unwanted, i.e., increased, levels of an alpha hydroxy neoactivity product, e.g., 2HG, *e.g.,* R-2HG.

In an embodiment the cell proliferation-related disorder is a prostate cancer, *e.g.,* prostate adenocarcinoma, characterized by a mutation, or preselected allele, of IDH2 associated with an alpha hydroxy neoactivity, e.g., 2HG neoactivity. *E.g*., in an embodiment, the IDH2 allele encodes an IDH2 having other than an Arg at residue 172. *E.g.,* the allele encodes Lys, Gly, Met, Trp, Thr, Ser, or any residue described in described in Yan *et al*., at residue 172, according to the sequence of SEQ ID NO:4(see also **Fig. 2**). In an embodiment the allele encodes an IDH2 having Lys at residue 172. In an embodiment the allele encodes an IDH2 having Met at residue 172.

In an embodiment the method comprises selecting a subject having a prostate cancer, **e.g**., prostate adenocarcinoma, wherein the cancer is characterized by having an IDH2 allele described herein, *e.g.,* an IDH2 allele having Lys or Met at residue 172 (SEQ ID NO:4).

In an embodiment the method comprises selecting a subject having a prostate cancer, *e.g*., prostate adenocarcinoma, on the basis of the cancer being characterized by an IDH2 allele described herein, *e.g.,* an IDH2 allele having Lys or Met at residue 172 (SEQ ID NO:4).

In an embodiment the method comprises selecting a subject having a prostate cancer, *e.g*., prostate adenocarcinoma, on the basis of the cancer being characterized by unwanted, i.e., increased, levels of an alpha hydroxy neoactivity product, e.g., 2HG, *e.g.,* R-2HG.

In an embodiment the cell proliferation-related disorder is ALL, e.g., B-ALL or T-ALL, characterized by a mutation, or preselected allele, of IDH2 associated with an alpha hydroxy neoactivity, e.g., 2HG neoactivity. *E.g*., in an embodiment, the IDH2 allele encodes an IDH2 having other than an Arg at residue 172. *E.g*., the allele encodes Lys, Gly, Met, Trp, Thr, Ser, or any residue described in described in Yan *et al*., at residue 172, according to the sequence of SEQ ID NO:4(see also **Fig. 2**). In an embodiment the allele encodes an IDH2 having Lys at residue 172. In an embodiment the allele encodes an IDH2 having Met at residue 172.

In an embodiment the method comprises selecting a subject having ALL, e.g., B-ALL or T-ALL, wherein the cancer is characterized by having an IDH2 allele described herein, *e.g.,* an IDH2 allele having Lys or Met at residue 172 (SEQ ID NO:4).

In an embodiment the method comprises selecting a subject having ALL, e.g., B-ALL or T-ALL, on the basis of the cancer being characterized by an IDH2 allele described herein, *e.g.,* an IDH2 allele having Lys or Met at residue 172 (SEQ ID NO:4).

In an embodiment the method comprises selecting a subject having ALL, e.g., B-ALL or T-ALL, on the basis of the cancer being characterized by unwanted, i.e., increased, levels of an alpha hydroxy neoactivity product, e.g., 2HG, *e.g.,* R-2HG.

In an embodiment the cell proliferation-related disorder is AML, characterized by a mutation, or preselected allele, of IDH2 associated with an alpha hydroxy neoactivity, e.g., 2HG neoactivity. *E.g*., in an embodiment, the IDH2 allele encodes an IDH2 having other than an Arg at residue 172. *E.g*., the allele encodes Lys, Gly, Met, Trp, Thr, Ser, or any residue described in described in Yan *et al*., at residue 172, according to the sequence of SEQ ID NO:4 (see also **Fig. 2**). In an embodiment the allele encodes an IDH2 having Lys at residue 172. In an embodiment the allele encodes an IDH2 having Met at residue 172.

In an embodiment the method comprises selecting a subject having AML, wherein the cancer is characterized by having an IDH2 allele described herein, *e.g.,* an IDH2 allele having Lys or Met at residue 172 (SEQ ID NO:4).

In an embodiment the method comprises selecting a subject having AML, on the basis of the cancer being characterized by an IDH2 allele described herein, *e.g.,* an IDH2 allele having Lys or Met at residue 172 (SEQ ID NO:4).

In an embodiment the method comprises selecting a subject having AML, on the basis of the cancer being characterized by unwanted, i.e., increased, levels of an alpha hydroxy neoactivity product, e.g., 2HG, *e.g.,* R-2HG.

In an embodiment the cell proliferation-related disorder is myelodysplasia or myelodysplastic syndrome, characterized by a mutation, or preselected allele, of IDH2. *E.g.,* in an embodiment, the IDH2 allele encodes an IDH2 having other than an Arg at residue 172. *E.g.,* the allele encodes Lys, Gly, Met, Trp, Thr, Ser, or any residue described in described in Yan *et al*., at residue 172, according to the sequence of SEQ ID NO:4 (see also **Fig. 2**). In an embodiment the allele encodes an IDH2 having Lys at residue 172. In an embodiment the allele encodes an IDH2 having Met at residue 172.

In an embodiment the method comprises selecting a subject having myelodysplasia or myelodysplastic syndrome,wherein the cancer is characterized by having an IDH2 allele described herein, *e.g.,* an IDH2 allele having Lys or Met at residue 172 (SEQ ID NO:4).

In an embodiment the method comprises selecting a subject having myelodysplasia or myelodysplastic syndrome, on the basis of the cancer being characterized by an IDH2 allele described herein, *e.g.,* an IDH2 allele having Lys or Met at residue 172 (SEQ ID NO:4).

In an embodiment the method comprises selecting a subject having myelodysplasia or myelodysplastic syndrome, on the basis of the cancer being characterized by unwanted, i.e., increased, levels of an alpha hydroxy neoactivity product, e.g., 2HG, *e.g.,* R-2HG.

In an embodiment a product of the neoactivity is 2HG (*e.g.,* R-2HG) which acts as a metabolite. In another

embodiment a product of the neoactivity is 2HG (*e.g.,* R-2HG) which acts as a toxin, *e.g.,* a carcinogen.

In some embodiments, the methods described herein can result in reduced side effects relative to other known methods of treating cancer.

In an embodiment, an IDH1 mutation include a mutation at residue 70 (e.g., a mutation having other than a Gly at residue 70, (e.g., G70V)), 130 (e.g., a mutation having other than an Ile at residue 130 (e.g., I130M)), 133 (e.g., a mutation having other than a His at residue 133 (e.g., H133Q)), 135 (e.g., a mutation having other than a His at residue 133 (e.g., H133Q)), or 178 (e.g., a mutation having a residue other than a Val at residue 178 (e.g., V178I)), where such mutation is associated with alpha hydroxy neoactivity, e.g., 2HG neoactivity.

In an embodiment, the cell proliferation-related disorder is thyroid cancer, fibrosarcoma or melanoma.

Compounds and compositions described herein (e.g., a compound of formula (I)) and methods of subject evaluation described herein can be combined with other therapeutic modalities, e.g., with art-known treatments.

In an embodiment the method comprises providing a second treatment, to the subject, *e.g.*, surgical removal, irradiation or administration of a chemotherapeutitc agent, *e.g.,* an administration of an alkylating agent. Administration (or the establishment of therapeutic levels) of the second treatment can: begin prior to the beginning or treatment with (or prior to the establishment of therapeutic levels of) the inhibitor; begin after the beginning or treatment with (or after the establishment of therapeutic levels of) the inhibitor, or can be administered concurrently with the inhibitor, *e.g.*, to achieve therapeiutc levels of both concurrently.

In an embodiment the cell proliferation-related disorder is a CNS tumor, *e.g.,* a glioma, and the second therapy comprises administration of one or more of: radiation; an alkylating agent, e.g., temozolomide, e.g., Temoader®, or BCNU; or an inhibitor of HER1/EGFR tyrosine kinase, e.g., erlotinib, e.g., Tarceva®.

The second therapy, *e.g.,* in the case of glioma, can comprise implantation of BCNU or carmustine in the brain, e.g., implantation of a Gliadel® wafer.

The second therapy, *e.g.,* in the case of glioma, can comprise administration of imatinib, e.g., Gleevec®.

In an embodiment the cell proliferation-related disorder is prostate cancer and the second therapy comprises one or more of: androgen ablation; administration of a microtubule stabilizer, e.g., docetaxol, e.g., Taxotere®; or administration of a topoisomerase II inhibitor, e.g., mitoxantrone.

In an embodiment the cell proliferation-related disorder is ALL, e.g., B-ALL or T-ALL, and the second therapy comprises one or more of:

induction phase treatment comprising the administration of one or more of: a steroid; an inhibitor of microtubule assembly, e.g., vincristine; an agent that reduces the availability of asparagine, e.g., asparaginase; an anthracycline; or an antimetabolite, e.g., methotrexate, e.g., intrathecal methotrexate, or 6-mercaptopurine;

consolidation phase treatment comprising the administration of one or more of: a drug listed above for the induction phase; an antimetabolite, e.g., a guanine analog, e.g., 6-thioguanine; an alkylating agent, e.g., cyclophosphamide; an anti-metabolite, e.g., AraC or cytarabine; or an inhibitor of topoisomerase I, e.g., etoposide; or

maintenance phase treatment comprising the administration of one or more of the drugs listed above for induction or consolidation phase treatment.

In an embodiment the cell proliferation-related disorder is AML and the second therapy comprises administration of one or more of: an inhibitor of topoisomerase II, e.g., daunorubicin, idarubicin, topotecan or mitoxantrone; an inhibitor of topoisomerase I, e.g., etoposide; or an anti-metabolite, e.g., AraC or cytarabine.

**Definitions:**

The term "halo" or "halogen" refers to any radical of fluorine, chlorine, bromine or iodine.

The term "alkyl" refers to a hydrocarbon chain that may be a straight chain or branched chain, containing the indicated number of carbon atoms. For example, $C_1$-$C_{12}$ alkyl indicates that the group may have from 1 to 12 (inclusive) carbon atoms in it. The term "haloalkyl" refers to an alkyl in which one or more hydrogen atoms are replaced by halo, and includes alkyl moieties in which all hydrogens have been replaced by halo (e.g., perfluoroalkyl). Alkyl may be optionally substituted. Suitable substituents on an alkyl include, without limitation, halo, alkoxy, haloalkoxy (e.g., perfluoroalkoxy such as $OCF_3$), hydroxy, carboxy, carboxylate, cyano, nitro, amino, alkyl amino, $SO_3H$, sulfate, phosphate, oxo, thioxo (e.g., C=S), imino (alkyl, aryl, aralkyl), $S(O)_n$alkyl (where n is 0-2), $S(O)_n$aryl (where n is 0-2), $S(O)_n$heteroaryl (where n is 0-2), $S(O)_n$ heterocyclyl (where n is 0-2), amine (mono-, di-, alkyl, cycloalkyl, aralkyl, heteroaralkyl, aryl, heteroaryl, and combinations thereof), ester (alkyl, aralkyl, heteroaralkyl, aryl, heteroaryl), amide (mono-, di-, alkyl, aralkyl, heteroaralkyl, aryl, heteroaryl, and combinations thereof), sulfonamide (mono-, di-, alkyl, aralkyl, heteroaralkyl, and combinations thereof).

The terms "arylalkyl" or "aralkyl" refer to an alkyl moiety in which an alkyl hydrogen atom is replaced by an aryl group. Aralkyl includes groups in which more than one hydrogen atom has been replaced by an aryl group. Examples

of "arylalkyl" or "aralkyl" include benzyl, 2-phenylethyl, 3-phenylpropyl, 9-fluorenyl, benzhydryl, and trityl groups.

The term "alkylene" refers to a divalent alkyl, e.g., $-CH_2-$, $-CH_2CH_2-$, and$-CH_2CH_2CH_2-$.

The term "alkenyl" refers to a straight or branched hydrocarbon chain containing 2-12 carbon atoms and having one or more double bonds. Examples of alkenyl groups include, but are not limited to, allyl, propenyl, 2-butenyl, 3-hexenyl and 3-octenyl groups. One of the double bond carbons may optionally be the point of attachment of the alkenyl substituent. The term "alkynyl" refers to a straight or branched hydrocarbon chain containing 2-12 carbon atoms and characterized in having one or more triple bonds. Examples of alkynyl groups include, but are not limited to, ethynyl, propargyl, and 3-hexynyl. One of the triple bond carbons may optionally be the point of attachment of the alkynyl substituent.

The terms "alkylamino" and "dialkylamino" refer to -NH(alkyl) and - $NH(alkyl)_2$ radicals respectively. The term "aralkylamino" refers to a -NH(aralkyl) radical. The term alkylaminoalkyl refers to a (alkyl)NH-alkyl- radical; the term dialkylaminoalkyl refers to a $(alkyl)_2N$-alkyl- radical The term "alkoxy" refers to an - O-alkyl radical. The term "mercapto" refers to an SH radical. The term "thioalkoxy" refers to an -S-alkyl radical. The term thioaryloxy refers to an -S-aryl radical.

The term "aralkyl", as used herein, refers to an alkyl group substituted with an aryl group (e.g., an aromatic or heteroaromatic group).

The term "aryl" refers to an aromatic monocyclic, bicyclic, or tricyclic hydrocarbon ring system, wherein any ring atom capable of substitution can be substituted (e.g., by one or more substituents). Examples of aryl moieties include, but are not limited to, phenyl, naphthyl, and anthracenyl.

The term "cycloalkyl" as employed herein includes saturated cyclic, bicyclic, tricyclic,or polycyclic hydrocarbon groups having 3 to 12 carbons. Any ring atom can be substituted (e.g., by one or more substituents). The cycloalkyl groups can contain fused rings. Fused rings are rings that share a common carbon atom. Examples of cycloalkyl moieties include, but are not limited to, cyclopropyl, cyclohexyl, methylcyclohexyl, adamantyl, and norbornyl.

The term "heteroaryl" refers to a fully aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms selected independently from N, O, or S if monocyclic, bicyclic, or tricyclic, respectively). Any ring atom can be substituted (e.g., by one or more substituents). The point of attachment of a heteroaryl is on the ring containing said heteroatom(s).

The term "heterocyclyl" refers to a nonaromatic 3-10 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively). The point of attachment of a heterocyclyl is on the ring containing said heteroatom(s). The heteroatom may optionally be the point of attachment of the heterocyclyl substituent. Any ring atom can be substituted (e.g., by one or more substituents). The heterocyclyl groups can contain fused rings. Fused rings are rings that share a common carbon atom. Examples of heterocyclyl include, but are not limited to, tetrahydrofuranyl, tetrahydropyranyl, piperidinyl, morpholino, pyrrolinyl, pyrimidinyl, and pyrrolidinyl.

Bicyclic and tricyclic ring systems containing one or more heteroatoms and both aromatic and non-aromatic rings are considered to be heterocyclyl groups according to the present definition.

The term "saturated or partially saturated heterocyclyl" refers to a non-aromatic cylic structure that includes at least one heteroatom. Heterocyclyl groups include, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring can be substituted at one or more positions with such substituents as described above, as for example, halogen, alkyl, aralkyl, alkenyl, alkynyl, cycloalkyl, hydroxyl, amino, nitro, sulfhydryl, imino, amido, phosphate, phosphonate, phosphinate, carbonyl, carboxyl, silyl, ether, alkylthio, sulfonyl, ketone, aldehyde, ester, a heterocyclyl, an aromatic or heteroaromatic moiety, $-CF_3$, -CN, or the like.

The term "heterocyclylalkyl", as used herein, refers to an alkyl group substituted with a heterocycle group.

The term "cycloalkenyl" refers to partially unsaturated, nonaromatic, cyclic, bicyclic, tricyclic, or polycyclic hydrocarbon groups having 5 to 12 carbons, preferably 5 to 8 carbons. The unsaturated carbon may optionally be the point of attachment of the cycloalkenyl substituent. Any ring atom can be substituted (e.g., by one or more substituents). The cycloalkenyl groups can contain fused rings. Fused rings are rings that share a common carbon atom. Examples of cycloalkenyl moieties include, but are not limited to, cyclohexenyl, cyclohexadienyl, or norbornenyl.

The term "heterocycloalkenyl" refers to a partially saturated, nonaromatic 5-10 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively). The unsaturated carbon or the heteroatom may optionally be the point of attachment of the heterocycloalkenyl substituent. Any ring atom can be substituted (e.g.,

by one or more substituents). The heterocycloalkenyl groups can contain fused rings. Fused rings are rings that share a common carbon atom. Examples of heterocycloalkenyl include but are not limited to tetrahydropyridyl and dihydropyranyl.

The terms "hetaralkyl" and "heteroaralkyl", as used herein, refers to an alkyl group substituted with a heteroaryl group.

The term "oxo" refers to an oxygen atom, which forms a carbonyl when attached to carbon, an N-oxide when attached to nitrogen, and a sulfoxide or sulfone when attached to sulfur.

The term "acyl" refers to an alkylcarbonyl, cycloalkylcarbonyl, arylcarbonyl, heterocyclylcarbonyl, or heteroarylcarbonyl substituent, any of which may be further substituted (e.g., by one or more substituents).

The term "substituents" refers to a group "substituted" on a cycloalkyl, cycloalkylalkyl, alkenyl, alkynyl, heterocyclyl, heterocyclylalkyl, heterocycloalkenyl, cycloalkenyl, aryl, aralkyl, heteroaryl or heteroaralkyl group at any atom of that group. Any atom can be substituted. Suitable substituents include, without limitation, alkyl (e.g., C1, C2, C3, C4, C5, C6, C7, C8, C9, C10, C11, C12 straight or branched chain alkyl), cycloalkyl, haloalkyl (e.g., perfluoroalkyl such as $CF_3$), aryl, heteroaryl, aralkyl, heteroaralkyl, heterocyclyl, alkenyl, alkynyl, cycloalkenyl, heterocycloalkenyl, alkoxy, haloalkoxy (e.g., perfluoroalkoxy such as $OCF_3$), halo, hydroxy, carboxy, carboxylate, cyano, nitro, amino, alkyl amino, $SO_3H$, sulfate, phosphate, methylenedioxy ($-O-CH_2-O-$ wherein oxygens are attached to vicinal atoms), ethylenedioxy, oxo, thioxo (e.g., C=S), imino (alkyl, aryl, aralkyl), $S(O)_n$alkyl (where n is 0-2), $S(O)_n$ aryl (where n is 0-2), $S(O)_n$ heteroaryl (where n is 0-2), $S(O)_n$ heterocyclyl (where n is 0-2), amine (mono-, di-, alkyl, cycloalkyl, aralkyl, heteroaralkyl, aryl, heteroaryl, and combinations thereof), ester (alkyl, aralkyl, heteroaralkyl, aryl, heteroaryl), amide (mono-, di-, alkyl, aralkyl, heteroaralkyl, aryl, heteroaryl, and combinations thereof), sulfonamide (mono-, di-, alkyl, aralkyl, heteroaralkyl, and combinations thereof). In one aspect, the substituents on a group are independently any one single, or any subset of the aforementioned substituents. In another aspect, a substituent may itself be substituted with any one of the above substituents.

The abbreviations Me, Et, Ph, Tf, Nf, Ts, Ms represent methyl, ethyl, phenyl, trifluoromethanesulfonyl, nonafluorobutanesulfonyl, *p*-toluenesulfonyl and methanesulfonyl, respectively. A more comprehensive list of the abbreviations utilized by organic chemists of ordinary skill in the art appears in the first issue of each volume of the *Journal of Organic Chemistry*; this list is typically presented in a table entitled Standard List of Abbreviations.

## BRIEF DESCRIPTION OF THE FIGURES

**Fig.1 depicts** the amino acid sequence of IDH1 (SEQ ID NO:1).
**Fig. 1a** depicts the cDNA sequence of IDH1 (SEQ ID NO:2).
**Fig. 1b** depicts the mRNA sequence of IDH1 (SEQ ID NO:3).
**Fig. 2** depicts the amino acid sequence of IDH2 (SEQ ID NO:4).
**Fig. 2a** depicts the cDNA sequence of IDH2 (SEQ ID NO:5).
**Fig. 2b** depicts the mRNA sequence of IDH2 (SEQ ID NO:6).

## DETAILED DESCRIPTION

The inventors have discovered that certain mutated forms of an IDH enzyme (*e.g.,* IDH1 or IDH2) have a gain of function, referred to herein as a neoactivity, which can be targeted in the treatment of a cell proliferation-related disorder such as cancer. Described herein are compounds, composition and methods for the treatment of cancer. The methods include, *e.g.*, treating a subject having a glioma or brain tumor, or AML by administering to the subject a therapeutically effective amount a compound of formula (I) or a pharmaceutical composition comprising a compound of formula (I).

This invention is not limited in its application to the details of construction and the arrangement of components set forth in the following description or illustrated in the drawings. The invention is capable of other embodiments and of being practiced or of being carried out in various ways. Also, the phraseology and terminology used herein is for the purpose of description and should not be regarded as limiting. The use of "including," "comprising," or "having," "containing", "involving", and variations thereof herein, is meant to encompass the items listed thereafter and equivalents thereof as well as additional items.

## Compounds

Described herein compounds and compositions that can be used to inhibit an isocitrate dehydrogenase (IDH) mutant (e.g., IDH1 or IDH2) having alpha hydroxyl neoactivity. Compounds that inhibit IDH, e.g., IDH1 can be used to

treat disorders such as cancer.

In one embodiment, disclosed herein is a compound and/or pharmaceutical composition comprising a compound of formula (I) or a pharmaceutically acceptable salt thereof:

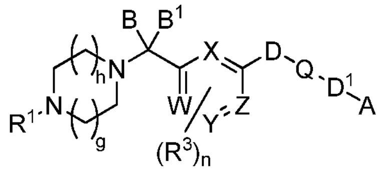

(I)

wherein:

W, X, Y and Z are each independently selected from CH or N;
B and $B^1$ are independently selected from hydrogen, alkyl or when taken together with the carbon to which they are attached form a carbonyl group;
Q is C=O or $SO_2$;
D and $D^1$ are independently selected from a bond, oxygen or $NR^c$;
A is optionally substituted aryl or optionally substituted heteroaryl;
$R^1$ is independently selected from alkyl, acyl, cycloalkyl, aryl, heteroaryl, heterocyclyl, heterocyclylalkyl, cycloalkylalkyl, aralkyl, and heteroaralkyl; each of which may be optionally substituted with 0-3 occurrences of $R^d$;
each $R^3$ is independently selected from halo, haloalkyl, alkyl and $-OR^a$;
each $R^a$ is independently selected from alkyl, and haloalkyl;
each $R^c$ is independently selected from hydrogen, alkyl and alkenyl;
each $R^d$ is independently selected from halo, haloalkyl, alkyl, nitro, cyano, and $-OR^a$, or two $R^d$ taken together with the carbon atoms to which they are attached form an optionally substituted heterocyclyl;
n is 0, 1, or 2;
h is 0, 1, 2; and
g is 0, 1 or 2.

In one embodiment, disclosed herein is a compound or pharmaceutical composition comprising a compound of formula (I) or a pharmaceutically acceptable salt thereof:

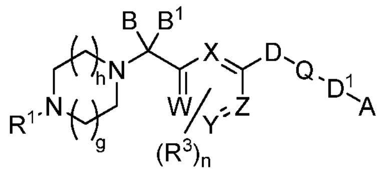

(I)

wherein:

W, X, Y and Z are each independently selected from CH or N;
B and $B^1$ are independently selected from hydrogen, alkyl or when taken together with the carbon to which they are attached form a carbonyl group;
Q is C=O or $SO_2$;
D and $D^1$ are independently selected from a bond, oxygen or $NR^c$;
A is aryl or heteroaryl each substituted with 0-3 occurrences of $R^2$;
$R^1$ is independently selected from alkyl, acyl, cycloalkyl, aryl, heteroaryl, heterocyclyl, heterocyclylalkyl, cycloalkylalkyl, aralkyl, and heteroaralkyl; each of which may be optionally substituted with 0-3 occurrences of $R^d$;
each $R^2$ is independently selected from halo, hydroxy, haloalkyl, aryl, heteroaryl, alkyl, $-NR^cR^{c'}$, alkyl-$NR^cR^{c'}$, $-OR^a$, -C(O)OH, $-C(O)OR^b$, $-C(O)NR^cR^{c'}$, cycloalkyl, heterocyclyl, heterocyclylalkyl, cycloalkylalkyl, aralkyl, or heteroaralkyl;
each $R^3$ is independently selected from halo, haloalkyl, alkyl, alkenyl, alkynyl, heterocyclyl and $-OR^a$, or two $R^3$s (when n is 2) taken together with the carbon atoms they are attached to form an optionally substituted heterocyclyl;
each $R^a$ is independently selected from alkyl, alkoxy, alkylalkoxy, alkylalkoxylalkoxy, alkyl-$C(O)OR^b$, alkyl-$C(O)OR^b$, and haloalkyl;
each $R^b$ is independently alkyl;

each $R^c$ and $R^{c'}$ is independently selected from hydrogen, alkyl, alkyl-C(O)O$R^b$ and alkenyl;
each $R^d$ is independently selected from halo, haloalkyl, alkyl, nitro, cyano, and -O$R^a$, or two $R^d$ taken together with the carbon atoms to which they are attached form an optionally substituted heterocyclyl;
n is 0, 1, or 2;
h is 0, 1, 2; and
g is 0, 1 or 2.

In some embodiments, $R^1$ is independently selected from alkyl, -C(O)$R^e$,-C(O)O$R^e$, -C(O)N$R^cR^{c'}$, cycloalkyl, aryl, heteroaryl, heterocyclyl, heterocyclylalkyl, cycloalkylalkyl, aralkyl, and heteroaralkyl; each of which may be optionally substituted with 0-3 occurrences of $R^d$; wherein $R^e$ is selected from alkyl, cycloalkyl, aryl, aralkyl, heteroaryl, heteroaralkyl and heterocyclyl.

In some embodiments, B and $B^1$ are taken together with the carbon to which they are attached form a carbonyl group.

In some embodiments, h is 1. In some embodiments, h is 2.

In some embodiments, g is 1. In some embodiments, g is 2.

In some embodiments, both h and g are 1. In some embodiments, h is 1 and g is 2. In some embodiments, g is 1 and h is 2.

In some embodiments, W, X, Y and Z are CH. In some embodiments, at least one of W, X, Y and Z is N. In some embodiments, at least two of W, X, Y and Z are N. In some embodiments, at least three of W, X, Y and Z are N.

In some embodiments, W, X, Y, Z and the carbons to which they are attached form a pyridyl ring. In some embodiments, W, X, Y, Z and the carbon atoms to which they are attached form a pyrimidyl ring. In some embodiments, W, X, Y, Z and the carbon atoms to which they are attached form a pyridazinyl ring.

In some embodiments, W, X and Y are CH and Z is N.

In some embodiments, Q is $SO_2$. In one aspect of these embodiments, D and $D^1$ are both N$R^c$. In another aspect of these embodiments, one of D and $D^1$ is a bond and the other of D and $D^1$ is N$R^c$. In another aspect of these embodiments, D is N$R^c$ and $D^1$ is a bond. In another aspect of these embodiments, D is a bond and $D^1$ is N$R^c$. In another aspect of these embodiments, $R^c$ is alkyl (e.g., methyl or ethyl). In another aspect of these embodiments, $R^c$ is hydrogen (H). In another aspect of these embodiments, $R^c$ is alkenyl (e.g., allyl).

In some embodiments, Q is C=O. In another aspect of these embodiments, one of D and $D^1$ is oxygen and the other of D and $D^1$ is N$R^c$. In another aspect of these embodiments, one of D and $D^1$ is a bond and the other of D and $D^1$ is N$R^c$. In another aspect of these embodiments, D is a bond and $D^1$ is N$R^c$. In another aspect of these embodiments, D is N$R^c$ and $D^1$ is a bond. In another aspect of these embodiments, $R^c$ is alkyl (e.g., methyl or ethyl). In another aspect of these embodiments, $R^c$ is hydrogen. In another aspect of these embodiments, $R^c$ is alkenyl (e.g., allyl).

In some embodiments, A is optionally substituted with 1 or 2 occurrences of $R^2$, wherein each $R^2$ is independently selected from halo, hydroxy, haloalkyl, aryl, heteroaryl, alkyl, -N$R^cR^{c'}$, alkyl-N$R^cR^{c'}$, -O$R^a$, -COOH, -COO$R^b$, or -CON-$R^cR^{c'}$.

In some embodiments, A is aryl. In an aspect of these embodiments, A is phenyl optionally substituted with 1 or 2 occurrences of $R^2$, wherein each $R^2$ is independently selected from halo, haloalkyl, aryl, heteroaryl, alkyl (e.g., $C_1$-$C_4$ alkyl), -O$R^a$, -COO$R^b$, or -CON$R^cR^{c'}$. In yet anotheraspect of these embodiments, A is optionally substituted phenyl (e.g., phenyl, para-tolyl, p-ethylphenyl, ortho-n-propylphenyl, para-n-propylphenyl, para-isopropylphenyl, para-n-butylphenyl, para-t-butylphenyl, para-sec-butylphenyl, ortho-anisolyl, para-anisolyl, meta-ethoxyphenyl, para-ethoxyphenyl, para-propoxyphenyl, meta-isopropoxyphenyl, pata-butoxyphenyl, para-(cyclopropylmethoxy)phenyl, ortho-fluorophenyl, para-chlorophenyl, para-fluoro-ortho-methylphenyl, para-methylsulfonylbenzene, 2,5-dimethoxy-5-chlorophenyl, para-ethylpyrrolidinylphenyl, para-propylaminophenyl).

In some embodiments, A is phenyl substituted with 1 occurrence of $R^2$. In some aspects of these embodiments, $R^2$ is alkyl (e.g., methyl, ethyl, *n*-propyl, isopropyl, *n*-butyl, *t*-butyl or *sec*-butyl). In some aspects of these embodiments, $R^2$ is halo. In a more particular aspect of theseembodiments, $R^2$ is fluorine (F). In another more particular aspect of these embodiments, $R^2$ is bromine (Br). In another more particular aspect of these embodiments, $R^2$ is chlorine (Cl). In another aspect of these embodiments, $R^2$ is alkyl-N$R^cR^{c'}$ (e.g., ethyl-N$R^cR^{c'}$). In a more particular aspect of these embodiments, $R^c$ and $R^{c'}$ are alkyl (e.g., methyl). In another aspect of these embodiments, $R^2$ is aralkyl (e.g., benzyl or 2-phenylethyl). In some embodiments, $R^2$ is N$R^cR^{c'}$. In one aspect of this embodiment, $R^c$ and $R^{c'}$ are alkyl (e.g., methyl). In some embodiments, $R^2$ is -O$R^a$. In some aspects of this embodiment, $R^a$ is alkyl (e.g., methyl, *n*- ethyl, propyl, isopropyl, *n*-butyl or methylcyclopropyl). In another aspect of this embodiment, $R^a$ is alkylalkoxy (e.g., methylmethoxy). In another aspect of this embodiment, $R^a$ is alkylalkoxylalkoxy (e.g., methylethyoxylmethoxy). In another aspect of this embodiment, $R^a$ is alkyl-C(O)O$R^b$ (e.g., methyl-C(O)O$R^b$ or ethyl-1-C(O)O$R^b$). In a further aspect of this embodiment, $R^b$ is ethyl.

In some embodiments, A is phenyl substituted with 2 occurrences of $R^2$. In some embodiments, both $R^2$ are halo (e.g., fluorine or chlorine). In some embodiments, both $R^2$ are alkyl (e.g, methyl). In some embodiments, both $R^2$

are-$OR^a$. In some embodiments, one $R^2$ is halo and the other is -$OR^a$. In some embodiments, one $R^2$ is bromine (BR) and the other is -$OR^a$. In some embodiments, one $R^2$ is chlorine (Cl) and the other is -$OR^a$. In some embodiments, one $R^2$ is fluorine (F) and the other is -$OR^a$. In some embodiments, $R^a$ is alkyl (e.g., methyl or ethyl). In some embodiments, one $R^2$ is alkyl (e.g., *n*-butyl) and the other $R^2$ is-COOH. In some embodiments, one $R^2$ is hydroxyl and one $R^2$ is -$OR^a$. In some aspect of this embodiments, $R^a$ is alkyl (e.g., methyl). In some embodiments, one $R^2$ is alkyl (e.g., n-butyl) and one $R^2$ is -$NR^cR^{c'}$. In one aspect of this embodiment, $R^c$ and $R^{c'}$ is alkyl (e.g., methyl).

In some embodiments, A is phenyl substituted with 3 occurrences of $R^2$. In one aspect of this embodiment, two $R^2$ are alkyl (e.g., methyl) and one is -$OR^a$. In one aspect of this embodiment, $R^a$ is alkyl (e.g., *n*-butyl).

In some embodiments, $R^1$ is acyl. In an aspect of this embodiment, $R^1$ is a ketone (e.g., phenylcarbonyl or benzylcarbonyl). In another aspect of this embodiment, $R^1$ is an ester (e.g., -C(O)Obenzyl, -C(O)Oisobutyl or-C(O)Oisopropyl).

In some embodiments, $R^1$ is aryl (e.g., monocyclic or bicyclic aryl). In some embodiments, $R^1$ is 5-8 membered monocyclic aryl (e.g., phenyl). In some embodiments, $R^1$ is optionally substituted phenyl.

In some embodiments, $R^1$ is optionally substituted phenyl. In some embodiments, $R^1$ is represented by the following structure:

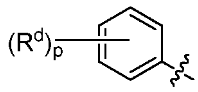

wherein p is 0, 1 or 2;

and each $R^d$ is independently selected from halo, haloalkyl, alkyl, aryl, -$OR^a$ wherein $R^a$ is as defined above.

In some embodiments, p is 0. In some embodiments, p is 1. In some embodiments, $R^d$ is ortho substituted. In some embodiments, $R^d$ is meta substituted. In some embodiments, $R^d$ is para substituted. In some embodiments, $R^d$ is halo (e.g., fluorine, chlorine or bromine). In some embodiments, $R^d$ is aryl (e.g., phenyl). In some embodiments, $R^d$ is -$OR^a$. In some embodiments, $R^a$ is alkyl (e.g., methyl, ethyl, *n*-propyl, isopropyl, isobutyl, methylcyclopropyl). In another aspect of these embodiments, $R^a$ is aryl (e.g., phenyl). In another aspect of this embodiment, $R^a$ is aralkyl (e.g., benzyl or 2-phenylethyl).

In some embodiments, p is 2. In some embodiment, the two $R^d$ are ortho and meta substituted. In some embodiments, the two $R^d$ are ortho and para substituted. In some embodiments, the two $R^d$ are meta and para substituted. In some embodiments, both $R^d$ are alkyl (e.g., methyl).

In some embodiments, $R^1$ is heteroaryl (e.g., N-containing monocyclic heteroaryl or N-containing bicyclic heteroaryl). In some embodiments, $R^1$ is a 5-8 membered monocyclic heteroaryl (e.g., pyridyl, pyrimidyl or pyrizyl). In some embodiments, $R^1$ is optionally substituted pyridyl (e.g., 2-pyridyl, 3-pyridyl, 4-pyridyl, 4-trifluoromethyl-6-chloro-2-pyridyl or 2-methoxy-3-pyridyl), optionally substituted pyrimidyl (e.g., 2-pyrimidyl or 5-pyrimidyl) or optionally substituted pyrizinyl (e.g., 2-pyrinzinyl). In some embodiments, $R^1$ is optionally substituted thiazolyl (e.g., 2-thiazolyl). In some embodiments, $R^1$ is an 8-12 membered bicyclic heteroaryl. In some embodiments, $R^1$ is pyrrolo[2,3-b]pyridyl (e.g., 4-pyrrolo[2,3-b]pyridyl).

In some embodiments, $R^1$ is alkyl. In some embodiments, $R^1$ is methyl. In some embodiments, $R^1$ is ethyl. In some embodiments, $R^1$ is acyl (e.g., acetyl). In some embodiments, $R^1$ is optionally substituted pyrimidyl (e.g., 2-pyrimidyl). In some embodiments, $R^1$ is 4-chloro-2-pyrimidyl. In some embodiments, $R^1$ is optionally substituted pyrazinyl.

In some embodiments, $R^1$ is optionally substituted aralkyl (e.g., benzyl, phenylethyl, 2-phenylethyl, 2-ethylbenzyl, 2-methylbenzyl, 3-methylbenzyl, 2,4,5-trimethylbenzyl, 2,3,4-trimethylbenzyl, 2-phenylpropyl or 3-phenylpropyl). In some embodiments, $R^1$ is optionally substituted heteroaralkyl (e.g., methyl-pyridyl or methyl-pyrimidyl).

In some embodiments, n is 0. In some embodiments, n is 1. In some embodiments, n is 1 and $R^3$ is positioned on W.

In some embodiments, $R^3$ is alkyl (e.g., methyl or ethyl). In some embodiments, $R^3$ is halo (e.g., fluorine, bromine or chlorine). In some embodiments, $R^3$ is haloalkyl (e.g., trifluoromethyl). In some embodiments, $R^3$ is alkenyl (e.g., vinyl). In some embodiments, $R^3$ is alkynyl (e.g., propynyl). In some embodiments, $R^3$ is heterocyclyl (e.g., morpholinyl or pyrrolidinyl).

In some embodiments, n is 2. In some embodiments, n is 2 and one $R^3$ is positioned on W and the other $R^3$ is positioned on Y.

In one aspect of this embodiment, two adjacent $R^3$s are taken together with the carbon atoms to which they are attached to form a heterocyclyl ring (e.g., 1,4-dioxane or morpholine).

In another embodiment, disclosed herein is a compound and/or a pharmaceutical composition comprising a compound of formula (Ia) or a pharmaceutically acceptable salt thereof:

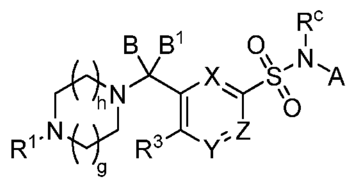

(Ia),

wherein A, $R^1$, $R^3$, $R^a$, $R^b$, $R^c$, B, $B^1$, n, h and g are as defined above.

In some embodiments, each of X, Y and Z are CH. In some embodiments, one of X, Y and Z are N and two of X, Y and Z are CH. In some embodiments, X is N and Y and Z are CH. In some embodiments, Y is N and X and Z are CH. In some embodiments, Z is N and X and Y are CH. In some embodiments, two of X, Y and Z are N and one of X, Y and Z are CH.

In another embodiment, disclosed herein is a compound and/or a pharmaceutical composition comprising a compound of formula (Ib):

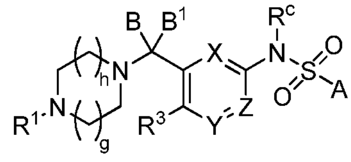

(Ib),

wherein A, $R^1$, $R^3$, $R^a$, $R^b$, $R^c$, B, $B^1$, n, h and g are as defined above.

In some embodiments, each of X, Y and Z are CH. In some embodiments, one of X, Y and Z are N and two of X, Y and Z are CH. In some embodiments, X is N and Y and Z are CH. In some embodiments, Y is N and X and Z are CH. In some embodiments, Z is N and X and Y are CH. In some embodiments, two of X, Y and Z are N and one of X, Y and Z are CH.

In another embodiment, disclosed herein is a compound of formula (Ic):

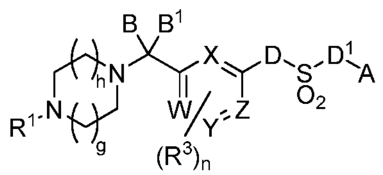

(Ic)

wherein:

W, X, Y and Z are each independently selected from CH or N;
B and $B^1$ are independently selected from hydrogen, alkyl or when taken together with the carbon to which they are attached form a carbonyl group;
D and $D^1$ are independently selected from a bond or $NR^c$;
A is aryl or heteroaryl, each substituted with 0-3 occurrences of $R^2$;
$R^1$ is independently selected from acyl, cycloalkyl, aryl, heteroaryl, heterocyclyl, heterocyclylalkyl, cycloalkylalkyl, aralkyl, and heteroaralkyl; each of which may be optionally substituted with 0-3 occurrences of $R^d$;
each $R^2$ is independently selected from halo, hydroxy, haloalkyl, aryl, heteroaryl, alkyl, $-NR^cR^{c'}$, alkyl-$NR^cR^{c'}$, $-OR^a$, -C(O)OH, $-C(O)OR^b$, or-$C(O)NR^cR^{c'}$;
each $R^3$ is independently selected from halo, haloalkyl, alkyl, alkenyl, alkynyl, heterocyclyl and $-OR^a$, or two adjacent $R^3$s (when n is 2) taken together with the carbon atoms to which they are attached form an optionally substituted heterocyclyl;
each $R^a$ is independently selected from alkyl, alkoxy, alkylalkoxy, alkylalkoxylalkoxy, alkyl-$C(O)OR^b$, alkyl-$C(O)OR^b$, and haloalkyl;
each $R^b$ is independently alkyl;
each $R^c$ and $R^{c'}$ is independently selected from hydrogen, alkyl, alkyl-$C(O)OR^b$ and alkenyl;
each $R^d$ is independently selected from halo, haloalkyl, alkyl, nitro, cyano, and $-OR^a$, or two $R^d$ taken together with the carbon atoms to which they are attached form an optionally substituted heterocyclyl;
n is 0, 1, or 2;

h is 0, 1, 2; and
g is 0, 1 or 2;

provided that:

(1) when W, X, Y and Z are each independently selected from CH;
B and $B^1$ taken together with the carbon to which they are attached form a carbonyl group;
each $R^3$ is independently selected from halo, alkyl and $-OR^a$;

(i) h and g are each 1; one of D and $D^1$ is a bond and the other is NH; $R^1$ is phenyl or monocyclic heteroaryl, each of which may be optionally substituted with 0-3 occurrences of $R^d$;
then A is not phenyl optionally substituted with unsubstituted alkyl, unsubstituted alkoxy, halo, $CF_3$, $CH_2CH_2NH_2$, $NO_2$, or acyl;
(ii) h and g are each 1; of D and $D^1$ is a bond and the other is NH; $R^1$ is acyl;
then n is 1, $R^3$ is alkyl and $R^3$ is connected to W, and A is not phenyl substituted by methyl, F, methoxy or ethoxy; and
(iii) the sum of h and g is 3, D is a bond and $D^1$ is NH; $R^1$ is o-methoxyphenyl;
then A is not phenyl substituted with unsubstituted alkyl, methoxy, ethoxy or halo;

(2) the compound is not N-(4-butylphenyl)-N'-[3-[[4-2-(methoxyphenyl)-1-piperazinyl]carbonyl]-4-methylphenyl]-sulfamide.

In another embodiment, disclosed herein is a compound of formula (Id):

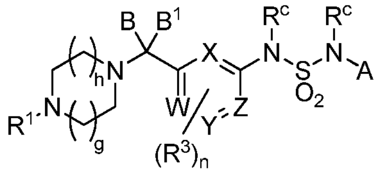

(Id)

wherein:

W, X, Y and Z are each independently selected from CH or N;
B and $B^1$ are independently selected from hydrogen, alkyl or when taken together with the carbon to which they are attached form a carbonyl group;
A is aryl or heteroaryl, each substituted with 0-3 occurrences of $R^2$;
$R^1$ is independently selected from acyl, cycloalkyl, aryl, heteroaryl, heterocyclyl, heterocyclylalkyl, cycloalkylalkyl, aralkyl, and heteroaralkyl; each of which may be optionally substituted with 0-3 occurrences of $R^d$;
each $R^2$ is independently selected from halo, hydroxy, haloalkyl, aryl, heteroaryl, alkyl, $-NR^cR^{c'}$, alkyl-$NR^cR^{c'}$, $-OR^a$, -C(O)OH, $-C(O)OR^b$, or-$C(O)NR^cR^{c'}$;
each $R^3$ is independently selected from halo, haloalkyl, alkyl, alkenyl, alkynyl, heterocyclyl and $-OR^a$, or two adjacent $R^3$s (when n is 2) taken together with the carbon atoms to which they are attached form an optionally substituted heterocyclyl;
each $R^a$ is independently selected from alkyl, alkoxy, alkylalkoxy, alkylalkoxylalkoxy, alkyl-$C(O)OR^b$, alkyl-$C(O)OR^b$, and haloalkyl;each $R^c$ and $R^{c'}$ is independently selected from hydrogen, alkyl, alkyl-$C(O)OR^b$ and alkenyl;
each $R^b$ is independently alkyl;
each $R^d$ is independently selected from halo, haloalkyl, alkyl, nitro, cyano, and $-OR^a$, or two $R^d$ taken together with the carbon atoms to which they are attached form an optionally substituted heterocyclyl;
n is 0, 1, or 2;
h is 0, 1, 2;
g is 0, 1 or 2; and
provided that (1) when W, X, Y and Z are each CH;
B and $B^1$ taken together with the carbon to which they are attached form a carbonyl group;
the sum of h and g is 3;
D is a bond and $D^1$ is NH; and
$R^1$ is o-methoxyphenyl;
then $A^1$ is not phenyl substituted with unsubstituted alkyl, methoxy, ethoxy or halo; and

(2) the compound is not N-(4-butylphenyl)-N'-[3-[[4-2-(methoxyphenyl)-1-piperazinyl]carbonyl]-4-methylphenyl]-sulfamide.

In some embodiments of formula (Ic) and (Id), h is 1. In some embodiments, his 2.
In some embodiments of formulas (Ic) and (Id), g is 1. In some embodiments, gis2.
In some embodiments of formula (Ic) and (Id), both h and g are 1. In some embodiments, h is 1 and g is 2. In some embodiments, g is 1 and h is 2.
In another embodiment, disclosed herein is a compound of formula (Ie):

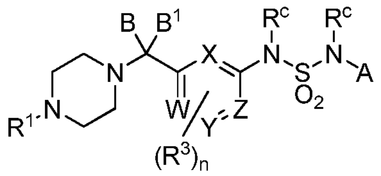

(Ie)

wherein:

W, X, Y and Z are each independently selected from CH or N;
B and $B^1$ are independently selected from hydrogen, alkyl or when taken together with the carbon to which they are attached form a carbonyl group;
A is aryl or heteroaryl, each substituted with 0-3 occurrences of $R^2$;
$R^1$ is independently selected from acyl, cycloalkyl, aryl, heteroaryl, heterocyclyl, heterocyclylalkyl, cycloalkylalkyl, aralkyl, and heteroaralkyl; each of which may be optionally substituted with 0-3 occurrences of $R^d$;
each $R^2$ is independently selected from halo, hydroxy, haloalkyl, aryl, heteroaryl, alkyl, $-NR^cR^{c'}$, alkyl-$NR^cR^{c'}$, $-OR^a$, -C(O)OH, $-C(O)OR^b$, or-$C(O)NR^cR^{c'}$;
each $R^3$ is independently selected from halo, haloalkyl, alkyl, alkenyl, alkynyl, heterocyclyl and $-OR^a$, or two $R^3$ (when n is 2) taken together with adjacent carbon atoms form an optionally substituted heterocyclyl;
each $R^a$ is independently selected from alkyl, alkoxy, alkylalkoxy, alkylalkoxylalkoxy, alkyl-$C(O)OR^b$, alkyl-$C(O)OR^b$, and haloalkyl;each $R^c$ and $R^{c'}$ is independently selected from hydrogen, alkyl, alkyl-$C(O)OR^b$ and alkenyl;
each $R^b$ is independently alkyl;
each $R^d$ is independently selected from halo, haloalkyl, alkyl, nitro, cyano, and $-OR^a$, or two $R^d$ taken together with the carbon atoms to which they are attached form an optionally substituted heterocyclyl;
n is 0, 1, or 2; and
provided that the compound is not N-(4-butylphenyl)-N'-[3-[[4-2-(methoxyphenyl)-1-piperazinyl]carbonyl]-4-methylphenyl]-sulfamide.

In some embodiments of formulas (Ic), (Id), (Ie) and (III), $R^1$ is independently selected from acyl, cycloalkyl, aryl, heteroaryl, heterocyclyl, heterocyclylalkyl, cycloalkylalkyl, aralkyl, and heteroaralkyl; each of which may be optionally substituted with 0-3 occurrences of $R^d$.
In some embodiments, $R^1$ is acyl. In a particular aspect of this embodiment, $R^1$ is a ketone (e.g., phenylcarbonyl or benzylcarbonyl). In another particular aspect of this embodiment, $R^1$ is an ester (e.g., -C(O)Obenzyl, -C(O)Oisobutyl or-C(O)Oisopropyl).
In some embodiments of formulas (Ic), (Id) and (Ie), $R^1$ is aryl (e.g., monocyclic). In one aspect of these embodiments, $R^1$ is 5-8 membered monocyclic aryl (e.g., phenyl). In another aspect of these embodiments, $R^1$ is optionally substituted phenyl.
In some embodiments of formulas (Ic), (Id) and (Ie), $R^1$ is optionally substituted phenyl. In some embodiments, $R^1$ is represented by the following structure:

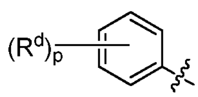

wherein p is 0, 1 or 2;
and each $R^d$ is independently selected from halo, haloalkyl, alkyl, aryl, $-OR^a$ wherein $R^a$ is as defined above.

In one aspect of these embodiments, p is 0. In another aspect of these embodiments, p is 1. In another aspect of these embodiments, $R^d$ is ortho substituted. In another aspect of these embodiments, $R^d$ is meta substituted. In

another aspect of these embodiments, $R^d$ is para substituted. In another aspect of these embodiments, $R^d$ is halo (e.g., fluorine, chlorine or bromine). In another aspect of these embodiments, $R^d$ is aryl (e.g., phenyl). In another aspect of these embodiments, $R^d$ is -$OR^a$. In a further aspect of these embodiments, $R^a$ is alkyl (e.g., methyl, ethyl, *n*-propyl, isopropyl, isobutyl, methylcyclopropyl). In another aspect of these embodiments, $R^a$ is aryl (e.g., phenyl). In another aspect of these embodiments, $R^a$ is aralkyl (e.g., benzyl or 2-phenylethyl).

In another aspect of these embodiments, p is 2. In another aspect of these embodiments, the two $R^d$ are ortho and meta substituted. In another aspect of these embodiments, the two $R^d$ are ortho and para substituted. In another aspect of these embodiments, the two $R^d$ are meta and para substituted. In another aspect of these embodiments, both $R^d$ are alkyl (e.g., methyl)..

In some embodiments of formulas (Ic), (Id) and (Ie), $R^1$ is heteroaryl (e.g., N-containing monocyclic heteroaryl or N-containing bicyclic heteroaryl). In some aspects of these embodiments, $R^1$ is a 5-8 membered monocyclic heteroaryl (e.g., pyridyl, pyrimidyl or pyrizyl). In some aspects of these embodiments, $R^1$ is optionally substituted pyridyl (e.g., 2-pyridyl, 3-pyridyl, 4-pyridyl, 4-trifluoromethyl-6-chloro-2-pyridyl or 2-methoxy-3-pyridyl), optionally substituted pyrimidyl (e.g., 2-pyrimidyl or 5-pyrimidyl) or optionally substituted pyrizinyl (e.g., 2-pyrinzinyl). In some aspects of these embodiments, $R^1$ is optionally substituted thiazolyl (e.g., 2-thiazolyl). In some aspects of these embodiments, $R^1$ is an 8-12 membered bicyclic heteroaryl. In some aspects of these embodiments, $R^1$ is pyrrolo[2,3-b]pyridyl (e.g., 4-pyrrolo[2,3-b]pyridyl). In some aspects of these embodiments, $R^1$ is optionally substituted pyrimidyl (e.g., 2-pyrimidyl). In some aspects of these embodiments, $R^1$ is 4-chloro-2-pyrimidyl. In some aspects of these embodiments, $R^1$ is optionally substituted pyrazinyl.

In some embodiments of formulas (Ic), (Id) and (Ie), $R^1$ is optionally substituted aralkyl (e.g., benzyl, phenylethyl, 2-phenylethyl, 2-ethylbenzyl, 2-methylbenzyl, 3-methylbenzyl, 2,4,5-trimethylbenzyl, 2,3,4-trimethylbenzyl, 2-phenylpropyl or 3-phenylpropyl). In some embodiments, $R^1$ is optionally substituted heteroaralkyl (e.g., methyl-pyridyl or methyl-pyrimidyl).

In another embodiment, disclosed herein is a compound of formula (II):

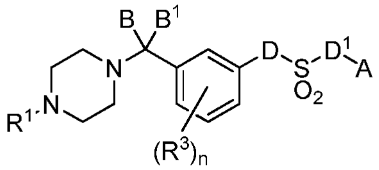

(II)

wherein:

B and $B^1$ are independently selected from hydrogen, alkyl or when taken together with the carbon to which they are attached form a carbonyl group;
D and $D^1$ are independently selected from a bond or $NR^c$;
A is aryl or heteroaryl, each substituted with 0-3 occurrences of $R^2$;
$R^1$ is independently selected from cycloalkyl, aryl, heteroaryl or heterocyclyl; each of which may be optionally substituted with 0-3 occurrences of $R^d$;
each $R^2$ is independently selected from halo, hydroxy, haloalkyl, aryl, heteroaryl, alkyl, -$NR^cR^{c'}$, alkyl-$NR^cR^{c'}$, -$OR^a$, -C(O)OH, -C(O)$OR^b$, or-C(O)$NR^cR^{c'}$;
each $R^3$ is independently selected from halo, haloalkyl, alkyl and -$OR^a$, or two adjacent $R^3$s (when n is 2) taken together with the carbon atoms to which they are attached form an optionally substituted heterocyclyl;
each $R^a$ is independently selected from alkyl, alkoxy, alkylalkoxy, alkylalkoxylalkoxy, alkyl-C(O)$OR^b$, alkyl-C(O)$OR^b$, and haloalkyl;each $R^c$ and $R^{c'}$ is independently selected from hydrogen, alkyl, alkyl-C(O)$OR^b$ and alkenyl;
each $R^b$ is independently alkyl;
each $R^d$ is independently selected from halo, haloalkyl, alkyl, nitro, cyano, and -$OR^a$, or two $R^d$ taken together with the carbon atoms to which they are attached form an optionally substituted heterocyclyl;
n is 0, 1, or 2; and
provided that when B and $B^1$ taken together with the carbon to which they are attached form a carbonyl group;
each $R^3$ is independently selected from halo, alkyl and -$OR^a$;
one of D and $D^1$ is a bond and the other is NH; and
$R^1$ is phenyl or monocyclic heteroaryl, each of which may be optionally substituted with 0-3 occurrences of $R^d$;
then A is not phenyl optionally substituted with unsubstituted alkyl, unsubstituted alkoxy, halo, $CF_3$, $CH_2CH_2NH_2$, $NO_2$, or acyl.
In some embodiments of formula (II), $R^1$ is aryl. In one aspect of these embodiments, $R^1$ is 5-8 membered monocyclic aryl (e.g., phenyl). In another aspect of these embodiments, $R^1$ is optionally substituted phenyl.

In some embodiments, $R^1$ is optionally substituted phenyl. In some embodiments, $R^1$ is represented by the following structure:

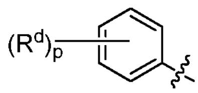

wherein p is 0, 1 or 2;
and each $R^d$ is independently selected from halo, haloalkyl, alkyl, aryl, $-OR^a$ wherein $R^a$ is as defined above.

In one aspect of these embodiments, p is 0. In another aspect of these embodiments, p is 1. In another aspect of these embodiments, $R^d$ is ortho substituted. In another aspect of these embodiments, $R^d$ is meta substituted. In another aspect of these embodiments, $R^d$ is para substituted. In another aspect of these embodiments, $R^d$ is halo (e.g., fluorine, chlorine or bromine). In another aspect of these embodiments, $R^d$ is aryl (e.g., phenyl). In another aspect of these embodiments, $R^d$ is $-OR^a$. In a further aspect of these embodiments, $R^a$ is alkyl (e.g., methyl, ethyl, *n*-propyl, isopropyl, isobutyl, methylcyclopropyl). In yet another aspect of these embodiments, $R^a$ is aryl (e.g., phenyl). In another aspect of these embodiments, $R^a$ is aralkyl (e.g., benzyl or 2-phenylethyl).

In another aspect of these embodiments, p is 2. In another aspect of these embodiments, the two $R^d$ are ortho and meta substituted. In another aspect of these embodiments, the two $R^d$ are ortho and para substituted. In another aspect of these embodiments, the two $R^d$ are meta and para substituted. In another aspect of these embodiments, both $R^d$ are alkyl (e.g., methyl)..

In some embodiments of formula (II), $R^1$ is heteroaryl (e.g., N-containing monocyclic heteroaryl or N-containing bicyclic heteroaryl). In some aspects of these embodiments, $R^1$ is a 5-8 membered monocyclic heteroaryl (e.g., pyridyl, pyrimidyl or pyrizyl). In some aspects of these embodiments, $R^1$ is optionally substituted pyridyl (e.g., 2-pyridyl, 3-pyridyl, 4-pyridyl, 4-trifluoromethyl-6-chloro-2-pyridyl or 2-methoxy-3-pyridyl), optionally substituted pyrimidyl (e.g., 2-pyrimidyl or 5-pyrimidyl) or optionally substituted pyrizinyl (e.g., 2-pyrinzinyl). In some aspects of these embodiments, $R^1$ is optionally substituted thiazolyl (e.g., 2-thiazolyl). In some aspects of these embodiments, $R^1$ is an 8-12 membered bicyclic heteroaryl. In some aspects of these embodiments, $R^1$ is pyrrolo[2,3-b]pyridyl (e.g., 4-pyrrolo[2,3-b]pyridyl). In some aspects of these embodiments, $R^1$ is optionally substituted pyrimidyl (e.g., 2-pyrimidyl). In some aspects of these embodiments, $R^1$ is 4-chloro-2-pyrimidyl. In some aspects of these embodiments, $R^1$ is optionally substituted pyrazinyl.

In another embodiment, disclosed herein is a compound of formula (IIa):

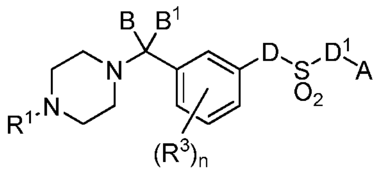

(IIa)

wherein:

B and $B^1$ are independently selected from hydrogen, alkyl or when taken together with the carbon to which they are attached form a carbonyl group;
D and $D^1$ are independently selected from a bond or $NR^c$;
A is aryl or heteroaryl, each substituted with 0-3 occurrences of $R^2$;
$R^1$ is independently selected from heterocyclylalkyl, cycloalkylalkyl, aralkyl and heteroaralkyl; each of which may be optionally substituted with 0-3 occurrences of $R^d$;
each $R^2$ is independently selected from halo, hydroxy, haloalkyl, aryl, heteroaryl, alkyl, $-NR^cR^{c'}$, alkyl-$NR^cR^{c'}$, $-OR^a$, -C(O)OH, $-C(O)OR^b$, or-$C(O)NR^cR^{c'}$;
each $R^3$ is independently selected from halo, haloalkyl, alkyl and $-OR^a$;
each $R^a$ is independently selected from alkyl, alkoxy, alkylalkoxy, alkylalkoxylalkoxy, alkyl-$C(O)OR^b$, alkyl-$C(O)OR^b$, and haloalkyl;each $R^c$ and $R^{c'}$ is independently selected from hydrogen, alkyl, alkyl-$C(O)OR^b$ and alkenyl;
each $R^b$ is independently alkyl;
each $R^d$ is independently selected from halo, haloalkyl, alkyl, nitro, cyano, and $-OR^a$, or two $R^d$ taken together with the carbon atoms to which they are attached form an optionally substituted heterocyclyl;
n is 0, 1, or 2.

In some embodiments of formula (IIa), $R^1$ is optionally substituted aralkyl (e.g., benzyl, phenylethyl, 2-phenylethyl, 2-ethylbenzyl, 2-methylbenzyl, 3-methylbenzyl, 2,4,5-trimethylbenzyl, 2,3,4-trimethylbenzyl, 2-phenylpropyl or 3-phenylpropyl). In some embodiments, $R^1$ is optionally substituted heteroaralkyl (e.g., methyl-pyridyl or methyl-pyrimidyl).

In some embodiments of formulas (Ic), (Id), (Ie), (II) and (IIa), n is 0. In some embodiments, n is 1. In some embodiments n is 1 and $R^3$ is positioned on W.

In some embodiments of formulas (Ic), (Id), (Ie), (II) and (IIa), $R^3$ is alkyl (e.g., methyl or ethyl). In some embodiments, $R^3$ is halo (e.g., fluorine, bromine or chlorine). In some embodiments, $R^3$ is haloalkyl (e.g., trifluoromethyl). In some embodiments, $R^3$ is alkenyl (e.g., vinyl). In some embodiments, $R^3$ is alkynyl (e.g., propynyl). In some embodiments, $R^3$ is heterocyclyl (e.g., morpholinyl or pyrrolidinyl).

In some embodiments of formulas (Ic), (Id), (Ie), (II) and (IIa), n is 2. In some embodiments, n is 2 and one $R^3$ is positioned on W and the other $R^3$ is positioned on Y. In one aspect of this embodiment, two adjacent $R^3$s are taken together with the carbon atoms to which they are attached to form a heterocyclyl ring (e.g., 1,4-dioxane or morpholine).

In another embodiment, disclosed herein is a compound of formula (III):

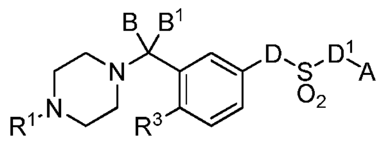
(III)

wherein:

B and $B^1$ are independently selected from hydrogen, alkyl or when taken together with the carbon to which they are attached form a carbonyl group;
D and $D^1$ are indepedently selected from a bond or $NR^c$;
A is aryl or heteroaryl, each substituted with 0-3 occurrences of $R^2$;
$R^1$ is independently selected from acyl, optionally substituted with 0-3 occurrences of $R^d$;
each $R^2$ is independently selected from halo, hydroxy, haloalkyl, aryl, heteroaryl, alkyl, $-NR^cR^{c'}$, alkyl-$NR^cR^{c'}$, $-OR^a$, -C(O)OH, $-C(O)OR^b$, or-$C(O)NR^cR^{c'}$;
$R^3$ is halo, haloalkyl, alkyl, or $-OR^a$;
each $R^a$ is independently selected from alkyl and haloalkyl;each $R^c$ and $R^{c'}$ is independently selected from hydrogen, alkyl, and alkenyl;
each $R^b$ is independently alkyl;
each $R^d$ is independently selected from halo, haloalkyl, alkyl, nitro, cyano, and $-OR^a$, or two $R^d$ taken together with the carbon atoms to which they are attached form an optionally substituted heterocyclyl; and
provided that when B and $B^1$ taken together with the carbon to which they are attached form a carbonyl group; D and $D^1$ is a bond and the other is NH;
then A is not phenyl substituted by methyl, fluorine, methoxy or ethoxy.

In some embodiments of formulas (Ic), (Id), (Ie), (II), (IIa) and (III), B and $B^1$ are taken together with the carbon to which they are attached form a carbonyl group.

In some embodiments of formula (III), $R^3$ is alkyl (e.g., methyl or ethyl). In some embodiments, $R^3$ is halo (e.g., fluorine, bromine or chlorine). In some embodiments, $R^3$ is haloalkyl (e.g., trifluoromethyl). In some embodiments, $R^3$ is alkenyl (e.g., vinyl). In some embodiments, $R^3$ is alkynyl (e.g., propynyl). In some embodiments, $R^3$ is heterocyclyl (e.g., morpholinyl or pyrrolidinyl).

In some embodiments, $R^1$ is acyl. In a particular aspect of this embodiment, $R^1$ is a ketone (e.g., phenylcarbonyl or benzylcarbonyl). In another particular aspect of this embodiment, $R^1$ is an ester (e.g., -C(O)Obenzyl, -C(O)Oisobutyl or - C(O)Oisopropyl).

In another embodiment, disclosed herein is a compound of formula (IV):

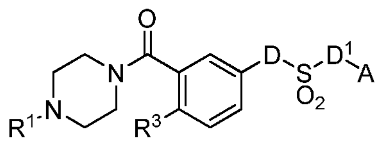
(IV)

wherein:

D and $D^1$ are indepedently selected from a bond or $NR^c$;

A is aryl or heteroaryl, each substituted with 0-3 occurrences of $R^2$;
$R^1$ is independently selected from heterocyclylalkyl, cycloalkylalkyl, aralkyl and heteroaralkyl; each of which may be optionally substituted with 0-3 occurrences of $R^d$;
each $R^2$ is independently selected from halo, hydroxy, haloalkyl, aryl, heteroaryl, alkyl, $-NR^cR^{c'}$, alkyl-$NR^cR^{c'}$, $-OR^a$, -C(O)OH, $-C(O)OR^b$, or-$C(O)NR^cR^{c'}$;
$R^3$ is alkyl;
each $R^a$ is independently selected from alkyl and haloalkyl;each $R^c$ and $R^{c'}$ is independently selected from hydrogen, alkyl, and alkenyl;
each $R^b$ is independently alkyl;
each $R^d$ is independently selected from halo, haloalkyl, alkyl, nitro, cyano, and $-OR^a$, or two $R^d$ taken together with the carbon atoms to which they are attached form an optionally substituted heterocyclyl; and
provided that when D is a bond and $D^1$ is NH, then A is not phenyl substituted with methyl or methoxy.

In some embodiments of formula (Ic), (II), (IIa), (III) and (IV), D and $D^1$ are both $NR^c$. In some embodiments, one of D and $D^1$ is a bond and the other of D and $D^1$ is $NR^c$. In some embodiments, D is $NR^c$ and $D^1$ is a bond. In some embodiments, D is a bond and $D^1$ is $NR^c$. In one aspect of these embodiments, $R^c$ is alkyl (e.g., methyl or ethyl). In another aspect of these embodiments, $R^c$ is hydrogen (H). In another aspect of these embodiments, $R^c$ is alkenyl (e.g., allyl).

In some embodiments of formula (Ic), (Id), (Ie), (II), (IIa), (III) and (IV), A is optionally substituted with 1 or 2 occurrences of $R^2$, wherein each $R^2$ is independently selected from halo, hydroxy, haloalkyl, aryl, heteroaryl, alkyl, $-NR^cR^{c'}$, alkyl-$NR^cR^{c'}$, $-OR^a$, -COOH, $-COOR^b$, or $-CONR^cR^{c'}$.

In some embodiments of formula (Ic), (Id), (Ie), (II), (IIa), (III) and (IV), A is aryl). In an aspect of these embodiments, A is phenyl optionally substituted with 1 or 2 occurrences of $R^2$, wherein each $R^2$ is independently selected from halo, haloalkyl, aryl, heteroaryl, alkyl (e.g., $C_1$-$C_4$ alkyl), $-OR^a$, $-COOR^b$, or $-CONR^cR^{c'}$. In a more particular aspect of these embodiments, A is optionally substituted phenyl (e.g., phenyl, para-tolyl, p-ethylphenyl, ortho-n-propylphenyl, para-n-propylphenyl, para-isopropylphenyl, para-n-butylphenyl, para-t-butylphenyl, para-sec-butylphenyl, ortho-anisolyl, para-anisolyl, meta-ethoxyphenyl, para-ethoxyphenyl, para-propoxyphenyl, meta-isopropoxyphenyl, pata-butoxyphenyl, para-(cyclopropylmethoxy)phenyl, ortho-fluorophenyl, para-chlorophenyl, para-fluoro-ortho-methylphenyl, para-methylsulfonylbenzene, 2,5-dimethoxy-5-chlorophenyl, para-ethylpyrrolidinylphenyl, para-propylaminophenyl).

In some embodiments of formula (Ic), (Id), (Ie), (II), (IIa), (III) and (IV), A is phenyl substituted with 1 occurrence of $R^2$. In some aspects of these embodiments, $R^2$ is alkyl (e.g., methyl, ethyl, *n*-propyl, isopropyl, *n*-butyl, *t*-butyl or *sec*-butyl). In some aspects of these embodiments, $R^2$ is halo. In another aspect of theseembodiments, $R^2$ is fluorine (F). In yet another aspect of these embodiments, $R^2$ is bromine (Br). In yet another aspect of these embodiments, $R^2$ is chlorine (Cl). In another aspect of these embodiments, $R^2$ is alkyl-$NR^cR^{c'}$ (e.g., ethyl-$NR^cR^{c'}$). In a further aspect of these embodiments, $R^c$ and $R^{c'}$ are alkyl (e.g., methyl). In another aspect of these embodiments, $R^2$ is aralkyl (e.g., benzyl or 2-phenylethyl). In some embodiments, $R^2$ is $NR^cR^{c'}$. In one aspect of this embodiment, $R^c$ and $R^{c'}$ are alkyl (e.g., methyl). In some embodiments, $R^2$ is $-OR^a$. In some aspects of this embodiment, $R^a$ is alkyl (e.g., methyl, *n*- ethyl, propyl, isopropyl, *n*-butyl or methylcyclopropyl). In another aspect of this embodiment, $R^a$ is alkylalkoxy (e.g., methylmethoxy). In another aspect of this embodiment, $R^a$ is alkylalkoxylalkoxy (e.g., methylethyoxylmethoxy). In another aspect of this embodiment, $R^a$ is alkyl-$C(O)OR^b$ (e.g., methyl-$C(O)OR^b$ or ethyl-1-$C(O)OR^b$). In another aspect of this embodiment, $R^b$ is ethyl.

In some embodiments, A is phenyl substituted with 2 occurrences of $R^2$. In some embodiments, both $R^2$ are halo (e.g., fluorine or chlorine). In some embodiments, both $R^2$ are alkyl (e.g, methyl). In some embodiments, both $R^2$ are - $OR^a$. In some embodiments, one $R^2$ is halo and the other is $-OR^a$. In some embodiments, one $R^2$ is bromine (BR) and the other is $-OR^a$. In some embodiments, one $R^2$ is chlorine (Cl) and the other is $-OR^a$. In some embodiments, one $R^2$ is fluorine (F) and the other is $-OR^a$. In some embodiments, $R^a$ is alkyl (e.g., methyl or ethyl). In some embodiments, one $R^2$ is alkyl (e.g., *n*-butyl) and the other $R^2$ is-COOH. In some embodiments, one $R^2$ is hydroxyl and one $R^2$ is $-OR^a$. In some aspect of this embodiments, $R^a$ is alkyl (e.g., methyl). In some embodiments, one $R^2$ is alkyl (e.g., n-butyl) and one $R^2$ is $-NR^cR^{c'}$. In one aspect of this embodiment, $R^c$ and $R^{c'}$ is alkyl (e.g., methyl).

In some embodiments of formula (Ic), (Id), (Ie), (II), (IIa), (III) and (IV), A is phenyl substituted with 3 occurrences of $R^2$. In one aspect of this embodiment, two $R^2$s are alkyl (e.g., methyl) and one is $-OR^a$. In one aspect of this embodiment, $R^a$ is alkyl (e.g., *n*-butyl).

In some embodiments of formula (IV), $R^3$ is alkyl (e.g., methyl or ethyl). In some embodiments, $R^3$ is halo (e.g., fluorine, bromine or chlorine). In some embodiments, $R^3$ is haloalkyl (e.g., trifluoromethyl). In some embodiments, $R^3$ is alkenyl (e.g., vinyl). In some embodiments, $R^3$ is alkynyl (e.g., propynyl). In some embodiments, $R^3$ is heterocyclyl (e.g., morpholinyl or pyrrolidinyl).

In some embodiments of formula (IV), $R^1$ is optionally substituted aralkyl (e.g., benzyl, phenylethyl, 2-phenylethyl, 2-ethylbenzyl, 2-methylbenzyl, 3-methylbenzyl, 2,4,5-trimethylbenzyl, 2,3,4-trimethylbenzyl, 2-phenylpropyl or 3-phenylpropyl). In some embodiments, $R^1$ is optionally substituted heteroaralkyl (e.g., methyl-pyridyl or methyl-pyrimidyl).

Exemplary compounds are shown in Table 1. A compound described herein may be an inhibitor of IDH1m. For simplicity, the inhibitory activity of these compounds is represented as an $IC_{50}$ (as measured in an assay similar to one described in Example 1) in the Table below and throughout the application. As shown in Table 1, A refers to an inhibitor of IDH1m with an $IC_{50}$ < 1 μM. B refers to an inhibitor of IDH1m with an $IC_{50}$ between 1 μM and 50 μM. C refers to an inhibitor of IDH1m with an $IC_{50}$ greater than 50 μM. D refers to a compound wherein an $IC_{50}$ is not available.

**Table 1**

| Compound | $IC_{50}$ | Compound | $IC_{50}$ |
|---|---|---|---|
| 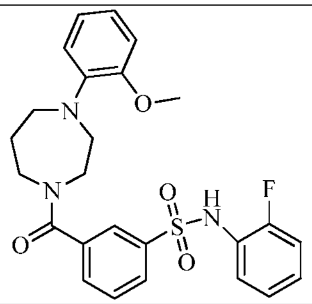 | B | 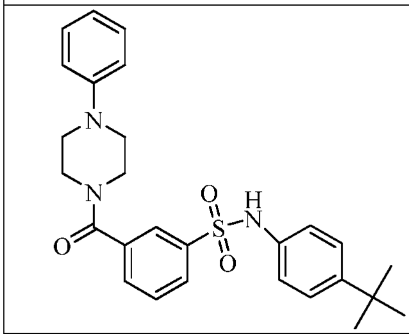 | B |
| 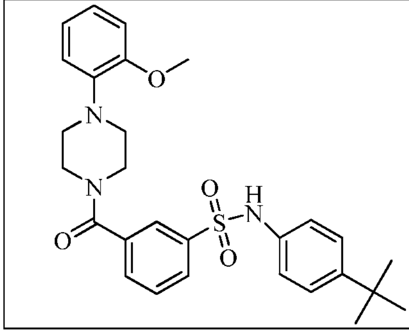 | B | 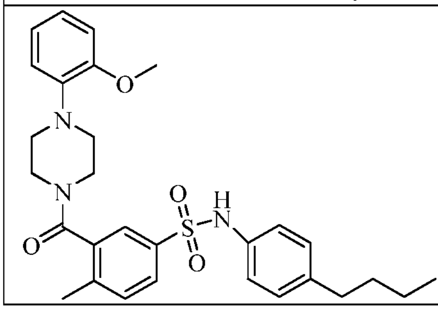 | A |

| Compound | $IC_{50}$ |
|---|---|
| 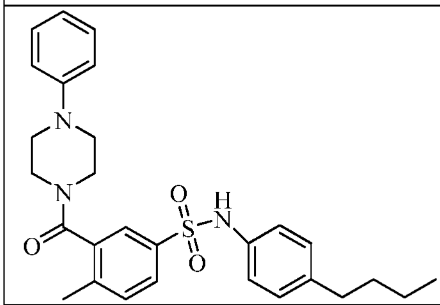 | B |
| 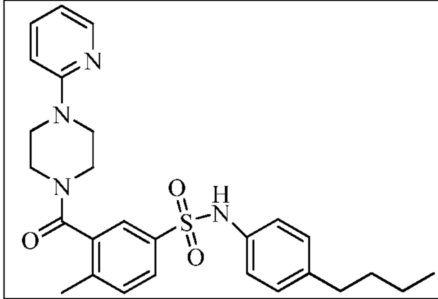 | B |
| 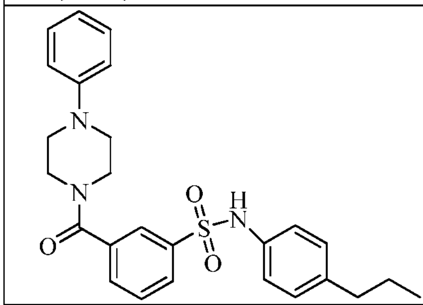 | B |
| 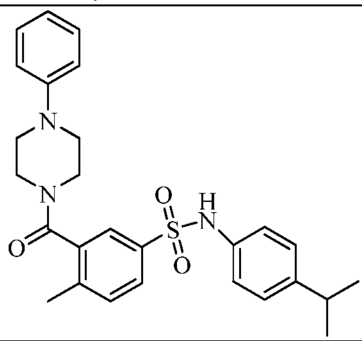 | B |
| 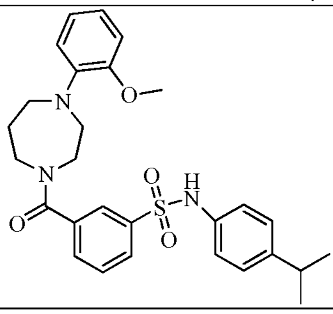 | B |

| Compound | $IC_{50}$ |
|---|---|
| 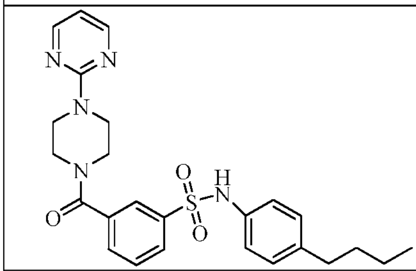 | B |
| 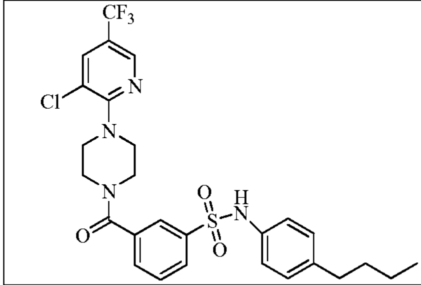 | B |
| 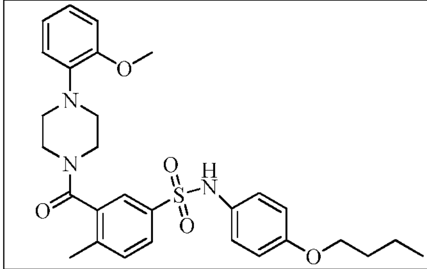 | B |
| 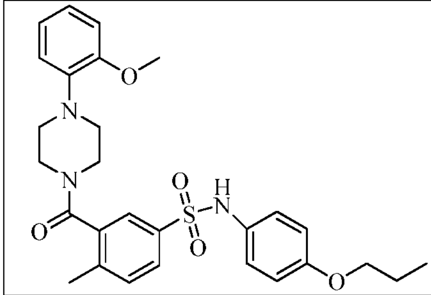 | B |
| 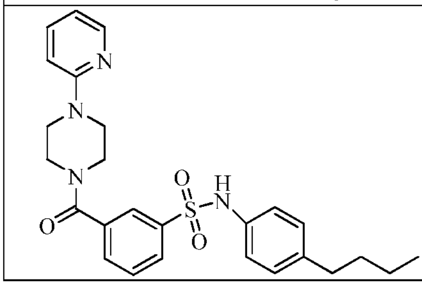 | B |

| Compound | IC50 |
|---|---|
| 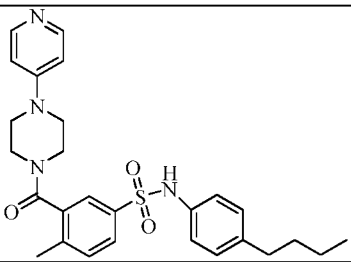 | B |
| 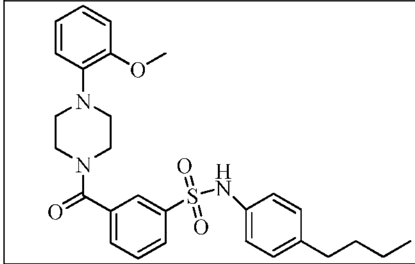 | B |
| 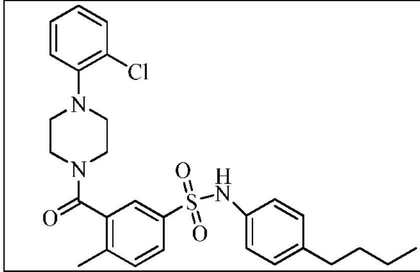 | A |
| 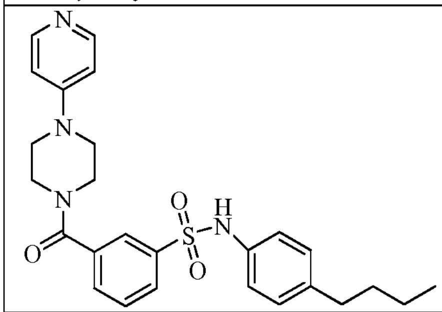 | B |
| 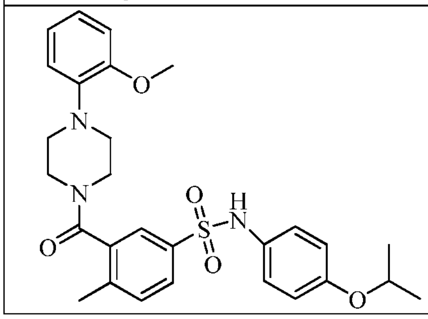 | B |

| Compound | IC50 |
|---|---|
| 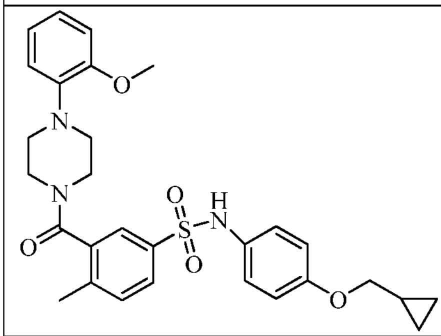 | B |
| 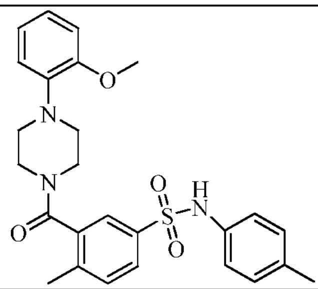 | B |
| 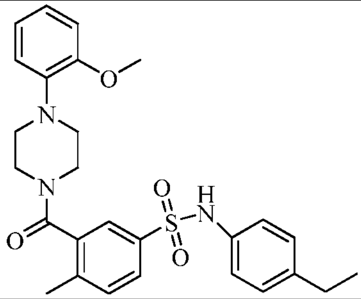 | B |
| 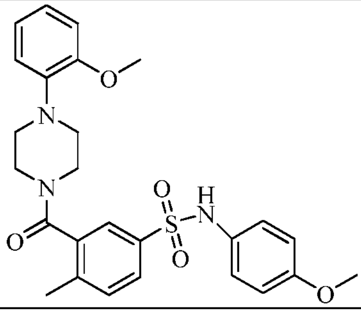 | B |

| Compound | $IC_{50}$ |
|---|---|
| 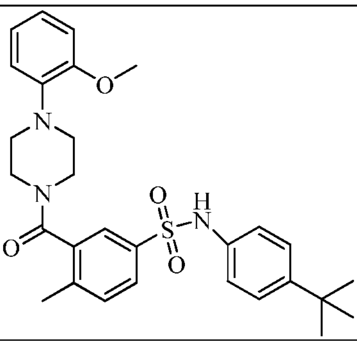 | B |
| 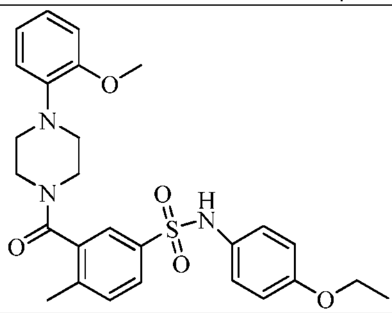 | B |
| 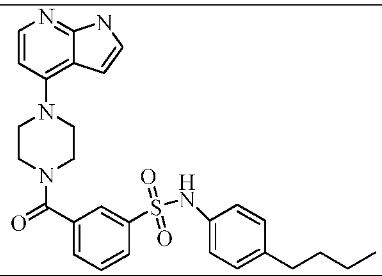 | B |
| 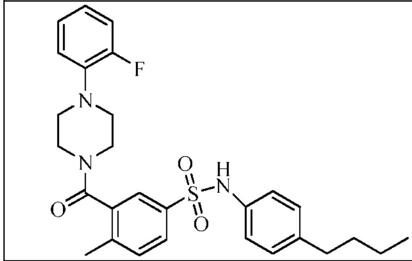 | B |
| 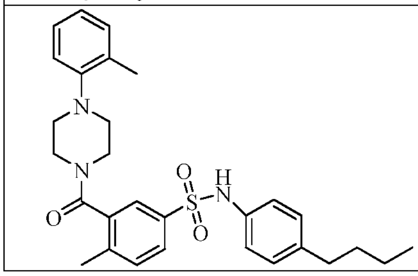 | A |

| Compound | $IC_{50}$ |
|---|---|
| 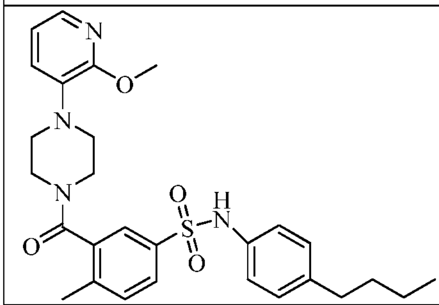 | B |
| 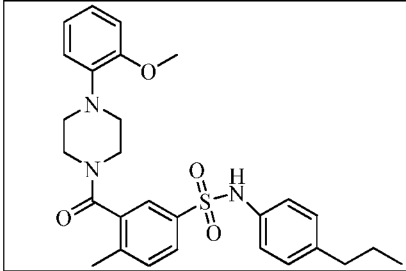 | B |
| 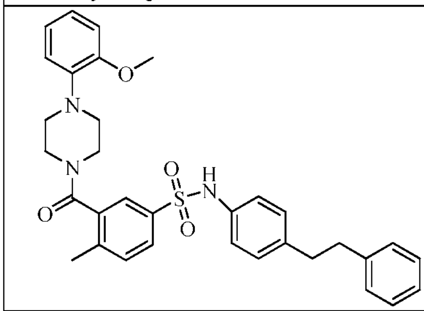 | B |
| 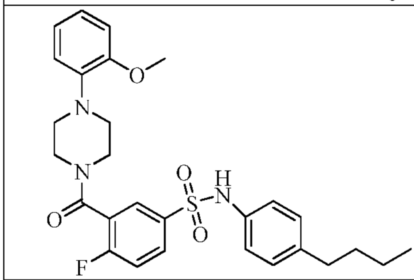 | B |
| 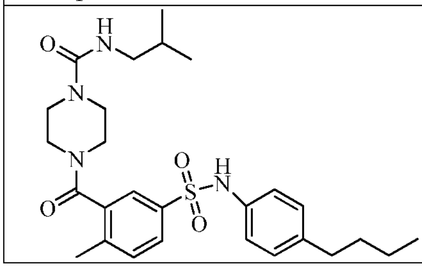 | B |

| Compound | $IC_{50}$ |
|---|---|
| 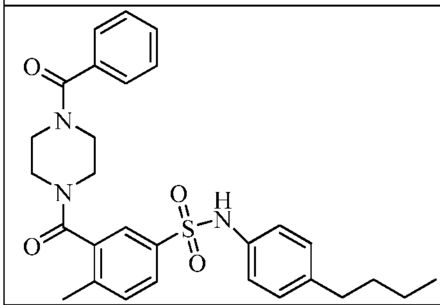 | **B** |
| 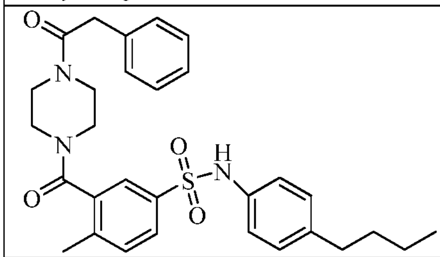 | **B** |
| 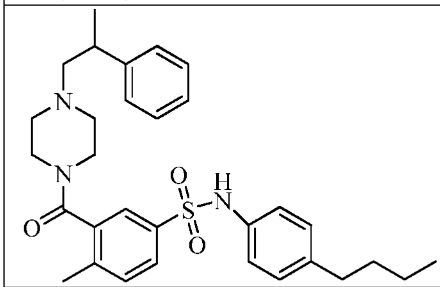 | **B** |
| 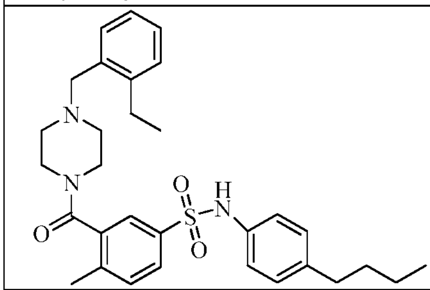 | **B** |
| 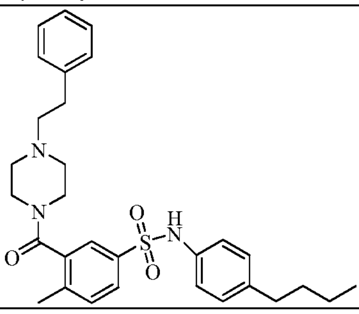 | **B** |

| Compound | $IC_{50}$ |
|---|---|
| 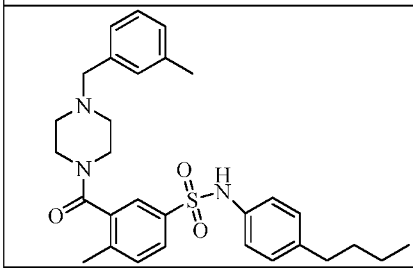 | **B** |
| 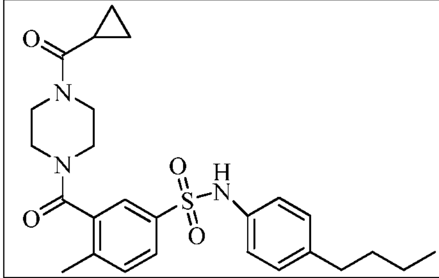 | **B** |
| 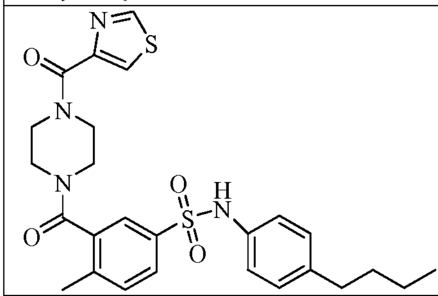 | **B** |
| 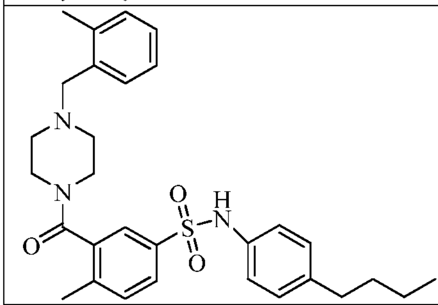 | **B** |
| 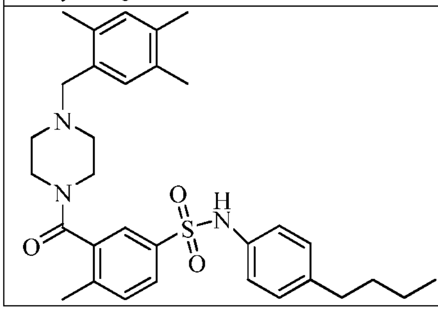 | **B** |

| Compound | $IC_{50}$ |
|---|---|
| 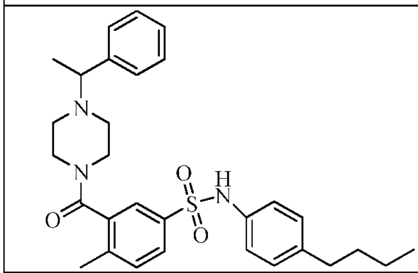 | B |
| 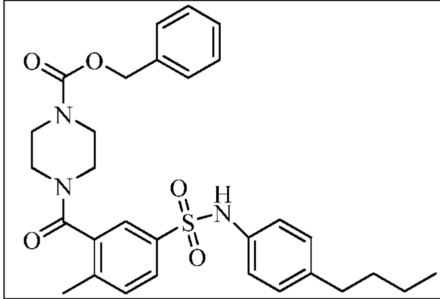 | B |
| 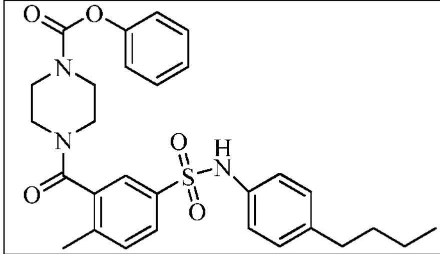 | B |
| 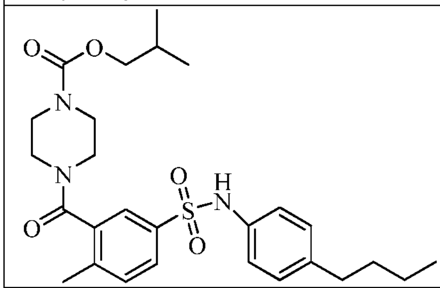 | B |
| 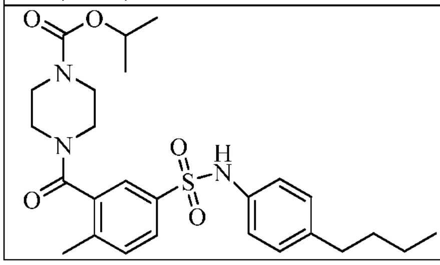 | B |

| Compound | $IC_{50}$ |
|---|---|
| 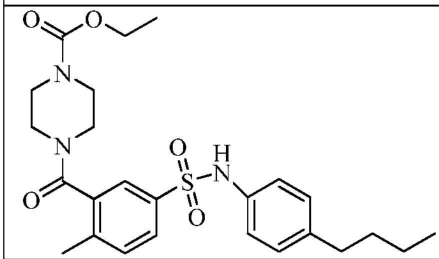 | B |
| 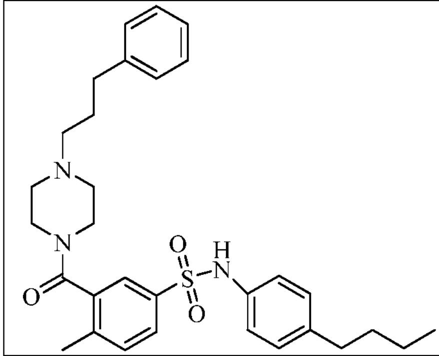 | B |
| 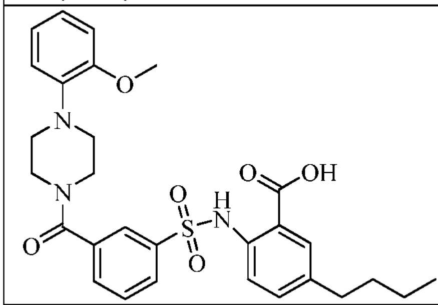 | B |
| 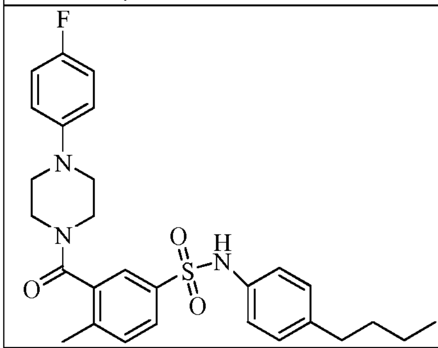 | B |
| 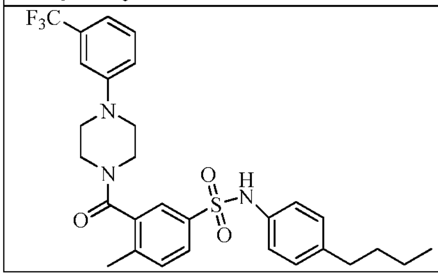 | B |

| Compound | $IC_{50}$ |
|---|---|
| 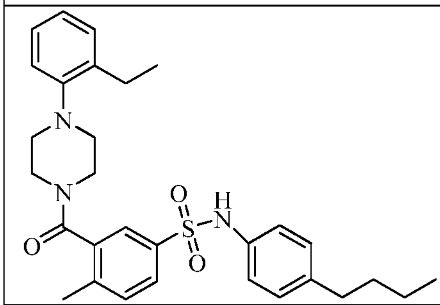 | A |
| 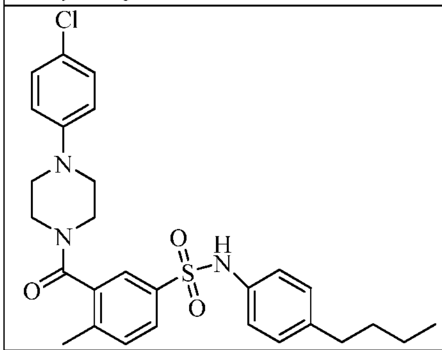 | B |
| 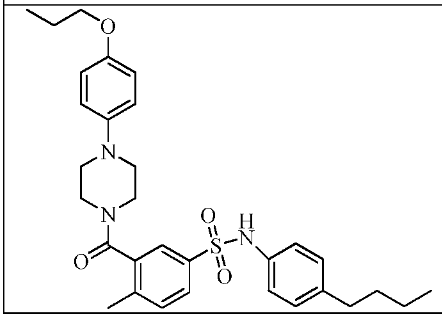 | B |
| 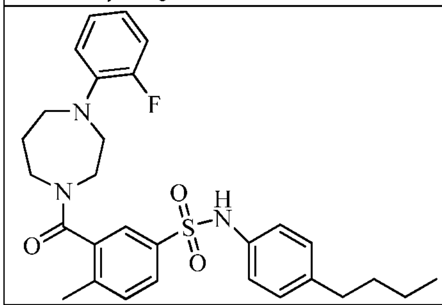 | B |
| 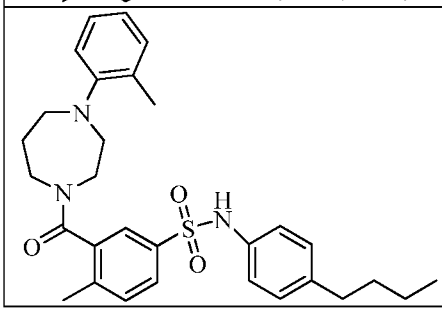 | B |

| Compound | $IC_{50}$ |
|---|---|
| 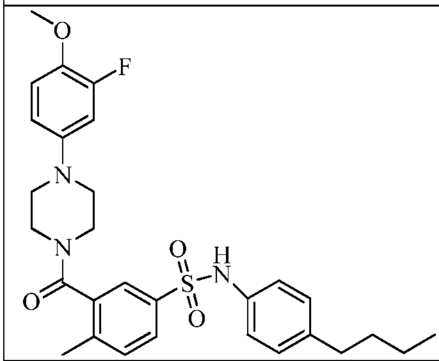 | B |
| 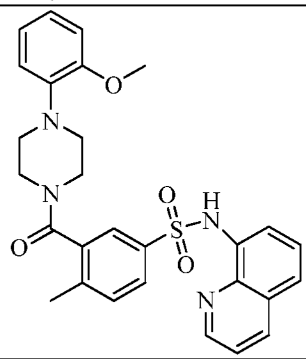 | B |
| 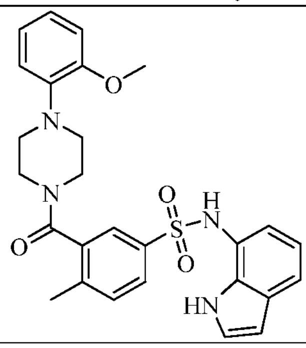 | B |
| 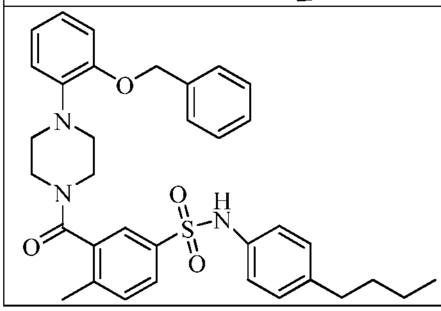 | B |

| Compound | $IC_{50}$ |
|---|---|
| 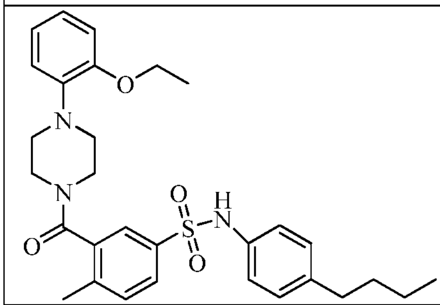 | B |
| 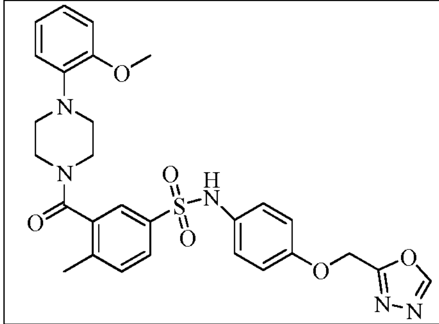 | B |
| 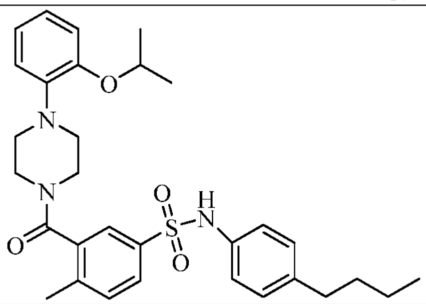 | B |
| 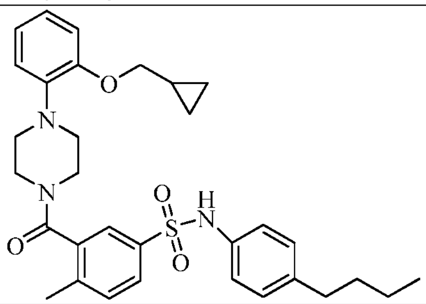 | B |
| 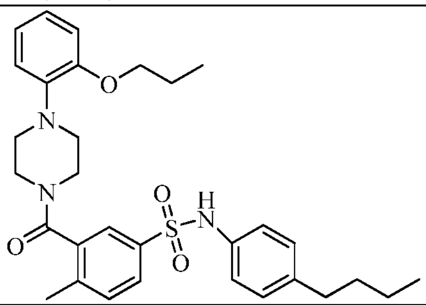 | B |

| Compound | $IC_{50}$ |
|---|---|
| 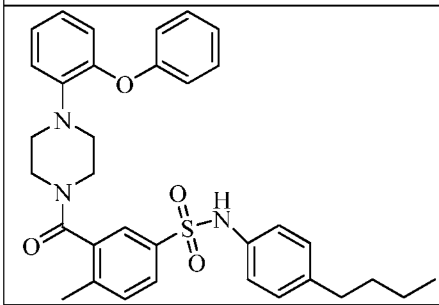 | B |
| 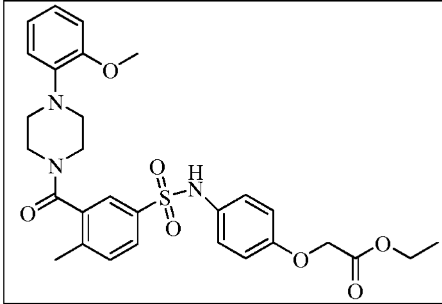 | B |
| 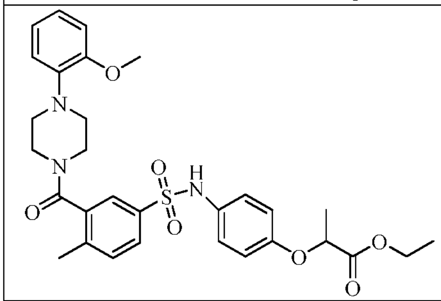 | B |
| 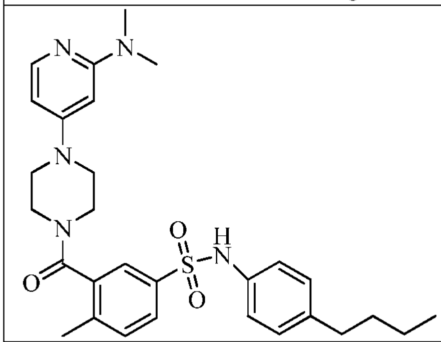 | B |
| 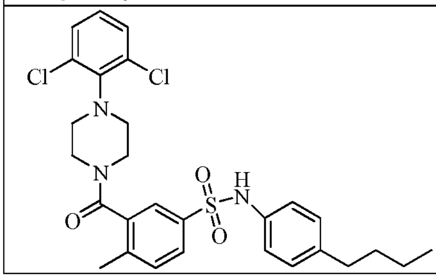 | B |

| Compound | IC50 |
|---|---|
| 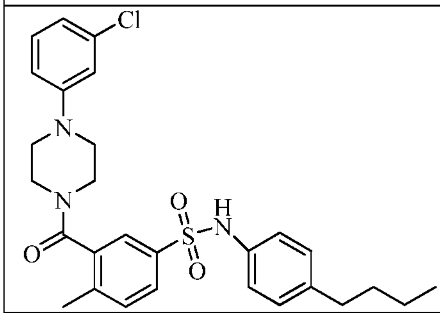 | B |
| 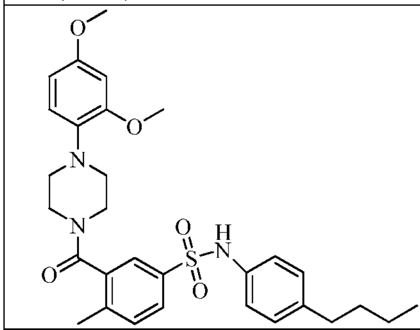 | B |
| 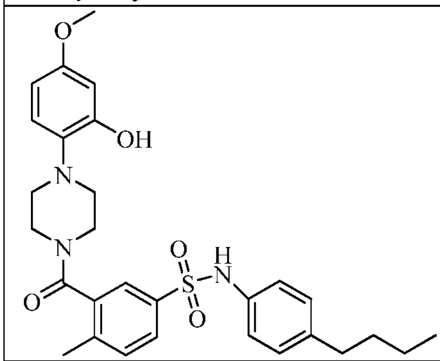 | B |
| 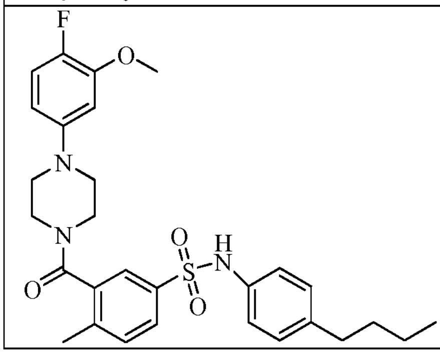 | B |
| 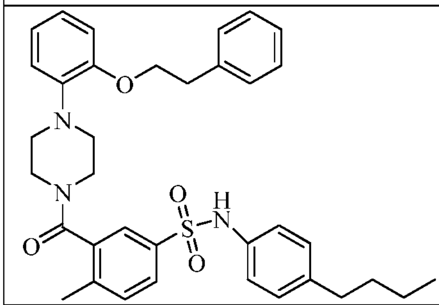 | B |
| 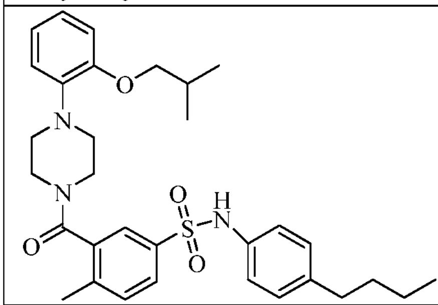 | B |
| 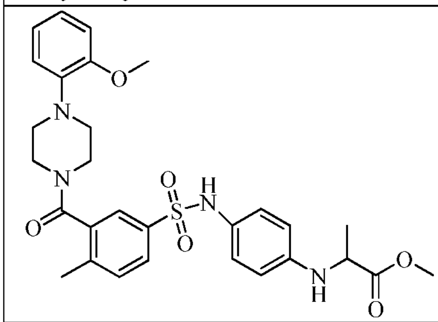 | B |
| 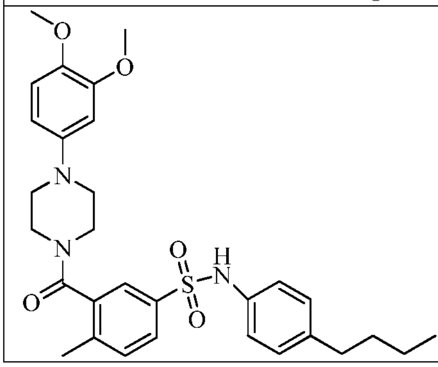 | B |

| Compound | $IC_{50}$ |
|---|---|
| 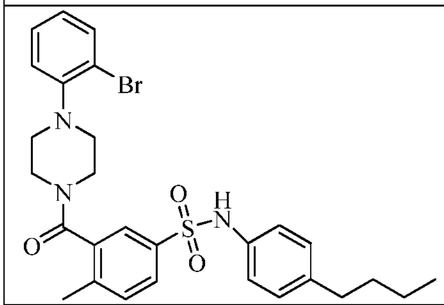 | B |
| 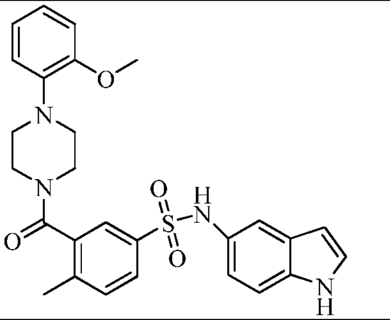 | B |
| 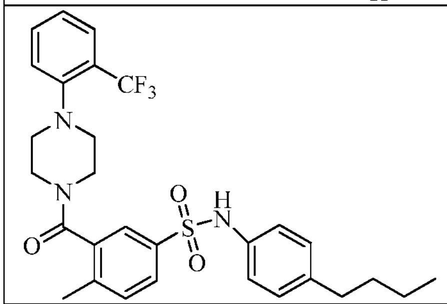 | B |
| 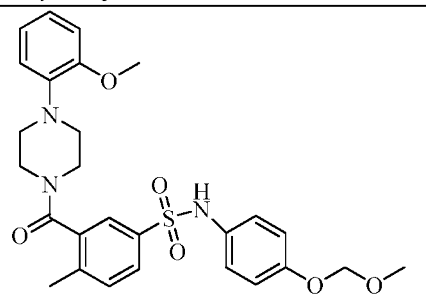 | B |
| 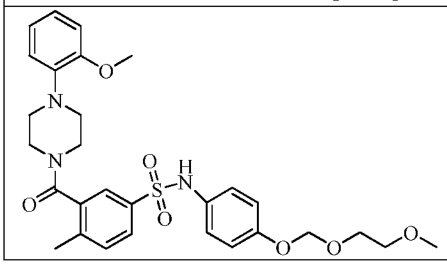 | B |

| Compound | $IC_{50}$ |
|---|---|
| 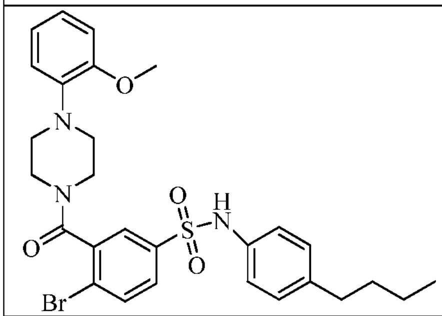 | B |
| 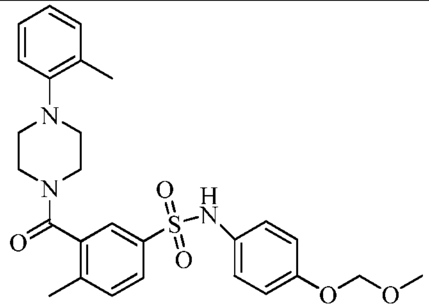 | B |
| 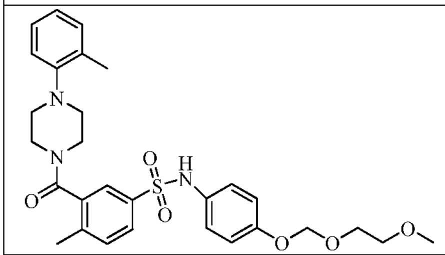 | B |
| 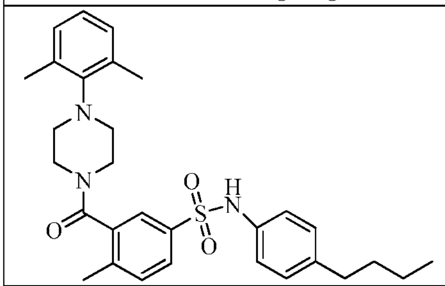 | B |
| 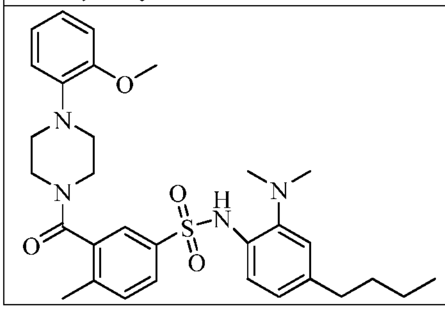 | B |

| Compound | IC50 |
|---|---|
| 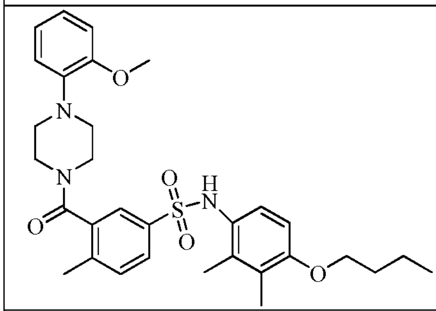 | B |
| 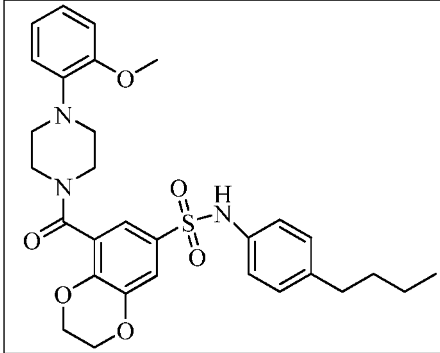 | B |
| 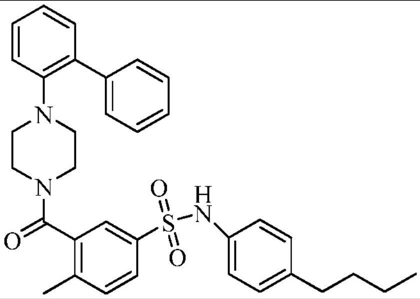 | B |
| 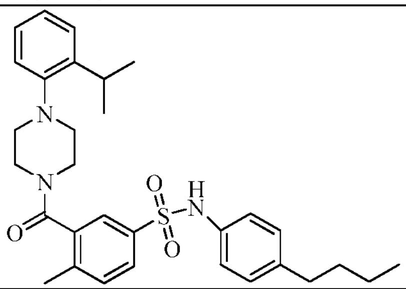 | B |
| 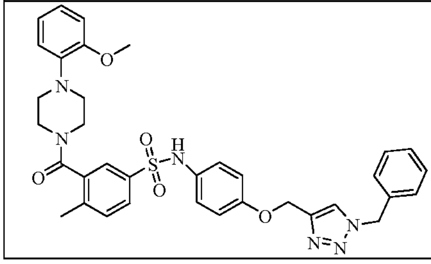 | B |

| Compound | IC50 |
|---|---|
| 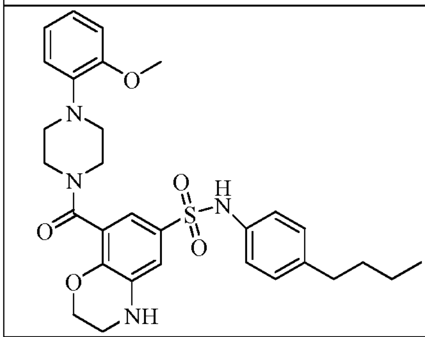 | B |
| 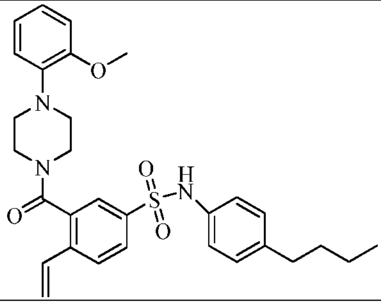 | B |
| 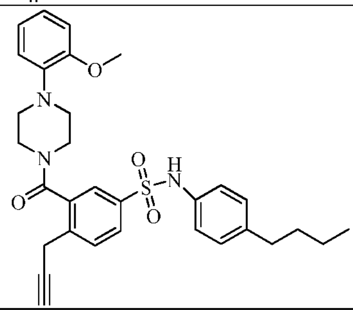 | B |
| 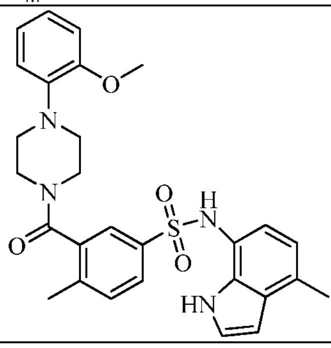 | B |

| Compound | $IC_{50}$ |
|---|---|
| 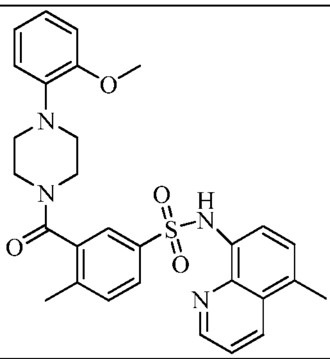 | B |
| 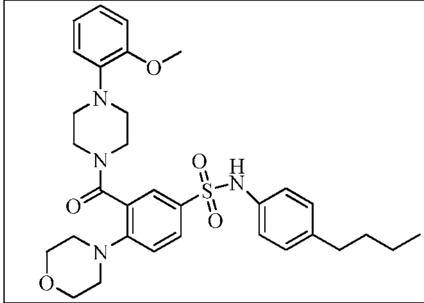 | B |
| 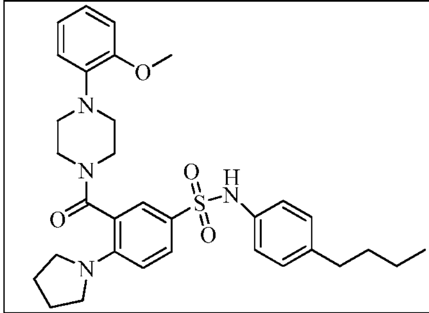 | B |
| 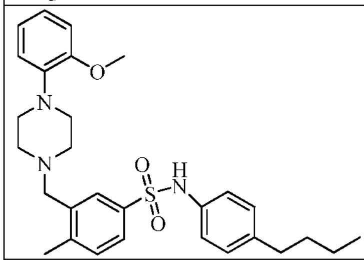 | D |
| 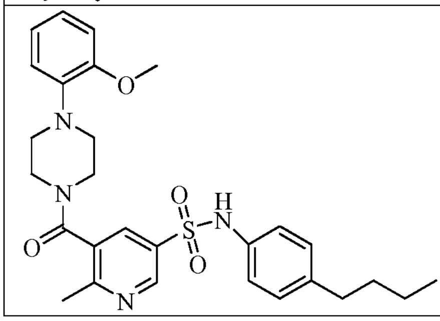 | D |

| Compound | $IC_{50}$ |
|---|---|
| 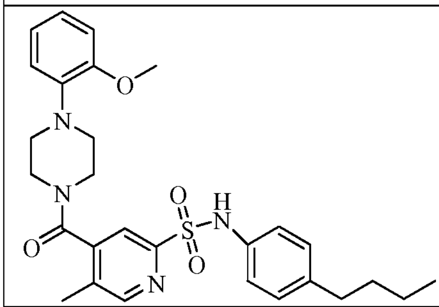 | D |
| 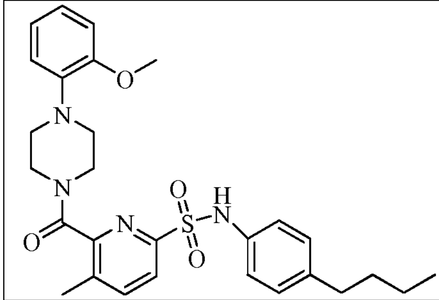 | D |
| 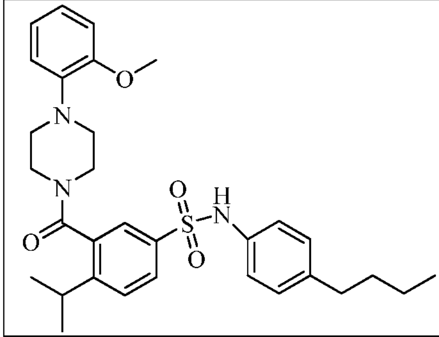 | D |
| 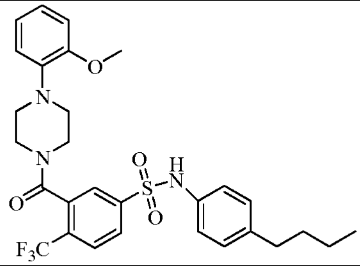 | D |
| 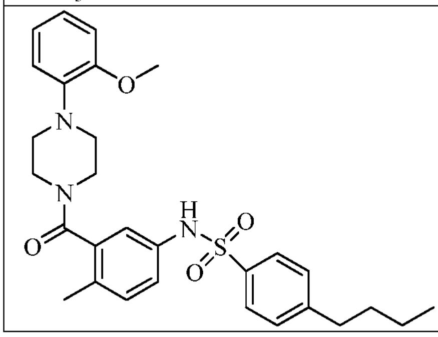 | D |

| Compound | $IC_{50}$ |
|---|---|
| 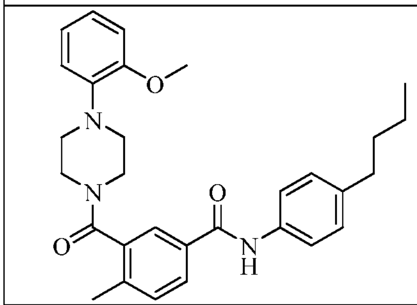 | D |
| 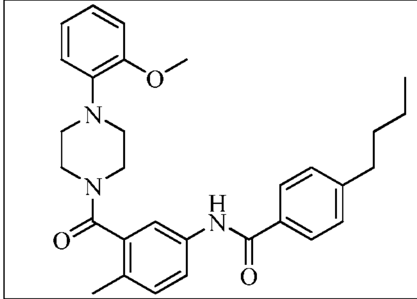 | B |
| 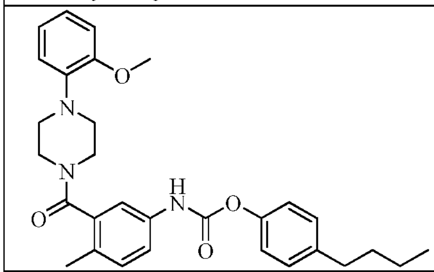 | C |
| 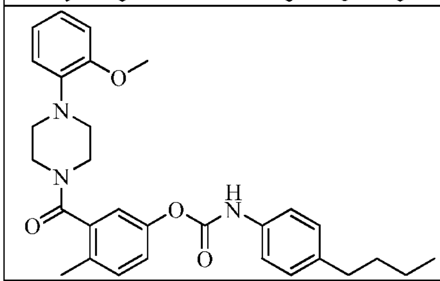 | D |
| 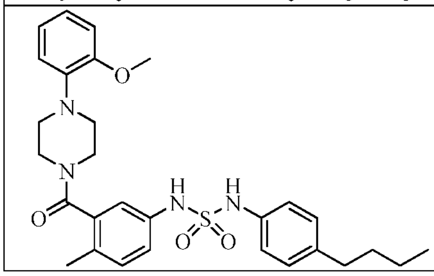 | D |

| Compound | $IC_{50}$ |
|---|---|
| 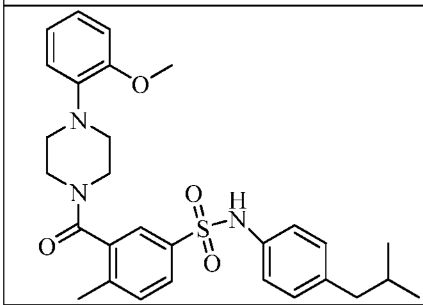 | D |
| 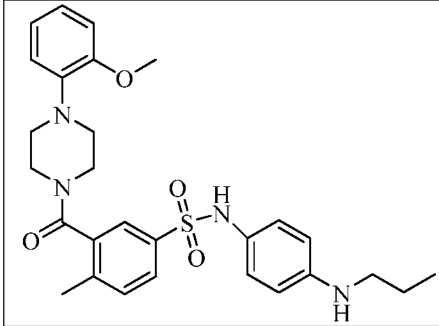 | D |
| 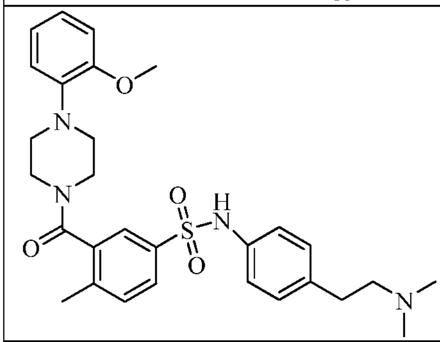 | D |
| 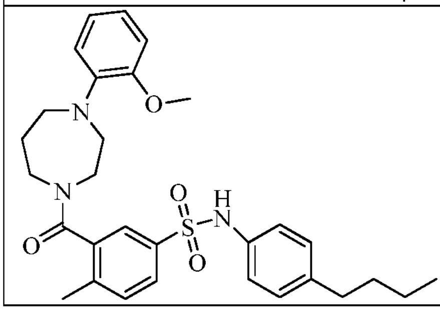 | D |

| Compound | $IC_{50}$ |
|---|---|
| 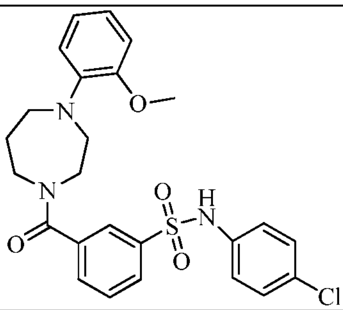 | D |
| 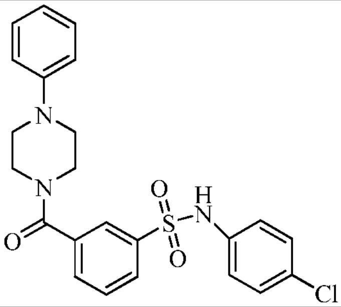 | D |
| 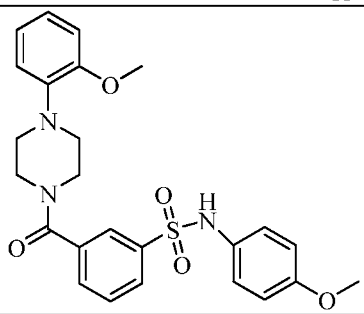 | D |
| 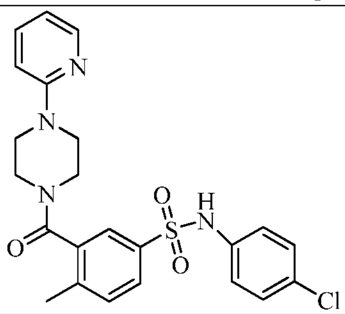 | C |
| 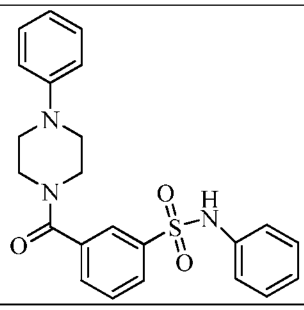 | D |

| Compound | $IC_{50}$ |
|---|---|
| 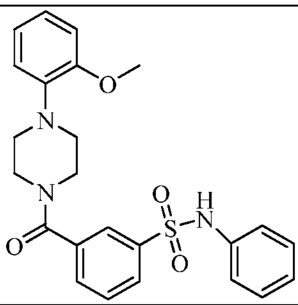 | C |
| 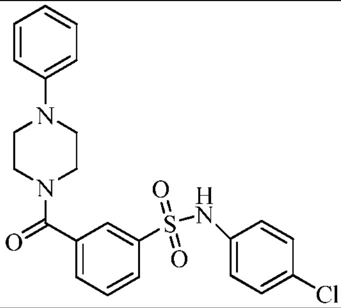 | C |
| 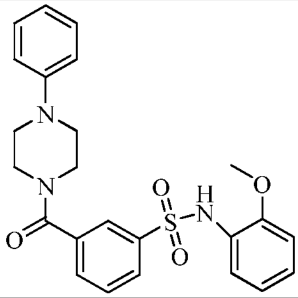 | C |
| 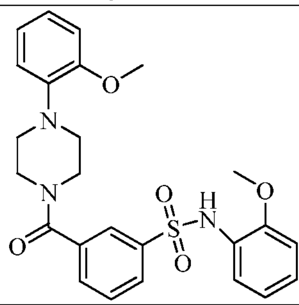 | C |
| 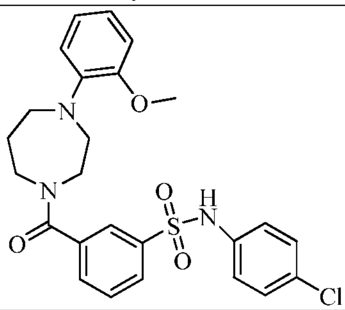 | C |

| Compound | IC$_{50}$ |
|---|---|
| 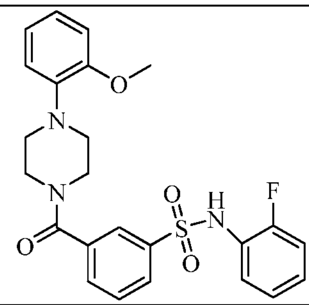 | C |
| 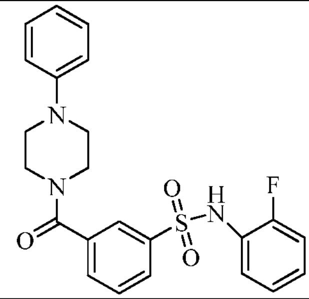 | C |
| 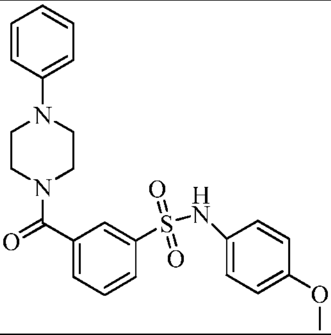 | C |
| 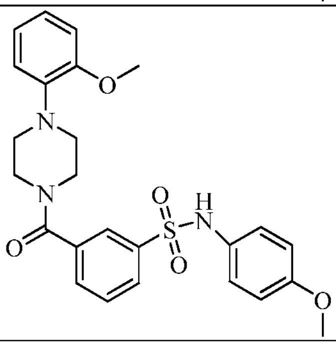 | C |

| Compound | IC$_{50}$ |
|---|---|
| 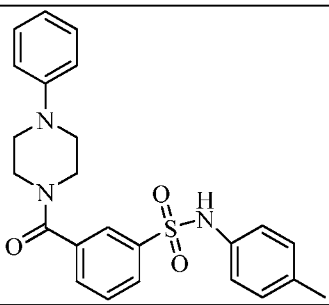 | C |
| 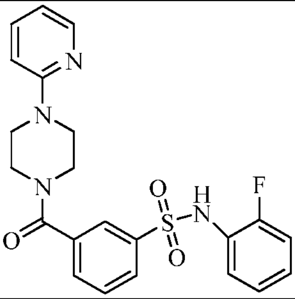 | C |
| 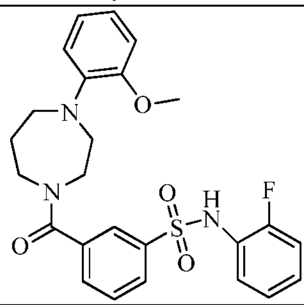 | B |
| 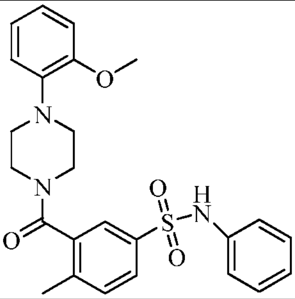 | C |
| 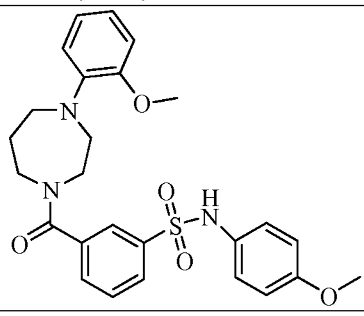 | C |

| Compound | $IC_{50}$ |
|---|---|
| 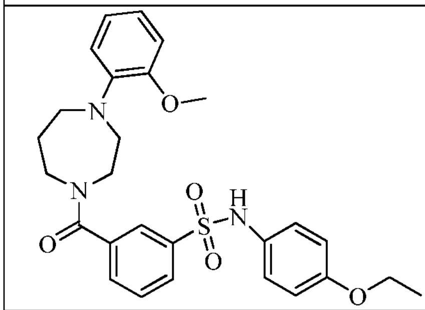 | C |
| 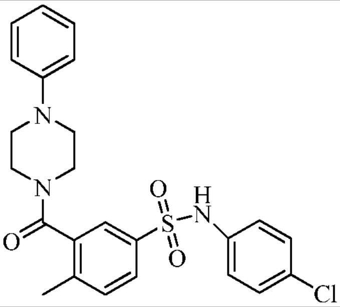 | C |
| 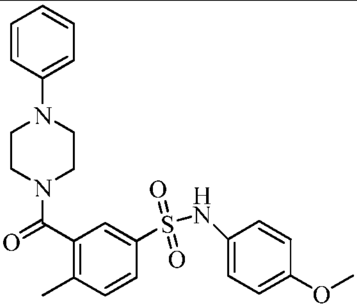 | 51 |
| 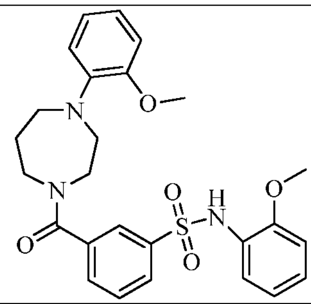 | C |
| 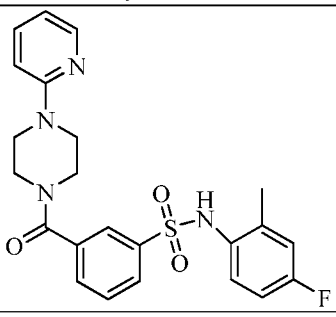 | C |

| Compound | $IC_{50}$ |
|---|---|
| 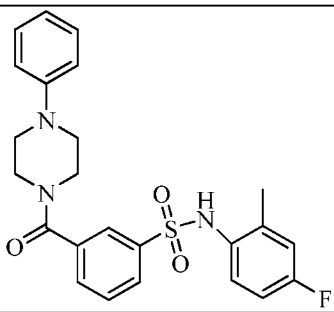 | C |
| 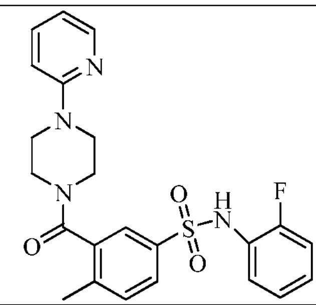 | C |
| 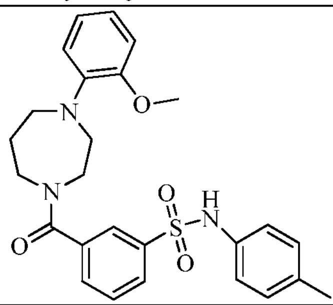 | C |
| 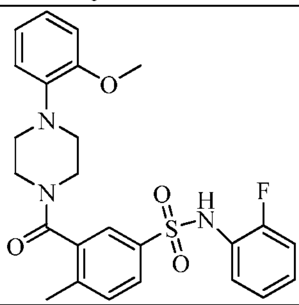 | C |
| 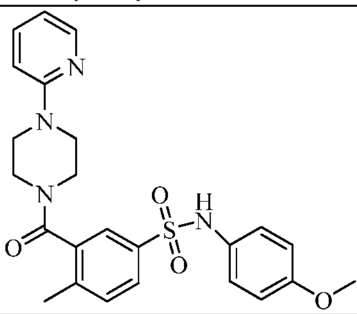 | C |

| Compound | $IC_{50}$ |
|---|---|
| 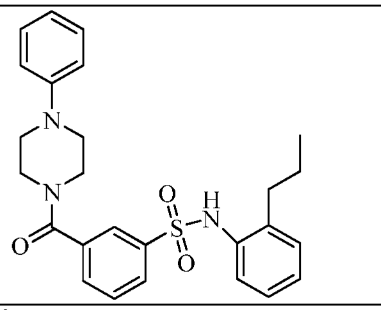 | B |
| 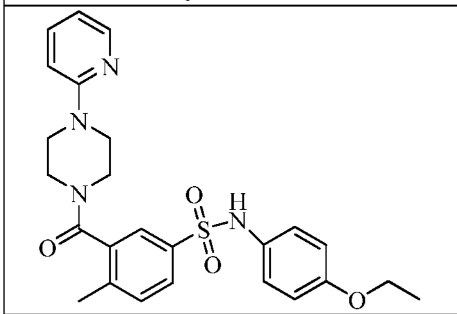 | C |
| 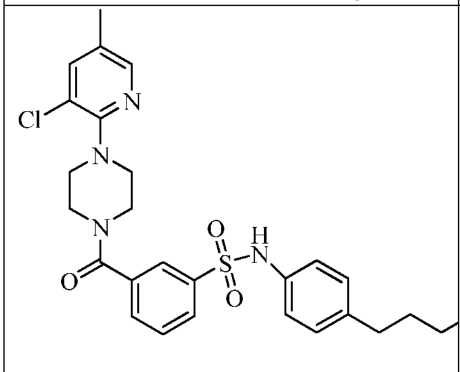 | B |
| 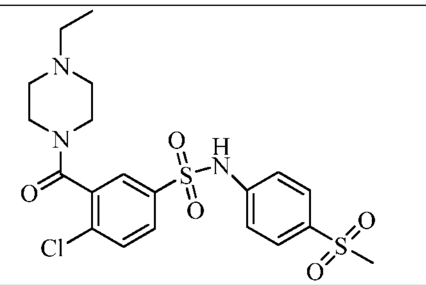 | D |
| 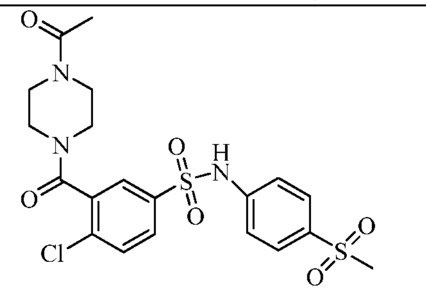 | D |

| Compound | $IC_{50}$ |
|---|---|
| 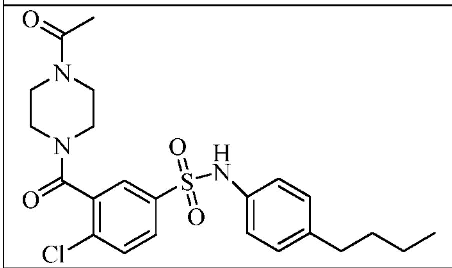 | C |
| 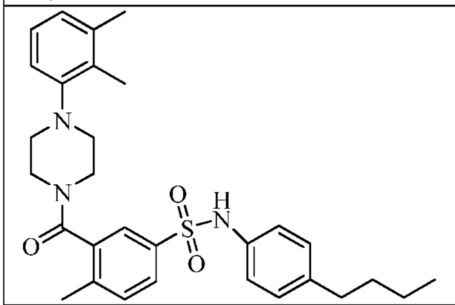 | D |
| 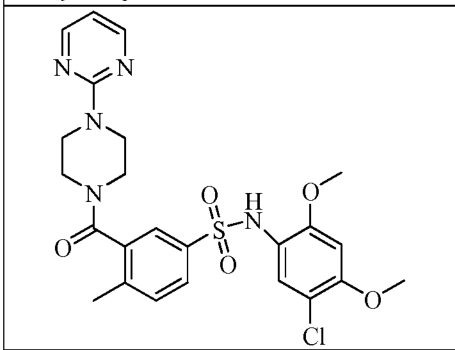 | D |
| 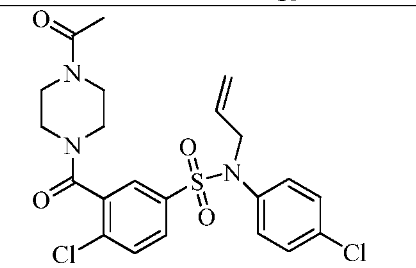 | D |
| 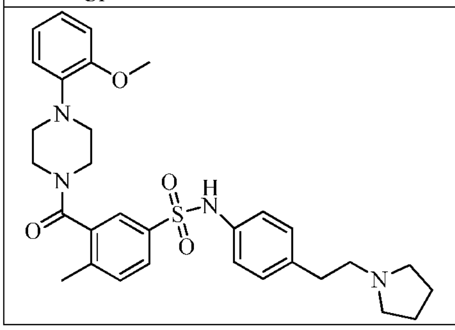 | C |

| Compound | IC50 |
|---|---|
| 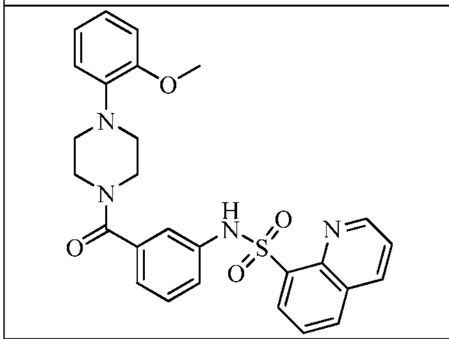 | C |
| 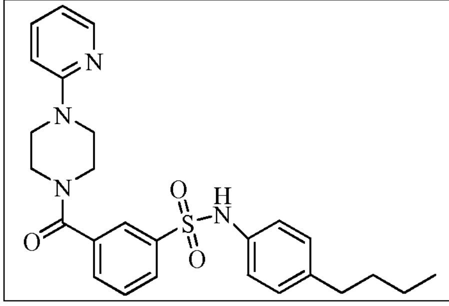 | B |

| Compound | IC50 |
|---|---|
| 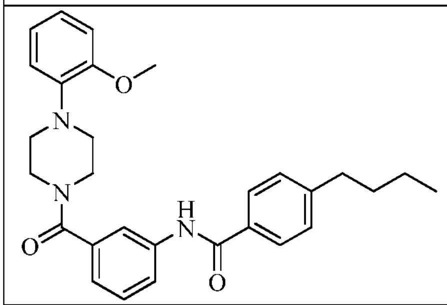 | B |
| 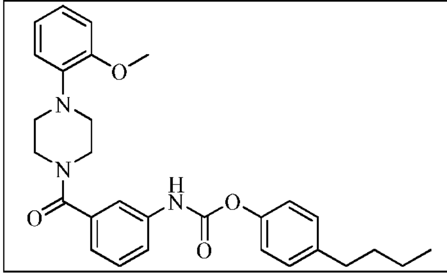 | C |

The compounds described herein can be made using a variety of synthetic techniques.

## Scheme 1.

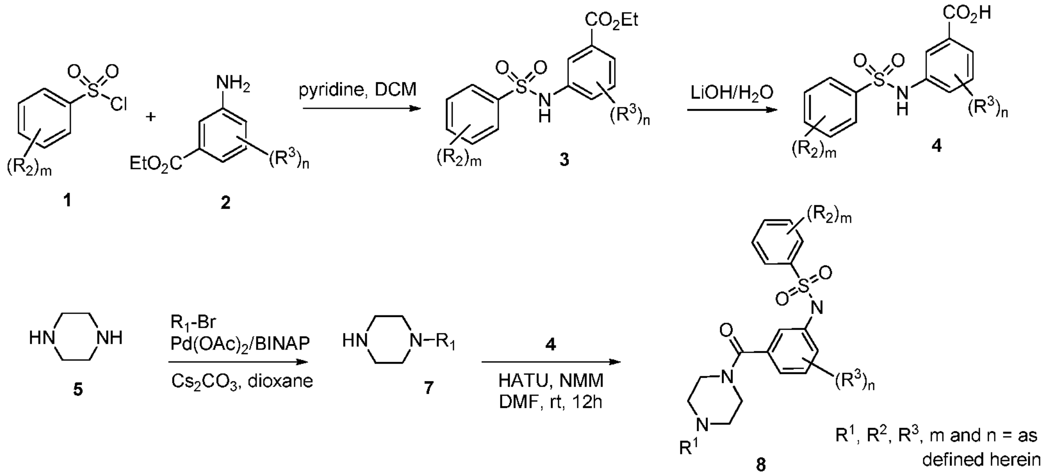


Scheme 1 above is an exemplary scheme that depicts a representative synthesis of certain compounds described herein. Sulfonyl chloride **1** is reacted with amine **2** under standard coupling conditions to produce ester 3. Hydrolysis of **3** using lithium hydroxide generates carboxylic acid **4**. Piperazine (**5**) is coupled with the appropriate bromide under standard palladium coupling conditions to provide **7**. Carboxylic acid **4** is then treated with piperazine derivative **7** to produce final compound **8**.

As can be appreciated by the skilled artisan, methods of synthesizing the compounds of the formulae herein will be evident to those of ordinary skill in the art. Additionally, the various synthetic steps may be performed in an alternate sequence or order to give the desired compounds. Synthetic chemistry transformations and protecting group methodologies (protection and deprotection) useful in synthesizing the compounds described herein are known in the art and include, for example, those such as described in R. Larock, Comprehensive Organic Transformations, VCH Publishers (1989); T.W. Greene and P.G.M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), and subsequent editions thereof.

The compounds of this invention may contain one or more asymmetric centers and thus occur as racemates and racemic mixtures, single enantiomers, individual diastereomers and diastereomeric mixtures. All such isomeric forms of these compounds are expressly included in the present invention as described below. The compounds of this invention include the compounds themselves, as well as their salts and their prodrugs, as described below.

The compounds of this invention may also be represented in multiple tautomeric forms, in such instances, the invention expressly includes all tautomeric forms of the compounds described herein, even though only a single tautomeric form may be represented (e.g., alkylation of a ring system may result in alkylation at multiple sites, the invention expressly includes all such reaction products). All such isomeric forms of such compounds are expressly included in the present invention. All crystal forms of the compounds described herein are expressly included in the present invention.

The compounds of this invention may be modified by appending appropriate functionalities to enhance selected biological properties, e.g., targeting to a particular tissue. Such modifications are known in the art and include those which increase biological penetration into a given biological compartment (e.g., blood, lymphatic system, central nervous system), increase oral availability, increase solubility to allow administration by injection, alter metabolism and alter rate of excretion.

In an alternate embodiment, the compounds described herein may be used as platforms or scaffolds that may be utilized in combinatorial chemistry techniques for preparation of derivatives and/or chemical libraries of compounds. Such derivatives and libraries of compounds have biological activity and are useful for identifying and designing compounds possessing a particular activity. Combinatorial techniques suitable for utilizing the compounds described herein are known in the art as exemplified by Obrecht, D. and Villalgrodo, J.M., Solid-Supported Combinatorial and Parallel Synthesis of Small-Molecular-Weight Compound Libraries, Pergamon-Elsevier Science Limited (1998), and include those such as the "split and pool" or "parallel" synthesis techniques, solid-phase and solution-phase techniques, and encoding techniques (see, for example, Czarnik, A.W., Curr. Opin. Chem. Bio., (1997) 1, 60. Thus, one embodiment relates to a method of using the compounds described herein for generating derivatives or chemical libraries comprising: 1) providing a body comprising a plurality of wells; 2) providing one or more compounds identified by methods described herein in each well; 3) providing an additional one or more chemicals in each well; 4) isolating the resulting one or more products from each well. An alternate embodiment relates to a method of using the compounds described herein for generating derivatives or chemical libraries comprising: 1) providing one or more compounds described herein attached to a solid support; 2) treating the one or more compounds identified by methods described herein attached to a solid support with one or more additional chemicals; 3) isolating the resulting one or more products from the solid support. In the methods described above, "tags" or identifier or labeling moieties may be attached to and/or detached from the compounds described herein or their derivatives, to facilitate tracking, identification or isolation of the desired products or their intermediates. Such moieties are known in the art. The chemicals used in the aforementioned methods may include, for example, solvents, reagents, catalysts, protecting group and deprotecting group reagents and the like. Examples of such chemicals are those that appear in the various synthetic and protecting group chemistry texts and treatises referenced herein.

## Isomers

Certain compounds may exist in one or more particular geometric, optical, enantiomeric, diasteriomeric, epimeric, atropic, stereoisomer, tautomeric, conformational, or anomeric forms, including but not limited to, cis- and trans-forms; E- and Z-forms; c-, t-, and r- forms; endo- and exo-forms; R-, S-, and meso-forms; D- and L-forms; d- and 1-forms; (+) and (-) forms; keto-, enol-, and enolate-forms; syn- and anti-forms; synclinal- and anticlinal-forms; $\alpha$- and $\beta$-forms; axial and equatorial forms; boat-, chair-, twist-, envelope-, and halfchair-forms; and combinations thereof, hereinafter collectively referred to as "isomers" (or "isomeric forms").

In one embodiment, a compound described herein, *e.g.,* an inhibitor of a neoactivity or 2-HG is an enantiomerically enriched isomer of a stereoisomer described herein. For example, the compound has an enantiomeric excess of at least about 10%, 15%, 20%, 25%, 30%, 35%, 40%, 45%, 50%, 55%, 60%, 65%, 70%, 75%, 80%, 85%, 90%, 95%, 96%, 97%, 98%, or 99%. Enantiomer, when used herein, refers to either of a pair of chemical compounds whose molecular structures have a mirror-image relationship to each other.

In one embodiment, a preparation of a compound disclosed herein is enriched for an isomer of the compound having a selected stereochemistry, *e.g.*, R or S, corresponding to a selected stereocenter, *e.g.,* the 2-position of 2-hydroxyglutaric acid. 2HG can be purchased from commercial sources or can be prepared using methods known in the art, for example, as described in Org. Syn. Coll vol., 7, P-99, 1990. For example, the compound has a purity corresponding to a compound having a selected stereochemistry of a selected stereocenter of at least about 60%, 65%, 70%, 75%, 80%, 85%, 90%, 95%, 96%, 97%, 98%, or 99%.

In one embodiment, a composition described herein includes a preparation of a compound disclosed herein that is enriched for a structure or structures having a selected stereochemistry, *e.g.,* R or S, at a selected stereocenter, *e.g.,* the 2-position of 2-hydroxyglutaric acid. Exemplary R/S configurations can be those provided in an example described herein.

An "enriched preparation," as used herein, is enriched for a selected stereoconfiguration of one, two, three or more selected stereocenters within the subject compound. Exemplary selected stereocenters and exemplary stereoconfigurations thereof can be selected from those provided herein, *e.g.,* in an example described herein. By enriched is meant at least 60%, *e.g.,* of the molecules of compound in the preparation have a selected stereochemistry of a selected stereocenter. In an embodiment it is at least 65%, 70%, 75%, 80%, 85%, 90%, 95%, 96%, 97%, 98%, or 99%. Enriched refers to the level of a subject molecule(s) and does not connote a process limitation unless specified.

Note that, except as discussed below for tautomeric forms, specifically excluded from the term "isomers," as used herein, are structural (or constitutional) isomers (*i.e.*, isomers which differ in the connections between atoms rather than merely by the position of atoms in space). For example, a reference to a methoxy group, $-OCH_3$, is not to be construed as a reference to its structural isomer, a hydroxymethyl group, $-CH_2OH$. Similarly, a reference to ortho-chlorophenyl is not to be construed as a reference to its structural isomer, meta-chlorophenyl. However, a reference to a class of structures may well include structurally isomeric forms falling within that class (*e.g.,* C1-7alkyl includes n-propyl and iso-propyl; butyl includes n-, iso-, sec-, and tert-butyl; methoxyphenyl includes ortho-, meta-, and para-methoxyphenyl).

The above exclusion does not pertain to tautomeric forms, for example, keto-, enol-, and enolate-forms, as in, for example, the following tautomeric pairs: keto/enol (illustrated below), imine/enamine, amide/imino alcohol, amidine/amidine, nitroso/oxime, thioketone/enethiol, N-nitroso/hydroxyazo, and nitro/aci-nitro.

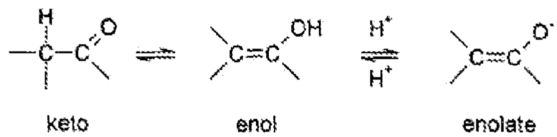

Note that specifically included in the term "isomer" are compounds with one or more isotopic substitutions. For example, H may be in any isotopic form, including 1H, 2H (D), and 3H (T); C may be in any isotopic form, including 12C, 13C, and 14C; O may be in any isotopic form, including 160 and 180; and the like. Unless otherwise specified, a reference to a particular compound includes all such isomeric forms, including (wholly or partially) racemic and other mixtures thereof. Methods for the preparation *(e.g.,* asymmetric synthesis) and separation *(e.g.,* fractional crystallisation and chromatographic means) of such isomeric forms are either known in the art or are readily obtained by adapting the methods taught herein, or known methods, in a known manner.

## Salts

It may be convenient or desirable to prepare, purify, and/or handle a corresponding salt of the active compound, for example, a pharmaceutically-acceptable salt. Examples of pharmaceutically acceptable salts are discussed in Berge et al., 1977, "Pharmaceutically Acceptable Salts." J. Pharm. ScL. Vol. 66, pp. 1-19.

For example, if the compound is anionic, or has a functional group which may be anionic *(e.g.,* -COOH may be -COO"), then a salt may be formed with a suitable cation. Examples of suitable inorganic cations include, but are not limited to, alkali metal ions such as Na+ and K+, alkaline earth cations such as Ca2+ and Mg2+, and other cations such as $Al^{+3}$. Examples of suitable organic cations include, but are not limited to, ammonium ion (*i.e.*, $NH^{4+}$) and substituted ammonium ions (*e.g.*, $NH_3R^+$, $NH_2R^{2+}$, $NHR^{3+}$, $NR^{4+}$). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is $N(CH_3)^{4+}$.

If the compound is cationic, or has a functional group that may be cationic (*e.g.,* $-NH_2$ may • be $-NH_3^+$), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose.

Unless otherwise specified, a reference to a particular compound also includes salt forms thereof.

## Chemically Protected Forms

It may be convenient or desirable to prepare, purify, and/or handle the active compound in a chemically protected form. The term "chemically protected form" is used herein in the conventional chemical sense and pertains to a compound in which one or more reactive functional groups are protected from undesirable chemical reactions under specified conditions (*e.g.*, pH, temperature, radiation, solvent, and the like). In practice, well known chemical methods are employed to reversibly render unreactive a functional group, which otherwise would be reactive, under specified conditions. In a chemically protected form, one or more reactive functional groups are in the form of a protected or protecting group (also known as a masked or masking group or a blocked or blocking group). By protecting a reactive functional group, reactions involving other unprotected reactive functional groups can be performed, without affecting the protected group; the protecting group may be removed, usually in a subsequent step, without substantially affecting the remainder of the molecule. See, for example, Protective Groups in Organic Synthesis (T. Green and P. Wuts; 3rd Edition; John Wiley and Sons, 1999). Unless otherwise specified, a reference to a particular compound also includes chemically protected forms thereof.

A wide variety of such "protecting," "blocking," or "masking" methods are widely used and well known in organic synthesis. For example, a compound which has two nonequivalent reactive functional groups, both of which would be reactive under specified conditions, may be derivatized to render one of the functional groups "protected," and therefore unreactive, under the specified conditions; so protected, the compound may be used as a reactant which has effectively only one reactive functional group. After the desired reaction (involving the other functional group) is complete, the protected group may be "deprotected" to return it to its original functionality.

For example, a hydroxy group may be protected as an ether (-OR) or an ester (-OC(=O)R), for example, as: a t-butyl ether; a benzyl, benzhydryl (diphenylmethyl), or trityl (triphenylmethyl) ether; a trimethylsilyl or t-butyldimethylsilyl ether; or an acetyl ester (-OC(=O)CH3, -OAc).

For example, an aldehyde or ketone group may be protected as an acetal (R-CH(OR)2) or ketal (R2C(OR)2), respectively, in which the carbonyl group (>C=O) is converted to a diether (>C(OR)2), by reaction with, for example, a primary alcohol. The aldehyde or ketone group is readily regenerated by hydrolysis using a large excess of water in the presence of acid.

For example, an amine group may be protected, for example, as an amide (-NRCO-R) or a urethane (-NRCO-OR), for example, as: a methyl amide (-NHCO-CH3); a benzyloxy amide (-NHCO-OCH2C6H5, -NH-Cbz); as a t-butoxy amide (-NHCO-OC(CH3)3, -NH-Boc); a 2-biphenyl-2-propoxy amide (-NHCO-OC(CH3)2C6H4C6H5, -NH-Bpoc), as a 9- fluorenylmethoxy amide (-NH-Fmoc), as a 6-nitroveratryloxy amide (-NH-Nvoc), as a 2-trimethylsilylethyloxy amide (-NH-Teoc), as a 2,2,2-trichloroethyloxy amide (-NH-Troc), as an allyloxy amide (-NH-Alloc), as a 2(-phenylsulphonyl)ethyloxy amide (-NH-Psec); or, in suitable cases *(e.g.,* cyclic amines), as a nitroxide radical (>N-O«).

For example, a carboxylic acid group may be protected as an ester for example, as: an alkyl ester (*e.g.,* a methyl ester; a t-butyl ester); a haloalkyl ester (*e.g.,* a C1-7trihaloalkyl ester); a triC1-7alkylsilyl-C1-7alkyl ester; or a C5.2oaryl-C1-7alkyl ester (*e.g.,* a benzyl ester; a nitrobenzyl ester); or as an amide, for example, as a methyl amide.

For example, a thiol group may be protected as a thioether (-SR), for example, as: a benzyl thioether; an acetamidomethyl ether (-S-CH2NHC(=O)CH3).

## Methods of treating a proliferative disorder

Described herein are methods of treating a cell proliferation-related disorder, *e.g.,* a cancer, *e.g.,* a glioma, AML, prostate cancer, thyroid cancer, fibrosarcoma or melanoma, *e.g.,* by inhibiting a neoactivity of a mutant IDH enzyme, *e.g.,* IDH1 or IDH2. The cancer can be characterized by the presence of a neoactivity. In some embodiments, the gain of function is the conversion of $\alpha$-ketoglurarate to 2-hydroxyglutarate, *e.g.*, R-2-hydroxyglutarate.

## Disorders

The IDH-related methods disclosed herein, *e.g.*, methods of evaluating or treating subjects, are directed to subjects having a cell proliferation-related disorder characterized by an IDH mutant, *e.g.*, an IDH1 or IDH2, mutant having neoactivity, *e.g.,* 2HG neoactivity. Examples of some of the disorders below have been shown to be characterized by an IDH1 or IDH2 mutation. Others can be analyzed, *e.g.,* by sequencing cell samples to determine the presence of a somatic mutation at amino acid 132 of IDH1 or at amino acid 172 of IDH2. Without being bound by theory it is expected that a portion of the tumors of given type of cancer will have an IDH, e.g., IDH1 or IDH2, mutant having 2HG neoactivity.

The disclosed methods are useful in evaluating or treating proliferative disorders, *e.g.* evaluating or treating solid tumors, soft tissue tumors, and metastases thereof wherein the solid tumor, soft tissue tumor or metastases thereof is a cancer described herein. Exemplary solid tumors include malignancies (*e.g.*, sarcomas, adenocarcinomas, and carcinomas) of the various organ systems, such as those of brain, lung, breast, lymphoid, gastrointestinal (*e.g.,* colon),

and genitourinary (*e.g.,* renal, urothelial, or testicular tumors) tracts, pharynx, prostate, and ovary. Exemplary adenocarcinomas include colorectal cancers, renal-cell carcinoma, liver cancer, non-small cell carcinoma of the lung, and cancer of the small intestine. The disclosed methods are also useful in evaluating or treating non-solid cancers.

The methods described herein can be used with any cancer, for example those described by the National Cancer Institute. A cancer can be evaluated to determine whether it is using a method described herein. Exemplary cancers described by the National Cancer Institute include: Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood; Carcinoid Tumor, Childhood; Carcinoid Tumor, Gastrointestinal; Carcinoma, Adrenocortical; Carcinoma, Islet Cell; Carcinoma of Unknown Primaiy; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma, Childhood Brain Stem; Glioma, Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS- Related; Lymphoma, Central Nervous System (Primary); Lymphoma, Cutaneous T-Cell; Lymphoma, Hodgkin's, Adult; Lymphoma, Hodgkin's, Childhood; Lymphoma, Hodgkin's During Pregnancy; Lymphoma, Non-Hodgkin's, Adult; Lymphoma, Non- Hodgkin's, Childhood; Lymphoma, Non-Hodgkin's During Pregnancy; Lymphoma, Primary Central Nervous System; Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplastic Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Non-Hodgkin's Lymphoma, Adult; Non-Hodgkin's Lymphoma, Childhood; Non- Hodgkin's Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood; Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland Cancer, Childhood; Sarcoma, Ewing's Family of Tumors; Sarcoma, Kaposi's; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial

Primitive Neuroectodermal Tumors, Childhood; T- Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom's Macro globulinemia; and Wilms' Tumor. Metastases of the aforementioned cancers can also be treated or prevented in accordance with the methods described herein.

The methods described herein are useful in treating cancer in nervous system, *e.g.,* brain tumor, *e.g.,* glioma, *e.g.,* glioblastoma multiforme (GBM), *e.g.,* by inhibiting a neoactivity of a mutant enzyme, *e.g.*, an enzyme in a metabolic pathway, *e.g.,* a metabolic pathway leading to fatty acid biosynthesis, glycolysis, glutaminolysis, the pentose phosphate shunt, the nucleotide biosynthetic pathway, or the fatty acid biosynthetic pathway, *e.g.*, IDH1 or IDH2.

Gliomas, a type of brain tumors, can be classified as grade I to grade IV on the basis of histopathological and clinical criteria established by the World Health Organization (WHO). WHO grade I gliomas are often considered benign. Gliomas of WHO grade II or III are invasive, progress to higher-grade lesions. WHO grade IV tumors (glioblastomas) are the most invasive form. Exemplary brain tumors include, *e.g.,* astrocytic tumor (*e.g.,* pilocytic astrocytoma, subependymal giant-cell astrocytoma, diffuse astrocytoma, pleomorphic xanthoastrocytoma, anaplastic astrocytoma, astrocytoma, giant cell glioblastoma, glioblastoma, secondary glioblastoma, primary adult glioblastoma, and primary pediatric glioblastoma); oligodendroglial tumor (*e.g.,* oligodendroglioma, and anaplastic oligodendroglioma); oligoastrocytic tumor (*e.g.*, oligoastrocytoma, and anaplastic oligoastrocytoma); ependymoma (*e.g.*, myxopapillary ependymoma, and anaplastic ependymoma); medulloblastoma; primitive neuroectodermal tumor, schwannoma, meningioma, meatypical meningioma, anaplastic meningioma; and pituitary adenoma. Exemplary cancers are described in Acta Neuropathol (2008) 116:597-602 and N Engl J Med. 2009 Feb 19;360(8):765-73.

In embodiments the disorder is glioblastoma.

In an embodiment the disorder is prostate cancer, e.g., stage T1 (*e.g.,* T1a, T1b and T1c), T2 (*e.g.,* T2a, T2b and T2c), T3 (*e.g.,* T3a and T3b) and T4, on the TNM staging system. In embodiments the prostate cancer is grade G1, G2, G3 or G4 (where a higher number indicates greater difference from normal tissue).. Types of prostate cancer include, *e.g.*, prostate adenocarcinoma, small cell carcinoma, squamous carcinoma, sarcomas, and transitional cell carcinoma.

Methods and compositions of the inventin can be combined with art-known treatment. Art-known treatment for prostate cancer can include, *e.g.*, active surveillance, surgery (*e.g.*, radical prostatectomy, transurethral resection of the prostate, orchiectomy, and cryosurgegry), radiation therapy including brachytherapy (prostate brachytherapy) and external beam radiation therapy, High-Intensity Focused Ultrasound (HIFU), chemotherapy, cryosurgery, hormonal therapy (*e.g.,* antiandrogens (*e.g.*, flutamide, bicalutamide, nilutamide and cyproterone acetate, ketoconazole, aminoglutethimide), GnRH antagonists (*e.g.*, Abarelix)), or a combination thereof.

## Methods of Treatment

The compounds and compositions described herein can be administered to cells in culture, e.g. *in vitro* or *ex vivo,* or to a subject, e.g., *in vivo,* to treat, prevent, and/or diagnose a variety of disorders, including those described herein. The compounds and compostions described herein also are useful for treating an aciduria subject (e.g., a 2-hydroxyglutaric aciduria subject).

As used herein, the term "treat" or "treatment" is defined as the application or administration of a compound, alone or in combination with, a second compound to a subject, e.g., a patient, or application or administration of the compound to an isolated tissue or cell, e.g., cell line, from a subject, e.g., a patient, who has a disorder (e.g., a disorder as described herein), a symptom of a disorder, or a predisposition toward a disorder, with the purpose to cure, heal, alleviate, relieve, alter, remedy, ameliorate, improve or affect the disorder, one or more symptoms of the disorder or the predisposition toward the disorder (e.g., to prevent at least one symptom of the disorder or to delay onset of at least one symptom of the disorder).

As used herein, an amount of a compound effective to treat a disorder, or a "therapeutically effective amount" refers to an amount of the compound which is effective, upon single or multiple dose administration to a subject, in treating a cell, or in curing, alleviating, relieving or improving a subject with a disorder beyond that expected in the absence of such treatment.

As used herein, an amount of a compound effective to prevent a disorder, or a "a prophylactically effective amount" of the compound refers to an amount effective, upon single- or multiple-dose administration to the subject, in preventing or delaying the occurrence of the onset or recurrence of a disorder or a symptom of the disorder.

As used herein, the term "subject" is intended to include human and non-human animals. Exemplary human subjects include a human patient having a disorder, e.g., a disorder described herein or a normal subject. The term "non-human animals" of the invention includes all vertebrates, e.g., non-mammals (such as chickens, amphibians, reptiles) and mammals, such as non-human primates, domesticated and/or agriculturally useful animals, e.g., sheep, dog, cat, cow, pig, etc.

**Combination therapies**

In some embodiments, a compound or composition described herein is administered together with an additional cancer treatment. Exemplary cancer treatments include, for example: surgery, chemotherapy, targeted therapies such as antibody therapies, immunotherapy, and hormonal therapy. Examples of each of these treatments are provided below.

*Chemotherapy*

In some embodiments, a compound or composition described herein is administered with a chemotherapy. Chemotherapy is the treatment of cancer with drugs that can destroy cancer cells. "Chemotherapy" usually refers to cytotoxic drugs which affect rapidly dividing cells in general, in contrast with targeted therapy. Chemotherapy drugs interfere with cell division in various possible ways, *e.g.*, with the duplication of DNA or the separation of newly formed chromosomes. Most forms of chemotherapy targ*et al*l rapidly dividing cells and are not specific for cancer cells, although some degree of specificity may come from the inability of many cancer cells to repair DNA damage, while normal cells generally can.

Examples of chemotherapeutic agents used in cancer therapy include, for example, antimetabolites (*e.g.*, folic acid, purine, and pyrimidine derivatives) and alkylating agents (*e.g.*, nitrogen mustards, nitrosoureas, platinum, alkyl sulfonates, hydrazines, triazenes, aziridines, spindle poison, cytotoxic agents, toposimerase inhibitors and others). Exemplary agents include Aclarubicin, Actinomycin, Alitretinon, Altretamine, Aminopterin, Aminolevulinic acid, Amrubicin, Amsacrine, Anagrelide, Arsenic trioxide, Asparaginase, Atrasentan, Belotecan, Bexarotene, endamustine, Bleomycin, Bortezomib, Busulfan, Camptothecin, Capecitabine, Carboplatin, Carboquone, Carmofur, Carmustine, Celecoxib, Chlorambucil, Chlormethine, Cisplatin, Cladribine, Clofarabine, Crisantaspase, Cyclophosphamide, Cytarabine, Dacarbazine, Dactinomycin, Daunorubicin, Decitabine, Demecolcine, Docetaxel, Doxorubicin, Efaproxiral, Elesclomol, Elsamitrucin, Enocitabine, Epirubicin, Estramustine, Etoglucid, Etoposide, Floxuridine, Fludarabine, Fluorouracil (5FU), Fotemustine, Gemcitabine, Gliadel implants, Hydroxycarbamide, Hydroxyurea, Idarubicin, Ifosfamide, Irinotecan, Irofulven, Ixabepilone, Larotaxel, Leucovorin, Liposomal doxorubicin, Liposomal daunorubicin, Lonidamine, Lomustine, Lucanthone, Mannosulfan, Masoprocol, Melphalan, Mercaptopurine, Mesna, Methotrexate, Methyl aminolevulinate, Mitobronitol, Mitoguazone, Mitotane, Mitomycin, Mitoxantrone, Nedaplatin, Nimustine, Oblimersen, Omacetaxine, Ortataxel, Oxaliplatin, Paclitaxel, Pegaspargase, Pemetrexed, Pentostatin, Pirarubicin, Pixantrone, Plicamycin, Porfimer sodium, Prednimustine, Procarbazine, Raltitrexed, Ranimustine, Rubitecan, Sapacitabine, Semustine, Sitimagene ceradenovec, Strataplatin, Streptozocin, Talaporfin, Tegafur-uracil, Temoporfin, Temozolomide, Teniposide, Tesetaxel, Testolactone, Tetranitrate, Thiotepa, Tiazofurine, Tioguanine, Tipifarnib, Topotecan, Trabectedin, Triaziquone, Triethylenemelamine, Triplatin, Tretinoin, Treosulfan, Trofosfamide, Uramustine, Valrubicin, Verteporfin, Vinblastine, Vincristine, Vindesine, Vinflunine, Vinorelbine, Vorinostat, Zorubicin, and other cytostatic or cytotoxic agents described herein.

Because some drugs work better together than alone, two or more drugs are often given at the same time. Often, two or more chemotherapy agents are used as combination chemotherapy. In some embodiments, the chemotherapy agents (including combination chemotherapy) can be used in combination with a compound described herein, *e.g.*, phenformin.

*Targeted therapy*

In some embodiments, a compound or composition described herein is administered with a targeted therapy. Targeted therapy constitutes the use of agents specific for the deregulated proteins of cancer cells. Small molecule targeted therapy drugs are generally inhibitors of enzymatic domains on mutated, overexpressed, or otherwise critical proteins within the cancer cell. Prominent examples are the tyrosine kinase inhibitors such as Axitinib, Bosutinib, Cediranib, desatinib, erlotinib, imatinib, gefitinib, lapatinib, Lestaurtinib, Nilotinib, Semaxanib, Sorafenib, Sunitinib, and Vandetanib, and also cyclin-depdendent kinase inhibitors such as Alvocidib and Seliciclib. Monoclonal antibody therapy is another strategy in which the therapeutic agent is an antibody which specifically binds to a protein on the surface of the cancer cells. Examples include the anti-HER2/neu antibody trastuzumab (HERCEPTIN®) typically used in breast cancer, and the anti-CD20 antibody rituximab and Tositumomab typically used in a variety of B-cell malignancies. Other exemplary antibodies include Cetuximab, Panitumumab, Trastuzumab, Alemtuzumab, Bevacizumab, Edrecolomab, and Gemtuzumab. Exemplary fusion proteins include Aflibercept and Denileukin diftitox. In some embodiments, the targeted therapy can be used in combination with a compound described herein, *e.g.,* a biguanide such as metformin or phenformin, preferably phenformin.

Targeted therapy can also involve small peptides as "homing devices" which can bind to cell surface receptors or affected extracellular matrix surrounding the tumor. Radionuclides which are attached to these peptides (*e.g.,* RGDs) eventually kill the cancer cell if the nuclide decays in the vicinity of the cell. An example of such therapy includes BEXXAR®.

*Immunotherapy*

In some embodiments, a compound or composition described herein is administered with an immunotherapy. Cancer immunotherapy refers to a diverse set of therapeutic strategies designed to induce the patient's own immune system to fight the tumor. Contemporary methods for generating an immune response against tumors include intravesicular BCG immunotherapy for superficial bladder cancer, and use of interferons and other cytokines to induce an immune response in renal cell carcinoma and melanoma patients.

Allogeneic hematopoietic stem cell transplantation can be considered a form of immunotherapy, since the donor's immune cells will often attack the tumor in a graft-versus-tumor effect. In some embodiments, the immunotherapy agents can be used in combination with a compound or composition described herein.

*Hormonal therapy*

In some embodiments, a compound or composition described herein is administered with a hormonal therapy. The growth of some cancers can be inhibited by providing or blocking certain hormones. Common examples of hormone-sensitive tumors include certain types of breast and prostate cancers. Removing or blocking estrogen or testosterone is often an important additional treatment. In certain cancers, administration of hormone agonists, such as progestogens may be therapeutically beneficial. In some embodiments, the hormonal therapy agents can be used in combination with a compound or a composition described herein.

In some embodiments, a compound or composition described herein is administered together with an additional cancer treatment (*e.g.*, surgical removal), in treating cancer in nervous system, *e.g.,* cancer in central nervous system, *e.g.,* brain tumor, *e.g.,* glioma, *e.g.,* glioblastoma multiforme (GBM).

Several studies have suggested that more than 25% of glioblastoma patients obtain a significant survival benefit from adjuvant chemotherapy. Meta-analyses have suggested that adjuvant chemotherapy results in a 6-10% increase in 1-year survival rate.

Temozolomide is an orally active alkylating agent that is used for persons newly diagnosed with glioblastoma multiforme. It was approved by the United States Food and Drug Administration (FDA) in March 2005. Studies have shown that the drug was well tolerated and provided a survival benefit. Adjuvant and concomitant temozolomide with radiation was associated with significant improvements in median progression-free survival over radiation alone (6.9 vs 5 mo), overall survival (14.6 vs 12.1 mo), and the likelihood of being alive in 2 years (26% vs 10%).

Nitrosoureas: BCNU (carmustine)-polymer wafers (Gliadel) were approved by the FDA in 2002. Though Gliadel wafers are used by some for initial treatment, they have shown only a modest increase in median survival over placebo (13.8 vs. 11.6 months) in the largest such phase III trial, and are associated with increased rates of CSF leak and increased intracranial pressure secondary to edema and mass effect.

MGMT is a DNA repair enzyme that contributes to temozolomide resistance. Methylation of the MGMT promoter, found in approximately 45% of glioblastoma multiformes, results in an epigenetic silencing of the gene, decreasing the tumor cell's capacity for DNA repair and increasing susceptibility to temozolomide.

When patients with and without MGMT promoter methylation were treated with temozolomide, the groups had median survivals of 21.7 versus 12.7 months, and 2-year survival rates of 46% versus 13.8%, respectively.

Though temozolomide is currently a first-line agent in the treatment of glioblastoma multiforme, unfavorable MGMT methylation status could help select patients appropriate for future therapeutic investigations.

O6-benzylguanine and other inhibitors of MGMT as well as RNA interference-mediated silencing of MGMT offer promising avenues to increase the effectiveness of temozolomide and other alkylating antineoplastics, and such agents are under active study.

Carmustine (BCNU) and cis -platinum (cisplatin) have been the primary chemotherapeutic agents used against malignant gliomas. All agents in use have no greater than a 30-40% response rate, and most fall into the range of 10-20%.

Data from the University of California at San Francisco indicate that, for the treatment of glioblastomas, surgery followed by radiation therapy leads to 1-, 3-, and 5-year survival rates of 44%, 6%, and 0%, respectively. By comparison, surgery followed by radiation and chemotherapy using nitrosourea-based regimens resulted in 1-, 3-, and 5-year survival rates of 46%, 18%, and 18%, respectively.

A major hindrance to the use of chemotherapeutic agents for brain tumors is the fact that the blood-brain barrier (BBB) effectively excludes many agents from the CNS. For this reason, novel methods of intracranial drug delivery are being developed to deliver higher concentrations of chemotherapeutic agents to the tumor cells while avoiding the adverse systemic effects of these medications.

Pressure-driven infusion of chemotherapeutic agents through an intracranial catheter, also known as convection-enhanced delivery (CED), has the advantage of delivering drugs along a pressure gradient rather than by simple diffusion. CED has shown promising results in animal models with agents including BCNU and topotecan.

Initial attempts investigated the delivery of chemotherapeutic agents via an intraarterial route rather than intravenously. Unfortunately, no survival advantage was observed.

Chemotherapy for recurrent glioblastoma multiforme provides modest, if any, benefit, and several classes of agents are used. Carmustine wafers increased 6-month survival from 36% to 56% over placebo in one randomized study of 222 patients, though there was a significant association between the treatment group and serious intracranial infections.

Genotyping of brain tumors may have applications in stratifying patients for clinical trials of various novel therapies.

The anti-angiogenic agent bevacizumab, when used with irinotecan improved 6-month survival in recurrent glioma patients to 46% compared with 21% in patients treated with temozolomide. This bevacizumab and irinotecan combination for recurrent glioblastoma multiforme has been shown to improve survival over bevacizumab alone. Anti-angiogenic agents also decrease peritumoral edema, potentially reducing the necessary corticosteroid dose.

Some glioblastomas responds to gefitinib or erlotinib (tyrosine kinase inhibitors). The simultaneous presence in glioblastoma cells of mutant EGFR (EGFRviii) and PTEN was associated with responsiveness to tyrosine kinase inhibitors, whereas increased p-akt predicts a decreased effect. Other targets include PDGFR, VEGFR, mTOR, farnesyltransferase, and PI3K.

Other possible therapy modalities include imatinib, gene therapy, peptide and dendritic cell vaccines, synthetic chlorotoxins, and radiolabeled drugs and antibodies.

## Compositions and routes of administration

The compositions delineated herein include the compounds delineated herein (e.g., a compound described herein), as well as additional therapeutic agents if present, in amounts effective for achieving a modulation of disease or disease symptoms, including those described herein.

The term "pharmaceutically acceptable carrier or adjuvant" refers to a carrier or adjuvant that may be administered to a patient, together with a compound of this invention, and which does not destroy the pharmacological activity thereof and is nontoxic when administered in doses sufficient to deliver a therapeutic amount of the compound.

Pharmaceutically acceptable carriers, adjuvants and vehicles that may be used in the pharmaceutical compositions of this invention include, but are not limited to, ion exchangers, alumina, aluminum stearate, lecithin, self-emulsifying drug delivery systems (SEDDS) such as d-$\alpha$-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as $\alpha$-, $\beta$-, and y-cyclodextrin, or chemically modified derivatives such as hydroxyalkylcyclodextrins, including 2- and 3-hydroxypropyl-$\beta$-cyclodextrins, or other solubilized derivatives may also be advantageously used to enhance delivery of compounds of the formulae described herein.

The pharmaceutical compositions of this invention may be administered orally, parenterally, by inhalation spray, topically, rectally, nasally, buccally, vaginally or via an implanted reservoir, preferably by oral administration or administration by injection. The pharmaceutical compositions of this invention may contain any conventional non-toxic pharmaceutically-acceptable carriers, adjuvants or vehicles. In some cases, the pH of the formulation may be adjusted with pharmaceutically acceptable acids, bases or buffers to enhance the stability of the formulated compound or its delivery form. The term parenteral as used herein includes subcutaneous, intracutaneous, intravenous, intramuscular, intraarticular, intraarterial, intrasynovial, intrasternal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, or carboxymethyl cellulose or similar dispersing agents which are commonly used in the formulation of pharmaceutically acceptable dosage forms such as emulsions and or suspensions. Other commonly used surfactants such as Tweens or Spans and/or other similar emulsifying agents or bioavailability enhancers which are commonly used

in the manufacture of pharmaceutically acceptable solid, liquid, or other dosage forms may also be used for the purposes of formulation.

The pharmaceutical compositions of this invention may be orally administered in any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions and/or emulsions are administered orally, the active ingredient may be suspended or dissolved in an oily phase is combined with emulsifying and/or suspending agents. If desired, certain sweetening and/or flavoring and/or coloring agents may be added.

The pharmaceutical compositions of this invention may also be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax and polyethylene glycols.

Topical administration of the pharmaceutical compositions of this invention is useful when the desired treatment involves areas or organs readily accessible by topical application. For application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene polyoxypropylene compound, emulsifying wax and water. Alternatively, the pharmaceutical composition can be formulated with a suitable lotion or cream containing the active compound suspended or dissolved in a carrier with suitable emulsifying agents. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions of this invention may also be topically applied to the lower intestinal tract by rectal suppository formulation or in a suitable enema formulation. Topically-transdermal patches are also included in this invention.

The pharmaceutical compositions of this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

When the compositions of this invention comprise a combination of a compound of the formulae described herein and one or more additional therapeutic or prophylactic agents, both the compound and the additional agent should be present at dosage levels of between about 1 to 100%, and more preferably between about 5 to 95% of the dosage normally administered in a monotherapy regimen. The additional agents may be administered separately, as part of a multiple dose regimen, from the compounds of this invention. Alternatively, those agents may be part of a single dosage form, mixed together with the compounds of this invention in a single composition.

The compounds described herein can, for example, be administered by injection, intravenously, intraarterially, subdermally, intraperitoneally, intramuscularly, or subcutaneously; or orally, buccally, nasally, transmucosally, topically, in an ophthalmic preparation, or by inhalation, with a dosage ranging from about 0.5 to about 100 mg/kg of body weight, alternatively dosages between 1 mg and 1000 mg/dose, every 4 to 120 hours, or according to the requirements of the particular drug. The methods herein contemplate administration of an effective amount of compound or compound composition to achieve the desired or stated effect. Typically, the pharmaceutical compositions of this invention will be administered from about 1 to about 6 times per day or alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (w/w). Alternatively, such preparations contain from about 20% to about 80% active compound.

Lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the disease, condition or symptoms, the patient's disposition to the disease, condition or symptoms, and the judgment of the treating physician.

Upon improvement of a patient's condition, a maintenance dose of a compound, composition or combination of this invention may be administered, if necessary. Subsequently, the dosage or frequency of administration, or both, may be reduced, as a function of the symptoms, to a level at which the improved condition is retained when the symptoms have been alleviated to the desired level. Patients may, however, require intermittent treatment on a long-term basis upon any recurrence of disease symptoms.

**Neoactivity of an enzyme**

As used herein, neoactivity refers to alpha hydroxy neoactivity. Neoactivity and alpha hydroxyl neoactivity are used interchanagly herein. Alpha hydroxy neoactivity is the ability to convert an alpha ketone to an alpha hydroxy. Neoactivity can arise as a result of a mutation, *e.g.,* a point mutation, *e.g.,* a substitution, *e.g.,* in the active site of an enzyme. In an embodiment the neoactivity is substantially absent from wild type or non-mutant enzyme. This is sometimes referred to herein as a first degree neoactivity. An example of a first degree neoactivity is a "gain of function" wherein the mutant enzyme gains a new catalytic activity. In an embodiment the neoactivity is present in wild type or non-mutant enzyme but at a level which is less than 10, 5, 1, 0.1, 0.01 or 0.001 % of what is seen in the mutant enzyme. This is sometimes referred to herein as a second degree neoactivity. An example of a second degree neoactivity is a "gain of function" wherein the mutant enzyme has an increase, for example, a 5 fold increase in the rate of a catalytic activity possessed by the enzyme when lacking the mutation.

In some embodiments, a non-mutant form the enzyme, *e.g.,* a wild type form, converts substance A (*e.g.*, isocitrate) to substance B (*e.g.*, α-ketoglutarate), and the neoactivity converts substance B (*e.g.*, α-ketoglutarate) to substance C, sometimes referred to as the neoactivity product (*e.g.,* 2-hydroxyglutarate, *e.g.,* R-2-hydroxyglutarate).

**Isocitrate Dehydrogenases**

Isocitrate dehydrogenases (IDHs) catalyze the oxidative decarboxylation of isocitrate to 2-oxoglutarate (*i.e.*, α-ketoglutarate). These enzymes belong to two distinct subclasses, one of which utilizes NAD(+) as the electron acceptor and the other NADP(+). Five isocitrate dehydrogenases have been reported: three NAD(+)-dependent isocitrate dehydrogenases, which localize to the mitochondrial matrix, and two NADP(+)-dependent isocitrate dehydrogenases, one of which is mitochondrial and the other predominantly cytosolic. Each NADP(+)-dependent isozyme is a homodimer.

IDH1 (isocitrate dehydrogenase 1 (NADP+), cytosolic) is also known as IDH; IDP; IDCD; IDPC or PICD. The protein encoded by this gene is the NADP(+)-dependent isocitrate dehydrogenase found in the cytoplasm and peroxisomes. It contains the PTS-1 peroxisomal targeting signal sequence. The presence of this enzyme in peroxisomes suggests roles in the regeneration of NADPH for intraperoxisomal reductions, such as the conversion of 2, 4-dienoyl-CoAs to 3-enoyl-CoAs, as well as in peroxisomal reactions that consume 2-oxoglutarate, namely the alpha-hydroxylation of phytanic acid. The cytoplasmic enzyme serves a significant role in cytoplasmic NADPH production.

The human IDH1 gene encodes a protein of 414 amino acids. The nucleotide and amino acid sequences for human IDH1 can be found as GenBank entries NM_005896.2 and NP_005887.2 respectively. The nucleotide and amino acid sequences for IDH1 are also described in, *e.g.,* Nekrutenko et al., Mol. Biol. Evol. 15:1674-1684(1998); Geisbrecht et al., J. Biol. Chem. 274:30527-30533(1999); Wiemann et al., Genome Res. 11:422-435(2001); The MGC Project Team, Genome Res. 14:2121-2127(2004); Lubec *et al.,* Submitted (DEC-2008) to UniProtKB; Kullmann *et al.,* Submitted (JUN-1996) to the EMBL/GenBank/DDBJ databases; and Sjoeblom et al., Science 314:268-274(2006).

IDH2 (isocitrate dehydrogenase 2 (NADP+), mitochondrial) is also known as IDH; IDP; IDHM; IDPM; ICD-M; or mNADP-IDH. The protein encoded by this gene is the NADP(+)-dependent isocitrate dehydrogenase found in the mitochondria. It plays a role in intermediary metabolism and energy production. This protein may tightly associate or interact with the pyruvate dehydrogenase complex. Human IDH2 gene encodes a protein of 452 amino acids. The nucleotide and amino acid sequences for IDH2 can be found as GenBank entries NM_002168.2 and NP_002159.2 respectively. The nucleotide and amino acid sequence for human IDH2 are also described in, *e.g.,* Huh *et al.,* Submitted (NOV-1992) to the EMBL/GenBank/DDBJ databases; and The MGC Project Team, Genome Res. 14:2121-2127(2004).

Non-mutant, *e.g.*, wild type, IDH1 catalyzes the oxidative decarboxylation of ioscitrate to α-ketoglutarate thereby reducing $NAD^+$ ($NADP^+$) to NADP (NADPH), *e.g.,* in the forward reaction:

$$\text{Isocitrate} + NAD^+\ (NADP^+) \rightarrow \alpha\text{-KG} + CO_2 + \text{NADH (NADPH)} + H^+.$$

In some embodiments, the neoactivity of a mutant IDH1 can have the ability to convert α-ketoglutarate to 2-hydroxyglutarate, *e.g.*, R-2-hydroxyglutarate:

$$\alpha\text{-KG} + \text{NADH (NADPH)} + H^+ \rightarrow \text{2-hydroxyglutarate, } e.g., \text{ R-2-hydroxyglutarate} + NAD^+\ (NADP^+).$$

In some embodiments, the neoactivity can be the reduction of pyruvate or malate to the corresponding α-hydroxy compounds.

In some embodiments, the neoactivity of a mutant IDH1 can arise from a mutant IDH1 having a His, Ser, Cys or Lys, or any other mutations described in Yan *et al.,* at residue 132. In some embodiments, the neoactivity of a mutant IDH2 can arise from a mutant IDH2 having a Gly, Met or Lys, or any other mutations described in Yan H *et al.,* at residue 140 or 172. Exemplary mutations include the following: R132H, R132C, R132S, R132G, R132L, R132V, and R140Q.

In some embodiments, the mutant IDH1 and/or IDH2 (*e.g.,* a mutant IDH1 and/or IDH2 having a neoactivity described herein) could lead to an increased level of 2-hydroxyglutarate, *e.g.*, R-2-hydroxyglutarate in a subject. The accumulation of 2-hydroxyglutarate, *e.g.,* R-2-hydroxyglutarate in a subject, *e.g.,* in the brain of a subject, can be harmful. For example, in some embodiments, elevated levels of 2-hydroxyglutarate, *e.g.*, R-2-hydroxyglutarate can lead to and/or be predictive of cancer in a subject such as a cancer of the central nervous system, *e.g.*, brain tumor, *e.g.,* glioma, *e.g.,* glioblastoma multiforme (GBM). Accordingly, in some embodiments, a method described herein includes administering to a subject an inhibitor of the neoactivity

**Detection of 2-hydroxyglutarate**

2-hydroxyglutarate can be detected, *e.g.,* by LC/MS. To detect secreted 2-hydroxyglutarate in culture media, 500 μL aliquots of conditioned media can be collected, mixed 80:20 with methanol, and centrifuged at 3,000 rpm for 20 minutes at 4 degrees Celsius. The resulting supernatant can be collected and stored at -80 degrees Celsius prior to LC-MS/MS to assess 2-hydroxyglutarate levels. To measure whole-cell associated metabolites, media can be aspirated and cells can be harvested, *e.g.,* at a non-confluent density. A variety of different liquid chromatography (LC) separation methods can be used. Each method can be coupled by negative electrospray ionization (ESI, -3.0 kV) to triple-quadrupole mass spectrometers operating in multiple reaction monitoring (MRM) mode, with MS parameters optimized on infused metabolite standard solutions. Metabolites can be separated by reversed phase chromatography using 10 mM tributyl-amine as an ion pairing agent in the aqueous mobile phase, according to a variant of a previously reported method (Luo et al. J Chromatogr A 1147, 153-64, 2007). One method allows resolution of TCA metabolites: t = 0,50% B; t = 5, 95% B; t= 7, 95% B; t= 8, 0% B, where B refers to an organic mobile phase of 100% methanol. Another method is specific for 2-hydroxyglutarate, running a fast linear gradient from 50% -95% B (buffers as defined above) over 5 minutes. A Synergi Hydro-RP, 100mm × 2 mm, 2.1 μm particle size (Phenomonex) can be used as the column, as described above. Metabolites can be quantified by comparison of peak areas with pure metabolite standards at known concentration. Metabolite flux studies from $^{13}$C-glutamine can be performed as described, *e.g.,* in Munger et al. Nat Biotechnol 26, 1179-86, 2008.

In an embodiment 2HG, e.g., R-2HG, is evaluated and the analyte on which the determination is based is 2HG, e.g., R-2HG. In an embodiment the analyte on which the determination is based is a derivative of 2HG, *e.g.,* R-2HG, formed in process of performing the analytic method. By way of example such a derivative can be a derivative formed in MS analysis. Derivatives can include a salt adduct, *e.g.,* a Na adduct, a hydration variant, or a hydration variant which is also a salt adduct, e.g., an Na adduct, e.g., as formed in MS analysis. In an embodiment an alpha hydroxy neoactivity product, e.g., 2HG, *e.g.,* R-2HG, can be assayed indirectly. In an indirect assay the analyte is a metabolic derivative of an alpha hydroxy neoactivity product, e.g., 2HG, *e.g.,* R-2HG, or another compound(s), e.g., a cellular compound, that is correlated to the level of an alpha hydroxy neoactivity product, e.g., 2HG, *e.g.,* R-2HG. Examples include species that build up or are elevated, or reduced, as a result of the presence of 2HG, *e.g.,* R-2HG. *E.g.,* in embodiments, cancer cells with the neoactive mutant have elevated levels of glutarate or glutamate that will be correlated to 2HG, *e.g.,* R-2HG.

Exemplary 2HG derivatives include dehydrated derivatives such as the compounds provided below or a salt adduct thereof:

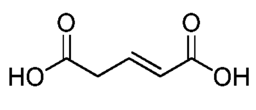 , 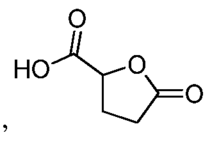 , 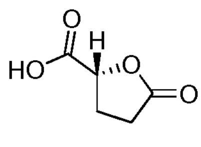 , and 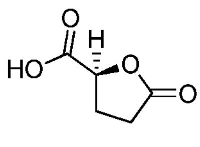 .

**Methods of evaluating samples and/or subjects**

In some embodiments, the methods described herein include evaluation of one or more parameters related to IDH, e.g., IDH1 or IDH2, an alpha hydroxy neoactivity, e.g., 2HG neoactivity, e.g., to evaluate the IDH1 or IDH2 2HG neoactivity genotype or phenotype. The evaluation can be performed, e.g., to select, diagnose or prognose the subject, to select a therapeutic agent, e.g., an inhibitor, or to evaluate response to the treatment or progression of disease. In an embodiment the evaluation, which can be performed before and/or after treatment has begun, is based, at least in part, on analysis of a tumor sample, cancer cell sample, or precancerous cell sample, from the subject. E.g., a sample

from the patient can be analyzed for the presence or level of an alpha hydroxy neoactivity product, e.g., 2HG, *e.g.,* R-2HG, by evaluating a parameter correlated to the presence or level of an alpha hydroxy neoactivity product, e.g., 2HG, *e.g.,* R-2HG. An alpha hydroxy neoactivity product, e.g., 2HG, *e.g.,* R-2HG, in the sample can be determined by a chromatographic method, *e.g.,* by LC-MS analysis. It can also be determined by contact with a specific binding agent, *e.g.,* an antibody, which binds the alpha hydroxy neoactivity product, e.g., 2HG, *e.g.,* R-2HG, and allows detection. In an embodiment the sample is analyzed for the level of neoactivity, *e.g.*, an alpha hydroxy neoactivity, e.g., 2HG neoactivity. In an embodiment, the sample is analysed for the presence of a mutant IDH, *e.g.,* IDH1 or IDH2, protein having an alpha hydroxy neoactivity, e.g., 2HG neoactivity (or a corresponding RNA). E.g., a mutant protein specific reagent, e.g., an antibody that specifically binds an IDH mutant protein, e.g., an antibody that specifically binds an IDH1-R132H mutant protein, can be used to detect neoactive mutant enzymeIn an embodiment a nucleic acid from the sample is sequenced to determine if a selected allele or mutation of IDH1 or IDH2 disclosed herein is present. In an embodiment the analysis is other than directly determining the presence of a mutant IDH, *e.g.,* IDH1 or IDH2, protein (or corresponding RNA) or sequencing of an IDH, *e.g.,* IDH1 or IDH2 gene. In an embodiment the analysis is other than directly determining, e.g., it is other than sequencing genomic DNA or cDNA, the presence of a mutation at residue 132 of IDH1 and/or a mutation at residue 140 or 172 of IDH2. In an embodiment the tumor is other than a tumor of the CNS, e.g., other than a glioma, and the analysis includes determing the sequence of a mutation at position 132 of IDH1, or a mutation at position 172 of IDH2. E.g., the sequence of IDH1 at any of position 71, or 100 or 109 can be determined, e.g., to detect the presence of a mutation having 2HG neoactivity. In an embodiment the tumor is a glioma and the presence of an IDH1 2HG neoactive mutation other than a mutation at 132 of IDH1 is determined. In an embodiment the tumor is a glioma and the presence of an IDH1 2HG neoactive mutation other than a mutation at 172 at IDH2 is determined. *E.g.*, the analysis can be the detection of an alpha hydroxy neoactivity product, e.g., 2HG, *e.g.,* R-2HG, or the measurement of the mutation's an alpha hydroxy neoactivity, e.g., 2HG neoactivity. In an embodiment the sample is removed from the patient and analyzed. In an embodiment the evaluation can include one or more of performing the analysis of the sample, requesting analysis of the sample, requesting results from analysis of the sample, or receiving the results from analysis of the sample. (Generally herein, analysis can include one or both of performing the underlying method or receiving data from another who has performed the underlying method.)

In an embodiment the evaluation, which can be performed before and/or after treatment has begun, is based, at least in part, on analysis of a tissue (e.g., a tissue other than a tumor sample), or bodily fluid, or bodily product. Exemplary tissues include lymph node, skin, hair follicles and nails. Exemplary bodily fluids include blood, serum, plasma, urine, lymph, tears, sweat, saliva, semen, and cerebrospinal fluid. Exemplary bodily products include exhaled breath. *E.g.*, the tissue, fluid or product can be analyzed for the presence or level of an alpha hydroxy neoactivity product, e.g., 2HG, *e.g.,* R-2HG, by evaluating a parameter correlated to the presence or level of an alpha hydroxy neoactivity product, e.g., 2HG, *e.g.,* R-2HG. An alpha hydroxy neoactivity product, e.g., 2HG, *e.g.,* R-2HG, in the sample can be determined by a chromatographic method, *e.g.,* by LC-MS analysis. It can also be determend by contact with a specific binding agent, *e.g.,* an antibody, which binds the alpha hydroxy neoactivity product, e.g., 2HG, *e.g.,* R-2HG, and allows detection. In embodiments where sufficient levels are present, the tissue, fluid or product can be analyzed for the level of neoactivity, *e.g.*, an alpha hydroxy neoactivity, e.g., the 2HG neoactivity. In an embodment the sample is analysed for the presence of a mutant IDH, *e.g.,* IDH1 or IDH2, protein having an alpha hydroxy neoactivity, e.g., 2HG neoactivity (or a corresponding RNA). E.g., a mutant protein specific reagent, e.g., an antibody that specifically binds an IDH mutant protein, e.g., an antibody that specifically binds an IDH1-R132H mutant protein, can be used to detect neoactive mutant enzyme. In an embodiment a nucleic acid from the sample is sequenced to determine if a selected allele or mutation of IDH1 or IDH2 disclosed herein is present. In an embodiment the analysis is other than directly determining the presence of a mutant IDH, *e.g.,* IDH1 or IDH2, protein (or corresponding RNA) or sequencing of an IDH, *e.g.,* IDH1 or IDH2 gene. *E.g.,* the analysis can be the detection of an alpha hydroxy neoactivity product, e.g., 2HG, *e.g.,* R-2HG, or the measurement of 2HG neoactivity. In an embodiment the tissue, fluid or product is removed from the patient and analyzed. In an embodiment the evaluation can include one or more of performing the analysis of the tissue, fluid or product, requesting analysis of the tissue, fluid or product, requesting results from analysis of the tissue, fluid or product, or receiving the results from analysis of the tissue, fluid or product.

In an embodiment the evaluation, which can be performed before and/or after treatment has begun, is based, at least in part, on alpha hydroxy neoactivity product, e.g., 2HG, *e.g.,* R-2HG, imaging of the subject. In embodiments magnetic resonance methods are is used to evaluate the presence, distribution, or level of an alpha hydroxy neoactivity product, e.g., 2HG, *e.g.,* R-2HG, in the subject. In an embodiment the subject is subjected to imaging and/or spectroscopic analysis, *e.g.*, magnetic resonance-based analysis, *e.g.,* MRI and/or MRS *e.g.*,analysis, and optionally an image corresponding to the presence, distribution, or level of an alpha hydroxy neoactivity product, e.g., 2HG, *e.g.,* R-2HG, or of the tumor, is formed. Optionally the image or a value related to the image is stored in a tangible medium and/or transmitted to a second site. In an embodiment the evaluation can include one or more of performing imaging analysis, requesting imaging analysis, requesting results from imaging analysis, or receiving the results from imaging analysis.

**Patient selection/monitoring**

Described herein are methods of treating a cell proliferation-related disorder, *e.g.,* cancer, in a subject and methods of identifying a subject for a treatment described herein. Also described herein are methods of predicting a subject who is at risk of developing cancer *(e.g.,* a cancer associate with a mutation in an IDH enzyme *(e.g.,* IDH1 and/or IDH2)). The cancer is generally characterized by the presence of a neoactivity, such as a gain of function in one or more mutant IDH enzymes (*e.g.*, IDH1 or IDH2). The subject can be selected on the basis of the subject having a mutant gene having a neoactivity, *e.g.*, a neoactivity described herein. As used herein, "select" means selecting in whole or part on said basis.
In some embodiments, a subject is selected for treatment with a compound described herein based on a determination that the subject has a mutant IDH enzyme described herein. In some embodiments, the mutant enzyme has a neoactivity and the patient is selected on that basis. The neoactivity of the enzyme can be identified, for example, by evaluating the subject or sample (*e.g.*, tissue or bodily fluid) therefrom, for the presence or amount of a substrate, cofactor and/or product of the enzyme. The presence and/or amount of substrate, cofactor and/or product can correspond to the wild-type/non-mutant activity or can correspond to the neoactivity of the enzyme. Exemplary bodily fluid that can be used to identifty (*e.g.*, evaluate) the neoactivity of the enzyme include amniotic fluid surrounding a fetus, aqueous humour, blood *(e.g.,* blood plasma), serum, Cerebrospinal fluid, cerumen, chyme, Cowper's fluid, female ejaculate, interstitial fluid, lymph, breast milk, mucus (*e.g.*, nasal drainage or phlegm), pleural fluid, pus, saliva, sebum, semen, serum, sweat, tears, urine, vaginal secretion, or vomit.
In some embodiments, a subject can be evaluated for neoactivity of an enzyme using magnetic resonance. For example, where the mutant enzyme is IDH1 and the neoactivity is conversion of $\alpha$-ketoglutarate to 2-hydroxyglutarate, the subject can be evaluated for the presence of and/or an elevated amount of 2-hydroxyglutarate, *e.g.*, R-2-hydroxyglutarate relative to the amount of 2-hydroxyglutarate, *e.g.*, R-2-hydroxyglutarate present in a subject who does not have a mutation in IDH1 having the above neoactivity. In some embodiments, neoactivity of IDH1 can be determined by the presence or elevated amount of a peak corresponding to 2-hydroxyglutarate, *e.g.*, R-2-hydroxyglutarate as determined by magnetic resonance. For example, a subject can be evaluated for the presence and/or strength of a signal at about 2.5 ppm to determine the presence and/or amount of 2-hydroxyglutarate, *e.g.*, R-2-hydroxyglutarate in the subject. This can be correlated to and/or predictive of a neoactivity described herein for the mutant enzyme IDH. Similarly, the presence, strength and/or absence of a signal at about 2.5 ppm could be predictive of a response to treatment and thereby used as a noninvasive biomarker for clinical response.
Neoactivity of a mutant IDH enzyme can also be evaluated using other techniques known to one skilled in the art. For example, the presence or amount of a labeled substrate, cofactor, and/or reaction product can be measured such as a $^{13}C$ or $^{14}C$ labeled substrate, cofactor, and/or reaction product. The neoactivity can be evaluated by evaluating the forward reaction of the wild-type/non mutant enzyme (such as the oxidative decarboxylation of ioscitrate to $\alpha$-ketoglutarate in a mutant IDH1 enzyme) and/or the reaction corresponding to the neoactivity (*e.g.*, the conversion of $\alpha$-ketoglutarate to 2-hydroxyglutarate, *e.g.*, R-2-hydroxyglutarate in a mutant IDH1 enzyme).

**Kits**

A compound described herein can be provided in a kit.
In an embodiment the kit includes (a) a compound described herein, *e.g.,* a composition that includes a compound described herein (wherein, *e.g.,* the compound can be an inhibitor described herein), and, optionally (b) informational material. The informational material can be descriptive, instructional, marketing or other material that relates to the methods described herein and/or the use of a compound described herein for the methods described herein.
In an embodiment the kit provides materials for evaluating a subject. The evaluation can be, *e.g.*,for: identifying a subject having unwanted, i.e., increased, levels (e.g., higher than present in normal or wildtype cells) of any of 2HG, 2HG neoactivity, or mutant IDH1 or IDH2 protien having 2HG neoactivity (or corresponding RNA), or having a somatic mutation in IDH1 or IDH2 characterized by 2HG neoactivity; diagnosing, prognosing, or staging, a subject, *e.g.,* on the basis of having unwanted, i.e., increased, levels of 2HG, 2HG neoactivity, or mutant IDH1 or IDH2 protien having 2HG neoactivity (or corresponding RNA), or having a somatic mutation in IDH1 or IDH2 characterized by 2HG neoactivity; selecting a treatment for, or evaluating the efficacy of, a treatment, *e.g.*, on the basis of the subject having unwanted, i.e., increased, levels of 2HG, 2HG neoactivity, or mutant IDH1 or IDH2 protien having 2HG neoactivity (or corresponding RNA), or having a somatic mutation in IDH1 or IDH2 characterized by 2HG neoactivity. The kit can include one or more reagent useful in the evaluation, *e.g.*, reagents mentioned elsewhere herein. A detection reagent, *e.g.,* an antibody or other specific bindng reagent can be included. Standards or reference samples, *e.g.,* a positive or negative control standard can be included. *E.g.*, if the evaluation is based on the presence of 2HG the kit can include a reagent, e.g, a positive or negative control standards for an assay, *e.g.,* a LC-MS assay.
If the evaluation is based on the presence of 2HG neoactivity, the kit can include a reagent, *e.g.,* one or more

of those mentioned elsewhere herein, for assaying 2HG neoactivity. If the evaluation is based on sequencing, the kit can include primers or other matierials useful for sequencing the relevant nucleic acids for identifying an IHD, e.g., IDH1 or IDH2, neoactive mutant. E.g., the kit can contain a reagent that provides for interrogation of the indentity, i.e., sequencing of, residue 132, 71, 100, 109, 70, 130, 133, 135, or 178 of IDH1 to determine if a neoactive mutant is present. The kit can include nucleic acids, e.g., an oligomer, e.g., primers, which allow sequencing of of the nucleotides that encode residue 132, 71, 100, 109, 70, 130, 133, 135, or 178 of IDH. In an embodiment the kit includes a nucleic acid whose hybridization, or ability to be amplified, is dependent on the indentity of residue 132, 71, 100, 109, 70, 130, 133, 135, or 178 of IDH. In other embodiments the kit includes a reagent, e.g., an antibody or other specific binding molecule, which can identify the presence of a neoactive mutant, *e.g*., a protein encoded by a neoactive mutant at 132, 71, 100, 109, 70, 130, 133, 135, or 178 of IDH. As described below, a kit can also include buffers, solvents, and information related to the evaluation.

In one embodiment, the informational material can include information about production of the compound, molecular weight of the compound, concentration, date of expiration, batch or production site information, and so forth. In one embodiment, the informational material relates to methods for administering the compound.

In one embodiment, the informational material can include instructions to administer a compound described herein in a suitable manner to perform the methods described herein, *e.g.,* in a suitable dose, dosage form, or mode of administration *(e.g.,* a dose, dosage form, or mode of administration described herein). In another embodiment, the informational material can include instructions to administer a compound described herein to a suitable subject, *e.g.,* a human, *e.g.,* a human having or at risk for a disorder described herein.

The informational material of the kits is not limited in its form. In many cases, the informational material, *e.g.,* instructions, is provided in printed matter, *e.g.,* a printed text, drawing, and/or photograph, *e.g.,* a label or printed sheet. However, the informational material can also be provided in other formats, such as Braille, computer readable material, video recording, or audio recording. In another embodiment, the informational material of the kit is contact information, *e.g*., a physical address, email address, website, or telephone number, where a user of the kit can obtain substantive information about a compound described herein and/or its use in the methods described herein. Of course, the informational material can also be provided in any combination of formats.

In addition to a compound described herein, the composition of the kit can include other ingredients, such as a solvent or buffer, a stabilizer, a preservative, a flavoring agent (*e.g.,* a bitter antagonist or a sweetener), a fragrance or other cosmetic ingredient, and/or a second agent for treating a condition or disorder described herein. Alternatively, the other ingredients can be included in the kit, but in different compositions or containers than a compound described herein. In such embodiments, the kit can include instructions for admixing a compound described herein and the other ingredients, or for using a compound described herein together with the other ingredients.

A compound described herein can be provided in any form, *e.g.*, liquid, dried or lyophilized form. It is preferred that a compound described herein be substantially pure and/or sterile. When a compound described herein is provided in a liquid solution, the liquid solution preferably is an aqueous solution, with a sterile aqueous solution being preferred. When a compound described herein is provided as a dried form, reconstitution generally is by the addition of a suitable solvent. The solvent, *e.g.,* sterile water or buffer, can optionally be provided in the kit.

The kit can include one or more containers for the composition containing a compound described herein. In some embodiments, the kit contains separate containers, dividers or compartments for the composition and informational material. For example, the composition can be contained in a bottle, vial, or syringe, and the informational material can be contained in a plastic sleeve or packet. In other embodiments, the separate elements of the kit are contained within a single, undivided container. For example, the composition is contained in a bottle, vial or syringe that has attached thereto the informational material in the form of a label. In some embodiments, the kit includes a plurality (*e.g.,* a pack) of individual containers, each containing one or more unit dosage forms (*e.g.,* a dosage form described herein) of a compound described herein. For example, the kit includes a plurality of syringes, ampules, foil packets, or blister packs, each containing a single unit dose of a compound described herein. The containers of the kits can be air tight, waterproof (*e.g*., impermeable to changes in moisture or evaporation), and/or light-tight.

The kit optionally includes a device suitable for administration of the composition, *e.g.,* a syringe, inhalant, pipette, forceps, measured spoon, dropper (*e.g.,* eye dropper), swab (*e.g.,* a cotton swab or wooden swab), or any such delivery device. In an embodiment, the device is a medical implant device, *e.g.,* packaged for surgical insertion.

## EXAMPLES

### Example 1: High Throughput Screening (HTS) for IDH1 R132H Inhibitors

Assays were conducted in a volume of 76 μl assay buffer (150 mM NaCl, 10 mM $MgCl_2$, 20 mM Tris pH 7.5, 0.03% bovine serum albumin) as follows in a standard 384-well plate: To 25 ul of substrate mix (8 uM NADPH, 2 mM aKG), 1 μl of test compound was added in DMSO. The plate was centrifuged briefly, and then 25 μl of enzyme mix was

added (0.2 μg/ml ICDH1 R132H) followed by a brief centrifugation and shake at 100 RPM. The reaction was incubated for 50 minutes at room temperature, then 25 μl of detection mix (30 μM resazurin, 36 μg/ml) was added and the mixture further incubated for 5 minutes at room temperature. The conversion of resazurin to resorufin was detected by fluorescent spectroscopy at Ex544 Em590 c/o 590.

Exemplary compounds tested in this assay include compound 1 from Table 1 which provided an $IC_{50}$ below 3 μM.

**Example 2: Compound Synthesis**

**Scheme 2:**

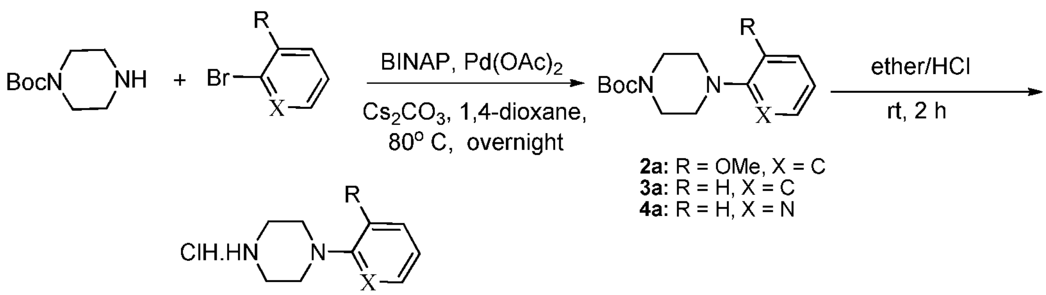


**2a:** R = OMe, X = C
**3a:** R = H, X = C
**4a:** R = H, X = N

**2b:** R = OMe, X = C
**3b:** R = H, X = C
**4b:** R = H, X = N

**General Procedure for Compound 2:** In a two neck round bottom flask, the appropriate bromide (26.7 mmoles) was solubilized in 1, 4-dioxane (100 ml) and purged with nitrogen for 30 minutes while stirring. $CS_2CO_3$ (21.7 gm, 66.8 mmoles), BINAP (1.49 gm, 2.4 mmoles) and Pd $(OAc)_2$ (0.96 gm, 0.42 mmoles) were added to the reaction mixture and purged with nitrogen for another 15 minutes. After 15 minutes, *tert*-butyl piperazine-1-carboxylate (4.97 gm, 26.7 moles) was added and the mixture was stirred with heating at 90 °C for 12 hrs. The progress of the reaction was monitored by TLC. After 12 hrs, the solvent was distilled off under reduced pressure, the resulting residue was diluted with water and extracted with ethyl acetate. The organic layer was separated, dried over $Na_2SO_4$ and concentrated under reduced pressure. The crude product was purified by column chromatography (silica gel 60-120, ethyl acetate-hexane) to obtain the desired.

**2a:** $^1$H NMR (500 MHz, $CDCl_3$): δ 1.42 (s, 9H), 3.0 (m, 4H), 3.60 (m, 4H), 3.84 (s, 3H), 6.80-7.00 (m, 4H); MS 293.1 (M+1 peak).

**Procedure for Compound 2b:** In a two neck RB flask, *tert*-butyl- 4-(2-methoxyphenyl) piperazine-1-carboxylate (**2a**, 0.6 gm, 2.05 mmoles) was treated with ether-HCl (10 mL). The resulting mixture was stirred for 2 hrs. After completion of the reaction as indicated by TLC, ether was removed under reduced pressure and a solid was obtained. The solid material was washed with ethyl acetate and dried to obtain the **2b** as a white solid (0.43 gm, 90.08% yield).

**Procedure for 3b:** The synthesis of compound **3b** was done by following the same procedure above using bromobenzene instead of 2-bromoanisole followed by Boc-deprotection with ether/HCl.

$^1$H NMR (500 MHz, DMSO-$d_6$): δ 3.10-3.20 (m, 4H), 3.40-3.42 (m, 4H), 6.80 (t, 1H), 7.00 (d, 2H), 7.22 (t, 2H), 9.0 (bs, 1H), 9.40 (bs, 2H).

**Procedure for 4b:** The synthesis of **4b** was done by following the procedure as described above using 2-bromopyrdine instead of 2-bromoanisole followed by Boc-deprotection with ether/HCl.

$^1$H NMR: δ 1.40 (s, 9H), 3.40-3.6- (m, 8H), 6.60 (t, 1H), 6.82 (d, 1H), 7.52 (t, 1H), 8.10 (d, 1H).

## Scheme 3

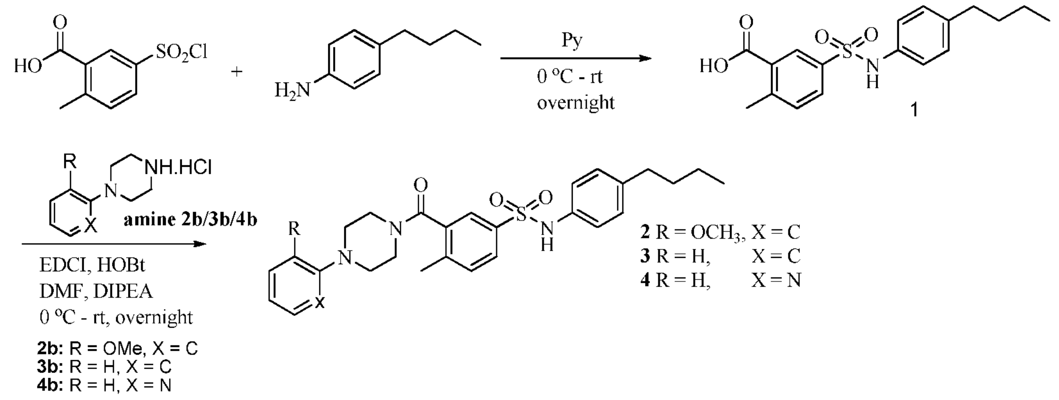


**Procedure for compound 1:**

To a solution of 4-butylaniline (0.2 gm, 1.34 mmoles) in pyridine (5 mL) was added sulfonyl chloride (0.34 gm, 1.47 mmoles) at 0 °C. The reaction was allowed to stir at room temperature overnight. After completion of the reaction, the resulting mixture was diluted with DCM and washed with 1N HCl (2 x 10 mL), brine, dried over $Na_2SO_4$ and concentrated under reduced pressure. The product was purified by column chromatography (silica gel, 60-120 mesh, EtOAc-hexane, 3:7) to afford acid **1** (0.40 gm) in 86.02% yield.
$^1$H NMR (400 MHz, $CDCl_3$): δ 0.85 (m, 5H), 1.58 (m, 2H), 2.58 (t, 2H), 2.71 (s, 3H), 7.01 (dd, 4H), 7.34 (d, 1H), 7.78 (d, 1H), 8.41 (s, 1H); MS 346.3 (M-1 peak).

**Procedure for compound 2:**

To a stirred solution of **2b** (0.065 gm, 0.28 mmoles) in DMF, EDCI (0.06 gm, 0.316 mmoles) and DIPEA (0.2 mL, 0.86 mmoles) were added at 0 °C and stirred for 5 minutes. HOBt was subsequently added (0.05 gm, 0.316 mmoles) followed by **1** (0.10 gm, 0.28 mmoles) at the same temperature. The resulting mixture was allowed to stir at room temperature overnight. After completion of the reaction, the mixture was diluted with water, extracted with ethyl acetate and the organic layer was washed again with water, brine, dried over $Na_2SO_4$ and concentrated under reduced pressure to obtain crude product. The crude mass obtained was purified via column chromatography (silica gel, 60-120 mesh, MeOH-DCM, 0.1:0.90) to afford **2** (0.05 gm, 30% yield).
$^1$H NMR (400 MHz, DMSO-$d_6$): δ 0.80 (t, 3H), 1.2 (m, 4H), 1.41 (p, 2H), 2.21 (s, 3H), 2.40 (t, 2H), 2.72 (br s, 2H), 3.01 (br s, 4H), 3.8 (s, 4H), 7.026.98-7 (m, 7H), 7.41 (d, 2H), 7.68 (d, 1H), 10.04 (s, 1H); MS 522.2 (M+1 peak).

**Procedure for compound 3:**

The synthesis of compound **3** was done by following the above procedure to produce **2** using **3b** instead of **2b** (31% yield from 0.1 g of acid **1**).
$^1$H NMR (400 MHz, DMSO-$d_6$): δ 0.81 (t, 3H), 1.21 (m, 2H), 1.41 (m, 2H), 2.21 (s, 3H), 2.45 (t, 3H), 2.91 (br s, 2H), 3.02 (br s, 2H), 3.22 (br s, 2H), 3.78 (br s, 2H), 6.81 (t, 1H), 6.98 (t, 4H), 7.02 (d, 2H), 7.22 (t, 2H), 7.44 (d, 2H), 7.64 (d, 1H), 10.02 (s, 1H); MS 492.1 (M+1 peak).
T

**Procedure for compound 4:**

The synthesis of compound **4** was done by following the above procedure to produce **2** using **4b** instead of **2b** (28% yield from 0.1 g of acid **1)**
$^1$H NMR (400 MHz, DMSO-$d_6$): δ 0.82 (t, 3H), 1.22 (m, 2H), 1.42 (p, 2H), 2.22 (s, 3H), 3.0 (br s, 2H), 3.59 (br s, 2H), 3.79 (br s, 2H), 6.71 (t, 1H), 6.82 (d, 1H), 7.01 (q, 4H), 7.45 (d, 2H), 7.59 (t, 1H), 7.68 (d, 1H), 8.18 (s, 1H), 10.01 (s, 1H); MS 493.2 (M+1 peak).

**Scheme 4**

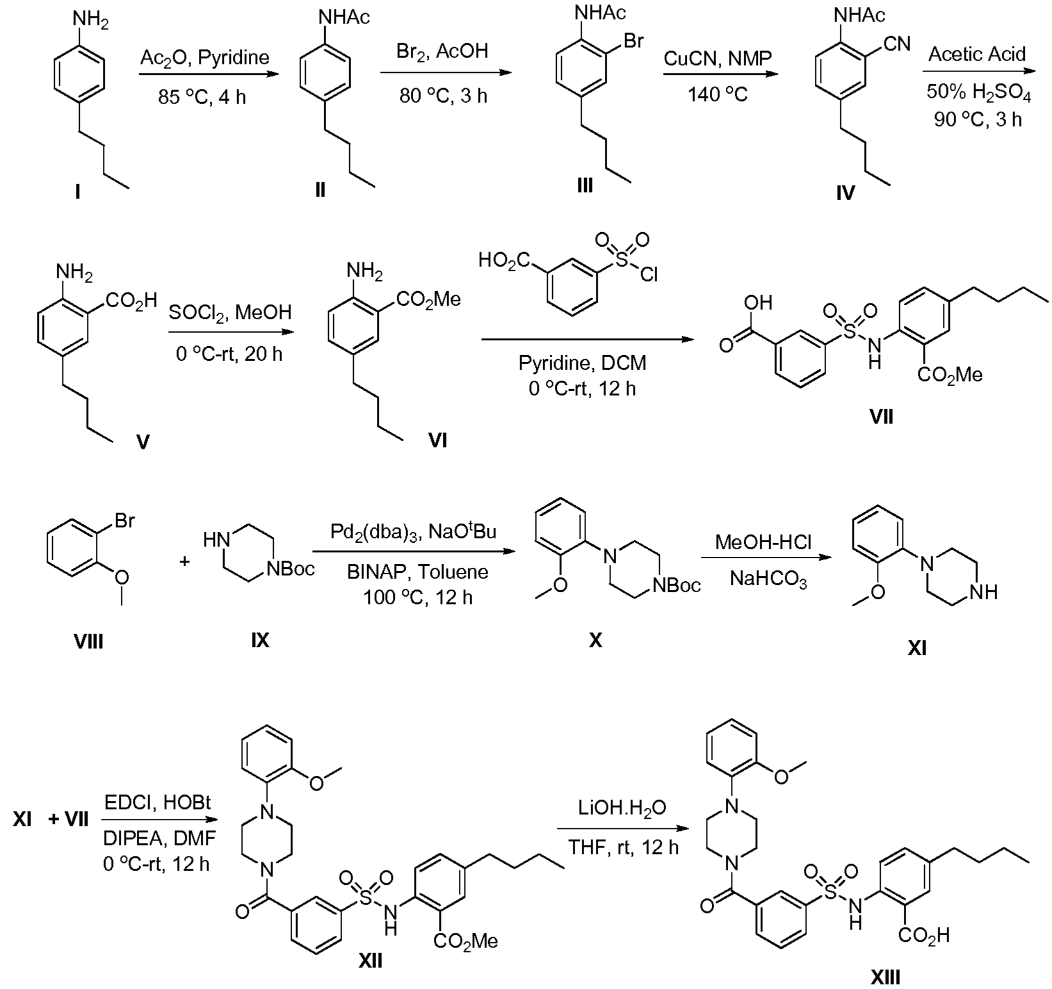


**Synthesis of N-(4-butylphenyl)acetamide (II):**

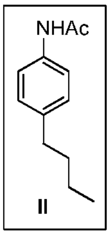

II

To a stirred solution of 4-butylaniline **I** (0.4 g, 2.68 mmol) in pyridine (10 ml) was added acetic anhydride (0.31 ml, 3.22 mmol) at 0°C under nitrogen atmosphere and the resulting mixture was heated at 90°C for 4 h. After completion of reaction, the reaction mixture was cooled, quenched with ice and extracted with ethyl acetate. The organic layer was dried over $Na_2SO_4$ and evaporated under reduced pressure to afford product **II** in 88% yield which was used as such for the next step.

**Synthesis of N-(2-bromo-4-butylphenyl)acetamide (III):**

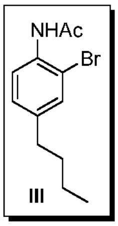

III

To a stirred solution of compound **II** (0.45 g, 2.35 mmol) in acetic acid (10 ml) was added bromine (0.145 ml, 2.82 mmol) at 0°C under nitrogen atmosphere and the resulting mixture was heated at 80°C for 3 h. After completion of reaction, the reaction mixture was cooled, quenched with water and extracted with ethyl acetate. The organic layer was dried over $Na_2SO_4$ and evaporated under reduced pressure. The crude product was then purified by column chromatography (60-120 silica gel) using 20 % EtOAc-Hexane to afford compound **III** in 86% yield.

**Synthesis of N-(4-butyl-2-cyanophenyl)acetamide (IV):**

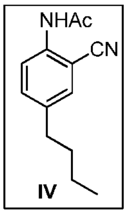

IV

To a stirred solution of compound **III** (1.5 g, 5.55 mmol) in NMP (15 ml) was added CuCN (0.650 g, 7.21 mmol) at room temperature under nitrogen atmosphere and the resulting mixture was heated at 140°C for 3 h. After completion of reaction, the reaction mixture was cooled, quenched with water and extracted with ethyl acetate. The organic layer was dried over $Na_2SO_4$ and evaporated under reduced pressure. The crude product was then purified by column chromatography (60-120 silica gel) using 30 % EtOAc-Hexane to afford compound **IV** in 66% yield.

**Synthesis of 2-amino-5-butylbenzoic acid (V):**

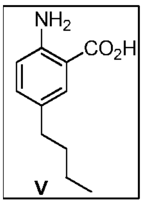

V

To a stirred solution of compound **IV** (0.3 g, 1.3 mmol) in acetic acid (1 ml) was added 50% $H_2SO_4$ (2 ml) at room temperature under nitrogen atmosphere and the resulting mixture was heated at 90°C for 3 h. After completion of reaction, the reaction mixture was cooled and concentrated. The pH of the solution was adjusted to 4 and extracted with ethyl acetate. The organic layer was dried over $Na_2SO_4$ and evaporated under reduced pressure to give the product **V** which was used as such for the next step.

**Synthesis of methyl 2-amino-5-butylbenzoate (VI):**

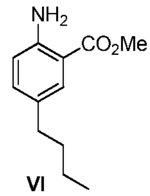

VI

To a stirred solution of compound **V** (0.26 g, 1.3 mmol) in MeOH (15 ml) was added $SOCl_2$ (0.3 ml, 4.04 mmol) at 0°C under nitrogen atmosphere and the resulting mixture was heated at 90°C for 3 h. After completion of reaction, the reaction mixture was cooled and evaporated the MeOH .The reaction mixture was neutralized with saturated solution of $NaHCO_3$ and extracted with ethyl acetate. The organic layer was dried over $Na_2SO_4$ and evaporated under reduced pressure to give the product **VI** which was used as such for the next step.

**Synthesis of 3-(N-(4-butyl-2-(methoxycarbonyl)phenyl)sulfamoyl)benzoic acid (VII):**

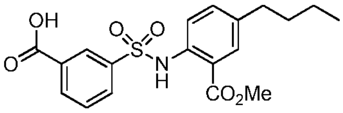

VII

To a stirred solution of amine **VI** (0.160 g, 0.772 mmol) in a mixture (1:1) of DCM and pyridine, 3-(chlorosulfonyl)benzoic acid (0.187 g, 0.85 mmol) was added at room temperature under $N_2$ atmosphere. The resulting mixture was allowed to stir for 16 h. After completion of reaction, the crude mixture was diluted with DCM, washed with water followed by 1N HCl. The organic layer was then dried over $Na_2SO_4$ and concentrated under reduced pressure to afford product **VII** in 76% yields.

**Synthesis of *tert*-butyl 4-(2-methoxyphenyl)piperazine-1-carboxylate (X):**

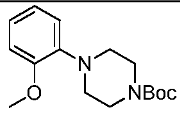

X

To a stirred solution of 2-Bromoanisole (**VIII**, 0.403 g, 2.15 mmol) in Toluene (20 ml) at room temperature nitrogen gas was purged for 30 minutes. BINAP (0.134 g, 0.215 mmol), $Pd_2(dba)_3$ (0.039 g, 0.043 mmol) and sodium *tert*-butoxide (0.412 g, 4.3 mmol) were added to the reaction mixture and the nitrogen purging was continued for another 20 minutes. Finally N-Boc piperazine (**IX**, 0.4 g, 2.15 mmol) was added to the reaction and stirred at 100°C overnight under nitrogen atmosphere. After completion of the reaction (monitored by TLC), the reaction mixture was concentrated under vacuum. The residue was dissolved in water, extracted with ethyl acetate (3 x 50 ml). The combined organic extracts were washed with brine (20 ml), dried over anhydrous Sodium sulfate, filtered and concentrated under reduced pressure. The crude product was then purified by column chromatography (60-120 silica gel) using 10 % ethyl acetate-hexane to afford compound **X** in 60% yield.

**Synthesis of 1-(2-methoxyphenyl)piperazine (XI):**

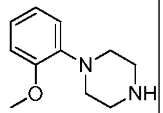

XI

To a stirred solution of MeOH·HCl (10 ml, 20%), Boc protected amine **X** (4.03 mmol) was added and the resulting mixture was stirred for 2 h. After completion of reaction, solvent was removed under reduced pressure, washed with water followed by addition of $NaHCO_3$ and extracted with DCM. The organic layer was dried over $Na_2SO_4$ and evaporated under reduced pressure to afford product XI in 94% yield.

**Synthesis of methyl 5-butyl-2-(3-(4-(2-methoxyphenyl)piperazine-1-carbonyl)phenylsulfonamido)benzoate (XII):**

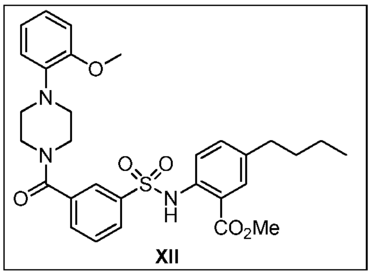

XII

To a stirred solution of acid **VII** (0.315 mmole) in DMF (5 ml), EDCI (0.066 g, 0.346 moles), HOBt (0.047 g, 0.346 mmole) and DIPEA (0.13 ml, 0.78 mmole) were added at 0°C and stirred for 15 minutes. A solution of amine **XI** (0.315 mmoles) was added at 0°C and then the resulting mixture was allowed to stir at room temperature for overnight. After completion of the reaction, water (20 mL) was added and extracted with ethyl acetate (2x30 ml). The combined organic layer was dried over anhydrous $Na_2SO_4$ and concentrated under reduced pressure. The crude product was purified by column chromatography (silica gel, 60-120 mess, MeOH-DCM, 2:98) to give **XII** in 55% yield.

**Synthesis of 5-butyl-2-(3-(4-(2-methoxyphenyl)piperazine-1-carbonyl)phenylsulfonamido)benzoic acid (XIII):**

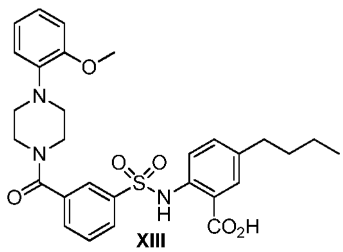

XIII

To a stirred solution of compound **XII** (0.040 g, 0.0707 mmole) in THF-MeOH (3 ml), was added $LiOH.H_2O$ (0.010 g, 0.212 mmol) at room temperature and stirred for 12 h. After completion of the reaction solvent was evaporated, acidified with citric acid and extracted with ethyl acetate (2x30 ml). The combined organic layer was dried over anhydrous $Na_2SO_4$ and concentrated under reduced pressure. The crude product was purified by column chromatography (silica gel, 60-120 mess, MeOH-DCM, 5:95) to give **XIII** in 45% yield.
**1H NMR (400 MHz, DMSO-$d_6$)** $\delta$**:** 0.9 (t, 3H), 1.1-1.5 (m, 2H), 1.4-1.6 (m, 2H), 2.4-2.6 (m, 2H), 2.69-3.3 (m, 6H), 3.6-3.8 (m, 2H), 3.81-4.0 (s, 3H), 6.8-7.2 (m, 4H), 7.3-7.5 (m, 3H), 7.6-7.8 (m, 2H), 7.8-8.0 (m, 2H), 10.2 (s, 1H), 12.1 (s, 1H); **HPLC Purity:** 96.95 %; **Mass (M+1):** 552.35.

**Scheme 5**

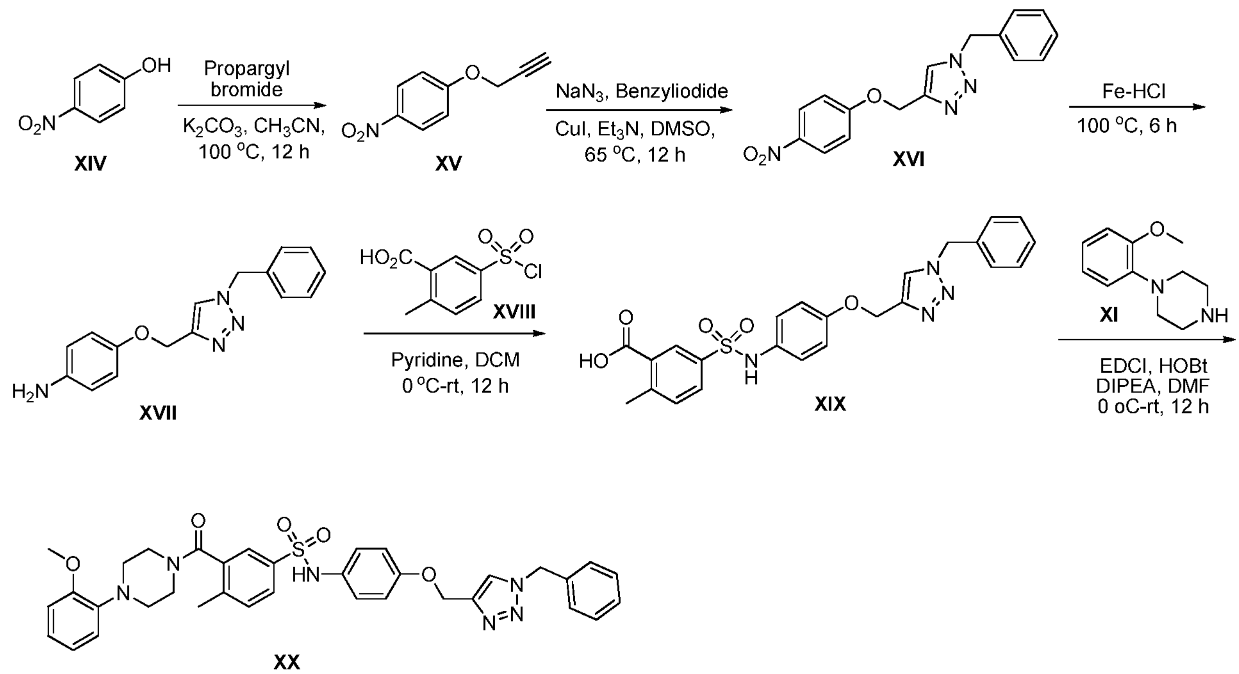


XX

**Synthesis of 1-nitro-4-(prop-2-yn-1-yloxy)benzene (XV):**

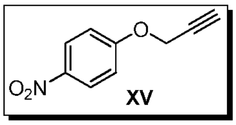

XV

To a stirred solution of compound **XV** (0.281 g, 2.01 mmole) in acetonitrile (10 ml), was added $K_2CO_3$ (0.416 g, 3.02 mmol) followed by propargyl bromide (0.236 g, 2.01 mmole) at room temperature and heated at 100°C for 12 h. After completion of the reaction solvent was evaporated, diluted with water and extracted with ethyl acetate (2x30 ml). The combined organic layer was dried over anhydrous $Na_2SO_4$ and concentrated under reduced pressure. The crude product was purified by column chromatography (silica gel, 60-120 mess, EtOAc-Hexane, 1:9) to give **XV** in 65% yield.

**Synthesis of 1-benzyl-4-((4-nitrophenoxy)methyl)-1H-1,2,3-triazole (XVI):**

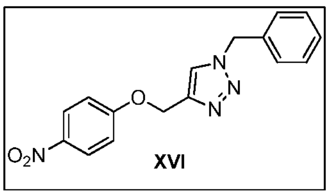

XVI

To a suspension of compound **XV** (0.531 g, 3.01 mmole) in DMSO (10 ml), was added $NaN_3$ (3.01 mmol), bezyliodide (6.02 mmol), $Et_3N$ (0.5 mmol), CuI (0.5 mmol) and proline (0.5 mmol) under nitrogen. The reaction was heated at 65°C for 12 h. After completion of the reaction solvent evaporated, diluted with water and extracted with ethyl acetate (2x30 ml). The combined organic layer was dried over anhydrous $Na_2SO_4$ and concentrated under reduced pressure. The crude product was purified by column chromatography (silica gel, 60-120 mess, EtOAc-Hexane, 3:7) to give **XVI** in 55% yield.

**Synthesis of 4-((1-benzyl-1H-1,2,3-triazol-4-yl)methoxy)aniline (XVII):**

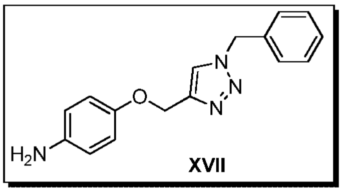

XVII

To a stirred solution of compound **XVI** (1.53 g, 4.96 mmole) in MeOH (10 ml), was added at Fe powder (0.831 g, 14.98 mmol) followed by 1 N HCl (10 ml) at room temperature and heated at reflux for 6 h. After completion of the reaction solvent was evaporated, diluted with ethyl acetate and filtered through celite. The filtrate was washed with water (2x30 ml) and the combined organic layer was dried over anhydrous $Na_2SO_4$ and concentrated under reduced pressure. The crude product was purified by column chromatography (silica gel, 60-120 mess, EtOAc-Hexane, 4:6) to give **XVII** in 65% yield.

**Synthesis of 5-(N-(4-((1-benzyl-1H-1,2,3-triazol-4-yl)methoxy)phenyl)sulfamoyl)-2-methylbenzoic acid (XIX):**

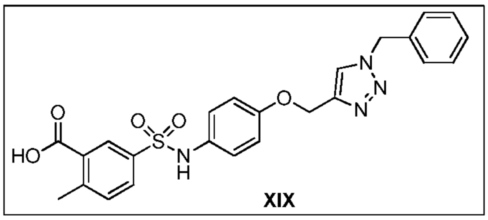

XIX

To a stirred solution of amine **XVII** (0.108 g, 0.386 mmol) in a mixture (1:1) of DCM and pyridine, 5-(chlorosulfonyl)-2-methylbenzoic acid **XVIII** (0.1 g, 0.425 mmol) was added at room temperature under $N_2$ atmosphere. The resulting mixture was allowed to stir for 16 h. After completion of reaction, the crude mixture was diluted with DCM, washed with water followed by 1N HCl. The organic layer was then dried over $Na_2SO_4$ and concentrated under reduced pressure to afford product **XIX** in 56% yields.

**Synthesis of 5-(N-(4-((1-benzyl-1H-1,2,3-triazol-4-yl)methoxy)phenyl)sulfamoyl)-2-methylbenzoic acid (XX):**

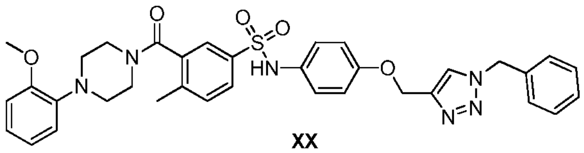

XX

To a stirred solution of acid **XIX** (0.315 mmole) in DMF (5 ml), EDCI (0.066 g, 0.346 mmoles), HOBt (0.047 g, 0.346 mmole) and DIPEA (0.13 ml, 0.78 mmole) were added at 0°C and stirred for 15 minutes. A solution of amine **XI** (0.315 mmoles) was added at 0°C and then the resulting mixture was allowed to stir at room temperature for overnight. After completion of the reaction, water (20 mL) was added and extracted with ethyl acetate (2x30 ml). The combined organic layer was dried over anhydrous $Na_2SO_4$ and concentrated under reduced pressure. The crude product was purified preparative HPLC to give **XX** in 55% yield.
**1H NMR (400 MHz, DMSO-$d_6$)** $\delta$**:** 2.3 (s, 3H), 2.5 (m, 2H), 2.6-2.8 (m, 8H), 3.8 (s, 3H), 5.0 (s, 2H), 5.6 (s, 2H), 6.8-7.0 (m, 8H), 7.2-7.5 (m, 6H), 7.6 (m, 1H), 8.1 (d, 1H), 9.99 (bs, 1H); **HPLC Purity:** 94.41 %; **Mass (M+1)**: 653.3.

## Scheme 6

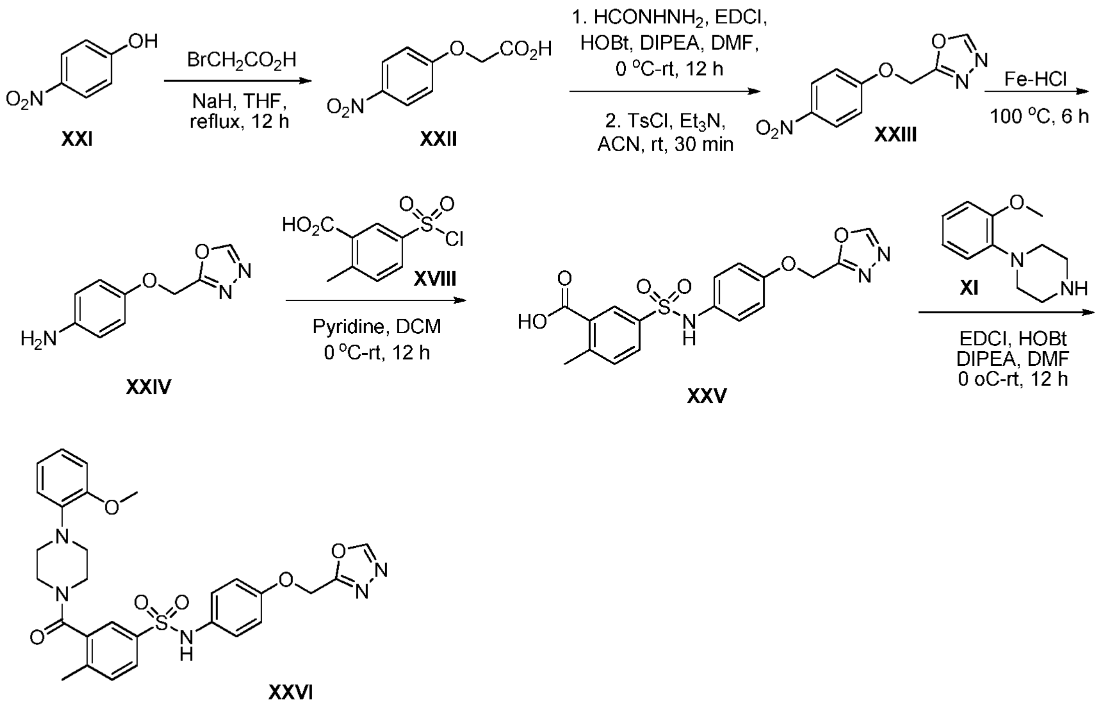


**Synthesis of 2-(4-nitrophenoxy)acetic acid (XXII):**

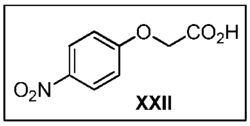

XXII

4-nitro-phenol **XXI** (5.0 g, 36 mmol) was added to a stirred suspension of sodium hydride (3.13 g; 55% in mineral oil; 71.9 mmol) in dry tetrahydrofuran (100 mL) and stirred for 30 min at ambient temperature. Bromoacetic acid (6.0 g, 43.2 mmol) was added and the mixture then heated at reflux overnight. The reaction mixture was cooled to ambient temperature, neutralised with dilute hydrochloric acid and extracted with ethyl acetate. The separated organic layer was extracted with sodium bicarbonate solution and the aqueous solution was acidified with concentrated HCl to pH~3 to afford a white precipitate, which was filtered and dried under vacuum to give (4-nitro-phenoxy)-acetic acid **XXII** (3.5 g, 45%).

**Synthesis of 2-((4-nitrophenoxy)methyl)-1,3,4-oxadiazole (XXIII):**

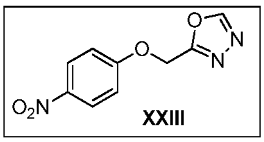

XXIII

To a stirred solution of acid **XXII** (2.31 mmole) in DMF (10 ml), EDCI (0.661 g, 3.46 mmoles), HOBt (0.47 g, 3.46 mmole) and DIPEA (0.89 g, 6.93 mmole) were added at 0°C and stirred for 15 minutes. A solution of formylhydrazide (2.31 mmole) was added at 0°C and then the resulting mixture was allowed to stir at room temperature for overnight. After completion of the reaction, water (20 mL) was added and extracted with ethyl acetate (2x30 ml). The combined

organic layer was dried over anhydrous $Na_2SO_4$ and concentrated under reduced pressure. The crude product was purified by column chromatography (silica gel, 60-120 mess, EtOAc-Hexane, 6:4) to give amide in 55% yield. To a stirred solution of amide (1.01 mmol) in acetonitrile (10 ml) was added triethylamine (0.306 g, 3.0 mmol) followed by p-toluenesulfonyl chloride (0.288 g, 1.5 mmol) and the reaction mixture was stirred for 30 minutes at room temperature. After completion of the reaction, water (20 mL) was added and extracted with ethyl acetate (2x30 ml). The combined organic layer was dried over anhydrous $Na_2SO_4$ and concentrated under reduced pressure. The crude product was purified column chromatography (silica gel, 60-120 mess, EtOAc-Hexane, 4:6) to give **XXIII** in 55% yield.

**Synthesis of 4-((1,3,4-oxadiazol-2-yl)methoxy)aniline (XXIV):**

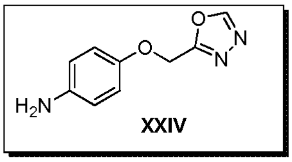

**XXIV**

To a stirred solution of compound **XXIII** (1.1 g, 4.96 mmole) in MeOH (10 ml), was added Fe powder (0.831 g, 14.98 mmol) followed by 1 N HCl (10 ml) at room temperature and heated under reflux for 6 h. After completion of the reaction solvent evaporated, diluted with ethyl acetate and filtered through celite. The filtrate washed with water (2×30 ml) and the combined organic layer was dried over anhydrous $Na_2SO_4$ and concentrated under reduced pressure. The crude product was purified by column chromatography (silica gel, 60-120 mess, EtOAc-Hexane, 4:6) to give **XXIV** in 65% yield.

**Synthesis of 5-(N-(4-((1,3,4-oxadiazol-2-yl)methoxy)phenyl)sulfamoyl)-2-methylbenzoic acid (XXV):**

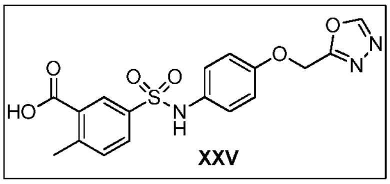

**XXV**

Compound **XXV** was prepared by following similar method dscribed for the preparation of compound **XIX (Scheme-2)** using sulfonyl chloride **XVIII** (0.325 mmol) and amine **XXIV** (0.325 mmol). Crude product was purified by column chromatography (60-120 silica gel, 80% Ethyl Acetate-Hexane) to afford the pure product **XXV** in 55% yields.

**Synthesis of N-(4-((1,3,4-oxadiazol-2-yl)methoxy)phenyl)-3-(4-(2-methoxyphenyl)piperazine-1-carbonyl)-4-methylbenzenesulfonamide (XXVI):**

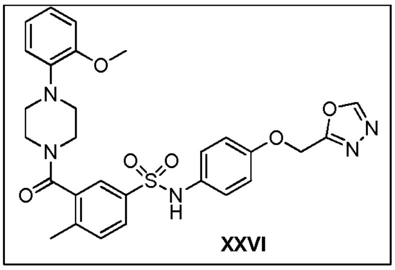

**XXVI**

Compound **XXVI** was prepared by following similar method described for the preparation of compound **XX** (**Scheme-2**) using acid **XXV** (0.325 mmol) and amine **XI** (0.325 mmol). Crude product was purified by preparative HPLC

to afford the product **XXVI** in 45% yields.
**1H NMR (400 MHz, $CDCl_3$) δ:** 2.3 (s, 3H), 2.8-3.2 (m, 8H), 3.8 (s, 3H), 5.3 (s, 2H), 6.8-7.0 (m, 7H), 7.4-7.55 (m, 2H), 7.4-7.5 (m, 2H), 9.1-9.2 (m, 1H), 10.1 (s, 1H); **HPLC Purity:** 96.45 %; **Mass (M+1):** 564.21.

**Scheme 7**

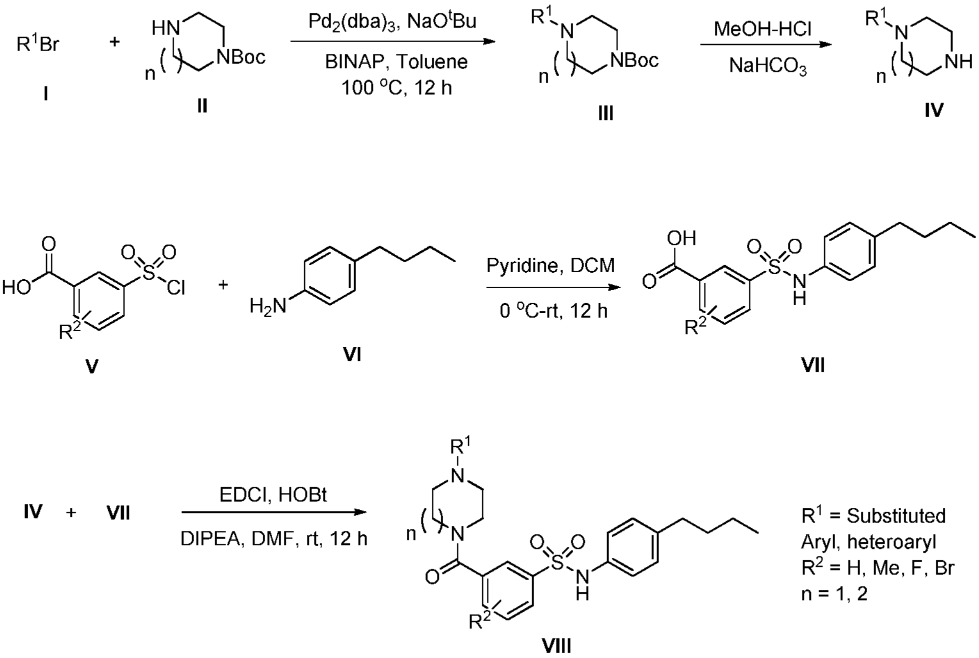


**General procedure for the synthesis of compound III:**

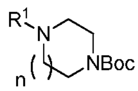

III

To a stirred solution of arylbromide (**I**, 2.15 mmol) in Toluene (20 ml) at room temperature nitrogen gas was purged for 30 min. BINAP (0.134 g, 0.215 mmol), $Pd_2(dba)_3$ (0.039 g, 0.043 mmol) and sodium *tert*-butoxide (0.412 g, 4.3 mmol) were added to the reaction mixture and the nitrogen purging was continued for another 20 min. and finally N-Boc amine (**II,** 2.15 mmol) was added and stirred at 100 °C overnight under nitrogen atmosphere. After completion of the reaction (monitored by TLC), the reaction mixture was concentrated under vacuum. The residue was dissolved in water, extracted with ethyl acetate (3 x 50 ml). Combined organic extracts were washed with brine (20 ml), dried over anhydrous Sodium sulfate, filtered and concentrated under reduced pressure. The crude product was then purified by column chromatography (60-120 silica gel) using 10 % ethyl acetate-hexane to yield compound **VI** (40-60%).

**General procedure for the synthesis of compound IV:**

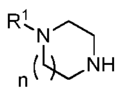

IV

To a stirred solution of MeOH·HCl (10 ml, 20%), Boc protected amine **III** (4.03 mmol) was added and the

resulting mixture was stirred for 2 hr. After completion of reaction, solvent was removed under reduced pressure, washed with water followed by addition of $NaHCO_3$ and extracted with DCM. The organic layer was dried over $Na_2SO_4$ and evaporated under reduced pressure to afford product **IV** in 94% yield.

**General procedure for the synthesis of compound VII:**

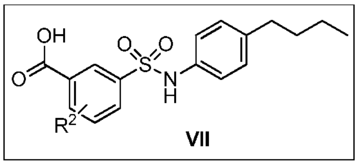

To a solution of 4-butylaniline **VI** (1.43 g, 9.6 mmol) in a mixture (1:1) of DCM and pyridine, appropriate sulfonyl chloride **II** (12.1 mmol) was added at room temperature under $N_2$ atmosphere. The resulting mixture was allowed to stir for 16 hrs. After completion of reaction, the crude mixture was diluted with DCM, washed with water followed by 1N HCl. The organic layer was then dried over $Na_2SO_4$ and concentrated under reduced pressure to afford product **VII** in 78% yields.

**General procedure for the synthesis of compound (VIII-1)-(VIII-36):**

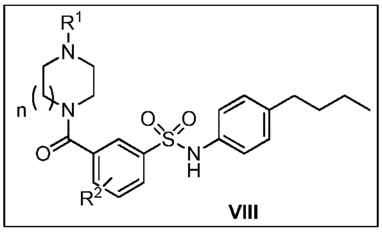

To a stirred solution of acid **VII** (0.315 mmole) in DMF (5 ml), EDCI (0.066 g, 0.000346 moles), HOBt (0.047 g, 0.346 mmole) and DIPEA (0.13 ml, 0.78 mmole) were added at 0°C and stirred for 15 minutes. A solution of appropriate amine **IV** (0.315 moles) was added at 0°C and then the resulting mixture was allowed to stir at room temperature for overnight. After completion of the reaction, water (20 mL) was added and extracted with ethyl acetate (2x30 ml). The combined organic layer was dried over anhydrous $Na_2SO_4$ and concentrated under reduced pressure. The crude product was purified by column chromatography (silica gel, 60-120 mess, ethyl acetate-hexane, 6:4) to give **VIII** in 55-70% yield.

**N-(4-butylphenyl)-3-(4-(2-isopropylphenyl)piperazine-1-carbonyl)-4-methylbenzenesulfonamide (VIII-1)**

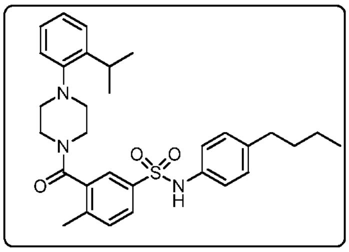

**$^1$H NMR (400 MHz, $CDCl_3$) δ:** 0.95 (t, 3H), 1.2-1.22 (d, 6H), 1.24-1.36 (m, 2H), 1.5-1.6 (m, 2H), 2.4 (s, 3H), 2.42-2.54 (t, 2H), 2.6-2.7 (m, 2H), 2.9-3.0 (m, 4H), 3.2-3.3 (m, 2H), 3.1-3.5 (m, 1H), 6.9-7.1 (m, 5H), 7.11-7.2 (m, 2H), 7.21-7.3 (m, 2H), 7.6-7.62 (m, 2H); **HPLC Purity:** 98.10%; **Mass (M+1)**: 534.34.

**3-(4-([1,1'-biphenyl]-2-yl)piperazine-1-carbonyl)-N-(4-butylphenyl)-4-methyl benzene sulfonamide (VIII-2)**

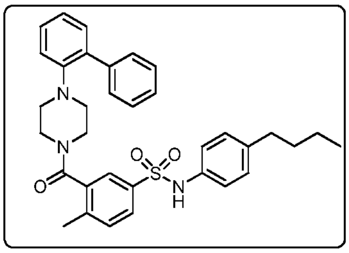

The starting material (2-bromo-1,1'-biphenyl) for Buchwald reaction was prepared from 1,2-dibromobenzene and phenylboronic acid in 25 % yield (Ref. - Synthesis **2009,** 1137).
**$^1$H NMR** (**400 MHz, $CDCl_3$**) δ**:** 0.95 (t, 3H), 1.2-1.36 (m, 2H), 1.5-1.6 (m, 2H), 2.3 (s, 3H), 2.2-2.5 (m, 2H), 2.6-2.7 (m, 2H), 2.9-3.0 (m, 4H), 3.7-3.72 (m, 2H), 6.9-7.0 (m, 5H), 7.1-7.4 (m, 7H), 7.58-7.6 (m, 4H); **HPLC Purity:** 97.14%; **Mass (M+1):** 568.35.

**N-(4-butylphenyl)-4-methyl-3-(4-(pyridin-2-yl)piperazine-1-carbonyl)benzenesulfonamide (VIII-3)**

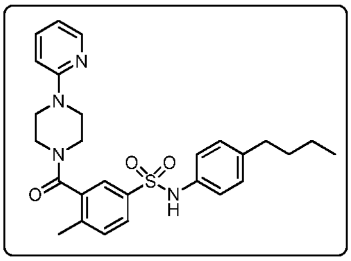

**$^1$H NMR** (**400 MHz, DMSO-$d_6$**) δ: 0.9 (t, 3H), 1.1-1.3 (m, 2H), 1.41-1.5 (m, 2H), 2.3 (s, 3H), 2.4-2.42 (m, 2H), 2.9-3.1 (m, 4H), 3.0-3.1 (m, 2H), 3.5-3.8 (m, 2H), 6.7-7.15 (m, 6H), 7.4-7.7 (m, 4H), 8.1-8.15 (m, 1H), 10.1 (s, 1H); **HPLC Purity:** 98.08%; **Mass (M+1)**: 493.28.

**N-(4-butylphenyl)-3-(4-(2-methoxyphenyl)piperazine-1-carbonyl)-4-methylbenzenesulfonamide (VIII-4)**

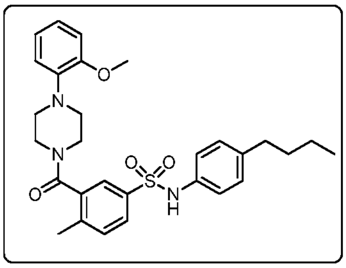

**$^1$H NMR** (**400 MHz, DMSO-$d_6$**) δ: 0.81 (t, 3H), 1.18-1.21 (m, 2H), 1.38-1.42 (m, 2H), 2.22 (s, 3H), 2.28-3.1 (t, 2H), 2.76-2.8 (m, 2H), 2.98-3.1 (m, 4H), 3.7-3.78 (m, 2H), 3.8 (s, 3H), 6.95-7.15 (m, 8H), 7.4-7.42 (m, 2H), 7.62-7.7 (m, 1H), 10.1 (s, 1H); **HPLC Purity:** 98.11%; **Mass (M+1)**: 522.23.

**N-(4-butylphenyl)-3-(4-(2-methoxypyridin-3-yl)piperazine-1-carbonyl)-4 methylbenzenesulfonamide (VIII-5)**

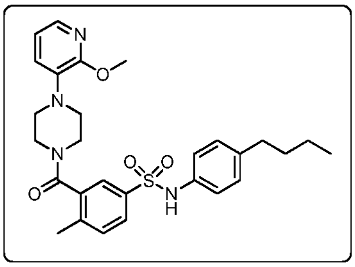

**$^1$H NMR** (**400 MHz, $CD_3OD$**) δ**:** 0.84 (t, 3H), 1.21-1.3 (m, 2H), 1.41-1.5 (m, 2H), 2.38 (s, 3H), 2.44 (t, 2H), 2.8-2.9 (m, 2H), 3.1-3.2 (m, 4H), 3.9-3.95 (m, 2H), 4.0 (s, 1H), 6.95-7.15 (m, 5H), 7.21-7.3 (m, 1H), 7.42-7.5 (m, 2H), 7.76-7.78 (m, 2H); **HPLC Purity:** 94.49%; **Mass (M+1):** 523.39.

**N-(4-butylphenyl)-4-methyl-3-(4-(o-tolyl)piperazine-1-carbonyl)benzenesulfonamide** (**VIII-6**)

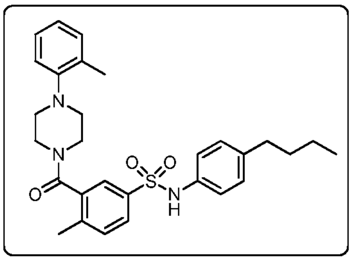

**$^1$H NMR** (**400** MHz, **$CD_3OD$**) δ: 0.84 (t, 3H), 1.21-1.3 (m, 2H), 1.41-1.5 (m, 2H), 2.3 (s, 3H), 3.36 (s, 3H), 2.4 (t, 2H), 2.6-2.7 (m, 2H),2.9-3.0 (m, 2H), 3.1-3.2 (m, 2H), 3.8-4.0 (m, 2H), 6.95-7.15 (m, 6H), 7.1-7.2 (m, 2H), 7.42-7.5 (m, 2H), 7.76-7.78 (m, 1H); **HPLC Purity:** 99.10%; **Mass (M+1):** 506.64.

**N-(4-butylphenyl)-3-(4-(2-fluorophenyl)piperazine-1-carbonyl)-4-methylbenzenesulfonamide** (**VIII-7**)

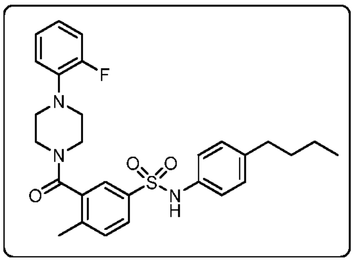

**$^1$H NMR** (**400 MHz, $CD_3OD$**) δ: 0.84 (t, 3H), 1.21-1.3 (m, 2H), 1.41-1.5 (m, 2H), 2.36 (s, 3H), 2.42 (t, 2H), 2.8 (b s, 2H),3.2 (b s, 4H), 3.9 (b s, 2H), 7.0-7.15 (m, 8H), 7.41-7.44 (m, 2H), 7.78-7.8 (m, 1H); **HPLC Purity:** 98.74%; **Mass (M+1):** 510.56.

**N-(4-butylphenyl)-3-(4-(4-fluorophenyl)piperazine-1-carbonyl)-4-methylbenzenesulfonamide** (**VIII-8**)

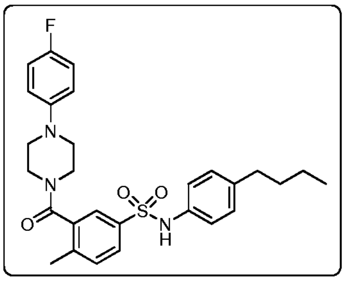

**$^1$H NMR** (**400 MHz, CDCl$_3$**) $\delta$**:** 0.95 (t, 3H), 1.2-1.38 (m, 2H), 1.5-1.6 (m, 2H), 2.38 (s, 3H), 2.5-2.58 (t, 2H), 2.8-3.0 (m, 2H), 3.1-3.21 (m, 4H), 3.9-4.0 (m, 2H), 6.9-7.1 (m, 8H), 7.2-7.3 (m, 1H), 7.6-7.64 (m, 2H); **HPLC Purity:** 99.30%; **Mass (M+1):** 510.77.

**N-(4-butylphenyl)-4-methyl-3-(4-(3-(trifluoromethyl)phenyl)piperazine-1-carbonyl)benzenesulfonamide** (**VIII-9**)

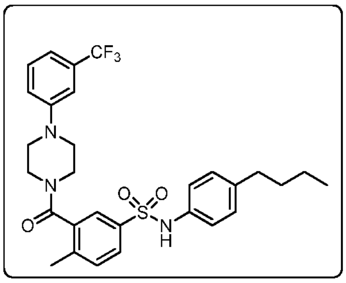

**$^1$H NMR** (**400 MHz, CDCl$_3$**) $\delta$**:** 0.95 (t, 3H), 1.2-1.38 (m, 2H), 1.48-1.6 (m, 2H), 2.38 (s, 3H), 2.5-2.58 (t, 2H), 2.9-3.1 (m, 2H), 3.2-3.3 (m, 4H), 3.9-4.0 (m, 2H), 6.9-7.2 (m, 7H), 7.21-7.4 (m, 2H), 7.6-7.64 (m, 2H); **HPLC Purity:** 99.56%; **Mass (M+1):** 560.89.

**N-(4-butylphenyl)-3-(4-(2-ethylphenyl)piperazine-1-carbonyl)-4-methylbenzenesulfonamide** (**VIII-10**)

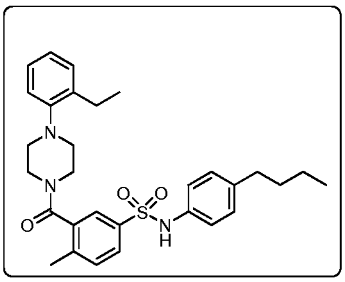

**$^1$H NMR** (**400 MHz, CDCl$_3$**) $\delta$**:** 0.95 (t, 3H), 1.2-1.38 (m, 5H), 1.48-1.6 (m, 2H), 2.38 (s, 3H), 2.42-2.52 (t, 2H), 2.62-2.78 (m, 4H), 2.9-3.0 (m, 2H), 3.2-3.22 (m, 2H), 3.9-4.0 (m, 2H), 6.95-7.2 (m, 8H), 7.21-7.3 (m, 1H), 7.6-7.64 (m, 2H); **HPLC Purity:** 96.52%; **Mass (M+1):** 520.80.

**N-(4-butylphenyl)-3-(4-(4-chlorophenyl)piperazine-1-carbonyl)-4-methylbenzenesulfonamide (VIII-11)**

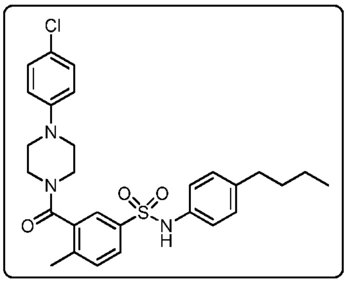

**$^1$H NMR (400 MHz, $CDCl_3$)** δ: 0.95 (t, 3H), 1.2-1.38 (m, 2H), 1.5-1.6 (m, 2H), 2.38 (s, 3H), 2.5-2.58 (t, 2H), 2.9-3.0 (m, 2H), 3.1-3.21 (m, 4H), 3.9-4.0 (m, 2H), 6.9-7.1 (m, 6H), 7.2-7.3 (m, 3H), 7.6-7.64 (m, 2H); **HPLC Purity:** 98.20%; **Mass (M+1):** 526.87.

**N-(4-butylphenyl)-4-methyl-3-(4-(4-propoxyphenyl)piperazine-1-carbonyl)benzenesulfonamide (VIII-12)**

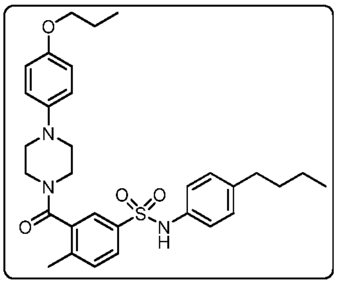

**$^1$H NMR (400 MHz, $CDCl_3$)** δ: 0.95 (t, 3H), 0.98-1.02 (t, 3H), 1.2-1.38 (m, 2H), 1.5-1.6 (m, 2H), 1.7-1.82 (m, 2H), 2.38 (s, 3H), 2.5-2.58 (t, 2H), 2.8-2.96 (m, 2H), 3.1-3.21 (m, 4H), 3.82-3.9 (t, 2H), 3.91-4.0 (m, 2H), 6.9-7.1 (m, 8H), 7.2-7.3 (m, 1H), 7.6-7.64 (m, 2H); **HPLC Purity:** 98.99%; **Mass (M+1):** 550.26.

**N-(4-butylphenyl)-3-(4-(2,6-dichlorophenyl)piperazine-1-carbonyl)-4-methylbenzenesulfonamide (VIII-13)**

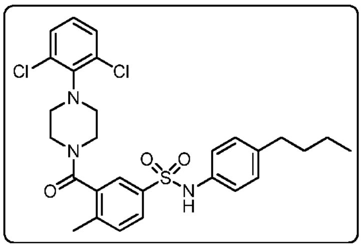

**$^1$H NMR (400 MHz, $CDCl_3$)** δ: 0.95 (t, 3H), 1.2-1.38 (m, 2H), 1.4-1.6 (m, 2H), 2.38 (s, 3H), 2.5-2.58 (t, 2H), 3.0-3.2 (m, 2H), 3.16-3.3 (m, 4H), 3.9-4.0 (m, 2H), 6.9-7.1 (m, 5H), 7.2-7.3 (m, 3H), 7.6-7.64 (m, 2H); **HPLC Purity:** 99.17%; **Mass (M+1):** 560.40.

**N-(4-butylphenyl)-3-(4-(2-(dimethylamino)pyridin-4-yl)piperazine-1-carbonyl)-4-methylbenzenesulfonamide (VIII-14)**

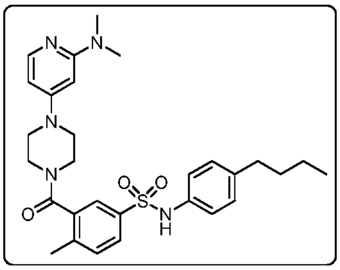

The starting material (4-bromo-N,N-dimethylpyridin-2-amine) for Buchwald reaction was prepared from 4-bromopyridin-2-amine and MeI in presence of NaI in 45 % yield.
**$^1$H NMR** (**400 MHz, DMSO-$d_6$**) δ**:** 0.95 (t, 3H), 1.1-1.3 (m, 2H), 1.4-1.5 (m, 2H), 2.22 (s, 3H), 2.4-2.5 (t, 2H), 2.52-2.58 (m, 2H), 2.92 (s, 6H), 3.02-3.1 (m, 4H), 3.7-3.8 (m, 2H), 6.9-7.1 (m, 5H), 7.2-7.3 (m, 3H), 7.6-7.8 (m, 2H), 10.1 (s, 1H); **HPLC Purity:** 96.83%; **Mass (M+1):** 536.40.

**N-(4-butylphenyl)-3-(4-(3-chlorophenyl)piperazine-1-carbonyl)-4-methylbenzenesulfonamide (VIII-15)**

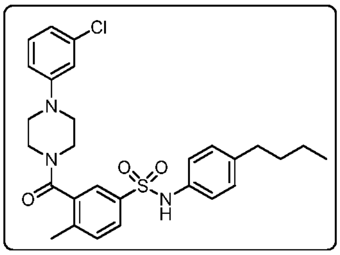

**$^1$H NMR** (**400 MHz, $CDCl_3$**) δ**:** 0.95 (t, 3H), 1.2-1.38 (m, 2H), 1.5-1.6 (m, 2H), 2.38 (s, 3H), 2.5-2.58 (t, 2H), 2.9-3.0 (m, 2H), 3.1-3.21 (m, 4H), 3.9-4.0 (m, 2H), 6.78-7.1 (m, 7H), 7.2-7.3 (m, 2H), 7.6-7.64 (m, 2H); **HPLC Purity:** 94.37%; **Mass (M+1):** 527.23.

**N-(4-butylphenyl)-3-(4-(2-hydroxy-4-methoxyphenyl)piperazine-1-carbonyl)-4-methylbenzenesulfonamide (VIII-16)**

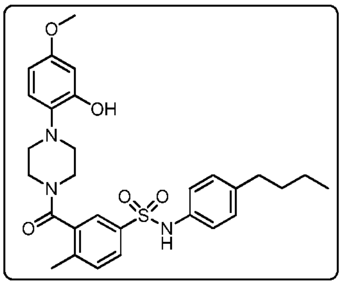

**$^1$H NMR** (**400 MHz, $CDCl_3$**) δ**:** 0.95 (t, 3H), 1.2-1.38 (m, 2H), 1.5-1.6 (m, 2H), 2.38 (s, 3H), 2.5-2.58 (t, 2H), 2.8-3.0 (m, 2H), 3.1-3.3 (m, 4H), 3.8 (s, 3H), 4.0-4.1 (m, 2H), 5.6-5.7 (b s, 1H), 6.4-6.5 (m, 1H), 6.7-6.74 (m, 1H), 6.9-7.0 (m, 4H), 7.2-7.4 (m, 2H), 7.5-7.7 (m, 2H); **HPLC Purity:** 97.77%; **Mass (M+1):** 538.40.

**N-(4-butylphenyl)-3-(4-(4-fluoro-2-methoxyphenyl)piperazine-1-carbonyl)-4-methylbenzenesulfonamide (VIII-17)**

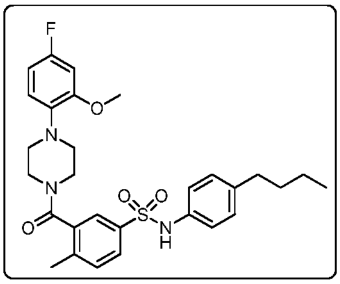

**$^1$H NMR** (**400 MHz, DMSO-d$_6$**) δ**:** 0.86 (t, 3H), 1.2-1.38 (m, 2H), 1.4-1.5 (m, 2H), 2.22 (s, 3H), 2.4-2.46 (t, 2H), 2.8-2.9 (m, 2H), 3.0-3.2 (m, 4H), 3.7-3.78 (m, 2H), 3.8 (s, 3H), 6.7-6.71 (m, 7H), 7.4-7.7 (m, 3H), 10.1 (s, 1H); **HPLC Purity:** 91.24%; **Mass (M+1):** 540.20.

**N-(4-butylphenyl)-3-(4-(2,4-dimethoxyphenyl)piperazine-1-carbonyl)-4-methylbenzenesulfonamide (VIII-18)**

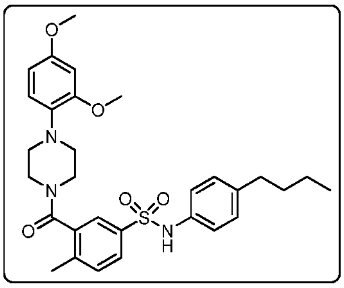

**$^1$H NMR** (**400 MHz, CDCl$_3$**) δ**:** 0.86 (t, 3H), 1.2-1.38 (m, 2H), 1.4-1.5 (m, 2H), 2.38 (s, 3H), 2.4-2.5 (t, 2H), 2.78-2.9 (m, 2H), 3.0-3.1 (m, 2H), 3.2-3.3 (m, 2H), 3.8 (s, 3H), 3.82 (s, 3H), 3.9-4.0 (m, 2H), 6.4-6.5 (m, 1H), 6.7-7.1 (m, 6H), 7.2-7.4 (m, 1H), 7.8-7.86 (m, 2H); **HPLC Purity:** 92.82%; **Mass (M+1):** 552.10.

**N-(4-butylphenyl)-4-fluoro-3-(4-(2-methoxyphenyl)piperazine-1-carbonyl)benzenesulfonamide (VIII-19)**

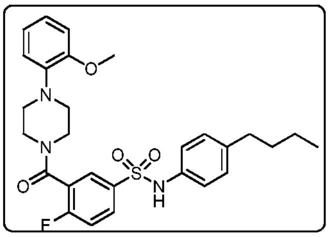

**$^1$H NMR** (**400 MHz, DMSO-d6)** δ**:** 0.9 (t, 3H), 1.2-1.4 (m, 2H), 1.5-1.6 (m, 2H), 2.5-2.6 (m, 2H), 2.75-3.0 (m, 3H), 3.0-3.2 (m, 2H), 3.35-3.4 (m, 2H), 3.9 (s, 3H), 6.65 (d, 1H), 6.8-7.1 (m, 8H), 7.6-7.8 (m, 1H), 7.8-7.98 (m, 2H); **HPLC Purity:** 95.26 %; **Mass (M+1):** 526.25.

**N-(4-butylphenyl)-3-(4-(pyridin-4-yl)piperazine-1-carbonyl)benzenesulfonamide(VIII-20)**

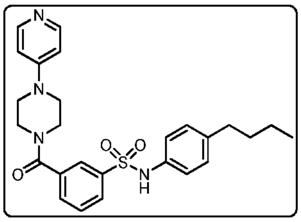

**1H NMR (400 MHz, DMSO-$d_6$) δ:** 0.9 (t, 3H), 1.2-1.4 (m, 2H), 1.6-1.7 (m, 2H), 2.4-2.6 (m, 2H), 3.6-4.0 (m, 7H), 6.9-7.25 (m, 6H), 7.5-8.0 (m, 4H), 8.2-8.4 (m, 2H), 10.2 (s, 1H); **HPLC Purity:** 99.99 %; **Mass (M+1):** 479.35.

**N-(4-butylphenyl)-3-(4-(2-methoxyphenyl)piperazine-1-carbonyl)benzenesulfonamide (VIII-21)**

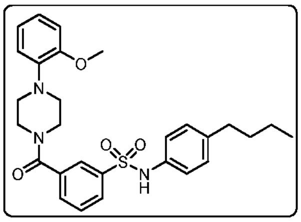

**1H NMR** (**400 MHz, $CDCl_3$**) **δ:** 0.9 (t, 3H), 1.2-1.4 (m, 4H), 1.6-1.7 (m, 2H), 2.4-2.6 (m, 2H), 2.8-3.2 (m, 2H), 3.4-3.6 (m, 1H), 3.8-4.0 (m, 3H), 6.5 (m, 1H), 6.9-7.25 (m, 7H), 7.39-7.8 (m, 4H); **HPLC Purity:** 95.87 %; **Mass (M+1):** 508.26.

**N-(4-butylphenyl)-3-(4-(3-chloro-5-(trifluoromethyl)pyridin-2-yl)piperazine-1-carbonyl)benzenesulfonamide (VIII-22)**

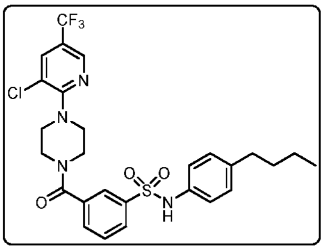

**1H NMR** (**400 MHz, $CDCl_3$**) **δ:** 0.9 (t, 3H), 1.1-1.2 (m, 2H), 1.3-1.5 (m, 2H), 2.6-2.62 (m, 2H), 3.2-3.4 (m, 6H), 3.8-4.0 (m, 2H), 6.4-6.45 (m, 1H), 6.96-7.4 (m, 4H), 7.4-7.8 (m, 4H), 8.4 (m, 1H); **HPLC Purity:** 97.33 %; **Mass (M+1):** 581.40.

**N-(4-butylphenyl)-3-(4-(pyridin-4-yl)piperazine-1-carbonyl)benzenesulfonamide (VIII-23)**

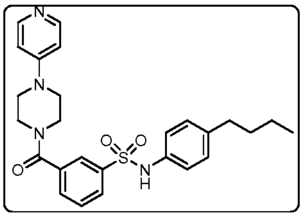

**1H NMR (400 MHz, $CDCl_3$) δ:** 1.0 (t, 3H), 1.2-1.4 (m, 2H), 1.5-1.6 (m, 2H), 2.4-2.6 (m, 2H), 3.2-4.0 (m, 8H), 6.6-6.8 (m, 3H), 7.0-7.25 (m, 4H), 7.5-7.6 (m, 3H), 7.8 (m, 2H), 8.2 (d, 1H); **HPLC Purity:** 98.85 %; **Mass (M+1):** 479.26.

**N-(4-butylphenyl)-3-(4-(2,6-dimethylphenyl)piperazine-1-carbonyl)-4-methylbenzenesulfonamide (VIII-24)**

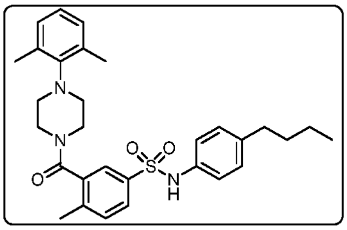

**1H NMR (400 MHz, $CDCl_3$) δ:** 0.99 (t, 3H), 1.0-1.2 (s, 6H), 1.4-1.6 (m, 2H), 2.2 (s, 3H), 2.35 (s, 3H), 2.4-2.6 (m, 2H), 2.8-3.0 (m, 2H), 3.2 (s, 3H), 3.8-4.0 (m, 2H), 6.8-7.18 (m, 6H), 7.2-7.4 (m, 2H), 7.59-7.8 (m, 3H); **HPLC Purity:** 97.19 %; **Mass (M+1):** 520.30.

**4-bromo-N-(4-butylphenyl)-3-(4-(2-methoxyphenyl)piperazine-1-arbonyl)benzenesulfonamide (VIII-25)**

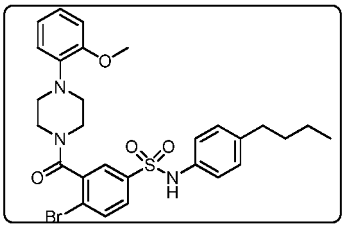

**1H NMR (400 MHz, DMSO-$d_6$) δ:** 0.9 (t, 3H), 1.0-1.2 (m, 2H), 1.4-1.6 (m, 2H), 2.4-2.2.5 (m, 2H), 2.7-2.9 (m, 3H), 3.0 (s, 3H), 3.76-3.85 (m, 5H), 6.8-7.18 (m, 8H), 7.59-7.8 (m, 2H), 7.9 (d, 1H), 10.2 (s, 1H); **HPLC Purity:** 92.66 %; **Mass (M+1):** 588.05.

**N-(4-butylphenyl)-4-methyl-3-(4-(2-(trifluoromethyl)phenyl)piperazine-1-carbonyl)benzenesulfonamide (VIII-26)**

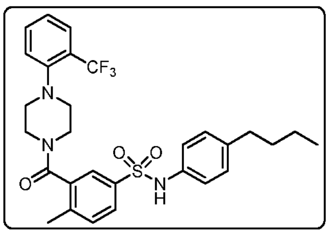

**1H NMR** (**400 MHz, DMSO**-$d_6$) δ**:** 2.39 (t, 3H), 2.55-2.6 (m, 2H), 3.0-3.2 (m, 4H), 3.8-3.9 (m, 2H), 3.9 (m, 3H), 6.4 (m, 1H), 6.8-7.2 (m, 5H), 7.3-7.8 (m, 8H), 8.2 (bs, 1H); **HPLC Purity:** 95.02 %; **Mass (M+1):** 505.31

**3-(4-(2-bromophenyl)piperazine-1-carbonyl)-N-(4-butylphenyl)-4-methylbenzenesulfonamide (VIII-27)**

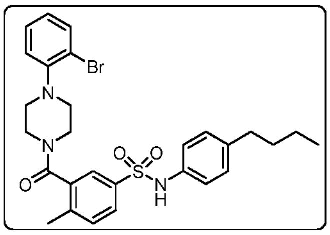

**1H NMR** (**400 MHz, DMSO-$d_6$**) δ**:** 0.9 (t, 3H), 1.1-1.5 (m, 4H), 2.1 (s, 3H), 2.12-2.4 (m, 2H), 2.7-2.8 (m, 2H), 3.0-3.2 (m, 4H), 3.8-3.9 (m, 2H), 6.4 (m, 1H), 6.9-7.1 (m, 5H), 7.19-7.5 (m, 3H), 7.6-7.8 (m, 2H), 10.1 (s, 1H); **HPLC Purity:** 99.55 %; **Mass (M+1):** 572.30.

**N-(4-butylphenyl)-3-(4-(pyrimidin-2-yl)piperazine-1-carbonyl)benzenesulfonamide (VIII-28)**

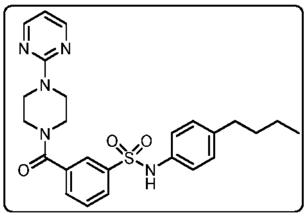

**1H NMR** (**400 MHz, $CDCl_3$**) δ**:** 0.9 (t, 3H), 1.1-1.2 (m, 2H), 1.3-1.5 (m, 2H), 2.6-2.62 (m, 2H), 3.2-3.4 (m, 2H), 3.8-4.0 (m, 6H), 6.4-6.6 (m, 2H), 7.0-7.4 (m, 4H), 7.3-7.8 (m, 2H), 8.36-8.4 (m, 2H); **HPLC Purity:** 92.98 %; **Mass** (**M+1)**: 480.24.

**N-(4-butylphenyl)-3-(4-(2-chlorophenyl)piperazine-1-carbonyl)-4-methylbenzenesulfonamide (VIII-29)**

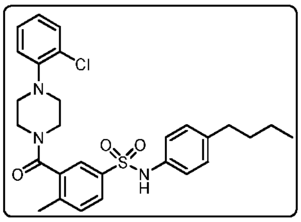

**1H NMR** (**400 MHz, $CDCl_3$**) δ**:** 0.9 (t, 3H), 1.2-1.4 (m, 2H), 1.6-1.7 (m, 2H), 2.4 (s, 3H), 2.5-2.6 (m, 2H), 2.8-3.0 (m, 2H), 3.0-3.6 (m, 7H), 6.6 (m, 1H), 6.9-7.25 (m, 7H), 7.3-7.5 (m, 1H), 7.6-7.7 (m, 4H); **HPLC Purity:** 98.23 %; **Mass (M+1):** 526.10.

**N-(4-butylphenyl)-4-methyl-3-(4-(pyridin-4-yl)piperazine-1-carbonyl)benzenesulfonamide (VIII-30)**

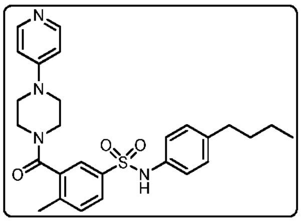

**1H NMR** (**400 MHz, DMSO**-**$d_6$**) δ**:** 0.9 (t, 3H), 0.91-1.0 (m, 1H), 1.2-1.25 (m, 4H), 1.4-1.42 (m, 1H), 2.3 (s, 3H), 3.0-3.2 (m, 4H), 3.4-3.8 (m, 4H), 6.9-7.25 (m, 6H), 7.39-7.6 (m, 2H), 7.7 (d, 1H), 8.2 (d, 2H), 10.1 bs, 1H) ; HPLC Purity: 97.79 %; **Mass** (**M+1**)**:** 493.10.

**N-(4-butylphenyl)-3-(4-(3-fluoro-4-methoxyphenyl)piperazine-1-carbonyl)-4-methylbenzenesulfonamide**(**VIII-31**)

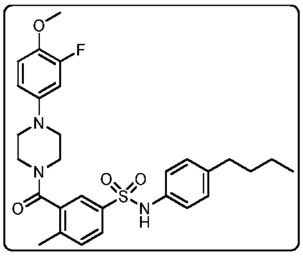

**1H NMR** (**400 MHz, DMSO**-**$d_6$**) δ: 1.0 (t, 3H), 1.2-1.3 (m, 2H), 1.4-1.5 (m, 2H), 2.1 (s, 3H), 2.3-2.6 (m, 2H), 2.8-3.4 (m, 6H), 3.8 (s, 3H), 6.6-7.2 (m, 6H), 7.4-7.5 (m, 2H), 7.7 (d, 1H) 10.1 (s, 1H); **HPLC Purity:** 98.45 %; **Mass** (**M+1**): 540.26.

**3-(4-(1H-pyrrolo[2,3-b]pyridin-4-yl)piperazine-1-carbonyl)-N-(4-butylphenyl)benzenesulfonamide (VIII-32)**

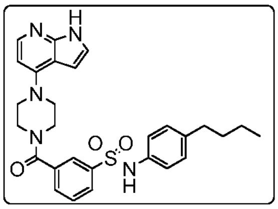

**1H NMR** (**400 MHz, $CDCl_3$**) δ**:** 1.0 (t, 3H), 1.2-1.4 (m, 2H), 1.5-1.6 (m, 2H), 2.4-2.6 (m, 2H), 3.2-4.0 (m, 8H), 6.6-6.8 (m, 3H), 7.0-7.25 (m, 4H), 7.5-7.6 (m, 3H), 7.8 (m, 2H), 8.2 (d, 1H); **HPLC Purity:** 94.35 %; **Mass** (**M+1**): 518.26.

**N-(4-butylphenyl)-4-methyl-3-(4-phenylpiperazine-1-carbonyl)benzenesulfonamide (VIII-33)**

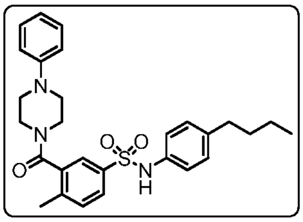

**1H NMR** (**400 MHz, DMSO**-$d_6$) δ**:** 2.39 (t, 3H), 2.2 (s, 3H), 2.55-2.6 (m, 2H), 3.0-3.2 (m, 4H), 3.8-3.9 (m, 2H), 3.9-4.0 (m, 6H), 6.4 (m, 1H), 6.8-7.2 (m, 5H), 7.3-7.8 (m, 8H), 8.2 (bs, 1H); **HPLC Purity:** 98.5 %; **Mass (M+1):** 492.31.

**N-(4-butylphenyl)-3-(4-(3,4-dimethoxyphenyl)piperazine-1-carbonyl)-4-methylbenzenesulfonamide (VIII-34)**

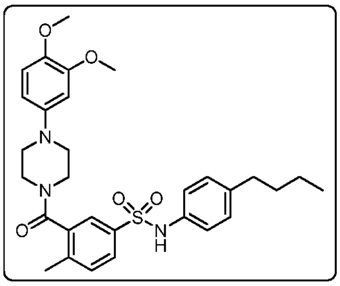

$^1$**H NMR** (**400 MHz, DMSO-**$d_6$) δ**:** 0.9 (t, 3H), 1.2-1.32 (m, 2H), 1.4-1.5 (m, 2H), 2.30 (s, 3H), 2.4-2.5 (t, 2H), 2.78-2.9 (m, 2H), 3.0-3.1 (m, 4H), 3.78 (s, 6H), 3.9-3.91 (m, 2H), 6.7-7.1 (m, 7H), 7.4-7.5 (m, 2H), 7.7-7.72 (m, 1H), 10.1 (s, 1H); **HPLC Purity:** 92.72%; **Mass (M+1):** 552.45.

**N-(4-butylphenyl)-4-methyl-3-(4-o-tolyl-1,4-diazepane-1-carbonyl)benzenesulfonamide (VIII-35)**

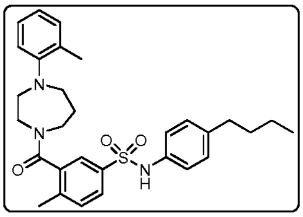

**1H NMR** (**400 MHz, $CDCl_3$)** δ**:** 1.0 (t, 3H), 1.2-1.4 (m, 2H), 1.5-1.6 (m, 2H), 1.7-1.8 (m, 1H), 2.0-2.1 (m, 1H), 2.2 (s, 3H), 2.21 (s, 3H), 2.25-2.6 (m, 2H), 2.9-3.2 (m, 3H), 3.8-4.0 (m, 2H), 6.8-7.2 (m, 6H), 7.21-7.5 (m, 5H), 7.6 (m, 1H); **HPLC Purity:** 93.96 %; **Mass (M+1):** 520.35.

**N-(4-butylphenyl)-3-(4-(2-fluorophenyl)-1,4-diazepane-1-carbonyl)-4-methylbenzenesulfonamide (VIII-36)**

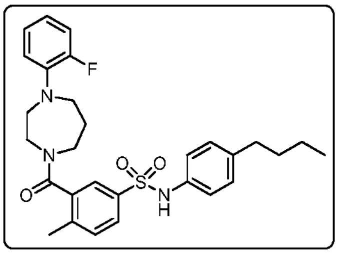

**$^1$H NMR (400 MHz, $CDCl_3$) δ:** 0.95 (t, 3H), 1.2-1.38 (m, 2H), 1.5-1.6 (m, 2H), 1.61-1.7 (m, 2H), 2.38 (s, 3H), 2.5-2.58 (t, 2H), 3.2-3.4 (m, 4H), 3.41-3.6 (m, 2H), 3.9-4.0 (m, 2H), 6.9-7.1 (m, 7H), 7.2-7.3 (m, 2H), 7.5-7.64 (m, 2H); **HPLC Purity:** 97.61%; **Mass (M+1):** 524.25.

## Scheme 8

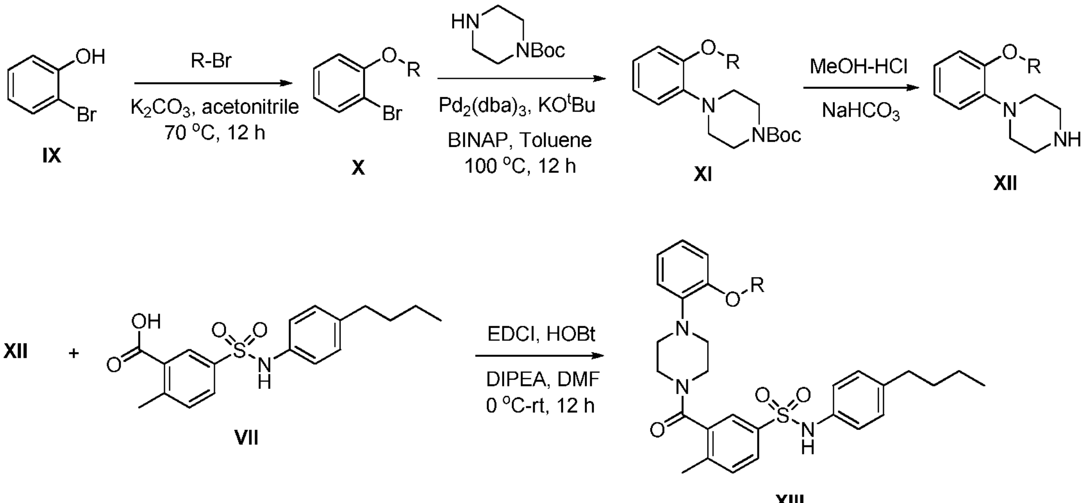


IX X XI XII

XII + VII → XIII

R = alkyl, aryl, cyclopropylmethyl, arylalkyl

**General procedure for the synthesis of compound X:**

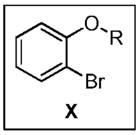

**X**

To a stirred solution of 2-bromo phenol **IX** (0.5 g, 2.89 mmole) in acetonitrile (20 ml) were added potassium carbonate (1.19 g, 8.67 mmol), appropriate alkyl halide (3.17 mmol) at room temperature under nitrogen atmosphere and the reaction mixture was heated at 70 °C for overnight. After completion of the reaction (checked by TLC), water (20 mL) was added and extracted with ethyl acetate (2x30 ml). The combined organic layer was dried over anhydrous $Na_2SO_4$ and concentrated under reduced pressure. The crude product was purified by column chromatography (silica gel, 60-120 mess, ethyl acetate-hexane, 1:10) to give **X** in 65-80% yield.

**General procedure for the synthesis of compound XI:**

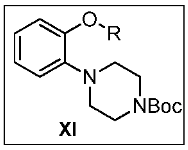

XI

The product **XI** was prepared by following similar method used for the preparation of compound **III** (**Scheme-1**) using aryl bromide **X** (0.92 mmol) and *tert*-butyl piperazine-1-carboxylate **X** (0.191 g, 1.02 mmol). Crude product was purified by column chromatography (60-120 silica gel, 20% Ethyl Acetate-Hexane) to afford the pure product **XI** in 41-65% yields.

**General procedure for the synthesis of compound XII:**

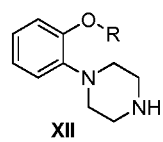

XII

To a stirred solution of MeOH·HCl (10 ml, 20%), Boc protected amine **XI** (4.03 mmol) was added and the resulting mixture was stirred for 2 hr. After completion of reaction, solvent was removed under reduced pressure, washed with water followed by addition of $NaHCO_3$ and extracted with DCM. The organic layer was dried over $Na_2SO_4$ and evaporated under reduced pressure to afford product **XII** in 85% yield.

**General procedure for the synthesis of compound XIII:**

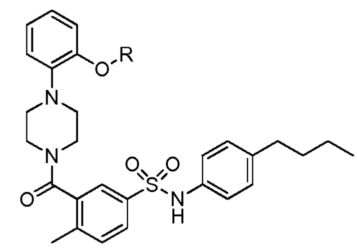

XIII

The product **XII** was prepared by following similar method used for the preparation of compound **VIII** (**Scheme-1**) using acid **VII** (0.167 mmol) and amine **XII** (0.167 mmol). Crude mixture was purified by column chromatography (60-120 silica gel, 50% Ethyl Acetate-Hexane) to afford the pure product **XIII** in 45-65% yields.

**3-(4-(2-(benzyloxy)phenyl)piperazine-1-carbonyl)-N-(4-butylphenyl)-4-methylbenzenesulfonamide** (**XIII-1**)

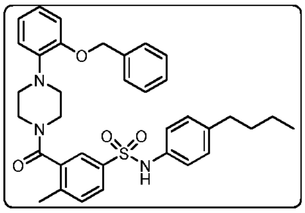

**1H NMR** (**400 MHz, DMSO**-**$d_6$**) δ**:** 0.9 (t, 3H), 1.0-1.4 (m, 6H), 2.2 (s, 3H), 2.3-2.4 (m, 3H), 3.0-3.4 (m, 7H), 3.8 (m, 1H), 5.1 (s, 2H), 6.8-7.2 (m, 8H), 7.4-7.8 (m, 8H), 10.1 (s, 1H); **HPLC Purity:** 96.06 %; **Mass (M+1):** 598.37.

**N-(4-butylphenyl)-3-(4-(2-ethoxyphenyl)piperazine-1-carbonyl)-4-methylbenzenesulfonamide (XIII-2)**

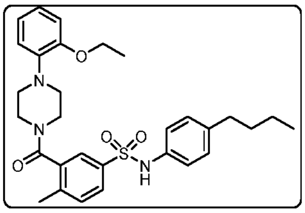

**1H NMR** (**400 MHz, $CDCl_3$**) δ**:** 0.9 (t, 3H), 1.2-1.4 (m, 3H), 1.41-1.6 (m, 4H), 2.2 (s, 3H), 2.4-2.6 (m, 2H), 2.8-3.0 (m, 2H), 3.1-3.4 (m, 4H), 3.8-4.1 (m, 4H), 6.4 (s, 1H), 6.8-7.2 (m, 8H), 7.2-7.4 (m, 1H), 7.6-7.7 (m, 2H); **HPLC Purity:** 97.33 %; **Mass (M+1):** 536.30.

**N-(4-butylphenyl)-4-methyl-3-(4-(2-phenoxyphenyl)piperazine-1-carbonyl)benzenesulfonamide (XIII-3)**

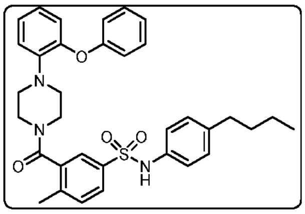

The starting material (1-bromo-2-phenoxybenzene) for Buchwald reaction was prepared from 2-bromophenol and phenylboronic acid in 45 % yield (Ref. - WO2009/66072 A2, 2009).
**1H NMR** (**400 MHz, $CDCl_3$)** δ: 0.9 (t, 3H), 1.2-1.3 (m, 2H), 2.39-2.4 (m, 2H), 2.2 (s, 3H), 2.3-2.6 (m, 2H), 2.8 (s, 3H), 3.0-3.2 (m, 2H), 3.4-4.8 (m, 4H), 6.8-7.0 (m, 6H), 7.1-7.2 (m, 3H), 7.3-7.5 (m, 4H), 7.6-7.7 (m, 2H); **HPLC Purity:** 98.39 %; **Mass (M+1):** 584.40.

**N-(4-butylphenyl)-4-methyl-3-(4-(2-propoxyphenyl)piperazine-1-arbonyl)benzenesulfonamide (XIII-4)**

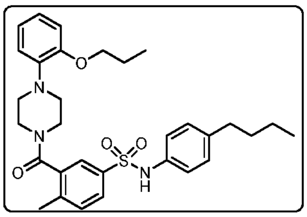

**1H NMR** (**400 MHz, $CDCl_3$)** δ**:** 0.9 (t, 3H), 1.0 (s, 3H), 1.1-1.2 (m, 2H), 1.3-1.4 (m, 2H), 1.7-1.8 (m, 2H), 2.3 (s, 3H), 2.4-2.5 (m, 2H), 2.8 (s, 3H), 3.0-3.2 (m, 2H), 3.4-4.8 (m, 4H), 6.8-7.0 (m, 6H), 7.1-7.2 (m, 2H), 7.4-7.5 (m, 2H), 7.6-7.7 (m, 1H), 10.1 (s, 1H); **HPLC Purity:** 99.67 %; **Mass (M+1):** 550.35.

**N-(4-butylphenyl)-3-(4-(2-(cyclopropylmethoxy)phenyl)piperazine-1-carbonyl)-4-methylbenzenesulfonamide (XIII-5)**

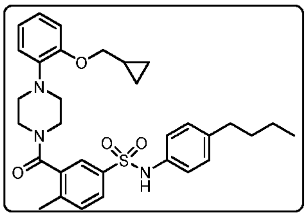

**1H NMR** (**400 MHz, $CDCl_3$) δ:** 0.2-0.6 (m, 4H), 0.8 (t, 3H), 1.1-1.3 (m, 5H), 2.3 (s, 3H), 2.25 (s, 3H), 2.8-3.0 (m, 2H), 3.0-3.4 (m, 4H), 3.6-3.8 (m, 4H), 6.8-7.0 (m, 5H), 7.4 -7.55 (m, 3H), 7.4-7.5 (m, 2H), 7.6-7.7 (m, 1H), 10.1 (s, 1H); **HPLC Purity:** 99.00 %; **Mass (M+1):** 562.20.

**N-(4-butylphenyl)-3-(4-(2-isopropoxyphenyl)piperazine-1-carbonyl)-4-methylbenzenesulfonamide (XIII-6)**

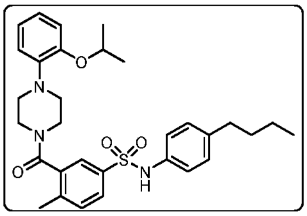

**1H NMR** (**400 MHz, $CDCl_3$) δ:** 0.8 (t, 3H), 1.1-1.15 (m, 2H), 1.3 (d, 6H), 1.35-1.4 (m, 4H), 2.3 (s, 3H), 2.25 (s, 3H), 2.8-3.2 (m, 4H), 3.6-3.8 (m, 4H), 4.6 (m, 1H), 6.8-7.0 (m, 5H), 7.4-7.55 (m, 2H), 7.4-7.5 (m, 2H), 7.6-7.7 (m, 4H), 10.1 (s, 1H); **HPLC Purity:** 98.08 %; **Mass (M+1):** 550.40.

**N-(4-butylphenyl)-3-(4-(2-isobutoxyphenyl)piperazine-1-carbonyl)-4-methylbenzenesulfonamide (XIII-7)**

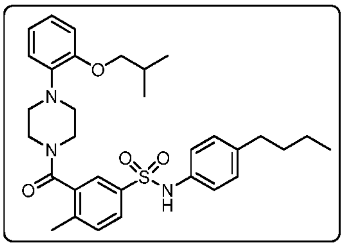

**$^1$H NMR** (**400 MHz, DMSO-$d_6$) δ:** 0.8-0.86 (t, 3H), 0.9-1.0 (d, 6H), 1.2-1.3 (m, 2H), 1.4-1.46 (m, 2H), 2.0-2.1 (m, 1H), 2.22 (s, 3H), 2.9-3.0 (t, 2H), 2.7-2.9 (m, 2H), 3.0-3.1 (m, 4H), 3.7-3.74 (d, 2H), 3.79-3.8 (m, 2H), 6.9-7.0 (m, 8H), 7.4-7.42 (m, 2H), 7.6-7.62 (m, 1H), 10.1 (s, 1H); **HPLC Purity:** 96.04%; **Mass (M+1):** 564.43.

**N-(4-butylphenyl)-4-methyl-3-(4-(2-phenethoxyphenyl)piperazine-1-carbonyl)benzenesulfonamide (XIII-8)**

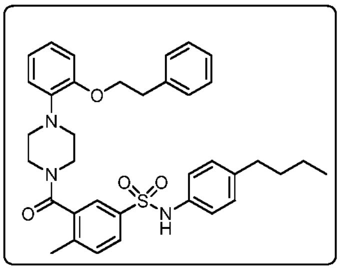

**$^1$H NMR (400 MHz, DMSO-$d_6$) δ:** 0.8-0.83 (t, 3H), 1.1-1.2 (m, 2H), 1.3-1.42 (m, 2H), 2.22 (s, 3H), 2.3-2.4 (t, 2H), 2.6-2.62 (m, 2H), 2.8-2.9 (m, 4H), 3.02-3.1 (t, 2H), 3.6-3.7 (m, 2H), 4.2-4.22 (t, 2H), 6.8-7.0 (m, 8H), 7.1-7.38 (m, 5H), 7.4-7.5 (m, 2H), 7.7-7.71 (m, 1H), 10.1 (s, 1H); **HPLC Purity:** 96.05%; **Mass (M+1):** 612.40.

## Scheme 9

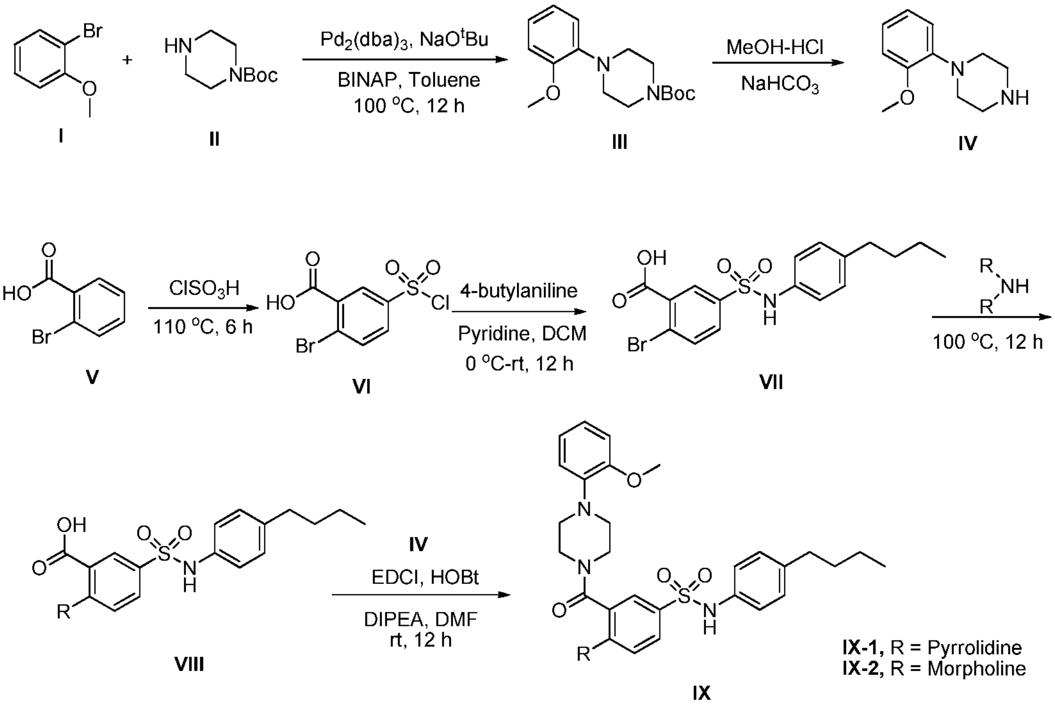


I II III IV V VI VII VIII IX

### Synthesis of *tert*-butyl 4-(2-methoxyphenyl)piperazine-1-carboxylate (III):

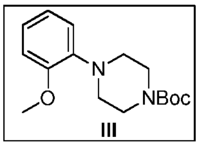

III

To a stirred solution of 2-Bromoanisole (**I**, 0.403 g, 2.15 mmol) in Toluene (20 ml) at room temperature, nitrogen gas was purged for 30 minutes. BINAP (0.134 g, 0.215 mmol), $Pd_2(dba)_3$ (0.039 g, 0.043 mmol) and sodium *tert*-butoxide (0.412 g, 4.3 mmol) were added to the reaction mixture and the nitrogen purging was continued for another 20 minutes and finally N-Boc piperazine (**II**, 0.4 g, 2.15 mmol) was added and stirred at 100°C overnight under nitrogen atmosphere. After completion of the reaction (monitored by TLC), the reaction mixture was concentrated under vacuum. The residue was dissolved in water, extracted with ethyl acetate (3 x 50 ml). Combined organic extracts were washed with brine (20 ml), dried over anhydrous Sodium sulfate, filtered and concentrated under reduced pressure. The crude product was then purified by column chromatography (60-120 silica gel) using 10 % ethyl acetate-hexane to afford compound **III** in 60% yield.

### Synthesis of 1-(2-methoxyphenyl)piperazine (IV):

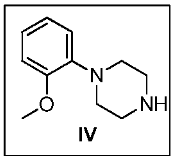

IV

To a stirred solution of MeOH·HCl (10 ml, 20%), Boc protected amine **III** (4.03 mmol) was added and the resulting mixture was stirred for 2 h. After completion of reaction, solvent was removed under reduced pressure, washed with water followed by addition of $NaHCO_3$ and extracted with DCM. The organic layer was dried over $Na_2SO_4$ and evaporated under reduced pressure to afford product **IV** in 94% yield.

**Synthesis of 2-bromo-5-(chlorosulfonyl)benzoic acid (VI):**

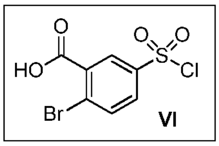

VI

To a stirred solution of 2-Bromobenzoic acid (1 g. 5.01 mmol) was added chlorosulphonic acid (5.8 g, 50 mmol) at 0°C under nitrogen atmosphere and the resulting mixture was heated at 110 °C for 6 h. After completion of reaction, the reaction mixture was cooled, quenched with ice and extracted with DCM. The organic layer was dried over $Na_2SO_4$ and evaporated under reduced pressure to afford product **VI** in 85% yield.

**Synthesis of 2-bromo-5-(N-(4-butylphenyl)sulfamoyl)benzoic acid (VII):**

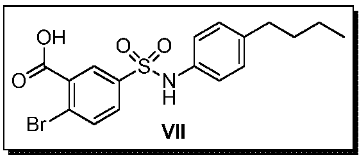

VII

To a stirred solution of 4-butylaniline (1.43 g, 9.6 mmol) in a mixture (1:1) of DCM and pyridine, sulfonyl chloride **VI** (4.98 g, 12.1 mmol) was added at room temperature under $N_2$ atmosphere. The resulting mixture was allowed to stir for 16 hrs. After completion of reaction, the crude mixture was diluted with DCM, washed with water followed by 1N HCl. The organic layer was then dried over $Na_2SO_4$ and concentrated under reduced pressure to afford product **VII** in 82% yields.

**General Procedure for the synthesis of compound VIII:**

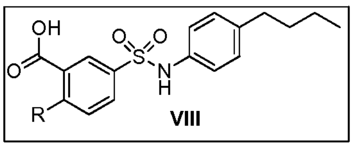

VIII

To a stirred solution of 2-bromo-5-(N-(4-butylphenyl)sulfamoyl) benzoic acid **VII** (0.2 g, 0.403 mmol) in morpholine/pyrrolidine (10 ml) was heated at 100°C under $N_2$ atmosphere for 16 h. After completion of reaction, the crude mixture was diluted with Ethyl acetate (30 ml), washed with 2N HCl followed by water. The organic layer was then dried over $Na_2SO_4$ and concentrated under reduced pressure. The crude product was then purified by column chromatography

(60-120 silica gel) using 2 % MeOH-DCM to afford compound **VIII** in 70% yield.

**General Procedure for the synthesis of compound (IX-1)-(IX-2):**

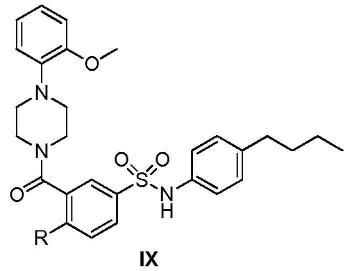

IX

To a stirred solution of acid **VII** (0.315 mmole) in DMF (5 ml), EDCI (0.066 g, 0.000346 moles), HOBt (0.047 g, 0.346 mmole) and DIPEA (0.13 ml, 0.78 mmole) were added at 0°C and stirred for 15 minutes. A solution of appropriate amine **IV** (0.315 moles) was added at 0°C and then the resulting mixture was allowed to stir at room temperature for overnight. After completion of the reaction, water (20 mL) was added and extracted with ethyl acetate (2x30 ml). The combined organic layer was dried over anhydrous $Na_2SO_4$ and concentrated under reduced pressure. The crude product was purified by column chromatography (silica gel, 60-120 mess, MeOH-DCM, 2:98) to give **VIII** in 45-50% yield.

**N-(4-butylphenyl)-3-(4-(2-methoxyphenyl)piperazine-1-carbonyl)-4-morpholinobenzenesulfonamide (IX-1):**

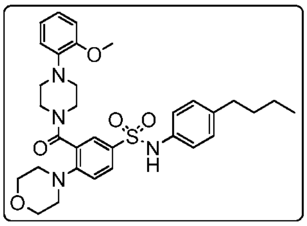

**1H NMR** (**400 MHz, DMSO**-$d_6$) δ**:** 1.0 (t, 3H), 1.2-1.4 (m, 2H), 1.5-1.6 (m, 2H), 2.4-2.6 (m, 2H), 2.6-2.8 (m, 2H), 2.8-3.0 (m, 4H), 3.2-3.3 (m, 4H), 3.4-3.6 (m, 4H), 3.6-3.79 (m, 2H), 3.8 (s, 3H), 6.55-6.6 (m, 2H), 6.8-7.0 (m, 4H), 7.2-7.25 (d, 2H), 7.5 (m, 1H), 7.78 (d, 1H), 9.78 (d, 1H), 10.75 (s, 1H); **HPLC Purity:** 96.47 %; **Mass (M+1):** 593.31.

**N-(4-butylphenyl)-3-(4-(2-methoxyphenyl)piperazine-1-carbonyl)-4-(pyrrolidin-1-yl)benzenesulfonamide (IX-2)**

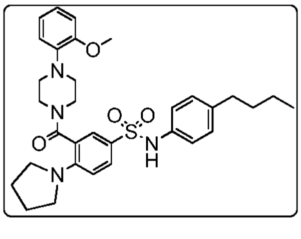

**1H NMR** (**400 MHz, DMSO-**$d_6$) δ**:** 1.0 (t, 3H), 1.2-1.4 (m, 2H), 1.5-1.6 (m, 2H), 1.62-1.7 (m, 4H), 2.4-2.6 (m, 2H), 2.6-2.8 (m, 2H), 2.8-3.0 (m, 4H), 3.2-3.3 (m, 2H), 3.4-3.6 (m, 4H), 3.8 (s, 3H), 6.55-6.6 (m, 2H), 6.8-7.0 (m, 4H), 7.2-7.25 (d, 2H), 7.5 (m, 1H), 7.78 (d, 1H), 9.78 (d, 1H), 10.75 (s, 1H); **HPLC Purity:** 96.20 %; **Mass (M+1):** 577.4.

## Scheme 10

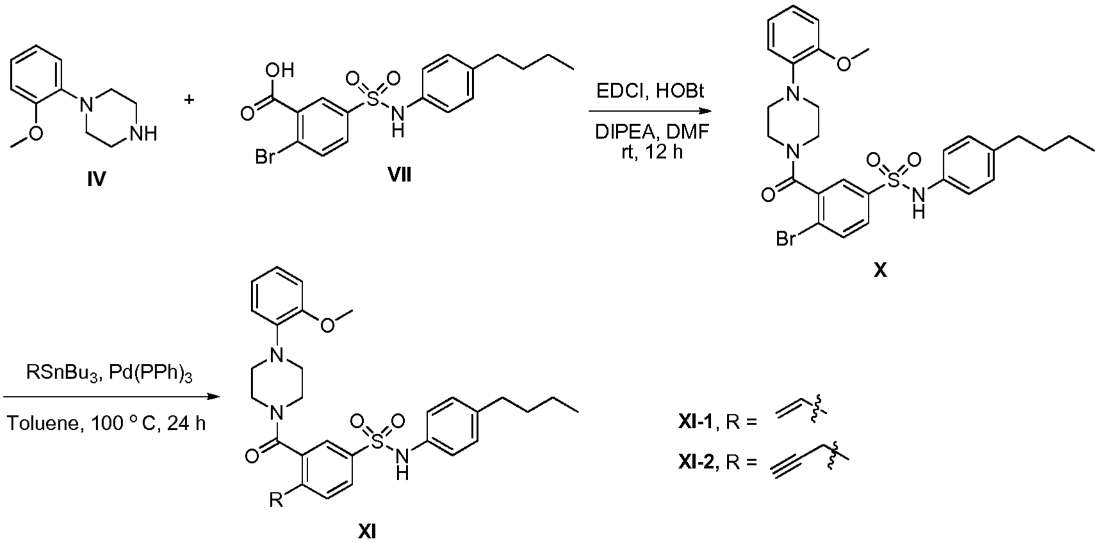


**Synthesis of 4-bromo-N-(4-butylphenyl)-3-(4-(2-methoxyphenyl)piperazine-1-carbonyl)benzenesulfonamide (X):**

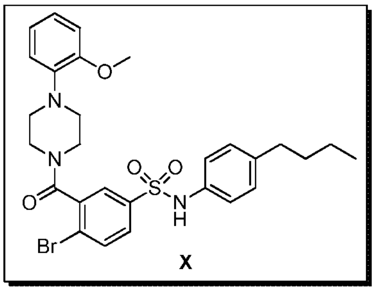

X

4-bromo-N-(4-butylphenyl)-3-(4-(2-methoxyphenyl) piperazine-1-carbonyl)benzenesulfonamide **X** was prepared by following similar method used for the preparation of compound **VII** (**Scheme-1**) using acid **VII** (0.325 mmol) and amine **IV** (0.325 mmol). Crude product was purified by column chromatography (60-120 silica gel, 70% Ethyl Acetate-Hexane) to afford the pure product **X** in 65% yields.

**General Procedure for the synthesis of compound (XI-1)-(XI-2):**

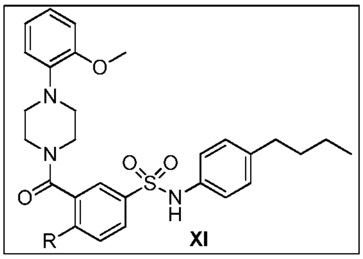

XI

To a stirred solution of compound **X** (0.1 g, 0.170 mmol) in Toluene (10 ml) at room temperature nitrogen gas was purged for 10 minutes. $Pd(PPh_3)_4$ (0.002 g, 0.0017 mmol) and Vinyl/propargyl tin (0.85 mmol) were added to the reaction mixture and stirred at 100°C overnight under nitrogen atmosphere. After completion of the reaction (monitored

by TLC), the reaction mixture was concentrated under vacuum. The residue was dissolved in water, extracted with ethyl acetate (3 x 50 ml). Combined organic extracts were washed with brine (20 ml), dried over anhydrous Sodium sulfate, filtered and concentrated under reduced pressure. The crude product was then purified by column chromatography (60-120 silica gel) using 2 % MeOH-DCM to afford compound **XI** in 60-65% yield.

**N-(4-butylphenyl)-3-(4-(2-methoxyphenyl)piperazine-1-carbonyl)-4-vinylbenzenesulfonamide (XI-1)**

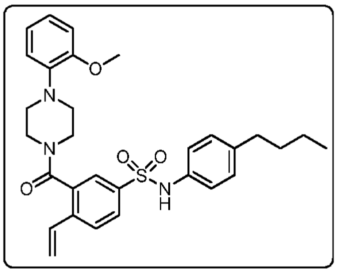

**$^1$H NMR** (**400 MHz, DMSO**-**$d_6$**) δ**:** 0.80-0.90 (t, 3H), 1.2-1.3 (m, 2H), 1.4-1.5 (m, 2H), 2.4-2.44 (t, 2H), 2.7-2.8 (m, 2H), 2.95-3.1 (m, 4H), 3.75-3.8 (m, 2H), 3.8 (s, 3H), 5.56-5.58 (d, 1H), 5.95-6.1 (d, 1H), 6.6-6.7 (m, 1H), 6.9-7.1 (m, 8H), 7.5-7.51 (m, 1H), 7.7-7.78 (m, 1H), 7.9-7.98 (m, 1H), 10.1 (s, 1H); **HPLC Purity:** 99.52%; **Mass (M+1):** 534.20.

**N-(4-butylphenyl)-3-(4-(2-methoxyphenyl)piperazine-1-carbonyl)-4-(prop-2-yn-1-yl)benzenesulfonamide (XI-2)**

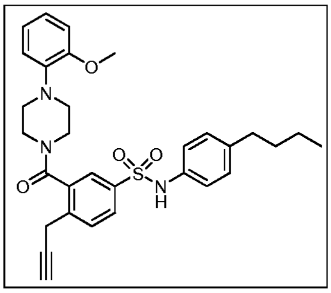

**$^1$H NMR** (**400 MHz, DMSO**-**$d_6$**) δ**:** 0.90-0.94 (t, 3H), 1.19-1.22 (m, 2H), 1.4-1.5 (m, 2H), 2.08-2.1 (s, 1H), 2.4-2.42 (t, 2H), 2.8-2.98 (m, 4H), 3.1 (s, 2H), 3.39-3.4 (m, 2H), 3.6-3.78 (m, 2H), 3.8 (s, 3H), 6.9-7.1 (m, 8H), 7.5-7.51 (m, 1H), 7.6-7.78 (m, 2H), 10.1 (s, 1H); **HPLC Purity:** 99.14%; **Mass (M+1):** 546.70.

**Scheme 11**

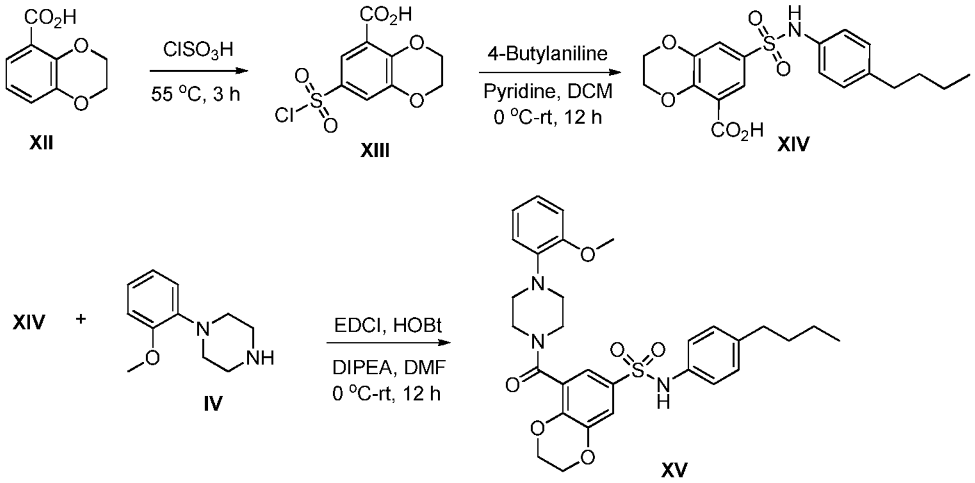


XII XIII XIV

XIV + IV

XV

**Synthesis of 7-(chlorosulfonyl)-2,3-dihydrobenzo[b][1,4]dioxine-5-carboxylic acid (XIII):**

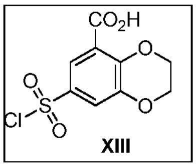

XIII

To a stirred solution of 2,3-dihydrobenzo[b][1,4]dioxine-5-carboxylic acid **XII** (0.5 g, 2.77 mmol) was added chlorosulphonic acid (1.2 ml, 16.6 mmol) at 0°C under nitrogen atmosphere and the resulting mixture was heated at 70°C for 3 h. After completion of reaction, the reaction mixture was cooled, quenched with ice and extracted with DCM. The organic layer was dried over $Na_2SO_4$ and evaporated under reduced pressure to afford product **XIII** in 75% yield.

**Synthesis of 7-(N-(4-butylphenyl)sulfamoyl)-2,3-dihydrobenzo[b][1,4]dioxine-5-carboxylic acid (XIV):**

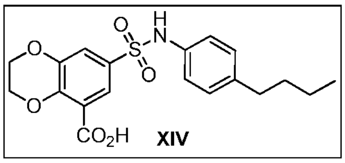

XIV

The compound **XIV** was prepared by following similar method used for the preparation of compound **VII** (**Scheme-1**) Sulfonyl chloride **XIII** (1.07 mmol) and 4-butylaniline (1.18 mmol). Crude product was purified by column chromatography (60-120 silica gel, 30% Ethyl Acetate-Hexane) to afford the pure product **XIV** in 45% yields.

**Synthesis of N-(4-butylphenyl)-8-(4-(2-methoxyphenyl)piperazine-1-carbonyl)-2,3-dihydrobenzo[b][1,4]dioxine-6-sulfonamide (XV):**

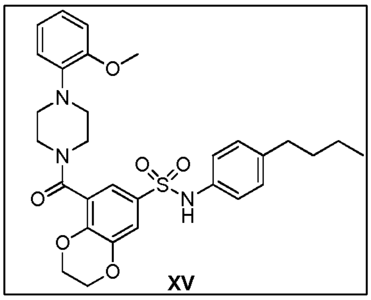

XV

Compound **XV** was prepared by following similar method as described for the preparation of compound **VII** (**Scheme-1**) using acid **XIV** (0.150 g, 0.382 mmol) and amine **IV** (0.081 g, 0.421 mmol). Crude product was purified by column chromatography (60-120 silica gel, 70% Ethyl Acetate-Hexane) to afford the pure product **XV** in 45% yields. **$^1$H NMR (400 MHz, DMSO-d$_6$) δ:** 0.9-0.95 (t, 3H), 1.2-1.3 (m, 2H), 1.4-1.5 (m, 2H), 2.4-2.42 (t, 2H), 2.7-3.0 (m, 4H), 3.1-3.2 (m, 2H), 3.6-3.7 (m, 2H), 3.8 (s, 3H), 4.2-4.4 (m, 4H), 6.9-7.15 (m, 9H), 7.2-7.21 (m, 1H), 10.0 (s, 1H); **HPLC Purity:** 97.61%; **Mass (M+1)**: 566.59.

## Scheme 12

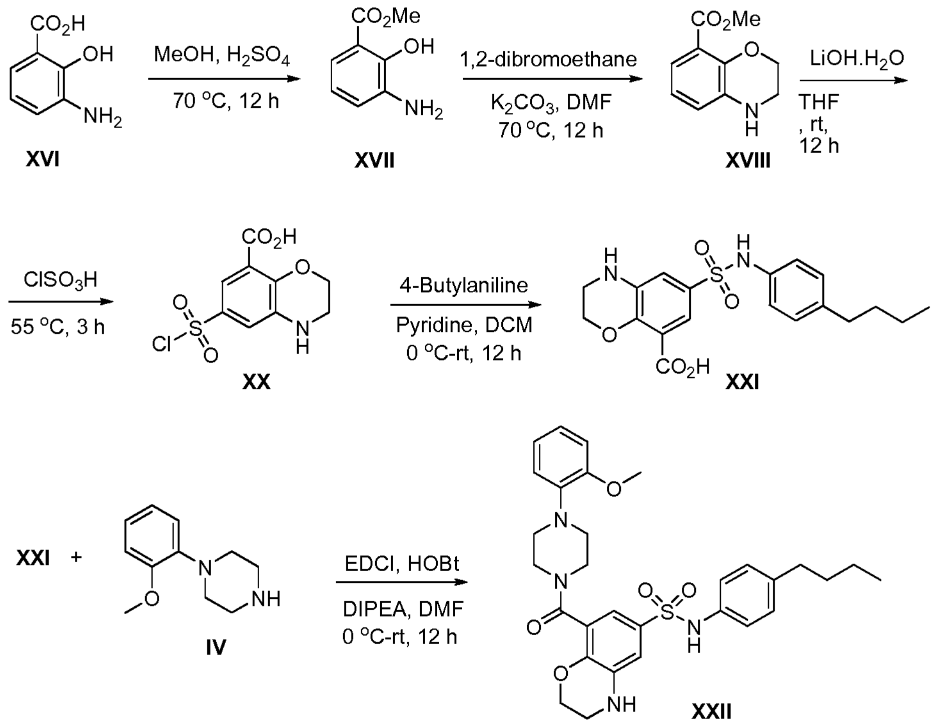


XVI XVII XVIII XIX XX XXI XXI + IV XXII

**Synthesis of methyl 3-amino-2-hydroxybenzoate (XVII):**

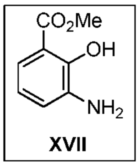

XVII

To a stirred solution of 3-amino-2-hydroxybenzoic acid **XII** (0.5 g, 3.26 mmol) in MeOH (15 ml) was added sulfuric acid (1 ml) at 0°C under nitrogen atmosphere and the resulting mixture was heated at 70°C for 12 h. After completion of reaction, the reaction mixture was cooled, concentrated, washed with saturated $NaHCO_3$ solution and

extracted with ethyl acetate. The organic layer was dried over $Na_2SO_4$ and evaporated under reduced pressure to afford product **XVII** in 45% yield.

**Synthesis of methyl 3,4-dihydro-2H-benzo[b][1,4]oxazine-8-carboxylate (XVIII):**

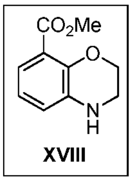

**XVIII**

To a stirred solution of methyl 3-amino-2-hydroxybenzoate **XVII** (0.15 g, 0.892 mmol) in DMF (20 ml) was added $K_2CO_3$ (0.308 g, 2.23 mmol) followed by 1,2-dibromoethane (1.2 ml, 16.6 mmol) at 0°C under nitrogen atmosphere and the resulting mixture was heated at 75°C for 16 h. After completion of reaction, the reaction mixture was cooled, added water and extracted with ethyl acetate. The organic layer was dried over $Na_2SO_4$ and evaporated under reduced pressure. The crude product was then purified by column chromatography (60-120 silica gel) using 40 % EtOAc-Hexane to afford compound **XVIII** in 45% yield.

**Synthesis of 3,4-dihydro-2H-benzo[b][1,4]oxazine-8-carboxylic acid (XIX):**

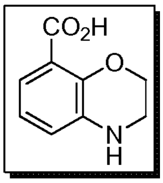

To a stirred solution of compound **XVIII** (0.1 g, 0.514 mmol) in THF-MeOH (6:2 ml) was added $LiOH.H_2O$ (0.082 g, 2.06 mmol) followed by 1,2-dibromoethane (1.2 ml, 16.6 mmol) at room temperature and the resulting mixture was stirred for 16 h. After completion of reaction, the solvent was removed under reduced pressure, added water and extracted with ethyl acetate. The organic layer was dried over $Na_2SO_4$ and evaporated under reduced pressure to afford product **XIX** which was used for the next step without further purification.

**Synthesis of 6-(chlorosulfonyl)-3,4-dihydro-2H-benzo[b][1,4]oxazine-8-carboxylic acid (XX):**

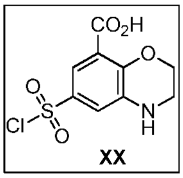

**XX**

Compound **XX** was prepared by following similar method described for the preparation of compound **XIII** (**Scheme-3**) using acid **XIX** (0.67 mmol) and chlorosulfonic acid (0.4 ml, 6.7 mmol) in 81% yield. Crude product was used as such for the next step without further purification.

**Synthesis of 6-(N-(4-butylphenyl)sulfamoyl)-3,4-dihydro-2H-benzo[b][1,4]oxazine-8-carboxylic acid (XXI):**

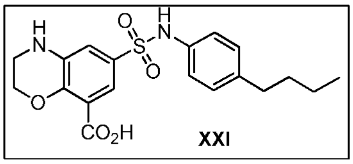

XXI

Compound **XXI** was prepared by following similar method described for the preparation of compound **XIV** (**Scheme-3**) using sulfonyl chloride **XX** (0.16 g, 0.577 mmol) and 4-butylaniline (0.072 g, 0.481 mmol) in 70% yield which was used for the next step without further purification.

**Synthesis of N-(4-butylphenyl)-8-(4-(2-methoxyphenyl)piperazine-1-carbonyl)-3,4-dihydro-2H-benzo[b][1,4]oxazine-6-sulfonamide (XXII):**

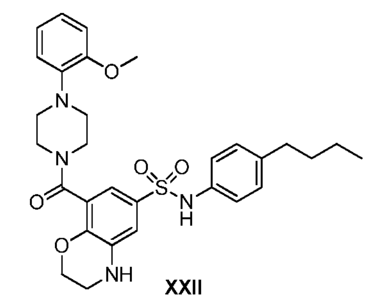

XXII

Compound **XXII** was prepared by following similar method described for the preparation of compound **VII** (**Scheme-1**) using acid **XX** (0.130 g, 0.333 mmol) and amine **IV** (0.070 g, 0.366 mmol). Crude product was purified by column chromatography (60-120 silica gel, 50% Ethyl Acetate-Hexane) to afford the pure product **XXII** in 35% yield. **$^1$H NMR (400 MHz, DMSO-$d_6$) δ:** 0.80-0.90 (t, 3H), 1.2-1.28 (m, 2H), 1.4-1.5 (m, 2H), 2.4-2.44 (t, 2H), 2.6-3.0 (m, 4H), 3.1-3.2 (m, 4H), 3.5-3.62 (m, 2H), 3.8 (s, 3H), 4.18-4.2 (m, 2H), 6.5-6.51 (m, 1H), 6.68-6.7 (m, 1H), 6.9-7.1 (m, 8H), 9.9 (s, 1H); **HPLC Purity:** 98.65%; **Mass (M+1):** 565.67.

**Scheme 13**

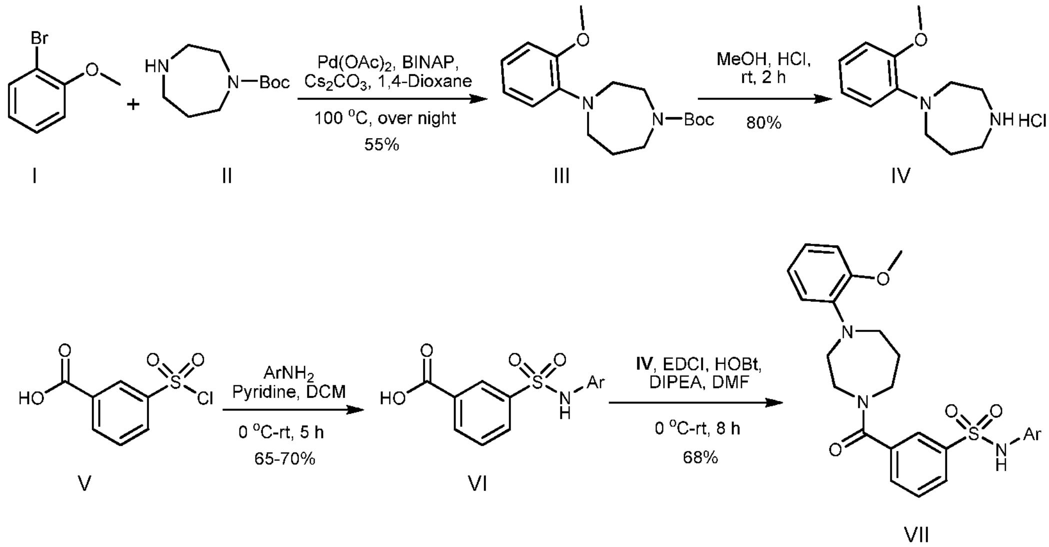


VII

Where Ar = Substituted aryl

**Procedure for synthesis of tert-butyl4-(2-methoxyphenyl)-1,4-diazepane-1-carboxylate (III):**

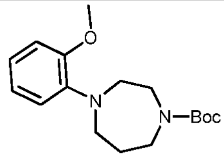

III

Nitrogen was purged through a stirred solution of 2-bromo anisole (**I**, 2.5 gm, 13.3 mmol) in 1,4-dioxane (30 mL) at room temperature for 30 minutes. BINAP (0.83 gm, 1.33 mmol), palladium acetate (0.058 gm, 0.26 mmol) and cesium carbonate (1.1 gm, 3.3 39.9 mmol) were added to the reaction mixture and the nitrogen purging was continued for another 20 minutes. Finally N-Boc homopiperazine (**II**, 2.7 gm, 13.3 mmol) was added and stirred at 100°C overnight under nitrogen atmosphere. After completion of the reaction (monitored by TLC), the reaction mixture was concentrated under vacuum. The residue was dissolved in water and extracted with ethyl acetate (3 x 50 mL). Combined organic extracts were washed with brine (20 mL), dried over anhydrous Sodium sulfate, filtered and concentrated under reduced pressure. The crude product was then purified by column chromatography (60-120 Silica gel) using 10 % ethyl acetate-hexane to afford product (III) (2.3 gm, 55% yield).

**Procedure for synthesis of 1-(2-methoxyphenyl)-1,4-diazepane HCl (IV):**

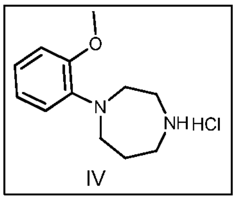

IV

tert-butyl 4-(2-methoxyphenyl)-1,4-diazepane-1-carboxylate (**III**, 2.2 gm, 7.18 mmol) was added methanolic-HCl (20 mL, 5%) which resulted in formation of a homogeneous solution and was stirred for 2 h at room temperature. After completion of the reaction (monitored by TLC), the solvent was removed under vacuum. The crude product was washed with ethyl acetate repeatedly and then dried well to afford product (**IV**) (1.3 gm, 85% yields) as a white solid.

**General procedure for synthesis of sulfonamide (VI):**

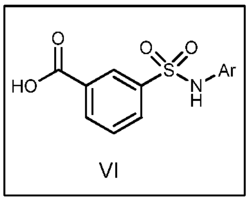

VI

To a solution of appropriate amine (0.7 mmol) in a 1:1 mixture of DCM-pyridine (8 mL) was added 3-chlorosulfonyl benzoic acid (**V**, 0.17 gm, 0.77 mmol) under nitrogen atmosphere. The resultant solution was stirred overnight at room temperature. On completion of the reaction (monitored by TLC), the reaction mixture was diluted with dichloromethane (50 mL), washed with water (2 x 10 mL), 1N HCl solution (2 x 10 mL) and brine (10 mL). The combined organic extracts were dried over anhydrous sodium sulfate, filtered and concentrated under vacuum. Crude product was co-distilled with toluene to remove the remnants of pyridine and dried to yield sulfonamide (**VI**) (65-70% yields) as an off-white solid and was used as such for the next step without further purification.

**General procedure for synthesis of sulfonamide (VII-1)-(VII-2):**

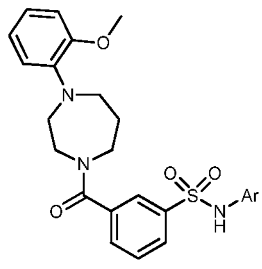

VII

To a stirred solution of the carboxylic acid (**VI**, 0.25 mmol) in DMF at 0°C under nitrogen atmosphere, EDCI (0.54 gm, 0.28 mmol), HOBt (0.38 gm, 0.28 mmol) and DIPEA (0.13 mL, 0.75 mmol) were added and the resultant solution was stirred at room temperature for 30 minutes. Amine hydrochloride (**IV**, 0.25 mmol) was then added at 0°C and stirred overnight at room temperature. After completion of the reaction (monitored by TLC), the reaction mixture was poured into 1.0 M HCl and extracted with EtOAc (3 x 25 mL). The organic layer was washed with saturated $NaHCO_3$ solution (10 mL), dried over anhydrous $Na_2SO_4$ and filtered. The solvent was removed by rotary evaporation and the product was isolated by column chromatography on silica gel (60-120 silica gel, 2% MeOH-DCM) or preparative HPLC to yield amide **(VII-1)-(VII-2)** (60-68% yields) as an off-white solid.

**N-(4-isopropylphenyl)-3-(4-(2-methoxyphenyl)-1,4-diazepane-1 carbonyl)benzene sulfonamide (VII-1):**

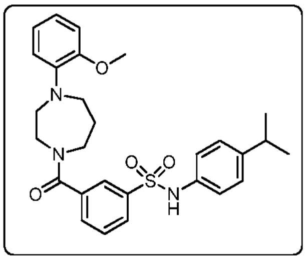

**$^1$H NMR** (**400 MHz, DMSO**-**$d_6$**) δ**:** 1.1 (d, 6H), 1.5-1.7 (m, 1H), 1.9-2.0 (m, 1H), 2.7-2.8 (m, 1H), 3.1-3.4 (m, 4H), 3.4 (s, 3H), 3.7-3.9 (m, 4H), 6.8-7.15 (m, 6H), 7.2-7.3 (m, 2H), 7.5-7.7 (m, 3H), 7.8-7.9 (m, 1H), 10.1 (s, 1H); **HPLC Purity:** 99.78%; **Mass (M+1):** 508.20.

**N-(2-fluorophenyl)-3-(4-(2-methoxyphenyl)-1,4-diazepane-1-carbonyl)benzenesulfonamide (VII-2):**

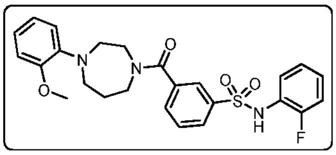

**$^1$H NMR** (**400 MHz, DMSO**-**$d_6$**) δ**:** 1.6-1.7 (m, 1H), 1.9-2.0 (m, 1H), 3.1-3.4 (m, 6H), 3.59-3.6 (m, 2H), 3.8 (s, 3H), 6.8-7.2 (m, 4H), 7.3-7.5 (m, 5H), 7.6-7.8 (m, 2H), 7.8-8.0 (m, 1H), 10.2 (s, 1H); **HPLC Purity:** 91.28 %; **Mass (M+1):** 484.17.

## Scheme 14

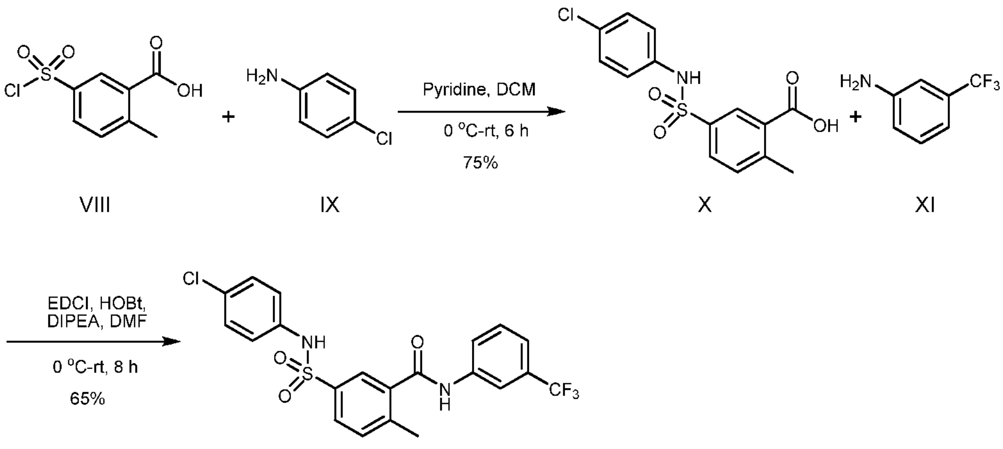


VIII IX X XI

XII

**Procedure for synthesis of 5-(N-(4-chlorophenyl)sulfamoyl)-2-methylbenzoic acid (X):**

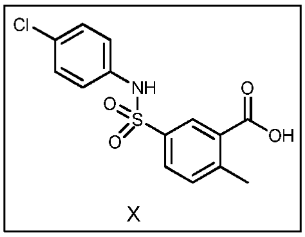

X

The sulfonamide **X** was prepared by following the similar method as followed for compound **VI** in scheme 1 using carboxylic acid **VIII** (0.1 gm, 0.42 mmol) and 4-chloroaniline (0.054 gm, 0.42 mmol) in (0.104 gm) 75% yield.

**Procedure for synthesis of 5-(N-(4-chlorophenyl)sulfamoyl)-2-methyl-N-(3-(trifluoromethyl) phenyl)benzamide (XII):**

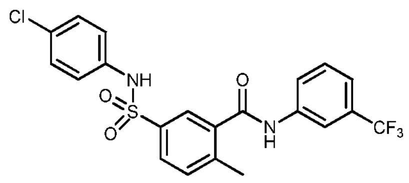

XII

The sulfonamide **XII** was prepared by following the similar method as followed for compound **VII** in scheme 1 using carboxylic acid **X** (0.08 gm, 0.25 mmol) and 3-(trifluoromethyl)aniline (0.040 gm, 0.25 mmol) in (0.075 gm) 65% yield. **$^1$H NMR** (**400 MHz, $CDCl_3$**) δ: 2.2 (s, 3H), 6.69 (s, 1H), 6.8-7.2 (m, 6H), 7.3-7.8 (m, 4H), 8.2 (d, 1H); **HPLC Purity:** 95.18 %; **Mass (M+1):** 469.3.

## Scheme 15

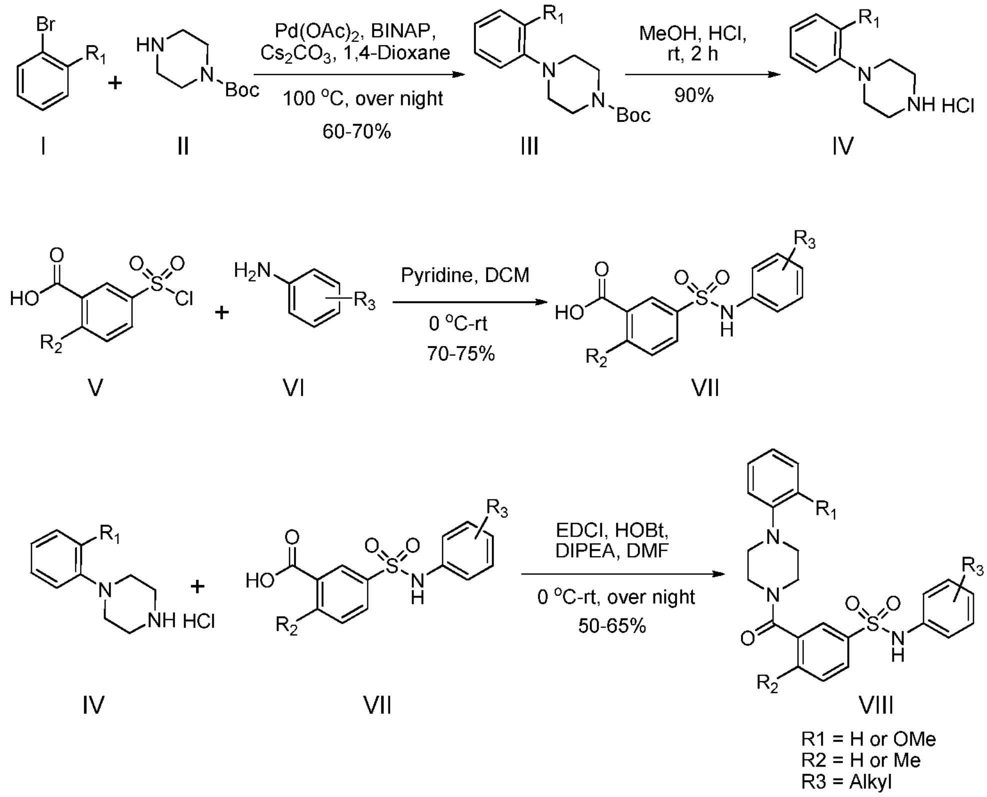


**General procedure for synthesis of *N⁴*-aryl-tert-butyl piperazine-*N¹*-carboxylate (III):**

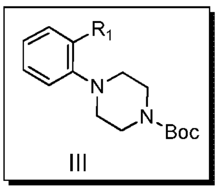

Nitrogen was purged through a stirred solution of aryl bromide (**I**, 1.1 mmol) in 1,4-dioxane (10 mL) at room temperature for 30 minutes. BINAP (0.069 gm, 0.11 mmol), palladium acetate (0.005 gm, 0.022 mmol) and cesium carbonate (1.1 gm, 3.3 mmol) were added to the reaction mixture and the nitrogen purging was continued for another 20 minutes. Finally, N-Boc piperazine (**II**, 0.204 gm, 1.1 mmol) was added and stirred at 100°C overnight under nitrogen atmosphere. After completion of the reaction (monitored by TLC), the reaction mixture was concentrated under vacuum. The residue was dissolved in water, extracted with ethyl acetate (3 x 50 mL). Combined organic extracts were washed with brine (20 mL), dried over anhydrous Sodium sulfate, filtered and concentrated under reduced pressure. The crude product was then purified by column chromatography (60-120 Silica gel) using 10 % ethyl acetate-hexane to afford product (III) (60-70% yield).

**General procedure for synthesis of *N¹*-aryl-piperazine HCl (III):**

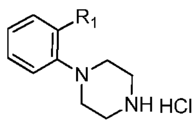

IV

$N^1$- Boc-$N^4$-arylpiperazine (**III**, 0.68 mmol) was added methanolic-HCl (10 mL, 5%) which resulted in formation of a homogeneous solution and was stirred for 2 h at room temperature. After completion of the reaction (monitored by TLC), the solvent was removed under vacuum. The crude product was washed with ethyl acetate repeatedly and then dried well to afford product (**IV**) (90% yields) as a white solid.

**General procedure for synthesis of sulfonamide (VII):**

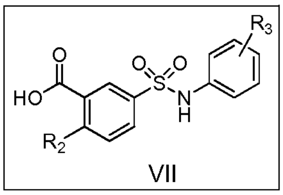

VII

To a solution of amine (**VI**, 0.5 mmol) in a 1:1 mixture of DCM-pyridine (10 mL) was added aryl sulfonyl chloride (**V**, 0.55 mmol) under nitrogen atmosphere. The resultant solution was stirred overnight at room temperature. On completion of the reaction (monitored by TLC), the reaction mixture was diluted with dichloromethane (50 mL), washed with water (2 x 10 mL), 1N HCl solution (2 x 10 mL) and brine (10 mL). The combined organic extracts were dried over anhydrous sodium sulfate, filtered and concentrated under vacuum. Crude product was co-distilled with toluene to remove the remnants of pyridine and dried to yield sulfonamide (**VII**) (70-75% yields) as an off-white solid and was used as such for the next step without further purification.

**General procedure for synthesis sulfonamide (VIII-1)-(VIII-9):**

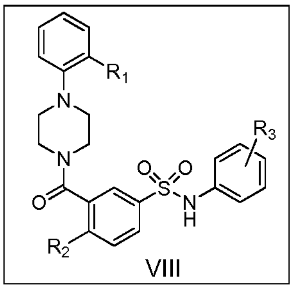

VIII

To a stirred solution of the carboxylic acid (**VII**, 0.25 mmol) in DMF at 0°C under nitrogen atmosphere, EDCI (0.54 gm, 0.28 mmol), HOBt (0.38 gm, 0.28 mmol) and DIPEA (0.13 mL, 0.75 mmol) were added and the resultant solution was stirred at room temperature for 30 minutes. Amine hydrochloride (**III**, 0.25 mmol) was then added at 0°C and stirred overnight at room temperature. After completion of the reaction (monitored by TLC), the reaction mixture was poured into 1.0 M HCl and extracted with EtOAc (3 x 25 mL). The organic layer was washed with saturated $NaHCO_3$ solution (10 mL), dried over anhydrous $Na_2SO_4$ and filtered. The solvent was removed by rotary evaporation and the product was isolated by column chromatography on silica gel (60-120 silica gel, 2% MeOH-DCM) or preparative HPLC to yield amide **(VIII-1)-(VIII-9)** (50-65% yields) as an off-white solid.

**3-(4-phenylpiperazine-1-carbonyl)-N-(4-propylphenyl)benzenesulfonamide (VIII-1):**

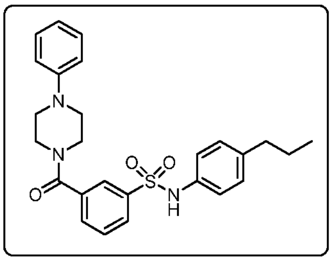

**$^1$H NMR** (**400 MHz, DMSO**-$d_6$) δ**:** 0.8 (t, 3H), 1.3-1.4 (m, 2H), 1.2-1.3 (t, 2H), 3.0-3.1 (m, 2H), 3.2-3.3 (m, 4H), 3.7-3.9 (m, 2H), 6.8-7.0 (m, 4H), 7.1-7.3 (m, 5H), 7.6-7.8 (m, 4H), 9.7 (s, 1H); **HPLC Purity:** 97.81%; **Mass (M+1):** 464.35.

**N-(4-(tert-butyl)phenyl)-3-(4-phenylpiperazine-1-carbonyl)benzenesulfonamide (VIII-2):**

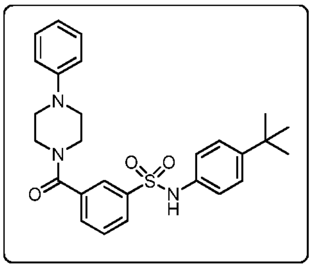

**$^1$H NMR** (**400 MHz, DMSO**-$d_6$) δ**:** 1.2 (s, 9H), 3.0-3.1 (m, 2H), 3.2-3.2.2 (m, 4H), 3.7-3.8 (m, 2H), 6.8-7.15 (m, 5H), 7.21-7.3 (m, 4H), 7.6-7.7 (m, 3H), 7.8-7.9 (m, 1H), 10.2 (s, 1H); **HPLC Purity:** 93.40%; **Mass (M+1):** 478.30.

**N-(4-(tert-butyl)phenyl)-3-(4-(2-methoxyphenyl)piperazine-1-carbonyl)benzenesulfonamide (VIII-3):**

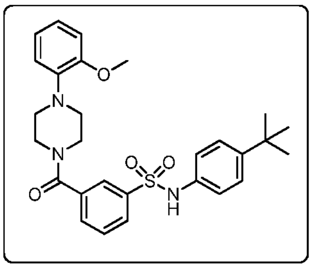

**$^1$H NMR** (**400 MHz, DMSO**-$d_6$) δ**:** 1.2 (s, 9H), 2.8-3.0 (m, 4H), 3.2-3.2.2 (m, 2H), 3.8 (s, 3H), 4.1-4.15 (m, 2H), 6.95-7.15 (m, 6H), 7.21-7.3 (m, 2H), 7.6-7.7 (m, 3H), 7.8-7.9 (m, 1H), 10.2 (s, 1H); **HPLC Purity:** 96.26%; **Mass (M+1):** 508.35.

**3-(4-(2-methoxyphenyl)piperazine-1-carbonyl)-4-methyl-N-(p-tolyl)benzenesulfonamide (VIII-4):**

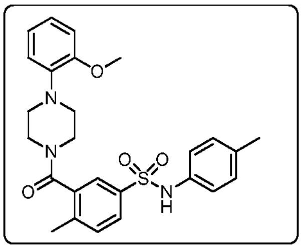

**$^1$H NMR** (**400 MHz, $CDCl_3$**) **δ:** 2.2 (s, 3H), 2.39 (s, 3H), 3.1-3.1 (m, 2H), 3.3-3.4 (m, 4H), 3.9 (s, 3H), 3.95-4.1 (m, 2H), 6.9-7.0 (m, 6H), 7.1-7.3 (m, 3H), 7.59-7.60 (m, 2H); **HPLC Purity:** 96.94%; **Mass (M+1):** 480.29.

**N-(4-ethylphenyl)-3-(4-(2-methoxyphenyl)piperazine-1-carbonyl)-4-methylbenzenesulfonamide (VIII-5):**

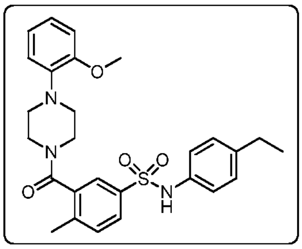

**$^1$H NMR** (**400 MHz, $CDCl_3$**) **δ:** 1.19-1.2 (t, 3H), 2.4 (s, 3H), 2.5-2.6 (q, 2H), 2.9-2.97 (m, 2H), 3.1-3.3 (m, 4H), 3.9 (s, 3H), 3.95-4.0 (m, 2H), 6.9-7.0 (m, 6H), 7.0-7.1 (m, 3H), 7.6-7.61 (m, 2H); **HPLC Purity:** 95.38%; **Mass (M+1):** 494.26.

**3-(4-(2-methoxyphenyl)piperazine-1-carbonyl)-4-methyl-N-(4-propylphenyl)benzenesulfonamide (VIII-6):**

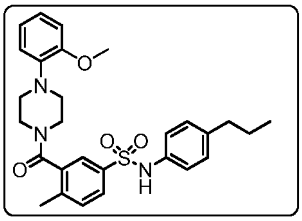

**$^1$H NMR** (**400 MHz, DMSO-$d_6$**) δ: 0.9 (t, 3H), 1.4-1.6 (m, 2H), 2.21 (s, 3H), 2.4 (t, 2H), 2.6-2.7 (m, 2H), 2.99-3.2 (m, 4H), 3.6-3.79 (m, 2H), 3.8 (s, 3H), 6.8-7.2 (m, 5H), 7.3-7.8 (m, 5H), 8.2 (bs, 1H) 10.2 (s, 1H); **HPLC Purity:** 99.41 %; **Mass (M+1):** 508.22.

**N-(4-isopropylphenyl)-4-methyl-3-(4-phenylpiperazine-1-carbonyl)benzenesulfonamide (VIII-7):**

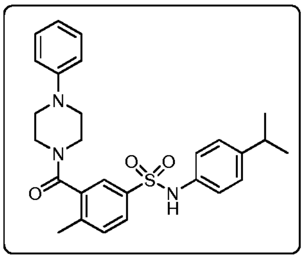

**$^1$H NMR** (**400 MHz, DMSO**-$d_6$) δ**:** 1.1 (d, 6H), 1.7-1.8 (m, 1H), 2.3 (s, 3H), 2.7-2.9 (m, 2H), 2.9-3.1 (m, 4H), 3.7-3.9 (m, 2H), 6.9-7.3 (m, 9H), 7.4-7.5 (m, 2H), 7.7-7.8 (m, 1H), 10.1 (s, 1H); **HPLC Purity:** 93.98%; **Mass (M+1):** 478.35.

**N-(4-(tert-butyl)phenyl)-3-(4-(2-methoxyphenyl)piperazine-1-carbonyl)-4-methylbenzenesulfonamide (VIII-8):**

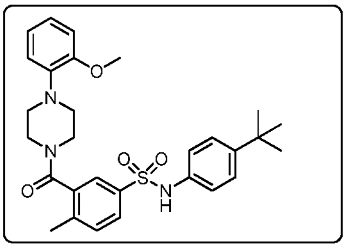

**$^1$H NMR** (**400 MHz, $CD_3OD$)** δ**:** 1.2 (s, 9H), 2.38 (s, 3H), 2.8-2.9 (m, 2H), 3.1-3.2 (m, 4H), 3.82 (s, 3H), 3.95-4.0 (m, 2H), 6.9-7.15 (m, 6H), 7.2-7.21 (m, 2H), 7.42-7.59 (m, 2H), 7.78-7.8 (m, 1H); **HPLC Purity:** 96.55%; **Mass (M+1):** 522.50.

**3-(4-(2-methoxyphenyl)piperazine-1-carbonyl)-4-methyl-N-(4-phenethylphenyl)benzenesulfonamide (VIII-9):**

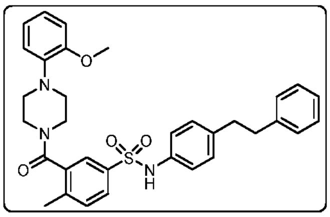

**1H NMR** (**400 MHz, DMSO**-$d_6$) δ**:** 1.1-1.5 (m, 2H), 1.4-1.6 (m, 2H), 2.1 (s, 3H), 2.69-3.3 (m, 6H), 3.6-3.8 (m, 2H), 3.81-4.0 (s, 3H), 6.8-7.2 (m, 7H), 7.3-7.5 (m, 5H), 7.6-7.8 (m, 2H), 7.8-8.0 (m, 2H), 10.2 (s, 1H); **HPLC Purity:** 98.95 %; **Mass (M+1):** 570.35.

## Scheme 16

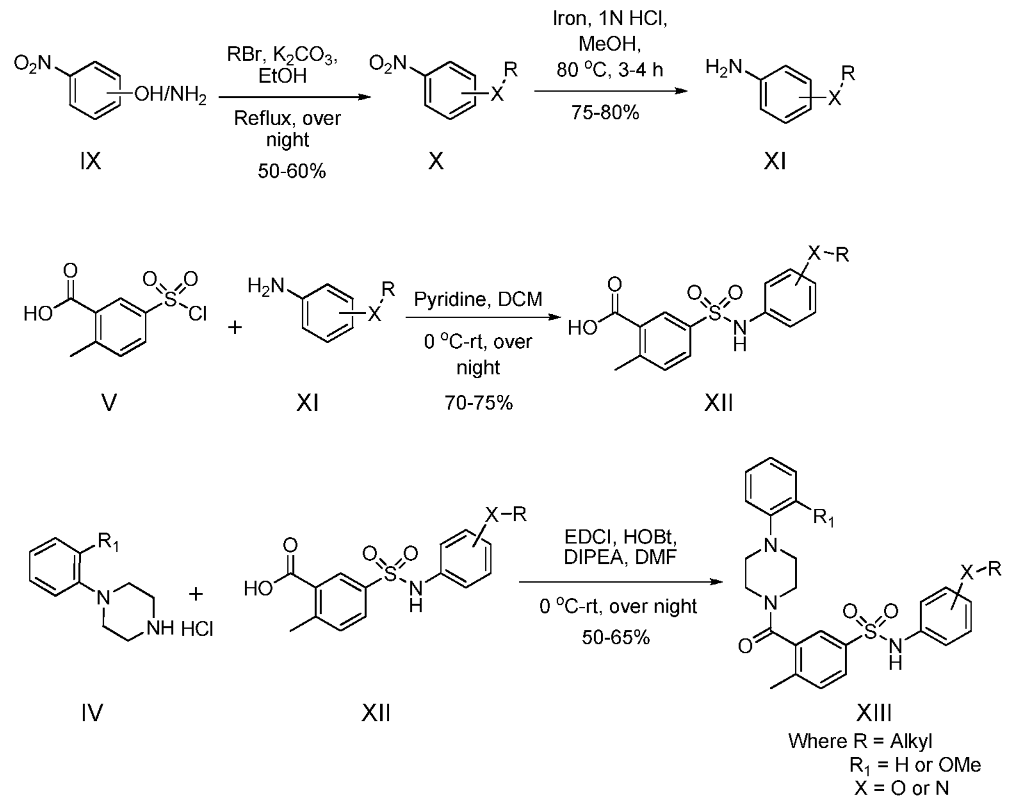


**General procedure for synthesis of nitrobenzene derivatives (X):**

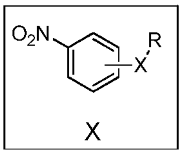

X

A solution of compound **IX** (3.6 mmol) in ethanol (15 mL), appropriate alkyl bromide (10.8 mmol when X=O & 3.6 mmol when X=N) and potassium carbonate (10.8 mmol) were added. The mixture was then refluxed over night. After completion of reaction (monitored by TLC), the mixture evaporated in vacuo, and the residue was diluted with ethyl acetate (100 mL), washed with water (2 x 20 mL), brine (20 mL), dried over anhydrous $Na_2SO_4$ and concentrated in vacuo. The crude product was purified by column chromatography (60:120 Silica gel, 5% ethylacetate: hexane) to afford product X (50-60% yields).

**General procedure for synthesis of aniline derivatives (XI):**

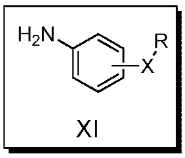

XI

To a solution of compound **X** (1.9 mmol) in methanol (20 mL), iron powder (9.5 mmol) and 1 N HCl solution

(0.5 mL) were added, and then the mixture was refluxed over night. After completion of reaction, the mixture was filtered through celite and the celite was washed with methanol (10 mL). The combined filtrates were evaporated to afford compound **XI** (75-80%). The material was used in the next step without purification.

**General procedure for synthesis of sulfonamide (XII):**

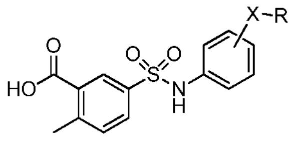

XII

Sulfonamides (**XII**) were prepared by following the similar method as described for sulfonamide **VII** in scheme 1 using amine **XI** (1.5 mmol) and 2-methyl-5-chlorosulfonyl benzoic acid (1.5 mmol) to sulfonamide XII (70-75%).

**General procedure for synthesis sulfonamide (XIII-1)-(XIII-13):**

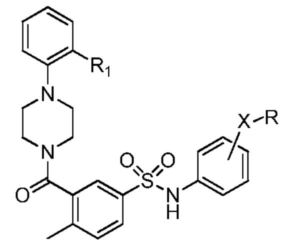

XIII

Sulfonamides **(XIII-1)-(XIII-13)** were prepared by following the similar method as described for sulfonamide **VIII** in scheme 1 using carboxylic acid **XII** (0.3 mmol) and amine HCl **(IV)** (0.3 mmol) in 50-65% yields.

**N-(4-methoxyphenyl)-3-(4-(2-methoxyphenyl)piperazine-1-carbonyl)-4-methylbenzenesulfonamide (XIII-1):**

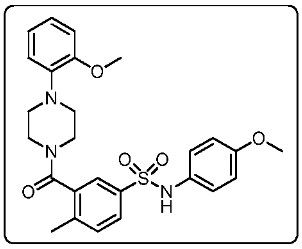

**$^1$H NMR (400 MHz, $CDCl_3$)** $\delta$: 2.4 (s, 3H), 2.84-2.9 (m, 2H), 3.0-3.19 (m, 2H), 3.2-3.26 (m, 2H), 3.7 (s, 3H), 3.86 (s, 3H), 3.95-4.0 (m, 2H), 6.78-7.1 (m, 8H), 7.2-7.4 (m, 1H), 7.58-7.60 (m, 2H); **HPLC Purity:** 94.54%; **Mass (M+1):** 496.18.

**N-(4-ethoxyphenyl)-3-(4-(2-methoxyphenyl)piperazine-1-carbonyl)-4-methylbenzenesulfonamide (XIII-2):**

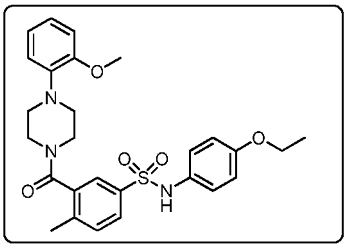

**$^1$H NMR (400 MHz, $CD_3OD$) δ:** 1.19-1.21 (t, 3H), 2.38 (s, 3H), 2.8-2.9 (m, 2H), 3.1-3.2 (m, 4H), 3.8-3.9 (m, 2H), 3.91 (s, 3H), 3.95-4.0 (m, 2H), 6.78-7.15 (m, 8H), 7.39-7.44 (m, 2H), 7.7-7.8 (m, 1H); **HPLC Purity:** 98.83%; **Mass (M+1):** 510.40.

**3-(4-(2-methoxyphenyl)piperazine-1-carbonyl)-4-methyl-N-(4-propoxyphenyl)benzenesulfonamide (XIII-3):**

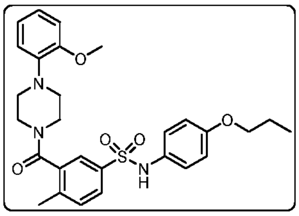

**$^1$H NMR (400 MHz, $CDCl_3$) δ:** 1.0 (t, 3H), 1.69-1.7 (m, 2H), 2.4 (s, 3H), 2.8-3.4 (m, 6H), 3.78-3.79 (m, 2H), 3.8 (s, 3H), 3.9-4.0 (m, 2H), 6.4 (m, 1H), 6.9-7.25 (m, 8H), 7.2-7.4 (m, 1H), 7.5-7.6 (m, 2H) ; **HPLC Purity:** 98.31 %; **Mass (M+1):** 524.27.

**N-(4-butoxyphenyl)-3-(4-(2-methoxyphenyl)piperazine-1-carbonyl)-4-methylbenzenesulfonamide (XIII-4):**

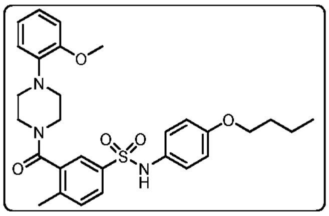

**$^1$H NMR (400 MHz, $CDCl_3$) δ:** 1.0 (t, 3H), 1.69-1.7 (m, 2H), 1.78-1.8 (m, 2H), 2.4 (s, 3H), 2.8-3.4 (m, 6H), 3.78-3.79 (m, 2H), 3.8 (s, 3H), 3.9-4.0 (m, 2H), 6.4 (m, 1H), 6.9-7.25 (m, 8H), 7.2-7.4 (m, 1H), 7.5-7.6 (m, 2H) ; **HPLC Purity:** 97.47 %; **Mass (M+1):** 538.28.

**N-(4-butoxy-2,3-dimethylphenyl)-3-(4-(2-methoxyphenyl)piperazine-1-carbonyl)-4-methylbenzene sulfonamide (XIII-5):**

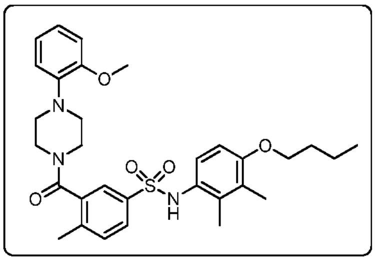

**$^1$H NMR (400 MHz, $CDCl_3$) δ:** 0.9-1.0 (t, 3H), 1.4-1.5 (m, 2H), 1.7-1.78 (m, 2H), 2.0 (s, 3H), 2.1 (s, 3H), 2.4 (s, 3H), 2.81-2.95 (m, 2H), 3.1-3.2 (m, 2H), 3.21-2.3 (m, 2H), 3.82 (s, 3H), 3.8-3.9 (t, 2H), 3.91-4.0 (m, 2H), 6.8-7.15 (m, 6H), 7.2-7.38 (m, 1H), 7.59-7.61 (m, 2H); **HPLC Purity:** 97.27%; **Mass (M+1):** 566.33.

**N-(4-isopropoxyphenyl)-3-(4-(2-methoxyphenyl)piperazine-1-carbonyl)-4-methylbenzenesulfonamide (XIII-6):**

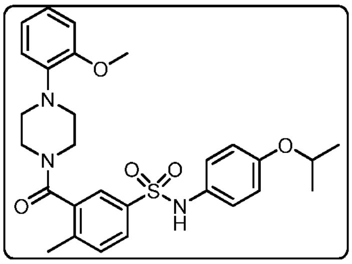

**$^1$H NMR (400 MHz, $CDCl_3$) δ:** 0.9 (d, 6H), 2.4 (s, 3H), 2.9-3.1 (m, 4H), 3.2-3.4 (m, 2H), 3.8 (s, 3H), 3.82-4.0 (m, 2H), 4.5-4.56 (m, 1H), 6.3 (m, 1H), 6.7-7.1 (m, 8H), 7.2-7.3 (m, 1H), 7.5-7.6 (m, 2H); **HPLC Purity:** 95.95 %; **Mass (M+1):** 524.45.

**N-(4-(cyclopropylmethoxy)phenyl)-3-(4-(2-methoxyphenyl)piperazine-1-carbonyl)-4-methyl benzenesulfonamide (XIII-7):**

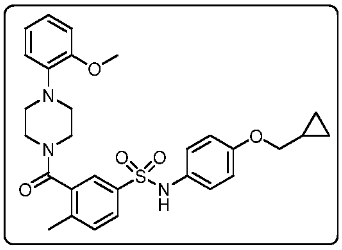

**$^1$H NMR (400 MHz, $CDCl_3$) δ:** 0.3-0.31, (m 2H), 0.59-0.61 (m, 2H), 1.19-1.21 (m, 1H), 2.2 (s, 3H), 2.8-3.1 (m, 4H), 3.2-3.3 (m, 2H), 3.5-3.53 (d, 2H), 3.9 (s, 3H), 3.95-4.1 (m, 2H), 6.78-7.1 (m, 8H), 7.2-7.3 (m, 1H), 7.5-7.6 (m, 2H); **HPLC Purity:** 97.69%; **Mass (M+1):** 536.45.

**N-(4-(methoxymethoxy)phenyl)-4-methyl-3-(4-o-tolylpiperazine-1-carbonyl)benzenesulfonamide (XIII-8):**

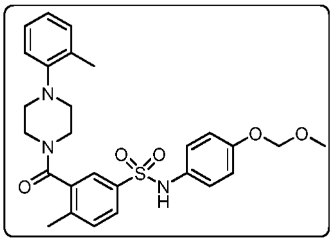

**$^1$H NMR (400 MHz, $CDCl_3$) δ:** 0.9-1.0 (m, 2H), 2.0 (m, 1H), 2.15 (s, 3H), 2.4 (s, 3H), 2.7-3.0 (m, 3H), 3.2 (m, 1H), 3.4 (s, 2H), 3.95-4.0 (m, 1H), 5.0 (m, 1H), 6.8-7.4 (m, 6H), 7.2-7.4 (m, 3H), 7.7-8.0 (m, 2H); **HPLC Purity:** 93.31 %; **Mass (M+Na):** 532.15.

**N-(4-((2-methoxyethoxy)methoxy)phenyl)-4-methyl-3-(4-o-tolylpiperazine-1 carbonyl)benzene sulfonamide (XIII-9):**

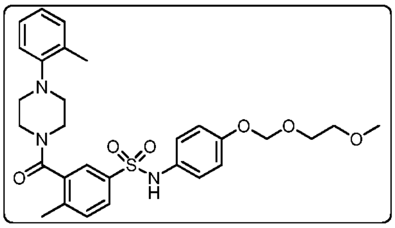

**$^1$H NMR (400 MHz, $CDCl_3$) δ:** 0.9-1.0 (m, 4H), 1.2-1.4 (m, 2H), 2.3 (s, 3H), 2.35 (s, 3H), 2.9-3.0 (m, 4H), 3.78 (s, 3H), 3.5-4.0 (m, 4H), 6.8-7.18 (m, 6H), 7.2-7.4 (m, 4H), 7.7-8.0 (m, 2H); **HPLC Purity:** 97.02 %; **Mass (M+Na):** 576.10.

**N-(4-(methoxymethoxy)phenyl)-3-(4-(2-methoxyphenyl)piperazine-1-carbonyl)-4-methylbenzene sulfonamide (XIII-10)**

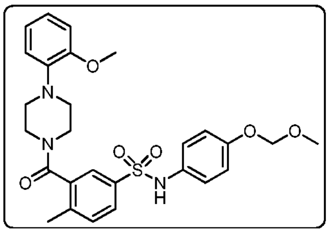

**$^1$H NMR (400 MHz, DMSO-$d_6$)** δ**:** 2.1 (s, 3H), 2.7-3.2 (m, 8H), 3.8 (s, 6H), 5.0-5.2 (m, 2H), 6.8-7.0 (m, 5H), 7.4-7.7 (m, 3H), 9.3 (d, 1H), 9.7 (d, 1H), 9.98 (s, 1H); **HPLC Purity:** 90.12 %; **Mass (M+1):** 526.1.

**N-(4-((2-methoxyethoxy)methoxy)phenyl)-3-(4-(2-methoxyphenyl)piperazine-1-carbonyl)-4-methylbenzenesulfonamide (XIII-11):**

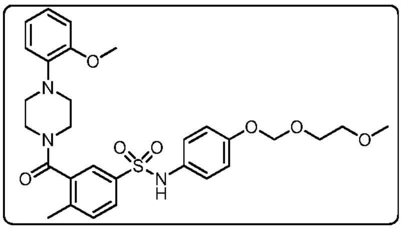

$^1$H NMR (400 MHz, DMSO-$d_6$) δ: 2.1 (s, 3H), 2.4-2.6 (s, 2H), 2.95-3.0 (m, 6H), 3.2 (s, 6H), 3.3-3.4 (m, 4H), 3.8 (s, 6H), 5.0-5.2 (m, 2H), 6.8-7.0 (m, 8H), 7.4-7.7 (m, 3H), 9.98 s, 1H); **HPLC Purity:** 97.063 %; **Mass (M+1):** 570.40.

**Ethyl-2-(4-(3-(4-(2-methoxyphenyl)piperazine-1-carbonyl)-4-methylphenylsulfonamido)phenoxy) propanoate (XIII-12):**

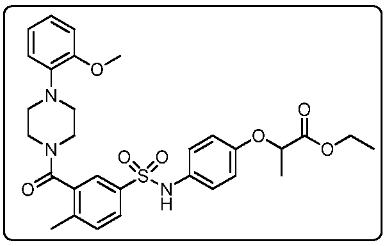

**$^1$H NMR (400 MHz, $CD_3OD$)** δ: 1.18-1.2 (t, 3H), 1.3-1.32 (d, 3H), 2.38 (s, 3H), 2.8-2.9 (m, 2H), 3.1-3.2 (m, 4H), 3.8 (s, 3H), 3.81-4.0 (m, 1H), 4.1-4.2 (m, 2H), 4.7-4.72 (q, 2H), 6.7-7.1 (m, 7H), 7.4-7.46 (m, 3H), 7.7-7.72 (m, 1H); **HPLC Purity:** 95.81%; **Mass (M+1):** 582.45.

**Methyl-2-((4-(3-(4-(2-methoxyphenyl)piperazine-1-carbonyl)-4-methylphenylsulfonamido)phenyl) amino)propanoate (XIII-13):**

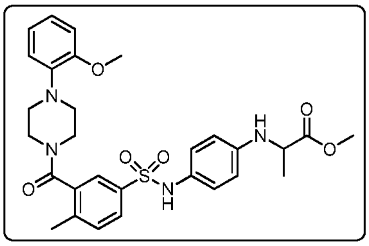

**$^1$H NMR (400 MHz, $CD_3OD$)** δ: 1.2-1.3 (d, 3H), 2.38 (s, 3H), 2.8-3.0 (m, 2H), 3.1-3.2 (m, 4H), 3.6 (s, 3H), 3.8 (s, 3H), 3.81-4.0 (m, 3H), 6.4-6.46 (m, 2H), 6.8-7.1 (m, 6H), 7.4-7.5 (m, 2H), 7.7-7.72 (m, 1H); **HPLC Purity:** 98.49%; **Mass (M+1):** 567.20.

**Scheme 17**

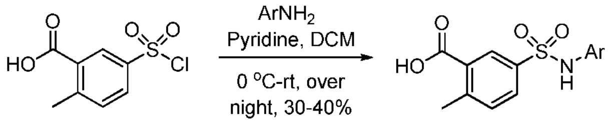


V XIV

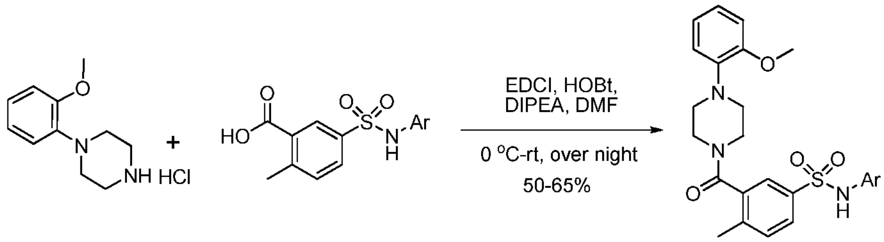


IV XIV XV

Where Ar =
Bicyclic aryl

**General procedure for synthesis of sulfonamide (XIV):**

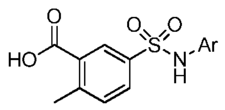

XIV

Sulfonamides (**XIV**) were prepared by following the similar method as described for sulfonamide **VII** in scheme 1 using appropriate amine (1.1 mmol) and 2-methyl-5-chlorosulfonyl benzoic acid (1.1 mmol) to afford sulfonamide **XIV** (30-40%).

**General procedure for synthesis of sulfonamide** (**XV-1**)-(**XV-5**):

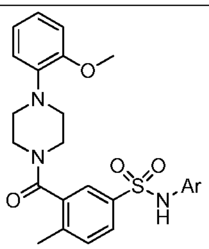

XV

Sulfonamides (**XV-1)-(XV-5**) were prepared by following the similar method as described for sulfonamide **VIII** in scheme 1 using compound **XIV** (0.25 mmol) and amine HCl (**IV**) (0.25 mmol) in 50-65% yields.

**N-(1H-indol-7-yl)-3-(4-(2-methoxyphenyl)piperazine-1-carbonyl)-4-methylbenzenesulfonamide** (**XV-1**):

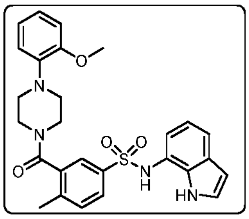

**1H NMR (400 MHz, $CDCl_3$)** δ: 2.39 (s, 3H), 2.69-2.8 (m, 2H), 3.0-3.2 (m, 4H), 3.8 (s, 3H), 3.9-4.0 (m, 2H), 6.4 (m, 1H), 6.6-6.7 (m, 2H), 6.8-7.1 (m, 3H), 7.15-7.2 (m, 2H), 7.4 (d, 1H), 7.6 (m, 2H) 8.1 (m, 1H); **HPLC Purity:** 98.88 %; **Mass (M+1):** 505.20.

**N-(1H-indol-5-yl)-3-(4-(2-methoxyphenyl)piperazine-1-carbonyl)-4-methylbenzenesulfonamide** (**XV-2**):

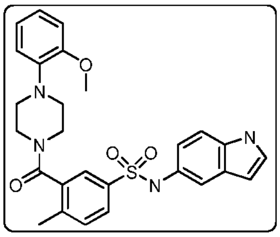

**$^1$H NMR** (**400 MHz, DMSO**-**$d_6$**) δ: 2.2 (s, 3H), 2.3-2.4 (m, 4H), 2.8-3.2 (m, 2H), 3.8 (s, 3H), 3.85-3.9 (m, 2H), 6.8-7.2 (m, 5H), 7.3-7.8 (m, 6H), 8.2 (d, 1H), 10.2 (s, 1H); **HPLC Purity:** 95.5 %; **Mass (M+1):** 505.31.

**3-(4-(2-methoxyphenyl)piperazine-1-carbonyl)-4-methyl-N-(quinolin-8-yl)benzenesulfonamide** (**XV-3**):

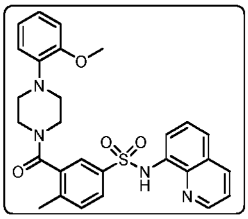

**$^1$H NMR** (**400 MHz, DMSO-$d_6$**) **δ:** 2.3 (s, 3H), 2.69-2.8 (m, 2H), 3.0-3.2 (m, 4H), 3.8 (s, 3H), 3.9-4.0 (m, 2H), 6.8-7.1 (m, 4H), 7.3-7.5 (m, 3H), 7.7-7.9 (m, 3H) 8.1 (m, 1H), 8.65-8.75 (m, 1H), 9.1-9.15 (m, 1H); **HPLC Purity:** 98.94 %; **Mass (M+1):** 517.20.

**3-(4-(2-methoxyphenyl)piperazine-1-carbonyl)-4-methyl-N-(4-methyl-1H-indol-7-yl)benzene sulfonamide** (**XV-4**):

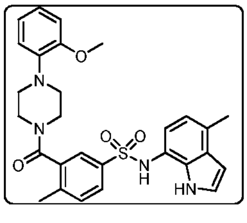

**$^1$H NMR (400 MHz, DMSO-$d_6$) δ:** 2.1 (s, 3H), 2.3 (s, 3H), 2.6-2.8 (m, 2H), 2.8-3.0 (m, 4H), 3.6-3.79 (m, 2H), 3.8 (s, 3H), 6.4 (d, 1H), 6.55-6.6 (m, 2H), 6.8-7.0 (m, 4H), 7.2-7.25 (d, 2H), 7.5 (m, 1H), 7.78 (d, 1H), 9.78 (d, 1H), 10.75 (s, 1H); **HPLC Purity:** 90.47 %; **Mass (M+1):** 519.31.

**3-(4-(2-Methoxyphenyl)piperazine-1-carbonyl)-4-methyl-N-(5-methylquinolin-8-yl)benzene sulfonamide (XV-5):**

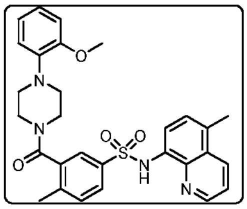

**$^1$H NMR (400 MHz, $CDCl_3$) δ:** 2.3 (s, 3H), 2.58 (s, 3H), 2.7-2.8 (m, 2H), 3.0-3.2 (m, 4H), 3.8 (s, 3H), 3.82-3.9 (m, 2H), 6.8-7.1 (m, 4H), 7.2-7.25 (m, 2H), 7.4-7.45 (m, 1H), 8.2 (d, 1H), 8.78 (d, 1H), 9.1 (s, 1H); **HPLC Purity:** 99.59 %; **Mass (M+1):** 531.34.

**Scheme 18**

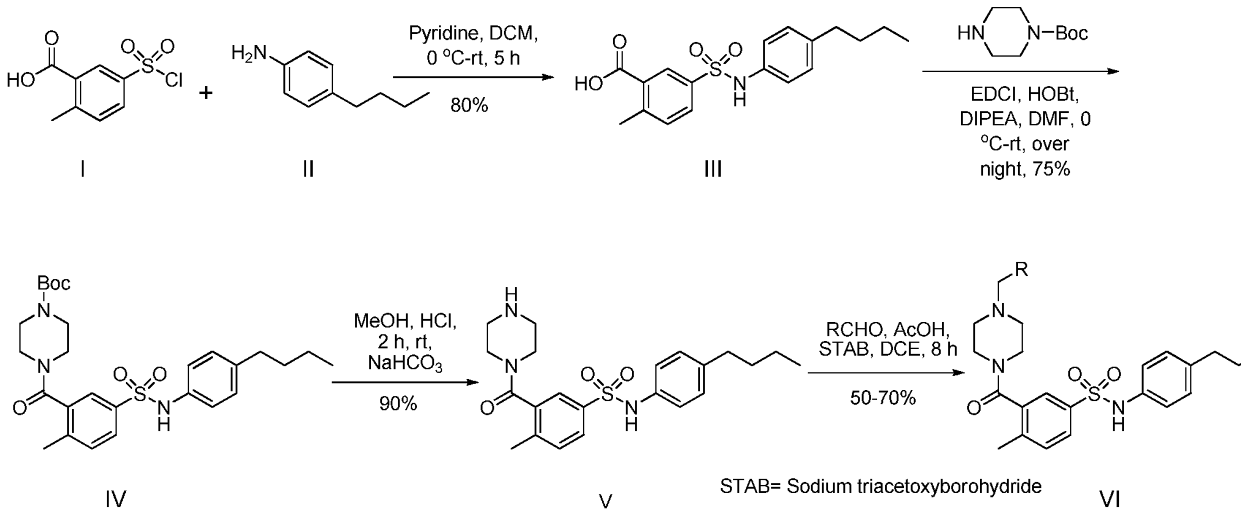


I II III

IV V VI

STAB= Sodium triacetoxyborohydride

Where R = Alkyl

**Procedure for synthesis of 5-(N-(4-butylphenyl)sulfamoyl)-2-methylbenzoic acid (III):**

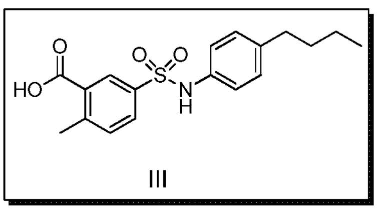

III

To a solution of 4-n-butylaniline (**II,** 2.0 gm, 13.4 mmol) in a 1:1 mixture of DCM-pyridine (25 mL) was added 2-methyl-5-chlorosulfonyl benzoic acid (**I,** 3.14 gm, 13.4 mmol) under nitrogen atmosphere. The resultant solution was stirred 5 h at room temperature. On completion of the reaction (monitored by TLC), the reaction mixture was diluted with dichloromethane (100 mL), washed with water (2 x 20 mL), 1N HCl solution (2 x 20 mL) and brine (20 mL). The combined organic extracts were dried over anhydrous sodium sulfate, filtered and concentrated under vacuum. Crude product was co-distilled with toluene to remove the remnants of pyridine and dried to yield sulfonamide (**III**) (3.7 gm, 80% yield) as

an off-white solid and was used as such for the next step without further purification.

**Procedure for synthesis of tert-butyl 4-(5-(N-(4-butylphenyl)sulfamoyl)-2-methylbenzoyl)piperazine-1-carboxylate** (**IV**)**:**

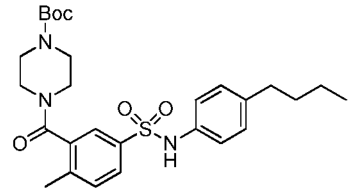
IV

To a stirred solution of the carboxylic acid (**III,** 3.5 gm, 10.07 mmol) in DMF at 0 °C under nitrogen atmosphere, EDCI (21.4 gm, 11.07 mmol), HOBt (15.0 gm, 11.07 mmol) and DIPEA (5.8 mL, 33.21 mmol) were added and the resultant solution was stirred at room temperature for 30 min. tert-Butyl piperaine-1-carboxylate (1.9 gm, 10.07 mmol) was then added at 0 °C and stirred overnight at room temperature. After completion of the reaction (monitored by TLC), the reaction mixture was poured into 1.0 M HCl and extracted with EtOAc (3 x 50 mL). The organic layer was washed with saturated $NaHCO_3$ solution (25 mL), dried over anhydrous $Na_2SO_4$ and filtered. The solvent was removed by rotary evaporation and the product was isolated by column chromatography on silica gel (60-120 silica gel, 2% MeOH-DCM) to yield amide (**IV**) (3.9 gm, 75% yield) as an off-white solid.

**Procedure for synthesis of N-(4-butylphenyl)-4-methyl-3-(piperazine-1-carbonyl)benzenesulfonamide** (**V**)**:**

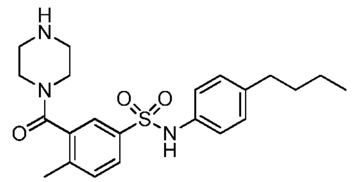
V

To the Boc-sulfonamide (**IV**, 3.85 gm, 7.46 mmol) was added methanolic-HCl (30 mL, 5%) which resulted in formation of a homogeneous solution and was stirred for 2 h at room temperature. After completion of the reaction (monitored by TLC), the solvent was removed under vacuum. To the crude product was added saturated solution of $NaHCO_3$ (50 mL) and extracted with ethyl acetate (3 x 50 mL), organic extracts were washed with ware (25 mL), brine (25 mL), concentrated in vacuo and then dried well to obtain product (V) (2.8 gm, 90% yields) as a white solid.

General Procedure for synthesis of compounds **(VI-1)-(VI-8):**

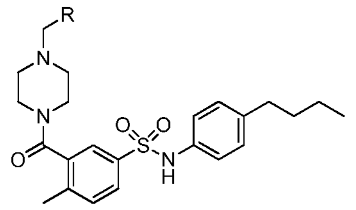
VI

To a solution of amine V (0.25 mmol) and appropriate aldehyde (0.27 mmol) in DCE, acetic acid (0.2 mL) was added at room temperature and the resulting mixture was allowed to stir for 30 min. Then sodium triacetoxyborohydride (0.75 mmol) was added to reaction mixture and the resulting mixture was allowed to stir at room temperature for 8 hr. After completion of reaction, the crude mixture was diluted with DCM washed with water, dried over $Na_2SO_4$ and concentrated under reduced pressure. The residue was purified by column chromatography (silica gel, 60-120 mesh; MeOH-DCM, 2:8) to afford product **(VI-1)-(VI-8)** in 50-70% yields.

**N-(4-butylphenyl)-4-methyl-3-(4-(2-methylbenzyl)piperazine-1-carbonyl)benzenesulfonamide** (**VI-1**):

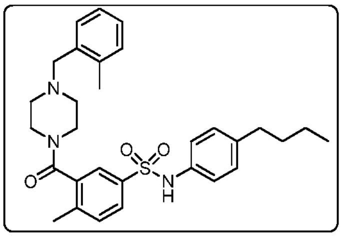

**$^1$H NMR (400 MHz, $CD_3OD$)** δ**:** 0.9-0.95 (t, 3H), 1.2-1.32 (m, 2H), 1.4-1.5 (m, 2H), 2.2-2.28 (m, 2H), 2.3 (s, 3H), 2.31 (s, 3H), 2.5-2.6 (t, 2H), 2.82-2.98 (m, 2H), 3.46-3.8 (m, 2H), 3.5 (s, 2H), 3.7-3.8 (m, 2H), 6.9-7.0 (m, 4H), 7.1-7.21 (m, 4H), 7.39-7.4 (m, 2H), 7.7-7.76 (m, 1H); **HPLC Purity:** 98.24%; **Mass (M+1):** 520.35.

**N-(4-butylphenyl)-4-methyl-3-(4-(3-methylbenzyl)piperazine-1-carbonyl)benzenesulfonamide** (**VI-2**):

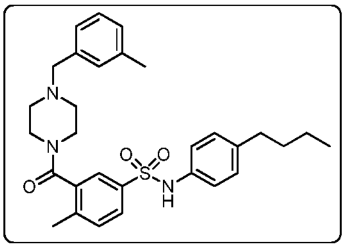

**$^1$H NMR (400 MHz, $CDCl_3$)** δ**:** 0.9-0.95 (t, 3H), 1.2-1.32 (m, 2H), 1.4-1.6 (m, 2H), 2.2-2.3 (m, 2H), 2.3 (s, 3H), 2.31 (s, 3H), 2.41-2.5 (t, 2H), 2.51-2.58 (m, 2H), 3.0-3.1 (m, 2H), 3.5 (s, 2H), 3.78-3.81 (m, 2H), 6.9-7.1 (m, 5H), 7.2-7.3 (m, 4H), 7.58-7.61 (m, 2H); **HPLC Purity:** 99.68%; Mass **(M+1):** 520.45.

**N-(4-butylphenyl)-3-(4-(2-ethylbenzyl)piperazine-1-carbonyl)-4-methylbenzenesulfonamide** (**VI-3**):

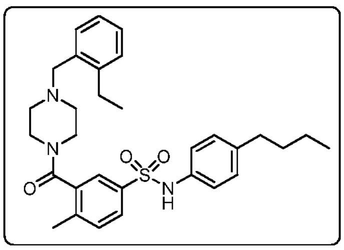

**$^1$H NMR** (**400 MHz, $CDCl_3$)** δ**:** 0.9-0.95 (t, 3H), 1.2-1.32 (m, 2H), 1.4-1.6 (m, 5H), 2.2-2.3 (m, 2H), 2.38 (s, 3H), 2.41-2.5 (t, 2H), 2.51-2.58 (m, 2H), 2.7-2.78 (q, 2H), 2.9-3.1 (m, 2H), 3.5 (s, 2H), 3.78-3.81 (m, 2H), 6.9-7.0 (m, 4H), 7.1-7.3 (m, 6H), 7.58-7.61 (m, 1H); **HPLC Purity:** 99.57%; **Mass (M+1):** 534.44.

**N-(4-butylphenyl)-4-methyl-3-(4-(2,3,4-trimethylbenzyl)piperazine-1-carbonyl)benzenesulfonamide** (**VI-4**):

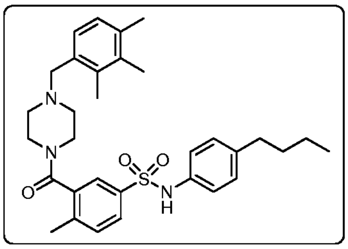

**$^1$H NMR** (**400 MHz, $CD_3OD$**) δ**:** 0.9-0.95 (t, 3H), 1.2-1.28 (m, 2H), 1.4-1.46 (m, 2H), 2.18-2.2 (m, 2H), 2.21-2.3 (m, 9H), 2.31 (s, 3H), 2.38-2.4 (t, 2H), 2.5-2.6 (m, 2H), 2.8-2.9 (m, 2H), 3.4-2.45 (m, 2H), 3.6-3.8 (m, 2H), 6.9-7.0 (m, 6H), 7.4-7.44 (m, 2H), 7.75-7.8 (m, 1H); **HPLC Purity:** 99.77%; **Mass (M+1):** 548.40.

**N-(4-butylphenyl)-4-methyl-3-(4-phenethylpiperazine-1-carbonyl)benzenesulfonamide** (**VI-5**):

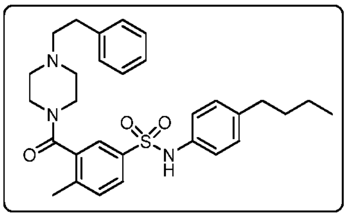

**$^1$H NMR** (**400 MHz, $CDCl_3$)** δ**:** 0.9-0.95 (t, 3H), 1.22-1.39 (m, 2H), 1.5-1.6 (m, 2H), 2.38 (s, 3H), 2.4-2.6 (m, 4H), 2.61-2.7 (m, 4H), 2.78-2.81 (m, 2H), 3.05-3.12 (m, 2H), 3.8-3.2 (m, 2H), 6.9-7.1 (m, 4H), 7.2-7.4 (m, 6H), 7.58-7.61 (m, 2H); **HPLC Purity:** 98.98%; **Mass (M+1):** 520.36.

**N-(4-butylphenyl)-4-methyl-3-(4-(3-phenylpropyl)piperazine-1-carbonyl)benzenesulfonamide** (**VI-6**):

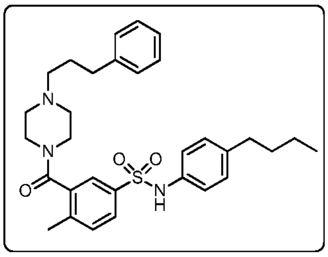

**$^1$H NMR** (**400 MHz, $CD_3OD$)** δ**:** 0.9 (t, 3H), 1.1-1.5 (m, 2H), 1.4-1.6 (m, 2H), 1.8-1.98 (m, 2H), 2.1 (s, 3H), 2.4-2.6 (m, 6H), 2.6-2.8 (m, 4H), 2.9-3.0 (m, 4H), 6.9-7.1 (m, 4H), 7.19-7.4 (m, 6H), 7.45-7.5 (m, 1H), 7.7 (d, 1H); **HPLC Purity:** 98.93 %; **Mass (M+1):** 534.34.

**N-(4-butylphenyl)-4-methyl-3-(4-(1-phenylethyl)piperazine-1-carbonyl)benzenesulfonamide** (**VI-7**):

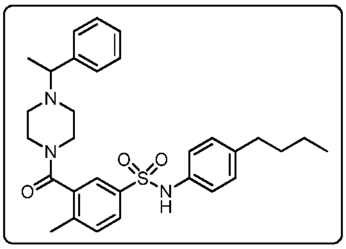

**$^1$H NMR (400 MHz, $CD_3OD$) δ:** 0.9-0.95 (t, 3H), 1.2-1.32 (m, 2H), 1.38-1.4 (d, 3H), 1.4-1.5 (m, 2H), 2.2 (s, 3H), 2.3-2.4 (m, 3H), 2.6-2.7 (m, 2H), 2.82-2.98 (m, 2H), 3.4-3.5 (m, 2H), 3.6-3.8 (m, 2H), 6.9-7.0 (m, 4H), 7.2-7.4 (m, 7H), 7.5-7.54 (m, 1H); **HPLC Purity:** 92.81%; **Mass (M+1):** 520.14.

**N-(4-butylphenyl)-4-methyl-3-(4-(2-phenylpropyl)piperazine-1-carbonyl)benzenesulfonamide (VI-8):**

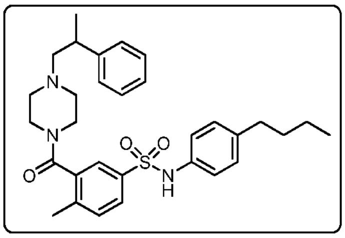

**$^1$H NMR (400 MHz, $CDCl_3$) δ:** 0.9-0.95 (t, 3H), 1.2-1.39 (m, 5H), 1.5-1.6 (m, 2H), 2.3 (s, 3H), 2.4-2.6 (m, 5H), 2.9-3.0 (m, 4H), 3.05-3.12 (m, 2H), 3.7-3.9 (m, 2H), 6.9-7.1 (m, 4H), 7.2-7.4 (m, 7H), 7.58-7.61 (m, 2H); **HPLC Purity:** 98.98%; **Mass (M+1):** 534.32.

**Scheme 19**

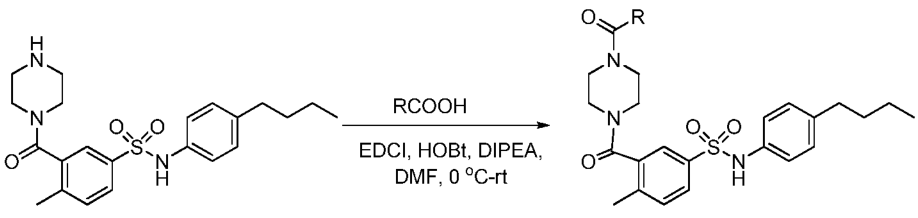


V VII

**General procedure for compound (VII-1)-(VII-4):**

Amide compounds (**VII-1**)-(**VII-4**) were prepared by following similar method as described for compound **IV** in scheme 1 using amine **V** (0.2 mmol) and appropriate carboxylic acids (0.2 mmol) to afford an off white solids in 60-65% yields.

**N-(4-butylphenyl)-3-(4-(cyclopropanecarbonyl)piperazine-1-carbonyl)-4-methylbenzenesulfonamide (VII-1):**

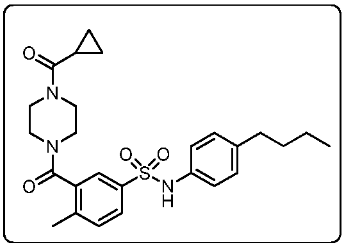

**$^1$H NMR** (**400 MHz, CDCl$_3$**) δ**:.** 0.8-0.9 (m, 2H), 0.9 -0.95 (t, 3H), 1.0-1.1 (m, 2H), 1.2-1.38 (m, 2H), 1.5-1.6 (m, 3H), 2.36 (s, 3H), 2.5-2.6 (t, 2H), 3.0-3.2 (m, 2H), 3.4-3.6 (m, 2H), 3.7-3.9 (m, 4H), 6.9-7.1 (m, 4H), 7.2-7.38 (m, 1H), 7.58-7.61 (m, 2H); **HPLC Purity:** 99.38%; **Mass (M+1):** 584.19.

**3-(4-benzoylpiperazine-1-carbonyl)-N-(4-butylphenyl)-4-methylbenzenesulfonamide** (**VII-2**)**:**

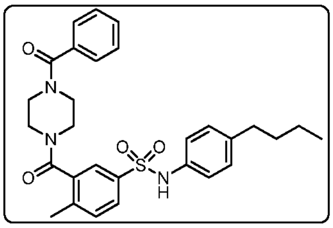

**$^1$H NMR** (**400 MHz, DMSO-d$_6$)** δ**:** 0.8 (t, 3H), 1.1-1.2 (m, 2H), 1.3-1.4 (m, 2H), 2.1 (s, 3H), 2.2 (m, 1H), 2.89-3.0 (m, 4H), 3.6-3.8 (m, 4H), 6.8-7.1 (m, 4H), 7.4-7.56 (m, 7H), 7.5-7.69 (m, 1H), 10.1 (m, 1H); **HPLC Purity:** 99.86 %; **Mass (M+1):** 520.21.

**N-(4-butylphenyl)-4-methyl-3-(4-(2-phenylacetyl)piperazine-1-carbonyl)benzenesulfonamide** (**VII-3**)**:**

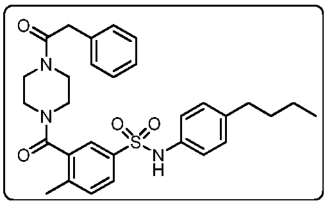

**$^1$H NMR** (**400 MHz, DMSO-d$_6$**) δ**:** 0.85 (t, 3H), 1.1-1.2 (m, 2H), 1.4-1.45 (m, 2H), 2.1 (s, 3H), 2.4-2.6 (m, 1H), 2.89-3.0 (s, 2H), 3.5-3.7 (m, 6H), 3.75-3.8 (m, 2H), 6.9-7.1 (m, 4H), 7.35-7.4 (m, 5H), 7.45-7.60 (m, 2H), 7.6-7.8 (m, 1H), 10.1 (m, 1H); **HPLC Purity:** 99.94%; **Mass (M+1):** 534.20.

**N-(4-butylphenyl)-4-methyl-3-(4-(thiazole-4-carbonyl)piperazine-1-carbonyl)benzenesulfonamide** (**VII-4**)**:**

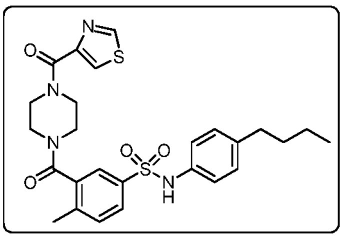

**$^1$H NMR** (**400 MHz, $CD_3OD$**) δ: 0.8-0.95 (t, 3H), 1.2-1.39 (m, 2H), 1.4-1.6 (m, 2H), 2.3 (s, 3H), 2.5-2.6 (t, 2H), 3.0-3.2 (m, 2H), 3.4-3.78 (m, 2H), 3.8-4.0 (m, 4H), 6.9-7.1 (m, 4H), 7.4-7.5 (m, 2H), 7.7-7.8 (m, 1H), 8.19-8.2 (m, 1H), 9.0-9.1 (m, 1H); **HPLC Purity:** 93.13%; **Mass** (**M+1**)**:** 527.23.

**Scheme 20**

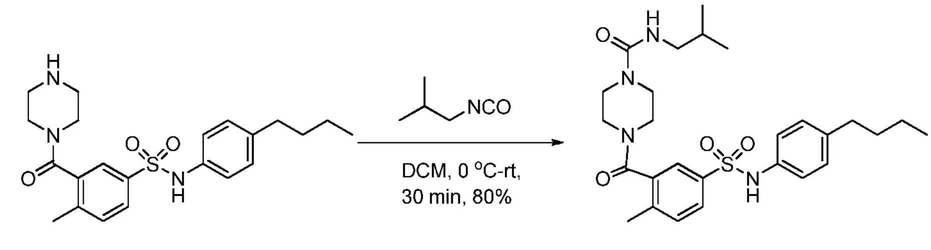


V VIII

To a solution of amine **V** (0.083 gm, 0.2 mmol) in dichloromethane (5 mL), isobutyl isocyacnate (0.022 gm, 0.22 mmol) was added at 0°C and the resultant mixture was allowed stir for 30 minutes at room temperature. After completion of reaction (monitored by TLC), the solvent was evaporated and the crude product was purified by column chromatography (60:120 Silica gel, 5% MeOH:DCM) to afford product **VIII** (80% yield) as an off white solid.
**1H NMR** (**400 MHz, DMSO-$d_6$**) **δ:** 0.8 (d, 6H), 0.81 (m, 1H), 0.9-0.95 (m, 2H), 1.2-1.4 (m, 2H), 1.4-1.6 (m, 2H), 2.1 (s, 3H), 2.4-2.6 (m, 3H), 2.89-3.0 (m, 4H), 3.1-3.2 (d, 2H), 3.6-3.7 (m, 2H), 6.6 (m, 1H), 6.9-7.1 (m, 4H), 7.4-7.42 (m, 2H), 7.45-7.6 (m, 1H), 10.1 (m, 1H); **HPLC Purity:** 99.64 %; **Mass (M+1):** 515.37.

**Scheme 21**

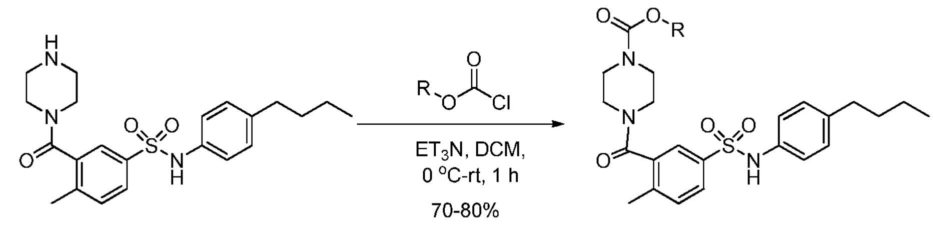


V IX

**General procedure for compound (IX-1)-(IX-5):**

To a solution of amine **V** (0.085 gm, 0.204 mmol) and triethyl amine (0.1 mL, 0.72 mmol) in dichloromethane (5 mL), appropriate alkyl chloroformate (0.22 mmol) was added at 0 °C and the resultant mixture was allowed stir for 1 h at room temperature. After completion of reaction (monitored by TLC), the solvent was evaporated and the crude product was purified by column chromatography (60:120 Silica gel, 5% MeOH:DCM) to afford product **(IX-1)-(IX-5)** (70-80% yields) as an off white solid.

**Ethyl 4-(5-(N-(4-butylphenyl)sulfamoyl)-2-methylbenzoyl)piperazine-1-carboxylate (IX-1):**

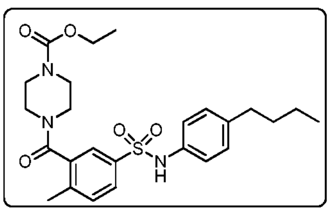

**$^1$H NMR (400 MHz, $CDCl_3$)** **δ:** 0.9 (t, 3H), 1.2-1.4 (m, 4H), 1.5-1.6 (m, 2H), 2.4 (s, 3H), 2.59-2.6 (m, 2H), 3.0-3.1 (m, 2H), 3.2-3.3 (m, 2H), 3.56-3.6 (m, 2H), 3.7-3.8 (m, 2H), 6.4 (s, 1H), 6.95-7.1 (m, 4H), 7.2-7.4 (m, 1H), 7.6-7.7 (m,

2H); **HPLC Purity:** 98.31 %; Mass **(M+1):** 488.35.

**Isopropyl 4-(5-(N-(4-butylphenyl)sulfamoyl)-2-methylbenzoyl)piperazine-1-carboxylate (IX-2):**

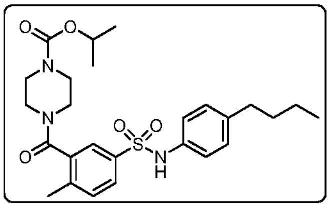

**$^1$H NMR (400 MHz, $CD_3OD$) δ:** 0.9 (t, 3H), 1.2-1.4 (m, 9H), 1.5-1.6 (m, 2H), 2.39 (s, 3H), 2.4-2.6 (m, 2H), 3.0-3.1 (m, 2H), 3.2-3.3 (m, 2H), 3.56-3.6 (m, 2H), 3.7-3.8 (m, 2H), 6.95-7.1 (m, 4H), 7.4-7.5 (m, 2H), 7.8-7.83 (m, 1H); **HPLC Purity:** 99.48 %; **Mass (M+1):** 502.35.

**Isobutyl 4-(5-(N-(4-butylphenyl)sulfamoyl)-2-methylbenzoyl)piperazine-1-carboxylate (IX-3):**

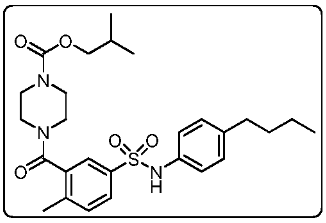

**$^1$H NMR (400 MHz, $CDCl_3$) δ:** 0.9 (d, 6H), 0.92-1.1 (m, 2H), 1.2-1.4 (m, 2H), 1.6 (s, 3H), 1.8-2.0 (m, 1H), 2.3 (s, 3H), 2.39-2.6 (m, 2H), 3.0-3.1 (m, 2H), 3.2-3.3 (m, 2H), 3.56-3.6 (m, 2H), 3.9-4.0 (m, 2H), 6.4 (s, 1H), 6.95-7.1 (m, 4H), 7.2-7.4 (m, 1H), 7.6-7.7 (m, 1H); **HPLC Purity:** 98.79 %; **Mass (M+1):** 516.40.

**Benzyl 4-(5-(N-(4-butylphenyl)sulfamoyl)-2-methylbenzoyl)piperazine-1-carboxylate (IX-4):**

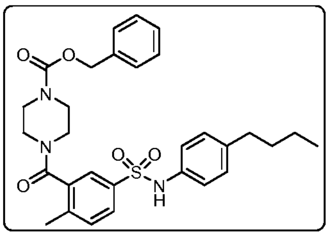

**[LNB.No: SCJ-E10044~017]: 1H NMR (400 MHz, $CD_3OD$) δ:** 0.9 (t, 3H), 1.2-1.4 (m, 2H), 1.4-1.6 (m, 2H), 2.1 (s, 3H), 2.3-2.6 (m, 2H), 2.39-3.12 (m, 2H), 3.5-4.0 (m, 4H), 5.2 (s, 2H), 6.95-7.2 (m, 4H), 7.2-7.4 (m, 7H), 7.8 (m, 1H); **HPLC Purity:** 96.74 %; **Mass (M+1):** 550.40.

**Phenyl 4-(5-(N-(4-butylphenyl)sulfamoyl)-2-methylbenzoyl)piperazine-1-carboxylate (IX-5):**

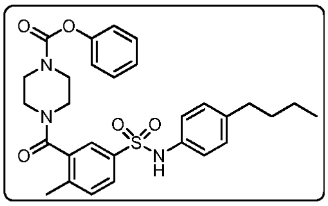

**$^1$H NMR (400 MHz, $CD_3OD$) δ:** 0.9 (t, 3H), 1.2-1.4 (m, 2H), 1.4-1.6 (m, 2H), 2.1 (s, 3H), 2.4-2.6 (m, 2H), 2.89-3.2 (m, 2H), 3.5-4.0 (m, 6H), 6.95-7.2 (m, 6H), 7.2-7.4 (m, 1H), 7.45-7.6 (m, 4H), 7.8 (m, 1H); **HPLC Purity:** 98.45 %; **Mass (M+1):** 536.35.

Having thus described several aspects of at least one embodiment of this invention, it is to be appreciated various alterations, modifications, and improvements will readily occur to those skilled in the art. Accordingly, the foregoing description and drawings are by way of example only.

## Claims

1. Use of a compound of formula (I) or a pharmaceutically acceptable salt thereof:

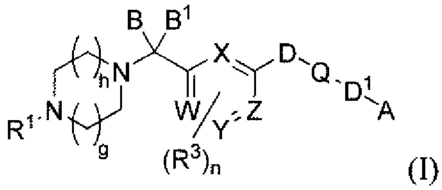

(I)

wherein:

W, X, Y and Z are each independently selected from CH or N;
B and $B^1$ are independently selected from hydrogen and alkyl or when taken together with the carbon to which they are attached form a carbonyl group;
Q is C=O or $SO_2$;
D and $D^1$ are independently selected from a bond, oxygen and $NR^c$; wherein at least one of D and $D^1$ is $NR^c$;
A is aryl or heteroaryl each substituted with 0-3 occurrences of $R^2$;
$R^1$ is independently selected from alkyl, acyl, cycloalkyl, aryl, heteroaryl, heterocyclyl, heterocyclylalkyl, cycloalkylalkyl, aralkyl, and heteroaralkyl;
each of which may be optionally substituted with 0-3 occurrences of $R^d$;
each $R^2$ is independently selected from halo, hydroxy, haloalkyl, aryl, heteroaryl, alkyl, $-NR^cR^{c'}$, alkyl-$NR^cR^{c'}$, $-OR^a$, -C(O)OH, $-C(O)OR^b$,$-C(O)NR^cR^{c'}$, cycloalkyl, heterocyclyl, heterocyclylalkyl, cycloalkylalkyl, aralkyl, and heteroaralkyl;
each $R^3$ is independently selected from halo, haloalkyl, alkyl, alkenyl, alkynyl, heterocyclyl and $-OR^a$, or two adjacent $R^3$s (when n is 2) taken together with the carbon atoms they are attached to form an optionally substituted heterocyclyl;
each $R^a$ is independently selected from alkyl, alkoxy, alkylalkoxy, alkylalkoxylalkoxy, alkyl-$C(O)OR^b$, alkyl-$C(O)OR^b$, and haloalkyl;
each $R^b$ is independently alkyl;
each $R^c$ and $R^{c'}$ is independently selected from hydrogen, alkyl, alkyl-$C(O)OR^b$ and alkenyl;
each $R^d$ is independently selected from halo, haloalkyl, alkyl, nitro, cyano, and $-OR^a$, or two $R^d$ taken together with the carbon atoms to which they are attached form an optionally substituted heterocyclyl;
n is 0, 1, or 2;
h is 0, 1, 2; and
g is 0, 1 or 2;
in the manufacture of a medicament for treating a cancer characterized as having an IDH mutation in a subject in need thereof.

2. The use of claim 1, wherein the compound is selected from any one of the compounds set forth in the table below:

| Compound |
|---|
| 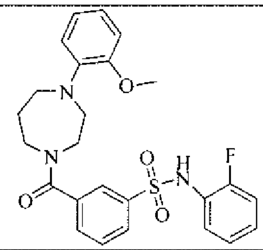 |
| 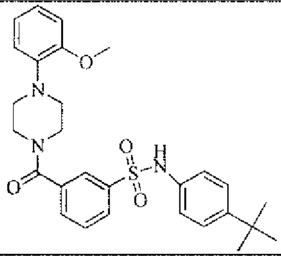 |
| 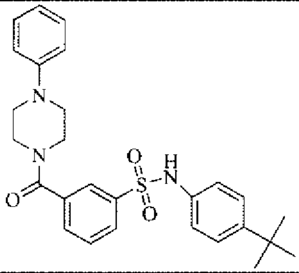 |
| 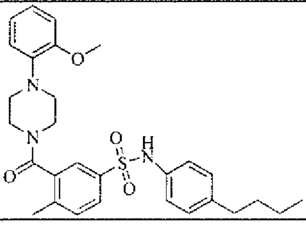 |

| Compound |
|---|
| 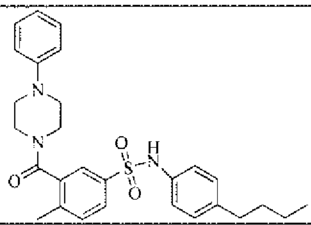 |
| 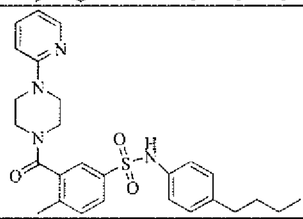 |
| 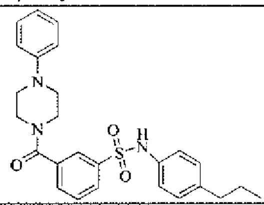 |
| 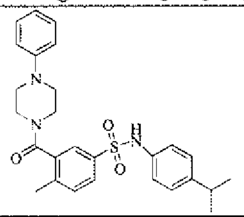 |
| 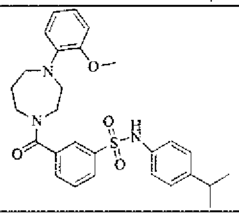 |

| Compound |
|---|
| 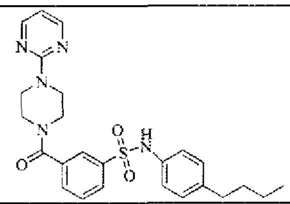 |
| 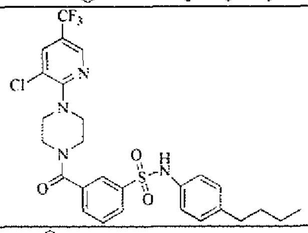 |
| 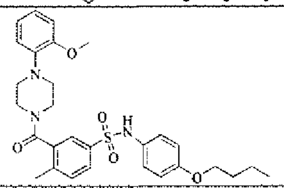 |
| 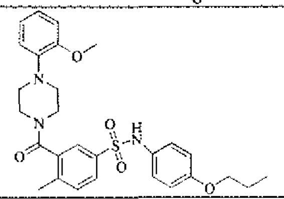 |
| 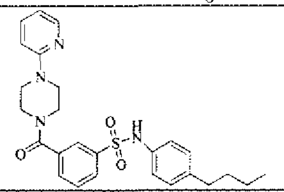 |
| 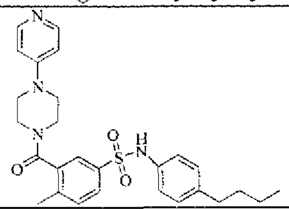 |
| 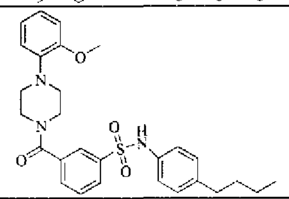 |
| 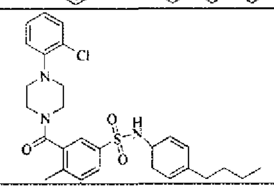 |

| Compound |
|---|
| 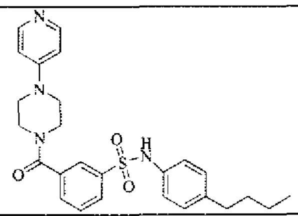 |
| 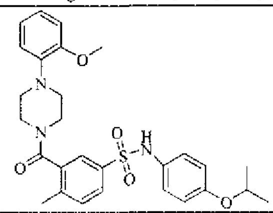 |
| 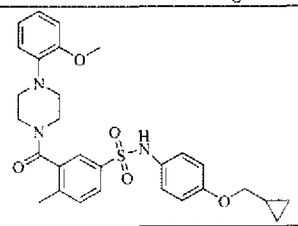 |
| 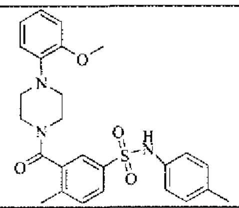 |
| 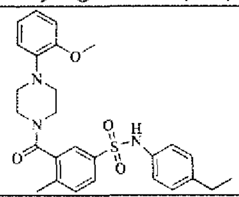 |
| 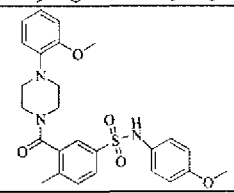 |
| 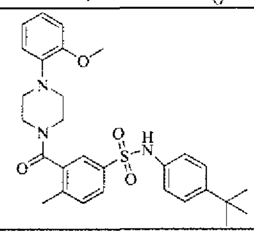 |
| 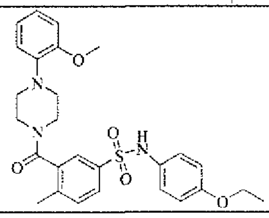 |

| Compound |
|---|
| 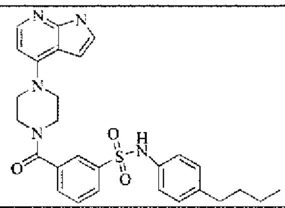 |
| 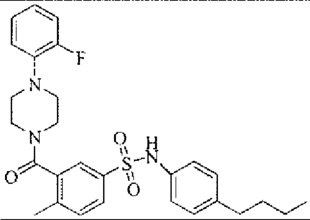 |
| 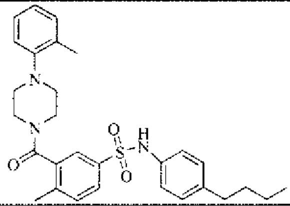 |
| 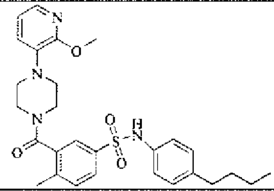 |
| 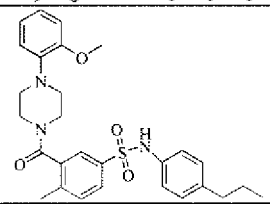 |
| 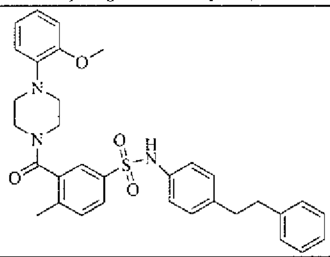 |
| 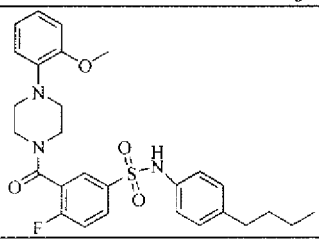 |

| Compound |
|---|
| 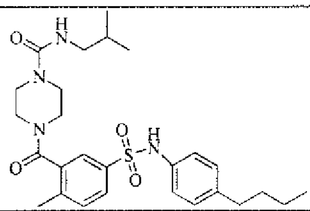 |
| 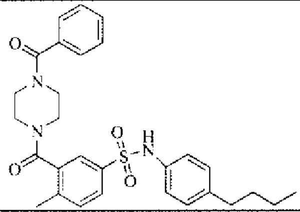 |
| 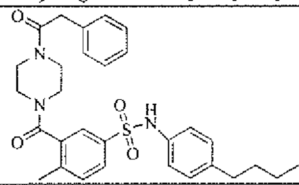 |
| 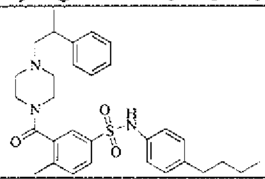 |
| 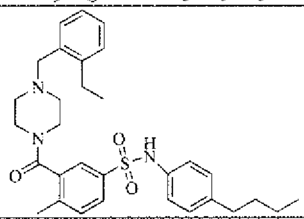 |
| 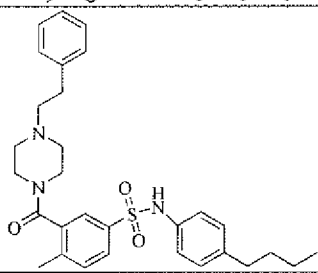 |
| 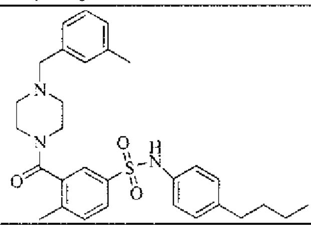 |
| 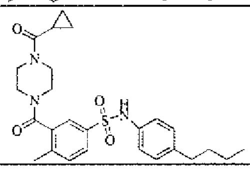 |

| Compound |
|---|
| 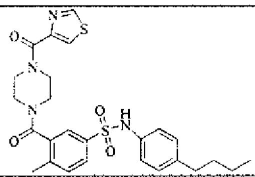 |
| 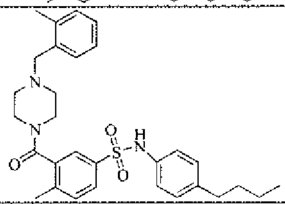 |
| 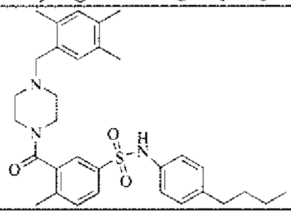 |
| 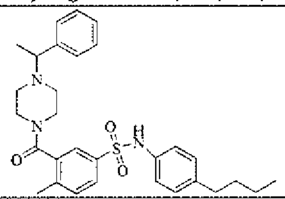 |
| 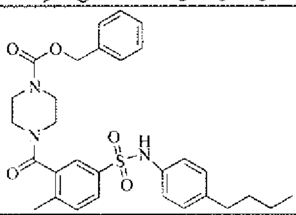 |
| 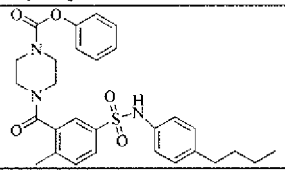 |
| 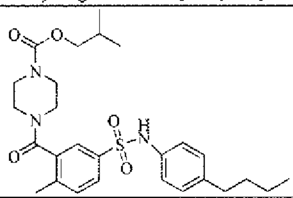 |
| 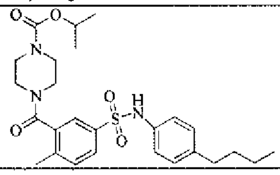 |

| Compound |
|---|
| 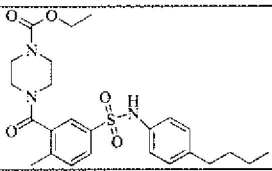 |
| 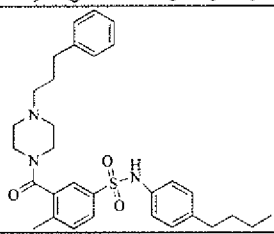 |
| 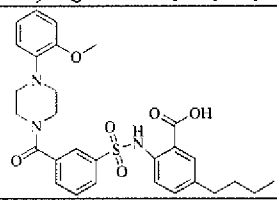 |
| 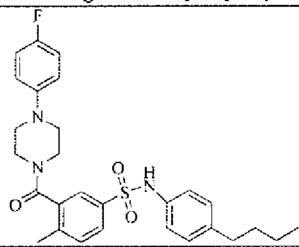 |
| 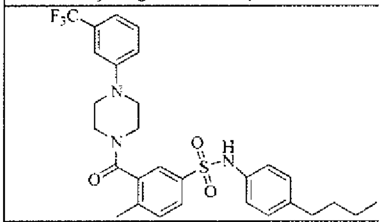 |
| 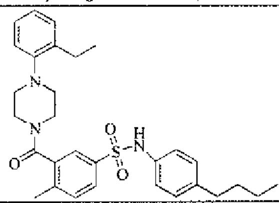 |
| 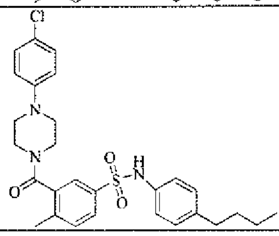 |

| Compound |
| --- |
| 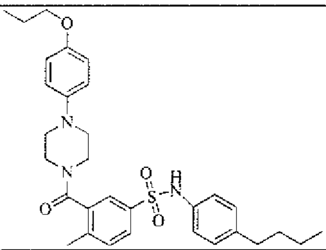 |
| 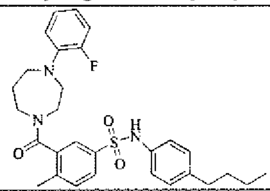 |
| 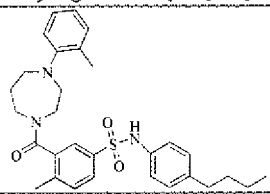 |
| 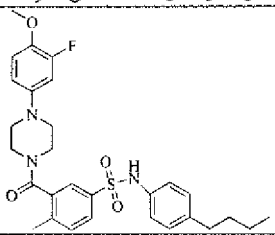 |
| 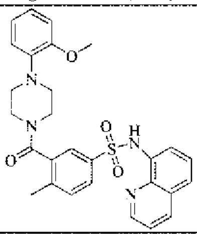 |
| 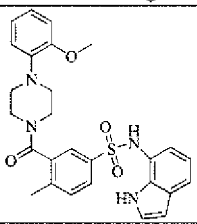 |
| 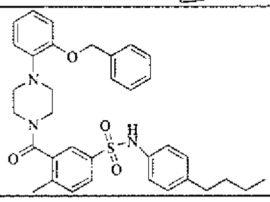 |

| Compound |
| --- |
| 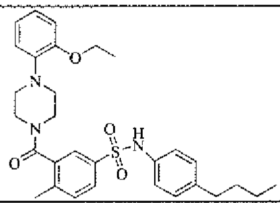 |
| 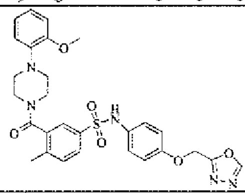 |
| 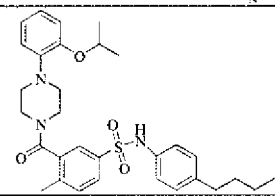 |
| 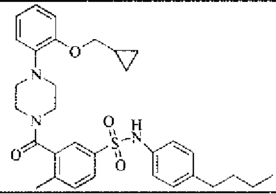 |
| 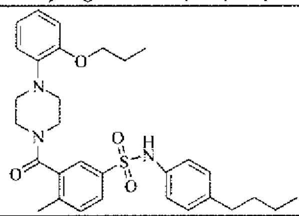 |
| 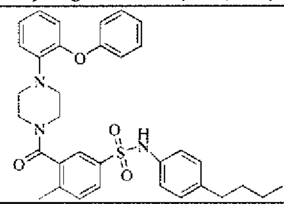 |
| 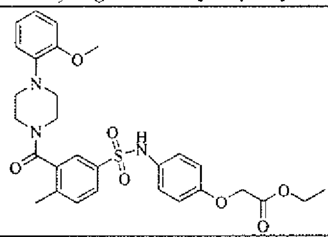 |
| 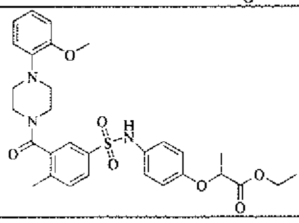 |

| Compound |
| --- |
| 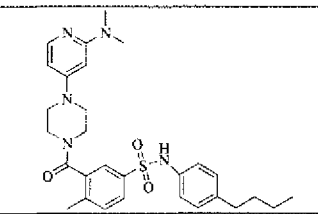 |
| 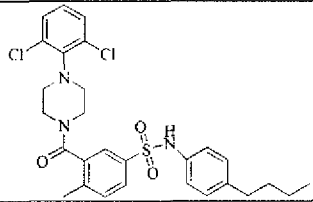 |
| 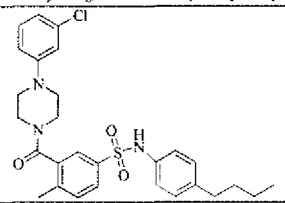 |
| 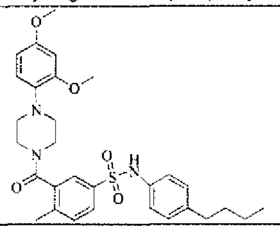 |
| 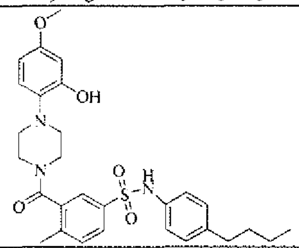 |
| 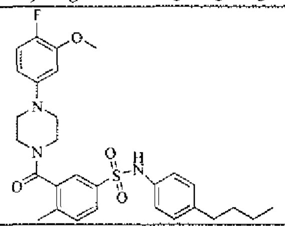 |
| 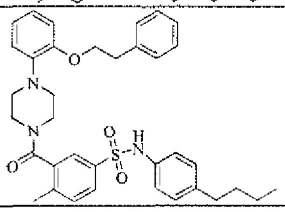 |

| Compound |
| --- |
| 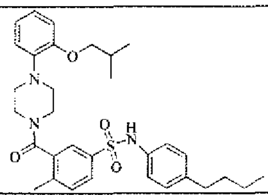 |
| 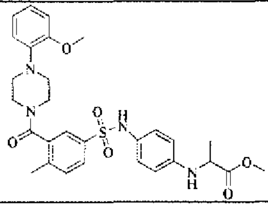 |
| 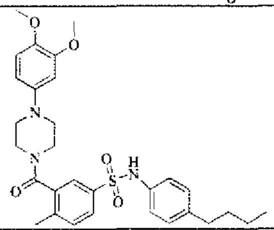 |
| 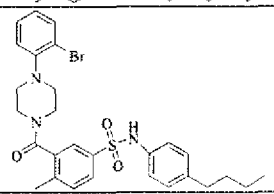 |
| 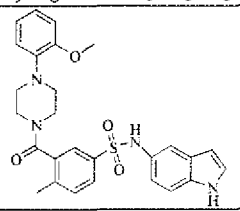 |
| 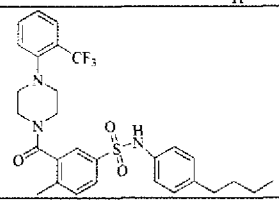 |
| 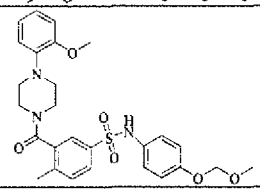 |
| 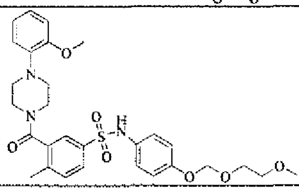 |

| Compound |
|---|
| 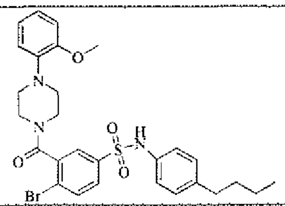 |
| 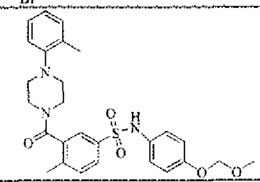 |
| 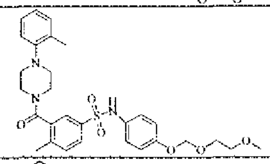 |
| 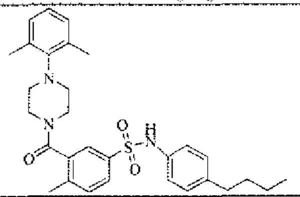 |
| 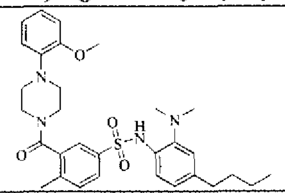 |
| 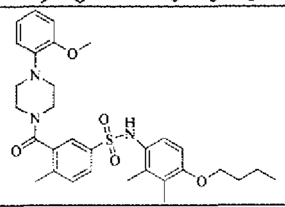 |
| 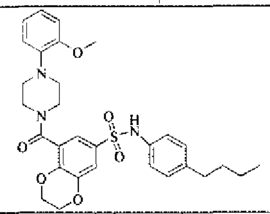 |
| 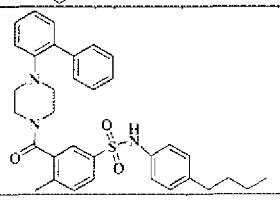 |

| Compound |
|---|
| 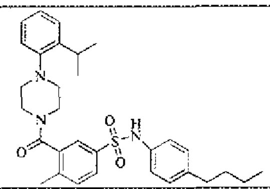 |
| 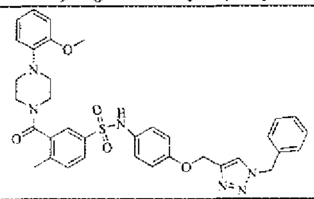 |
| 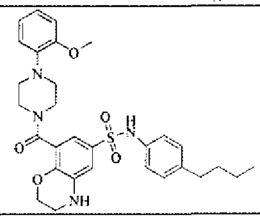 |
| 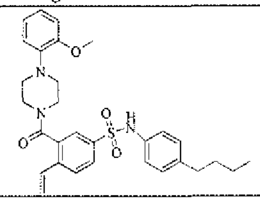 |
| 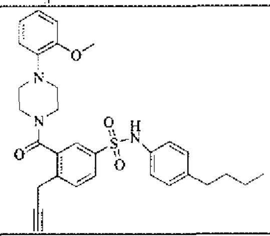 |
| 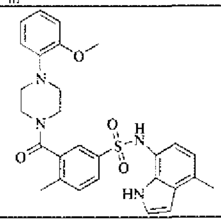 |
| 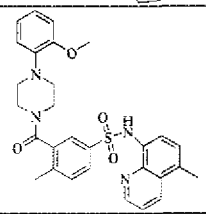 |
| 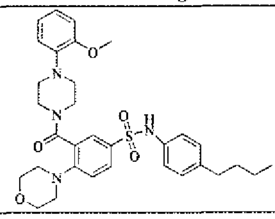 |

| Compound |
|---|
| 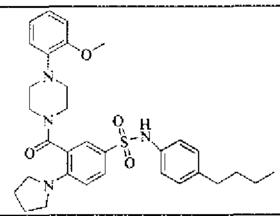 |
| 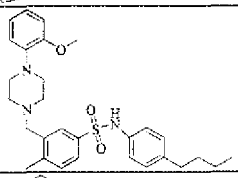 |
| 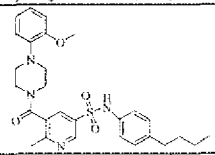 |
| 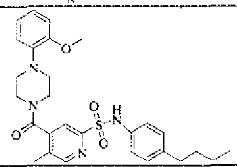 |
| 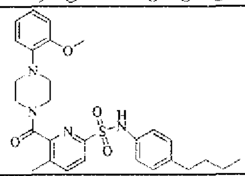 |
| 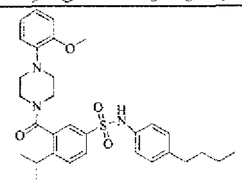 |
| 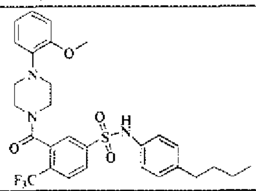 |
| 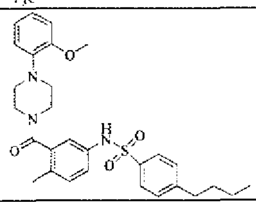 |
| 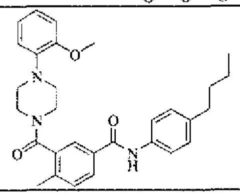 |

| Compound |
|---|
| 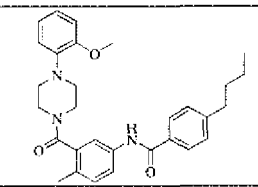 |
| 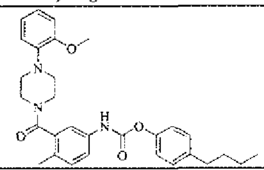 |
| 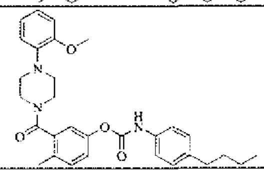 |
| 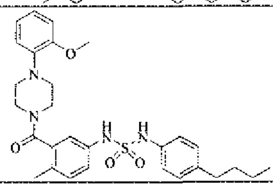 |
| 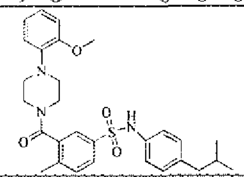 |
| 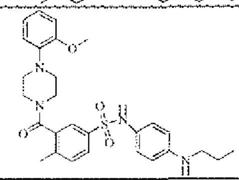 |
| 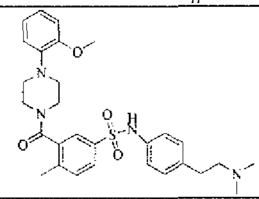 |
| 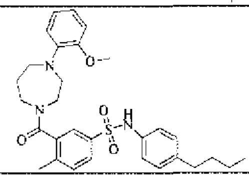 |
| 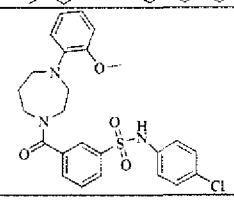 |

| Compound |
| --- |
| 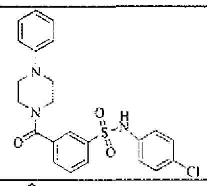 |
| 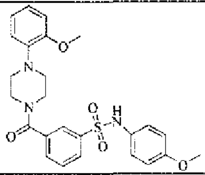 |
| 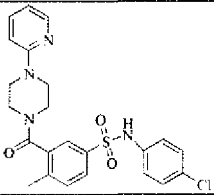 |
| 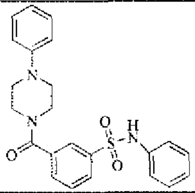 |
| 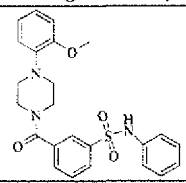 |
| 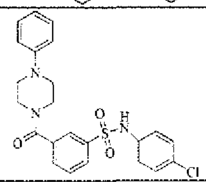 |
| 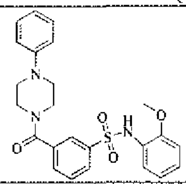 |
| 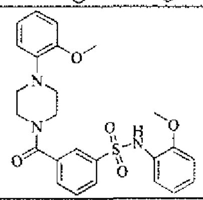 |
| 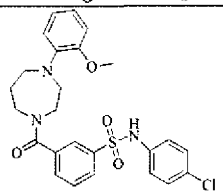 |

| Compound |
| --- |
| 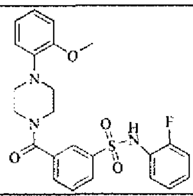 |
| 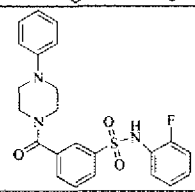 |
| 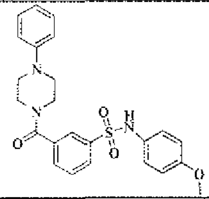 |
| 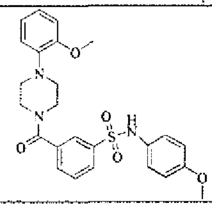 |
| 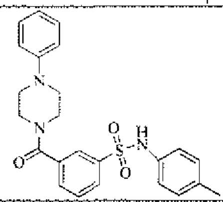 |
| 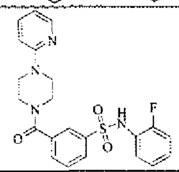 |
| 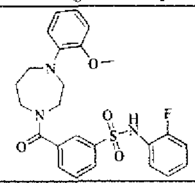 |
| 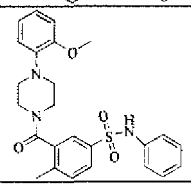 |

| Compound |
|---|
| 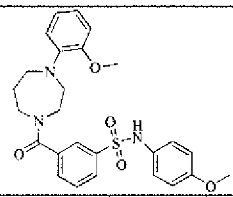 |
| 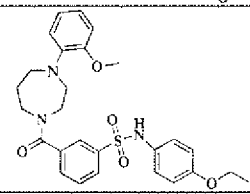 |
| 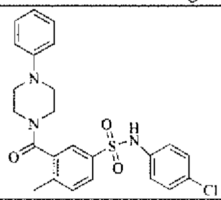 |
| 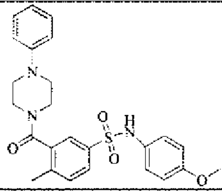 |
| 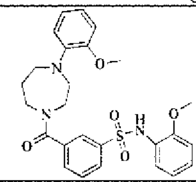 |
| 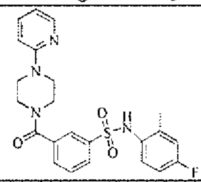 |
| 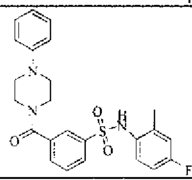 |
| 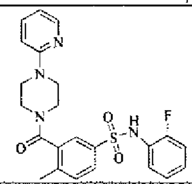 |
| 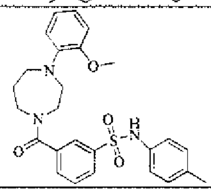 |

| Compound |
|---|
| 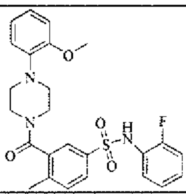 |
| 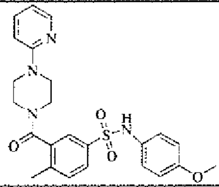 |
| 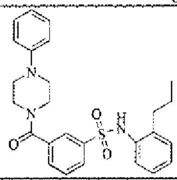 |
| 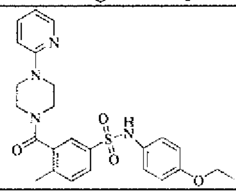 |
| 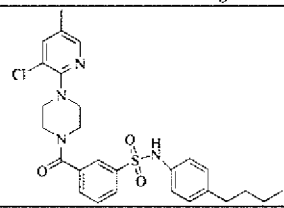 |
| 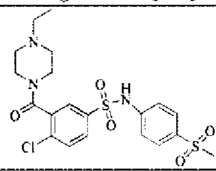 |
| 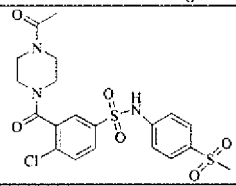 |
| 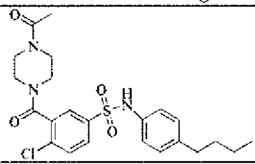 |
| 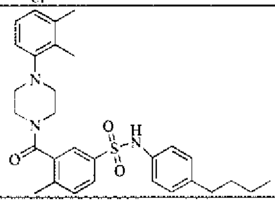 |

| Compound |
|---|
| 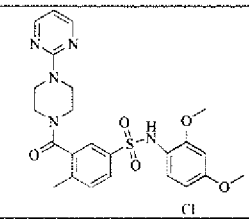 |
| 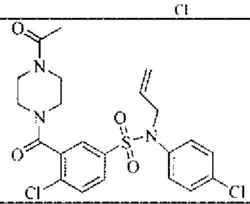 |
| 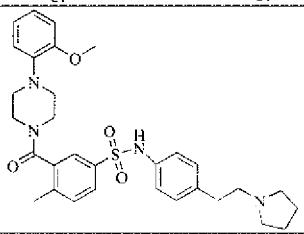 |
| 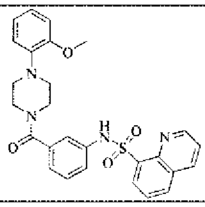 |

| Compound |
|---|
| 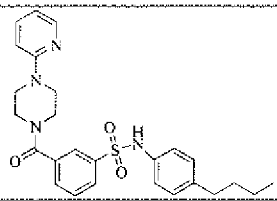 |
| 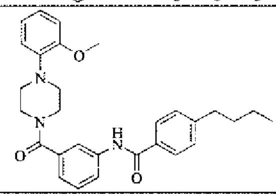 |
| 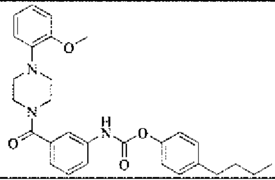 |

**3.** The use of any one of claims 1 to 2, wherein the subject is evaluated for the presence of an IDH1 R132X mutant allele prior to administration of the compound.

**4.** The use of any one of claims 1 to 2, wherein the subject is evaluated for the presence of an elevated level of 2HG prior to administration of the compound.

**5.** The use of any one of claims 1 to 2, wherein efficacy of treatment of cancer comprises monitoring the level of 2HG in a subject during treatment.

**6.** A compound of formula (I) or a pharmaceutically acceptable salt thereof:

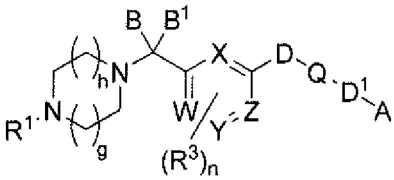

(I)

wherein:

W, X, Y and Z are each independently selected from CH or N;
B and $B^1$ are independently selected from hydrogen and alkyl or when taken together with the carbon to which they are attached form a carbonyl group;
Q is C=O or $SO_2$;
D and $D^1$ are independently selected from a bond, oxygen and $NR^c$; wherein at least one of D and $D^1$ is $NR^c$;
A is aryl or heteroaryl each substituted with 0-3 occurrences of $R^2$;
$R^1$ is independently selected from alkyl, acyl, cycloalkyl, aryl, heteroaryl, heterocyclyl, heterocyclylalkyl, cycloalkylalkyl, aralkyl, and heteroaralkyl;
each of which may be optionally substituted with 0-3 occurrences of $R^d$;
each $R^2$ is independently selected from halo, hydroxy, haloalkyl, aryl, heteroaryl, alkyl, -$NR^cR^{c'}$, alkyl-$NR^cR^{c'}$, -$OR^a$, -C(O)OH, -C(O)$OR^b$,-C(O)$NR^cR^{c'}$, cycloalkyl, heterocyclyl, heterocyclylalkyl, cycloalkylalkyl, aralkyl, and heteroaralkyl;
each $R^3$ is independently selected from halo, haloalkyl, alkyl, alkenyl, alkynyl, heterocyclyl and -$OR^a$, or two adjacent $R^3$s (when n is 2) taken together with the carbon atoms they are attached to form an optionally substituted heterocyclyl;
each $R^a$ is independently selected from alkyl, alkoxy, alkylalkoxy, alkylalkoxylalkoxy, alkyl-C(O)$OR^b$, alkyl-C(O)$OR^b$, and haloalkyl;
each $R^b$ is independently alkyl;
each $R^c$ and $R^{c'}$ is independently selected from hydrogen, alkyl, alkyl-C(O)$OR^b$ and alkenyl;
each $R^d$ is independently selected from halo, haloalkyl, alkyl, nitro, cyano, and -$OR^a$, or two $R^d$ taken together with the carbon atoms to which they are attached form an optionally substituted heterocyclyl;
n is 0, 1, or 2;
h is 0, 1, 2; and
g is 0, 1 or 2;
for use in a method of treating a cancer characterized as having an IDH mutation in a subject in need thereof.

## Patentansprüche

1. Verwendung einer Verbindung der Formel (I) oder ein pharmazeutisch verträgliches Salz davon:

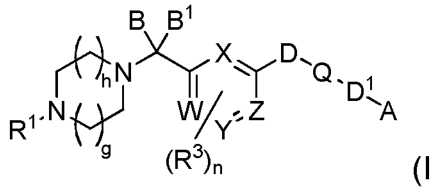

(I)

wobei:

W, X, Y und Z jeweils unabhängig ausgewählt aus CH oder N sind;
B und $B^1$ unabhängig ausgewählt aus Wasserstoff und Alkyl sind oder wenn sie zusammen mit dem Kohlenstoff, an dem sie gebunden sind, genommen sind, eine Carbonlygruppe bilden;
Q C=O oder $SO_2$ ist;
D und $D^1$ jeweils unabhängig ausgewählt aus einer Bindung, Sauerstoff und $NR^c$ sind; wobei mindestens eines von D und $D^1$ $NR^c$ ist;
A Aryl oder Heteroaryl jeweils mit 0-3 Vorkommnissen von $R^2$ substituiert ist;
$R^1$ unabhängig ausgewählt aus Alkyl, Acyl, Cycloalkyl, Aryl, Heteroaryl, Heterocyclyl, Heterocyclylalkyl, Cycloalkylalkyl, Aralkyl, und Heteroaralkyl ist; jedes davon kann gegebenenfalls mit 0-3 Vorkommnissen von $R^d$ substituiert sein;
jedes $R^2$ unabhängig ausgewählt aus Halo, Hydroxy, Haloalkyl, Aryl, Heteroaryl, Alkyl, -$NR^cR^{c'}$, Alkyl-$NR^cR^{c'}$, -$OR^a$, -C(O)OH, - C(O)$OR^b$, -C(O)$NR^cR^{c'}$, Cycloalkyl, Heterocyclyl, Heterocyclylalkyl, Cycloalkylalkyl, Aralkyl, und Heteroaralkyl ist;
jedes $R^3$ unabhängig ausgewählt aus Halo, Haloalkyl, Alkyl, Alkenyl, Alkynyl, Heterocyclyl und -$OR^a$ ist, oder zwei benachbarte $R^3$ (wenn n 2 ist) zusammen mit den Kohlenstoffatomen, an die sie gebunden sind, ein gegebenenfalls substituiertes Heterocyclyl bilden;
jedes $R^a$ unabhängig ausgewählt aus Alkyl, Alkoxy, Alkylalkoxy, Alkylalkoxylalkoxy, Alkyl-C(O)$OR^b$, Alkyl-C(O)$OR^b$ und Haloalkyl ist;
jedes $R^b$ unabhängig Alkyl ist;
jedes $R^c$ und $R^{c'}$ unabhängig ausgewählt aus Wasserstoff, Alkyl, Alkyl-C(O)$OR^b$ und Alkenyl ist;
jedes $R^d$ unabhängig ausgewählt aus Halo, Haloalkyl, Alkyl, Nitro, Cyano, und -$OR^a$ ist, oder zwei $R^d$ zusammen mit den Kohlenstoffatomen, an die sie gebunden sind, ein gegebenenfalls substituiertes Heterocyclyl bilden;
n 0, 1, oder 2 ist;
h 0, 1, 2 ist; und

g 0, 1 oder 2 ist;
in der Herstellung eines Medikaments zur Behandlung eines Krebses, **dadurch gekennzeichnet, dass** er eine IDH-Mutation hat, in einem Subjekt, das dessen Bedarf.

**2.** Verwendung nach Anspruch 1, wobei die Verbindung ausgewählt ist aus irgendeiner der Verbindungen in der folgenden unten stehenden Tabelle:

| Verbindung |
|---|
| 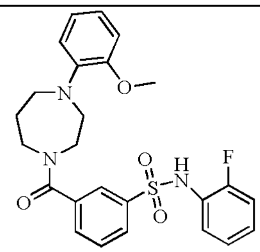 |

| Verbindung |
|---|
| 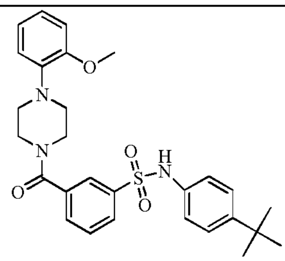 |

| Verbindung |
| --- |
| 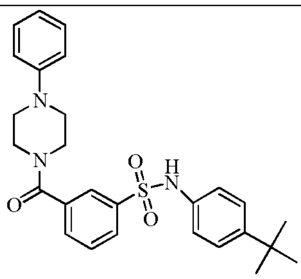 |
| 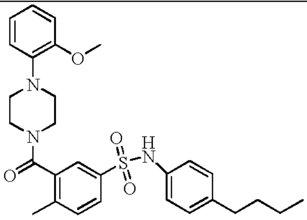 |
| 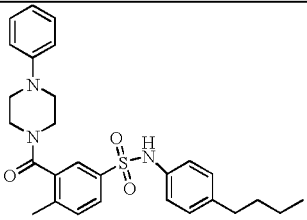 |
| 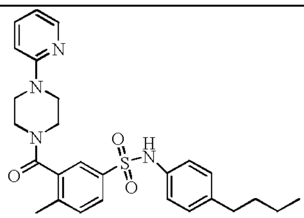 |
| 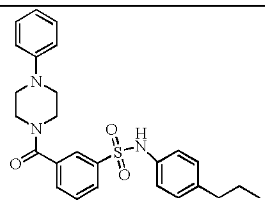 |
| 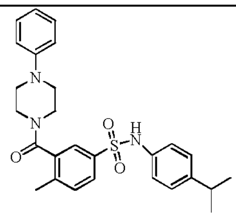 |

| Verbindung |
| --- |
| 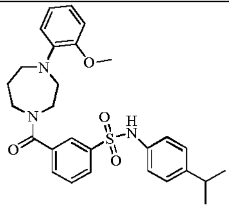 |
| 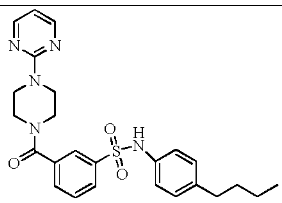 |
| 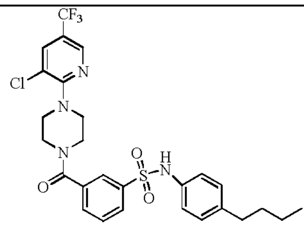 |
| 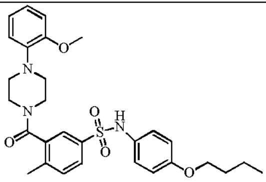 |
| 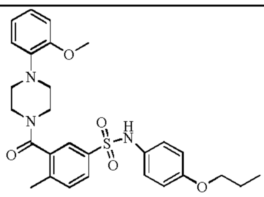 |
| 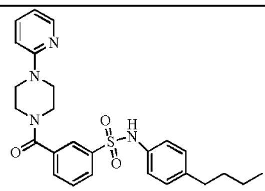 |
| 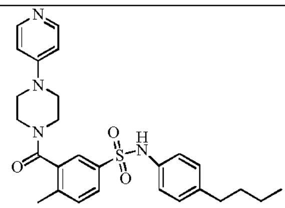 |

| Verbindung |
| --- |
| 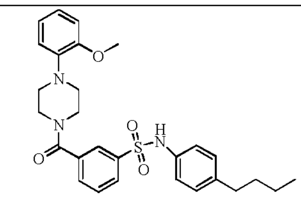 |
| 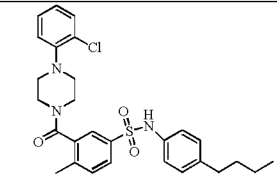 |
| 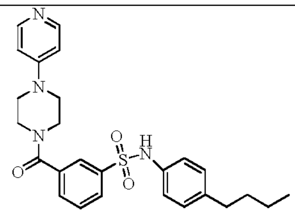 |
| 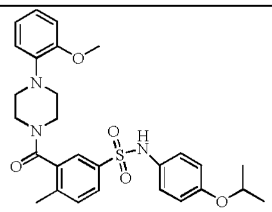 |
| 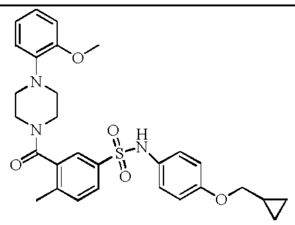 |
| 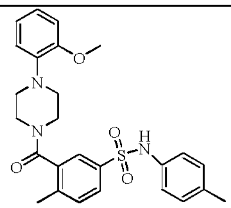 |
| 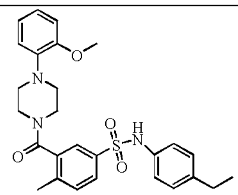 |

| Verbindung |
| --- |
| 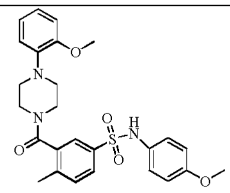 |
| 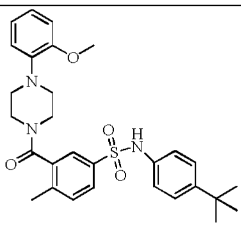 |
| 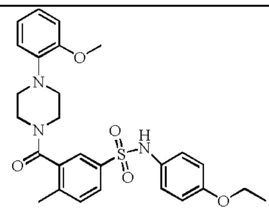 |
| 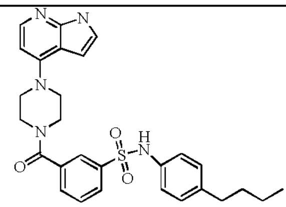 |
| 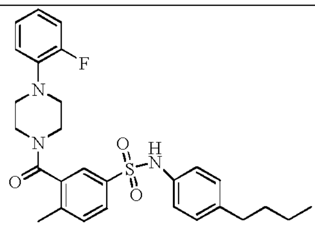 |
| 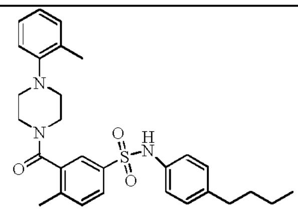 |
| 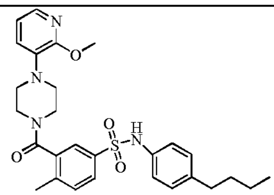 |

| Verbindung |
|---|
| 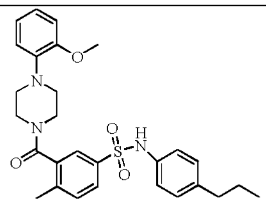 |
| 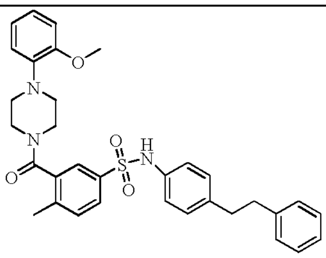 |
| 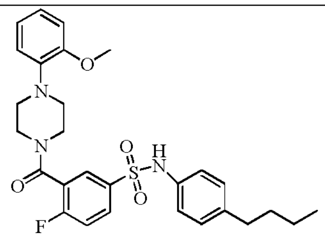 |
| 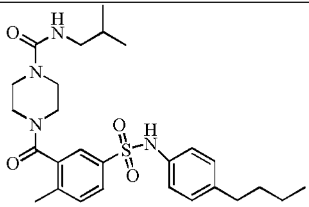 |
| 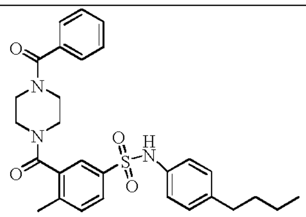 |
| 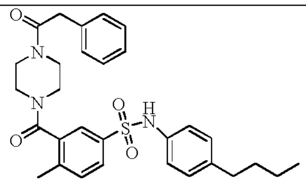 |
| 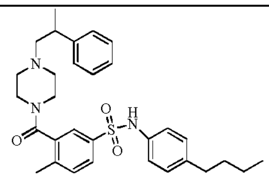 |

| Verbindung |
|---|
| 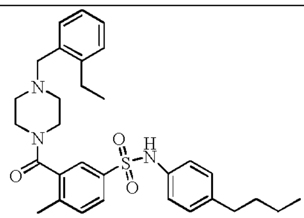 |
| 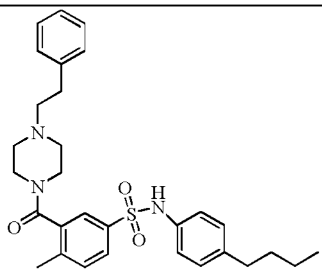 |
| 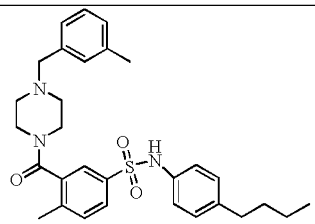 |
| 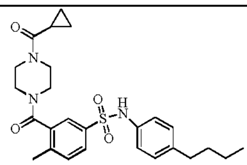 |
| 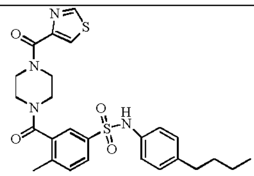 |
| 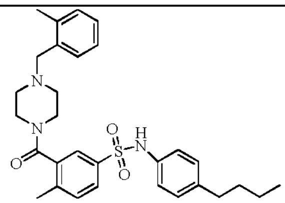 |
| 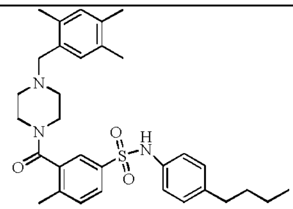 |

| Verbindung |
| --- |
| 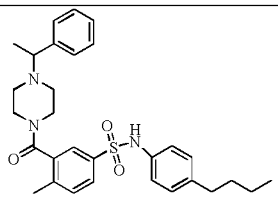 |
| 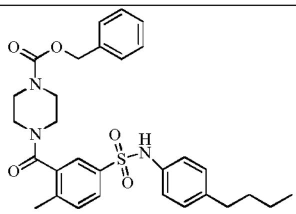 |
| 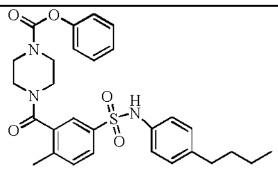 |
| 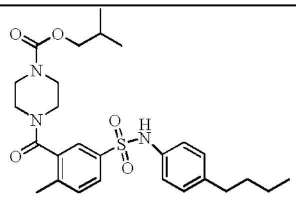 |
| 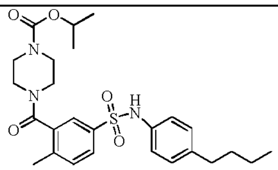 |
| 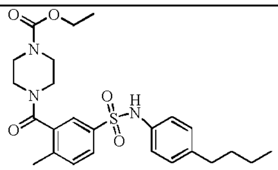 |
| 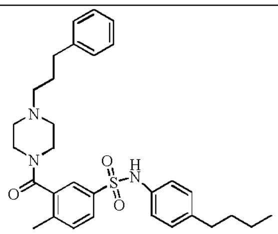 |

| Verbindung |
| --- |
| 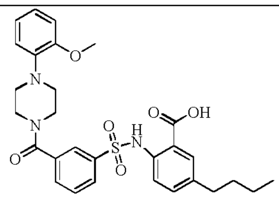 |
| 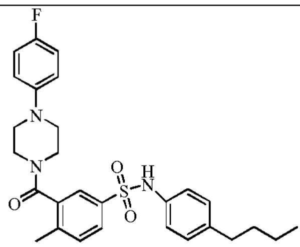 |
| 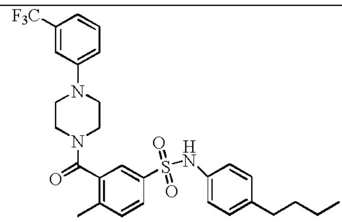 |
| 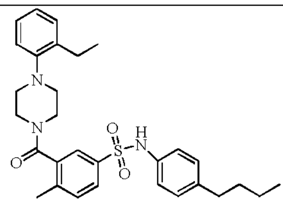 |
| 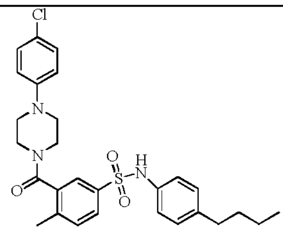 |
| 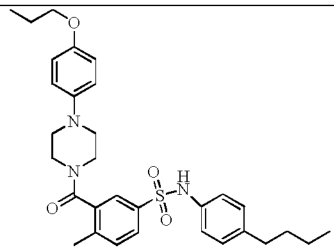 |

| Verbindung |
|---|
| 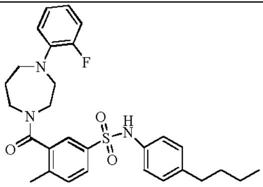 |
| 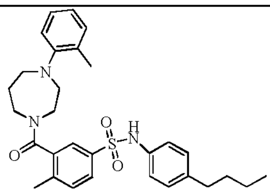 |
| 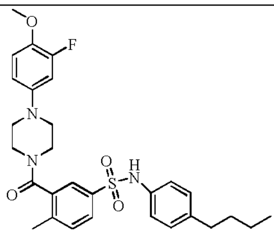 |
| 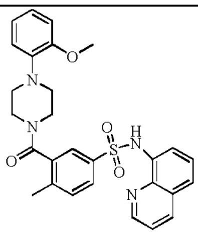 |
| 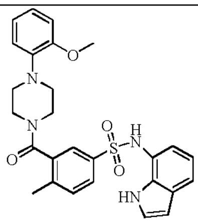 |
| 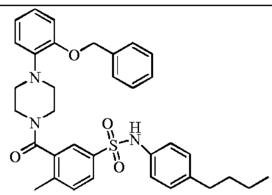 |
| 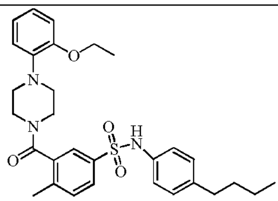 |

| Verbindung |
|---|
| 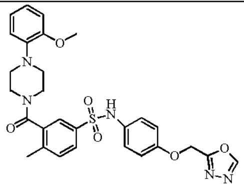 |
| 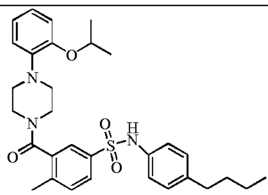 |
| 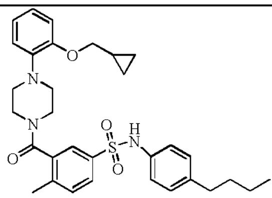 |
| 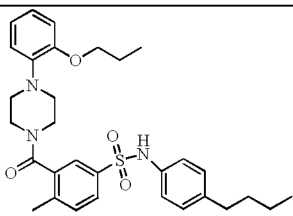 |
| 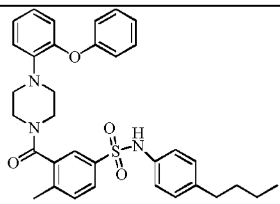 |
| 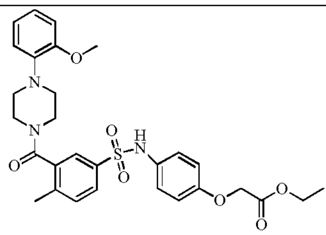 |
| 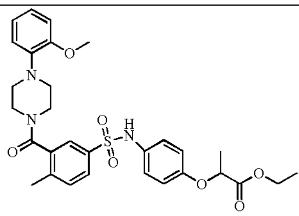 |

| Verbindung |
| --- |
| 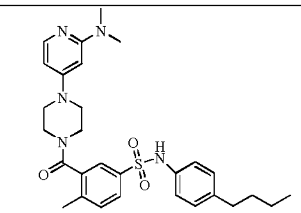 |
| 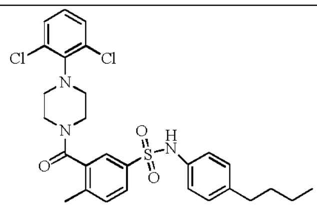 |
| 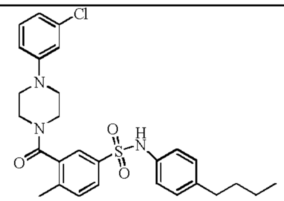 |
| 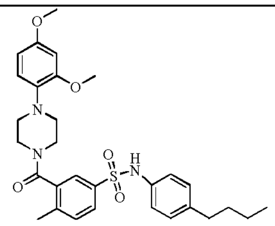 |
| 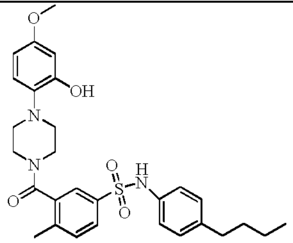 |
| 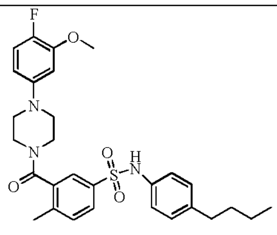 |

| Verbindung |
| --- |
| 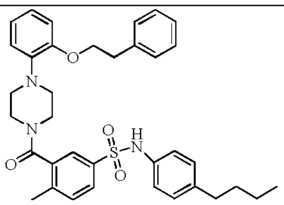 |
| 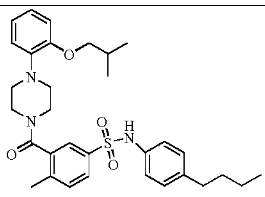 |
| 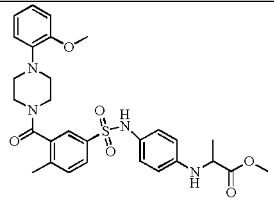 |
| 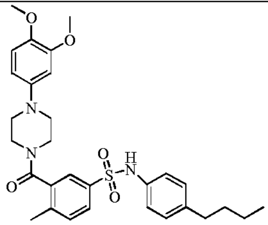 |
| 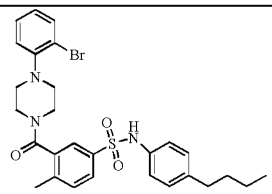 |
| 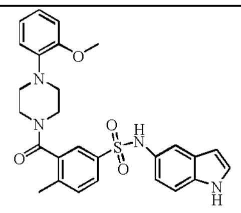 |
| 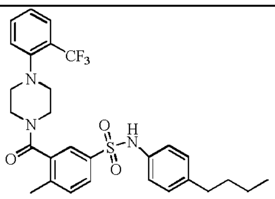 |

| Verbindung |
|---|
| 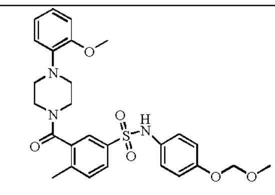 |
| 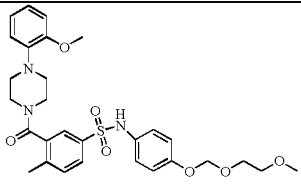 |
| 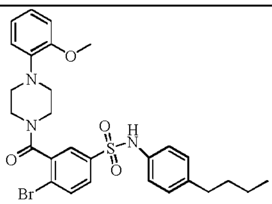 |
| 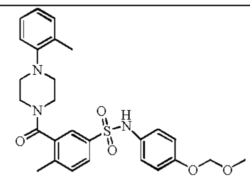 |
| 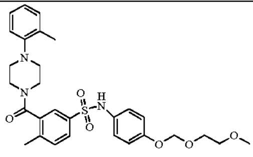 |
| 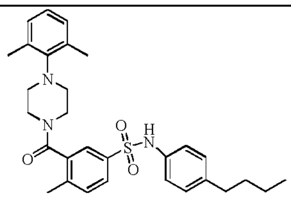 |
| 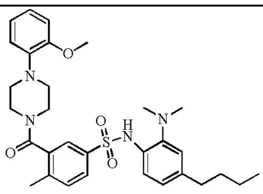 |

| Verbindung |
|---|
| 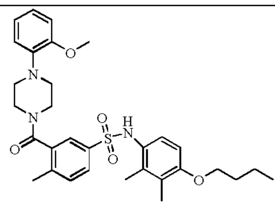 |
| 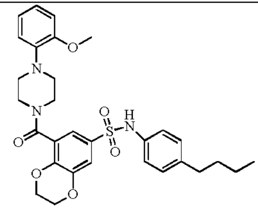 |
| 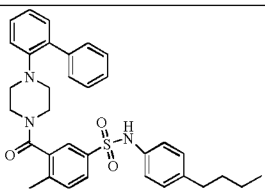 |
| 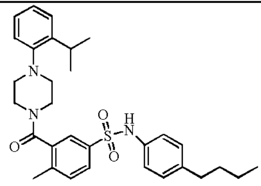 |
| 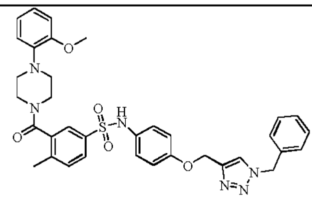 |
| 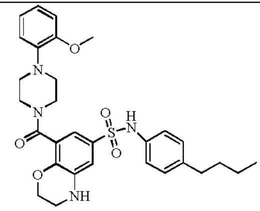 |
| 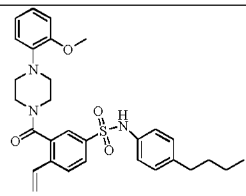 |

| Verbindung | Verbindung |
| --- | --- |
| 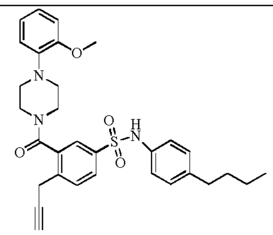 | 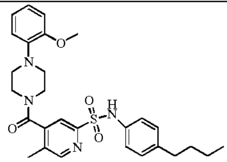 |
| 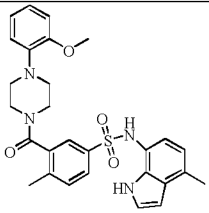 | 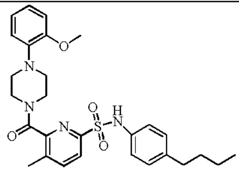 |
| 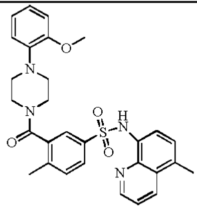 | 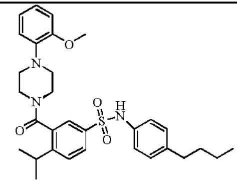 |
| 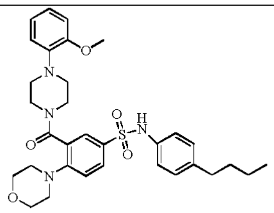 | 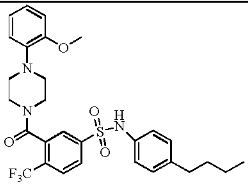 |
| 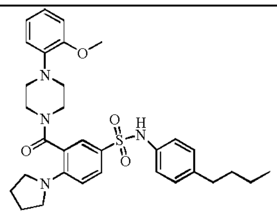 | 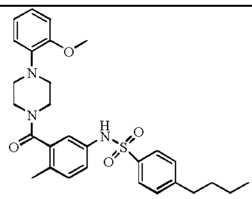 |
| 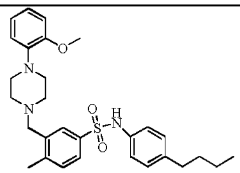 | 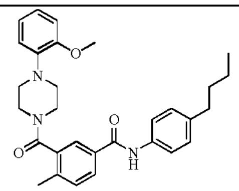 |
| 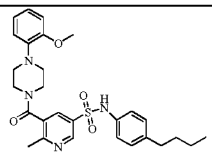 | 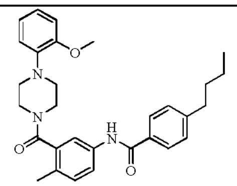 |
| | 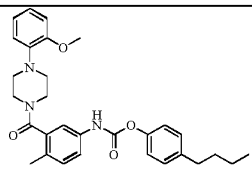 |

| Verbindung |
| --- |
| 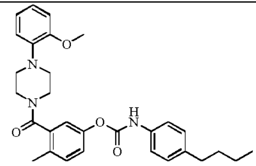 |
| 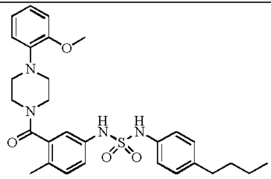 |
| 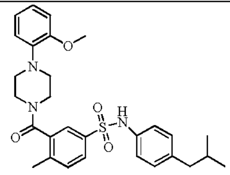 |
| 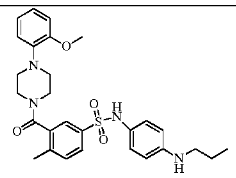 |
| 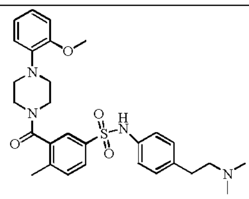 |
| 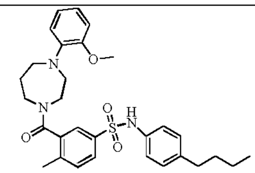 |
| 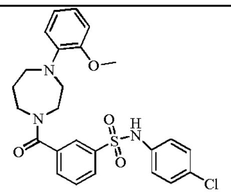 |
| 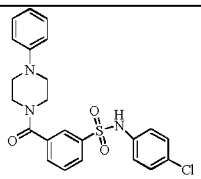 |

| Verbindung |
| --- |
| 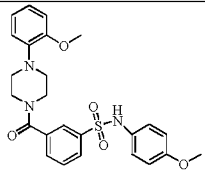 |
| 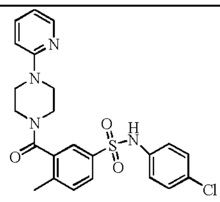 |
| 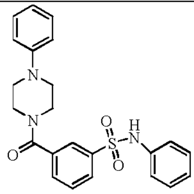 |
| 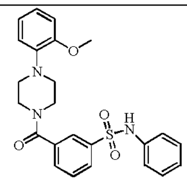 |
| 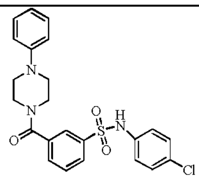 |
| 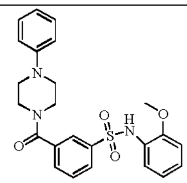 |
| 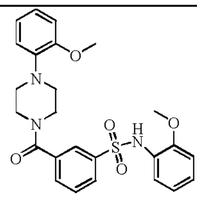 |

| Verbindung |
|---|
| 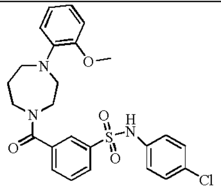 |
| 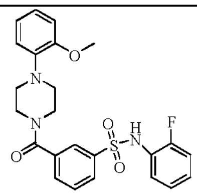 |
| 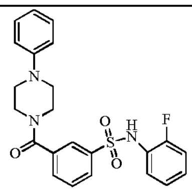 |
| 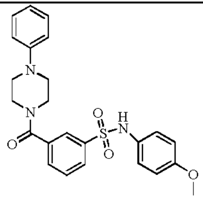 |
| 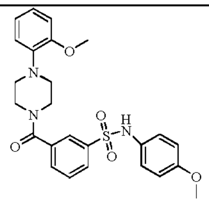 |
| 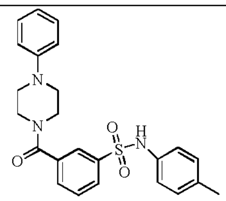 |
| 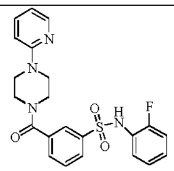 |

| Verbindung |
|---|
| 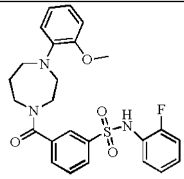 |
| 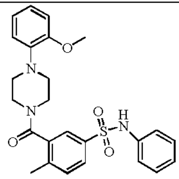 |
| 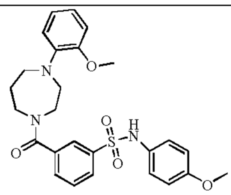 |
| 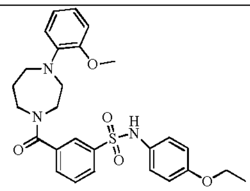 |
| 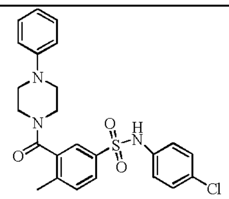 |
| 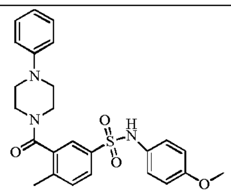 |
| 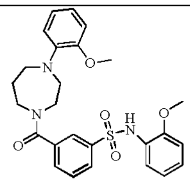 |

| Verbindung |
|---|
| 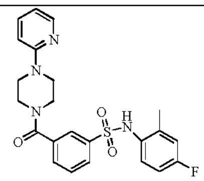 |
| 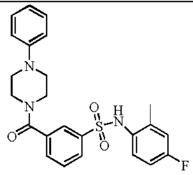 |
| 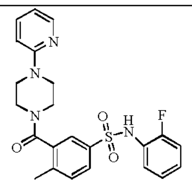 |
| 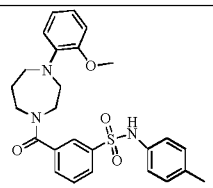 |
| 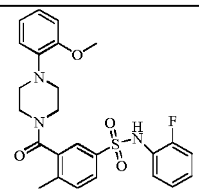 |
| 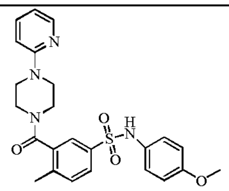 |
| 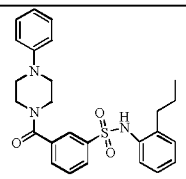 |
| 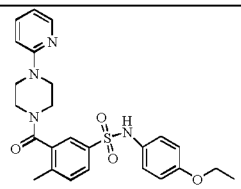 |

| Verbindung |
|---|
| 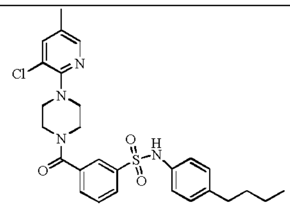 |
| 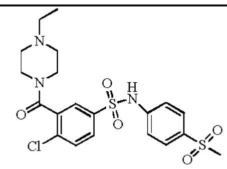 |
| 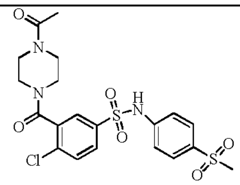 |
| 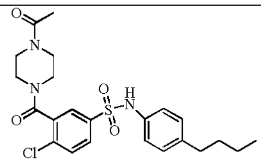 |
| 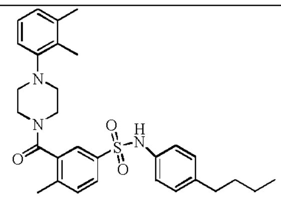 |
| 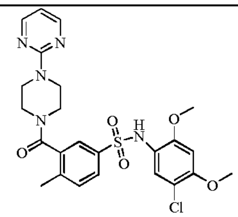 |
| 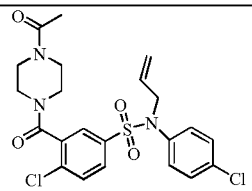 |

| Verbindung |
|---|
| 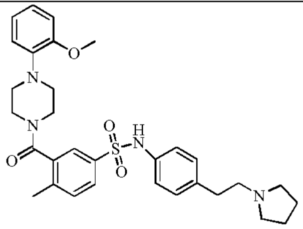 |
| 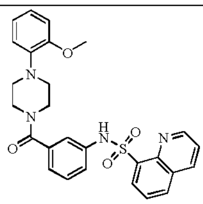 |
| 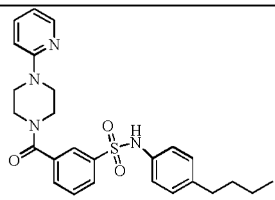 |
| 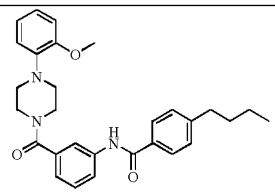 |
| 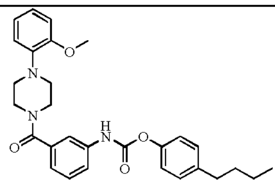 |

**3.** Verwendung nach irgendeinem der Ansprüche 1 bis 2, wobei das Subjekt auf die Anwesenheit eines IDH1 R132X Mutanten Allels vor der Gabe der Verbindung untersucht wird.

**4.** Verwendung nach irgendeinem der Ansprüche 1 bis 2, wobei das Subjekt auf die Anwesenheit eines erhöhten Levels an 2HG vor der Gabe der Verbindung untersucht wird.

**5.** Verwendung nach irgendeinem der Ansprüche 1 bis 2, wobei die Effizienz der Krebsbehandlung das Überwachen des Levels an 2HG in einem Subjekt während der Behandlung umfasst.

**6.** Verbindung nach Formel (I) oder ein pharmazeutisch verträgliches Salz davon:

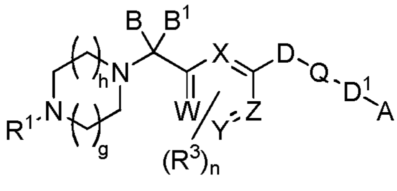

(I)

wobei:

W, X, Y und Z jeweils unabhängig ausgewählt aus CH oder N sind;
B und $B^1$ unabhängig ausgewählt aus Wasserstoff und Alkyl sind oder wenn sie zusammen mit dem Kohlenstoff, an dem sie gebunden sind, genommen sind, eine Carbonlygruppe bilden;
Q C=O oder $SO_2$ ist;
D und $D^1$ jeweils unabhängig ausgewählt aus einer Bindung, Sauerstoff und $NR^c$ sind; wobei mindestens eines von D und $D^1$ $NR^c$ ist;
A Aryl oder Heteroaryl jeweils mit 0-3 Vorkommnissen von $R^2$ substituiert ist;
$R^1$ unabhängig ausgewählt aus Alkyl, Acyl, Cycloalkyl, Aryl, Heteroaryl, Heterocyclyl, Heterocyclylalkyl, Cycloalkylalkyl, Aralkyl, und Heteroaralkyl ist; jedes davon kann gegebenenfalls mit 0-3 Vorkommnissen von $R^d$ substituiert sein;
jedes $R^2$ unabhängig ausgewählt aus Halo, Hydroxy, Haloalkyl, Aryl, Heteroaryl, Alkyl, $-NR^cR^{c'}$, Alkyl-$NR^cR^{c'}$, $-OR^a$, -C(O)OH, $-C(O)OR^b$,$-C(O)NR^cR^{c'}$, Cycloalkyl, Heterocyclyl, Heterocyclylalkyl, Cycloalkylalkyl, Aralkyl, und Heteroaralkyl ist;
jedes $R^3$ unabhängig ausgewählt aus Halo, Haloalkyl, Alkyl, Alkenyl, Alkynyl, Heterocyclyl und $-OR^a$ ist, oder zwei benachbarte $R^3$ (wenn n 2 ist) zusammen mit den Kohlenstoffatomen, an die sie gebunden sind, ein gegebenenfalls substituiertes Heterocyclyl bilden;
jedes $R^a$ unabhängig ausgewählt aus Alkyl, Alkoxy, Alkylalkoxy, Alkylalkoxylalkoxy, Alkyl-$C(O)OR^b$, Alkyl-$C(O)OR^b$ und Haloalkyl ist;
jedes $R^b$ unabhängig Alkyl ist;
jedes $R^c$ und $R^{c'}$ unabhängig ausgewählt aus Wasserstoff, Alkyl, Alkyl-$C(O)OR^b$ und Alkenyl ist;
jedes $R^d$ unabhängig ausgewählt aus Halo, Haloalkyl, Alkyl, Nitro, Cyano, und $-OR^a$ ist, oder zwei $R^d$ zusammen mit den Kohlenstoffatomen, an die sie gebunden sind, ein gegebenenfalls substituiertes Heterocyclyl bilden;
n 0, 1, oder 2 ist;
h 0, 1, 2 ist; und
g 0, 1 oder 2 ist;
zur Verwendung in einem Verfahren zur Behandlung eines Krebses **dadurch gekennzeichnet, dass** er eine IDH-Mutation in einem Subjekt hat, das dessen Bedarf hat.

## Revendications

**1.** Utilisation d'un composé de formule (I) ou d'un de ses sels pharmaceutiquement acceptables :

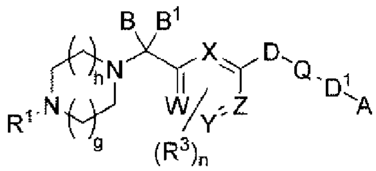
(I)

dans laquelle :

W, X, Y et Z sont choisis chacun indépendamment parmi CH ou N ;
B et $B^1$ sont choisis indépendamment parmi un hydrogène et un groupe alkyle ou, quand ils sont pris conjointement avec le carbone auquel ils sont attachés, forment un groupe carbonyle ;
Q est un groupe C=O ou $SO_2$ ;
D et $D^1$ sont choisi indépendamment parmi une liaison, un oxygène et $NR^c$ ; où au moins un de D et D1 est $NR^c$ ;
A est un groupe aryle ou hétéroaryle chacun substitué avec 0 à 3 occurrences de $R^2$ ;
$R^1$ est choisi indépendamment parmi des groupes alkyle, acyle, cycloalkyle, aryle, hétéroaryle, hétérocyclyle, hétérocyclylalkyle, cycloalkylalkyle, aralkyle et hétéroaralkyle ; dont chacun peut être éventuellement substitué avec 0 à 3 occurrences de $R^d$ ;
chaque $R^2$ est indépendamment choisi parmi des groupes halogéno, hydroxy, halogénalkyle, aryle, hétéroaryle, alkyle, $-NR^cR^{c'}$, alkyl-$NR^cR^{c'}$, $-OR^a$, -C(O)OH, $-C(O)OR^b$, $-C(O)NR^cR^{c'}$, cycloalkyle, hétérocyclyle, hétérocyclylalkyle, cycloalkylalkyle, aralkyle et hétéroaralkyle ;
chaque $R^3$ est choisi indépendamment parmi des groupes halogéno, halogénalkyle, alkyle, alcényle, alcynyle, hétérocyclyle et $-OR^a$, ou deux $R^3$ adjacents (quand n vaut 2) pris conjointement avec les atomes de carbone auxquels ils sont attachés forment un groupe hétérocyclyle éventuellement substitué ;
chaque $R^a$ est choisi indépendamment parmi des groupes alkyle, alkoxy, alkylalkoxy, alkylalkoxylalkoxy, alkyl-C(O)OR$^b$, alkyl-C(O)OR$^b$ et halogénalkyle ;
chaque R$^b$ est indépendamment un groupe alkyle ;
chaque R$^c$ et R$^{c'}$ est choisi indépendamment parmi un hydrogène, des groupes alkyle, alkyl-C(O)OR$^b$ et alcényle ;
chaque R$^d$ est choisi indépendamment parmi des groupes halogéno, halogénalkyle, alkyle, nitro, cyano et -OR$^a$, ou deux R$^d$ pris conjointement avec les atomes de carbone auxquels ils sont attachés forment un groupe hétérocyclyle éventuellement substitué ;
n vaut 0, 1 ou 2 ;
h vaut 0, 1, 2 ; et
g vaut 0, 1 ou 2 ;
dans la fabrication d'un médicament pour traiter un cancer caractérisé comme ayant une mutation de IDH chez un sujet en ayant besoin.

**2.** Utilisation selon la revendication 1, le composé étant choisi parmi l'un quelconque des composés présentés dans le tableau ci-dessous :

| Composé |
| --- |
| 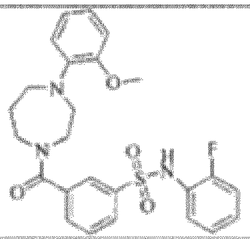 |
| 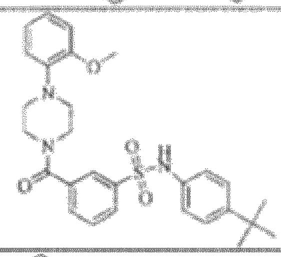 |
| 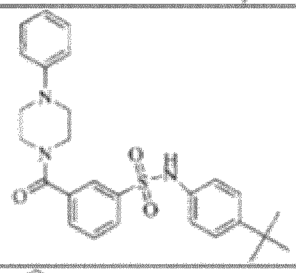 |
| 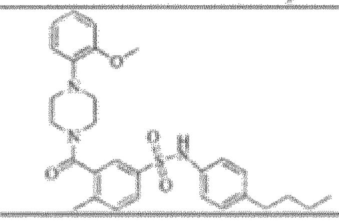 |

| Composé |
| --- |
| 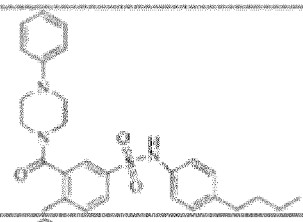 |
| 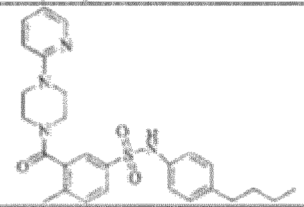 |
| 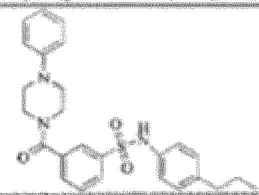 |
| 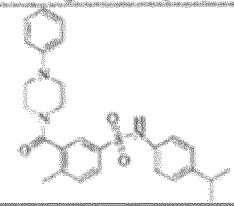 |
| 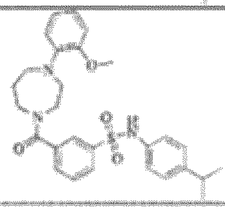 |

| Composé |
|---|
| 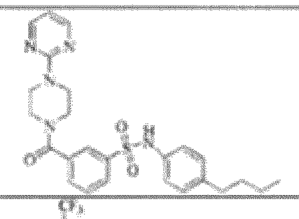 |
| 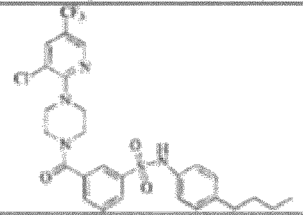 |
| 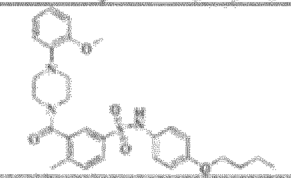 |
| 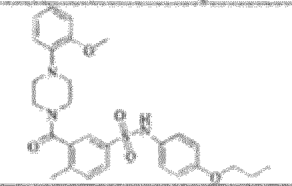 |
| 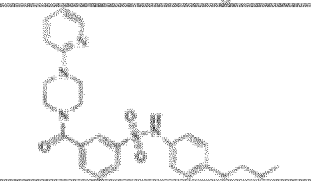 |
| 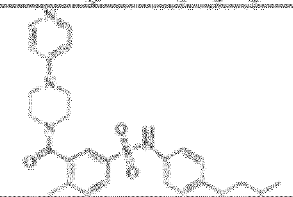 |
| 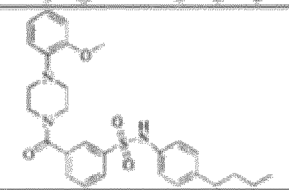 |
| 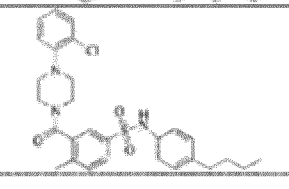 |

| Composé |
|---|
| 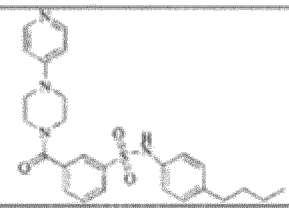 |
| 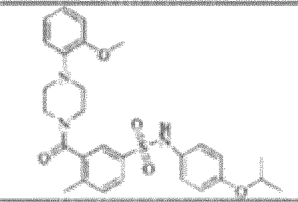 |
| 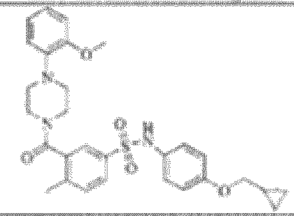 |
| 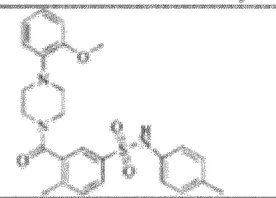 |
| 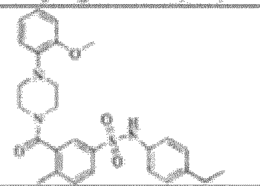 |
| 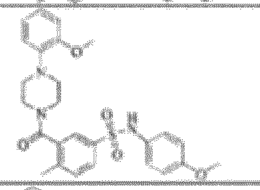 |
| 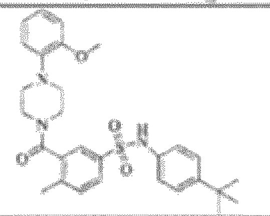 |
| 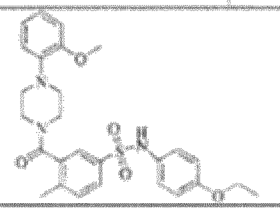 |

| Composé |
|---|
| 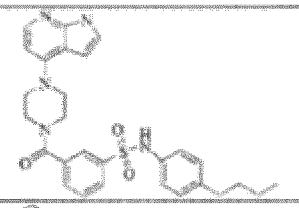 |
| 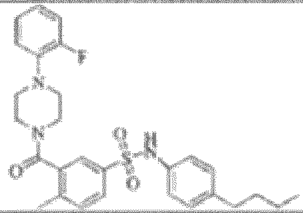 |
| 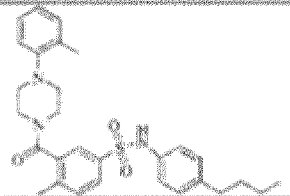 |
| 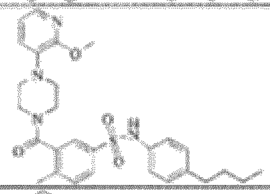 |
| 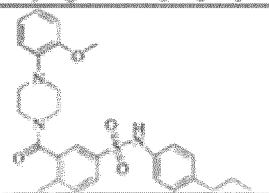 |
| 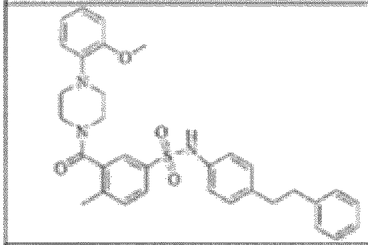 |
| 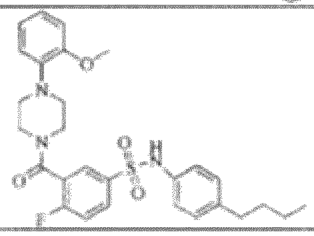 |

| Composé |
|---|
| 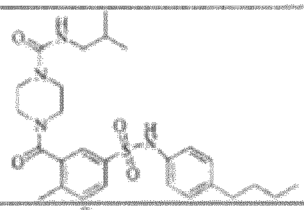 |
| 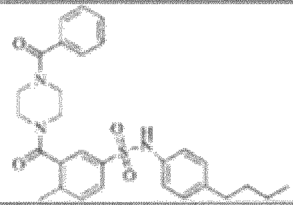 |
| 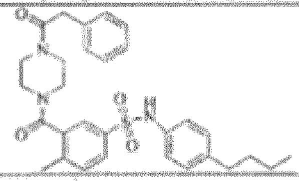 |
| 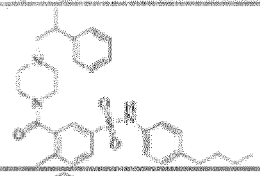 |
| 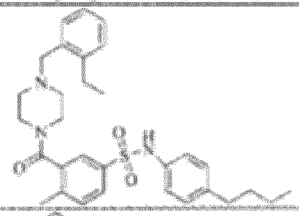 |
| 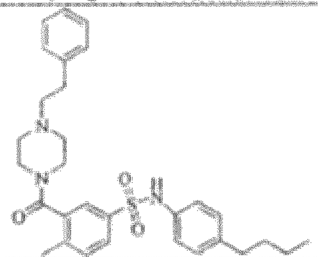 |
| 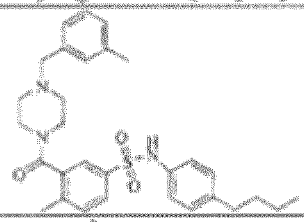 |
| 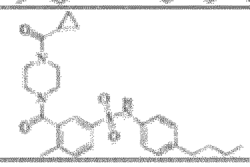 |

| Composé |
|---|
| 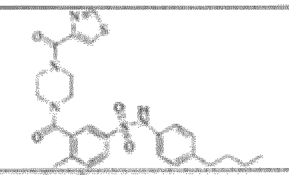 |
| 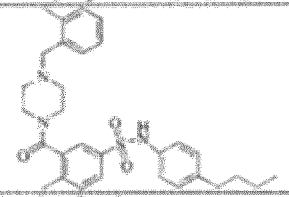 |
| 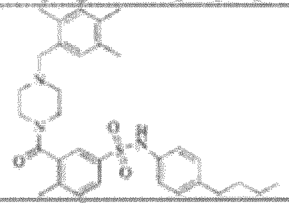 |
| 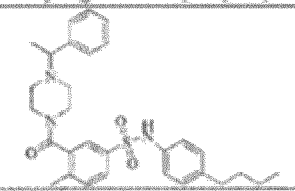 |
| 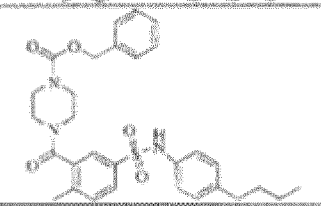 |
| 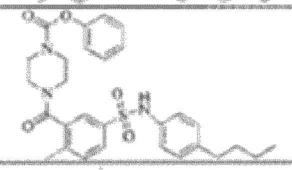 |
| 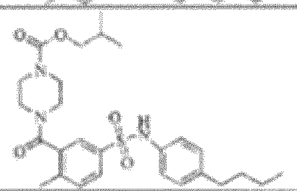 |
| 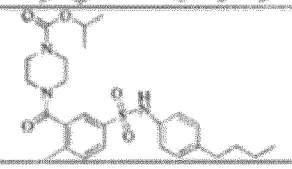 |

| Composé |
|---|
| 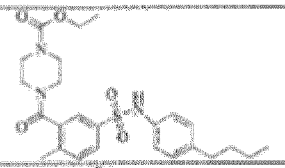 |
| 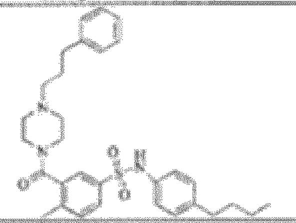 |
| 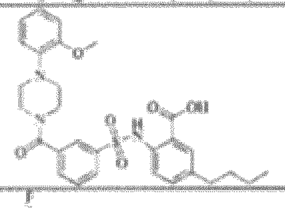 |
| 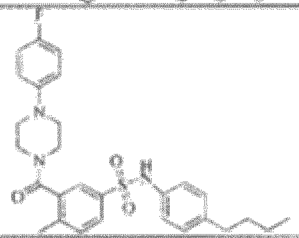 |
| 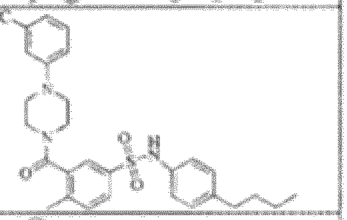 |
| 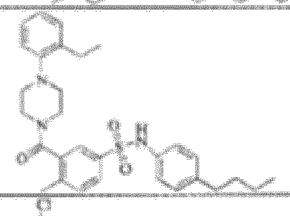 |
| 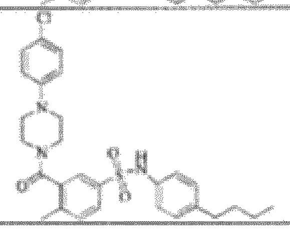 |

| Composé |
|---|
| 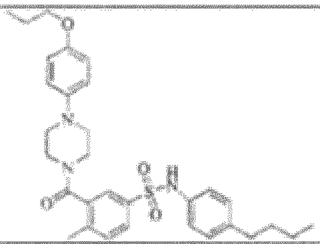 |
| 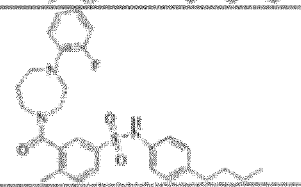 |
| 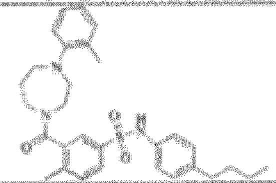 |
| 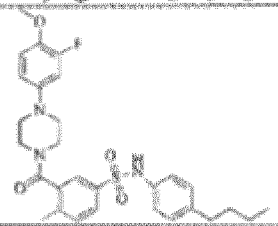 |
| 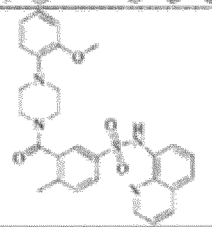 |
| 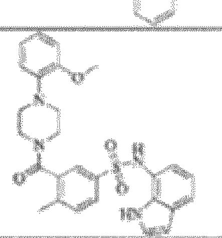 |
| 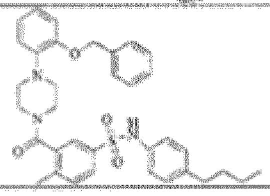 |

| Composé |
|---|
| 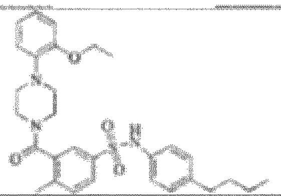 |
| 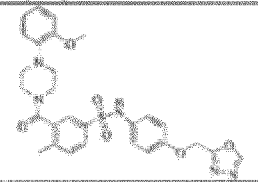 |
| 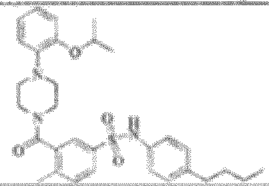 |
| 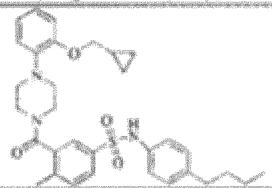 |
| 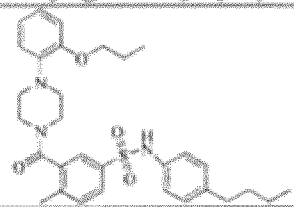 |
| 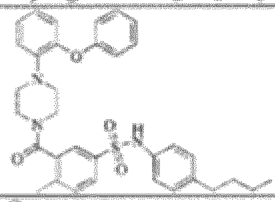 |
| 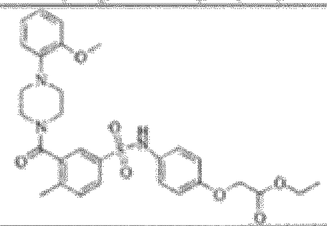 |
| 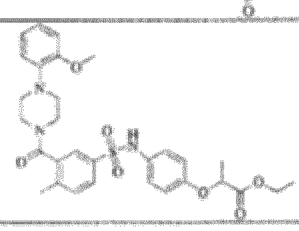 |

| Composé |
|---|
| 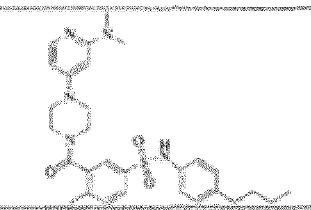 |
| 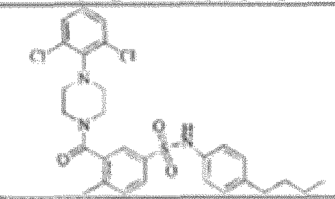 |
| 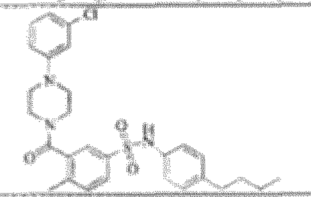 |
| 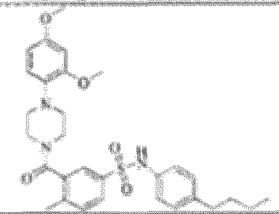 |
| 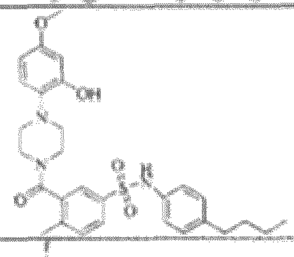 |
| 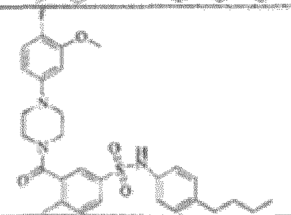 |
| 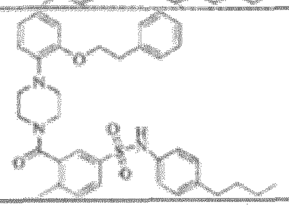 |

| Composé |
|---|
| 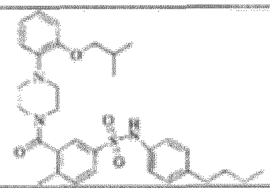 |
| 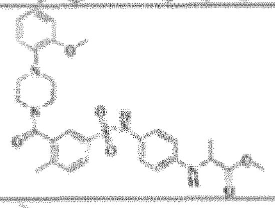 |
| 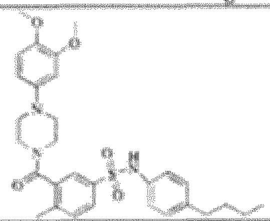 |
| 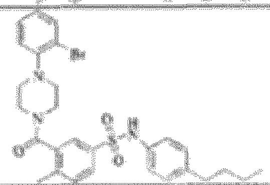 |
| 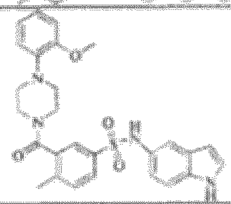 |
| 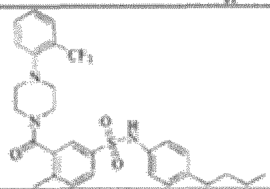 |
| 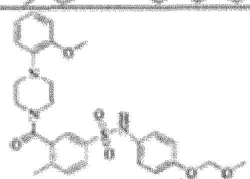 |
| 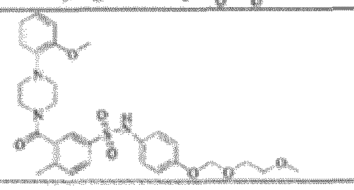 |

| Composé |
|---|
| 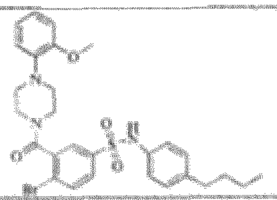 |
| 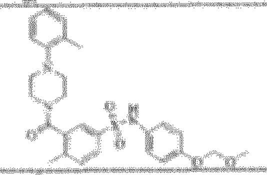 |
| 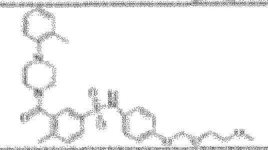 |
| 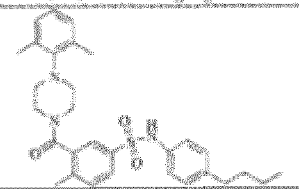 |
| 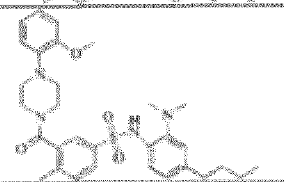 |
| 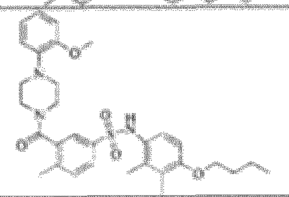 |
| 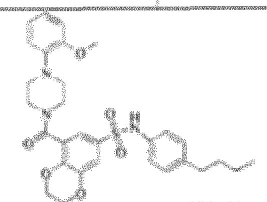 |
| 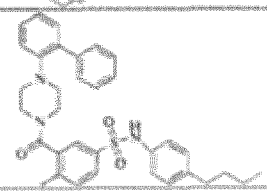 |

| Composé |
|---|
| 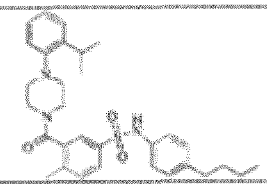 |
| 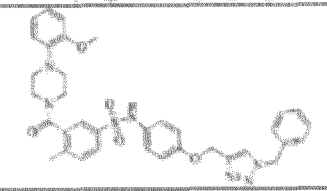 |
| 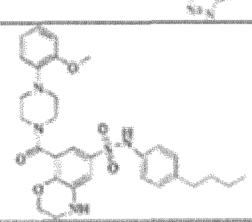 |
| 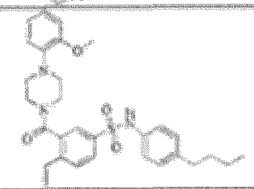 |
| 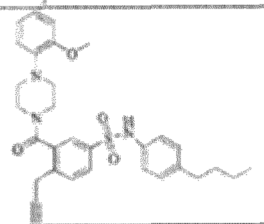 |
| 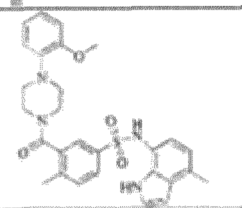 |
| 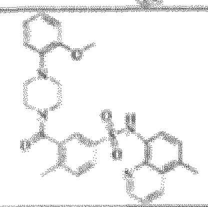 |
| 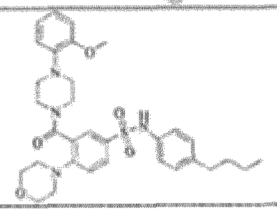 |

| Composé |
|---|
| 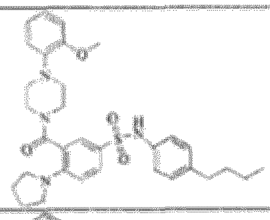 |
| 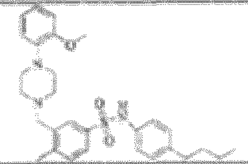 |
| 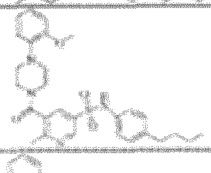 |
| 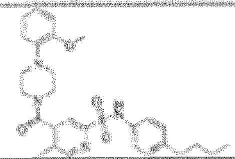 |
| 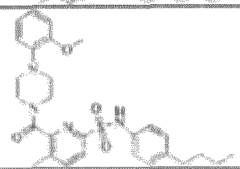 |
| 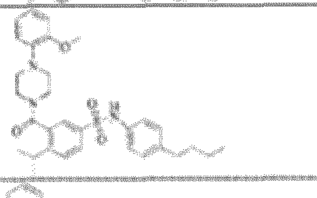 |
| 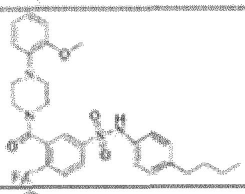 |
| 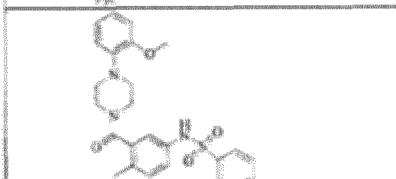 |
| 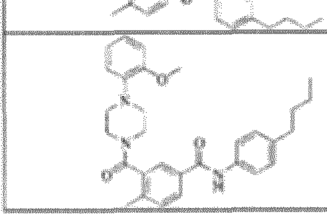 |

| Composé |
|---|
| 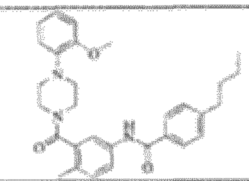 |
| 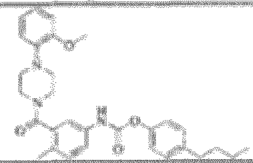 |
| 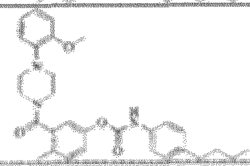 |
| 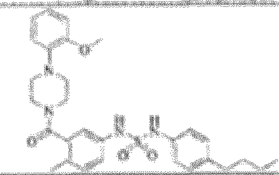 |
| 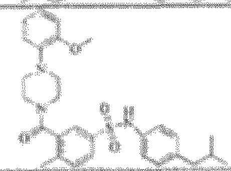 |
| 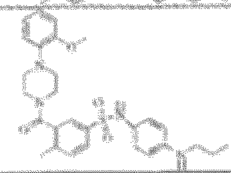 |
| 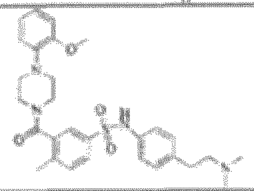 |
| 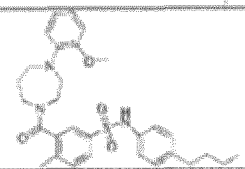 |
| 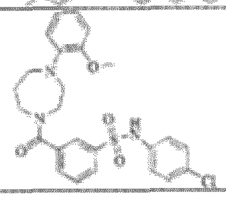 |

| Composé |
|---|
| 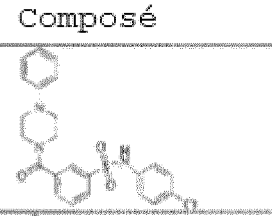 |
| 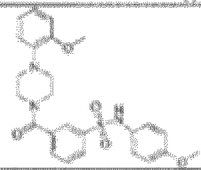 |
| 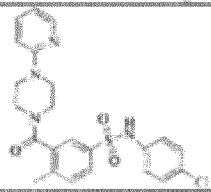 |
| 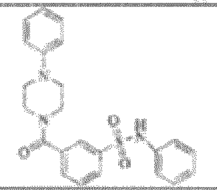 |
| 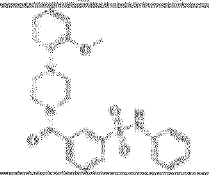 |
| 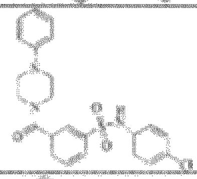 |
| 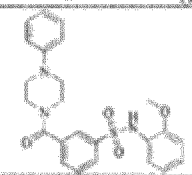 |
| 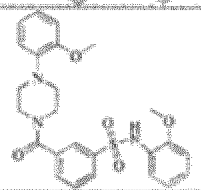 |
| 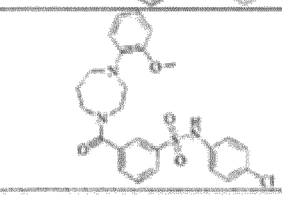 |

| Composé |
|---|
| 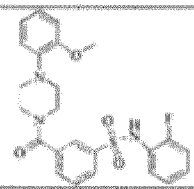 |
| 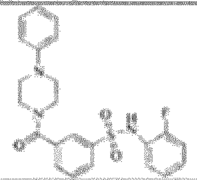 |
| 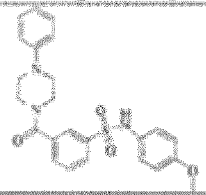 |
| 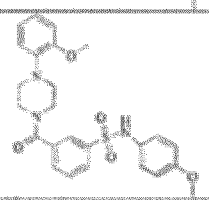 |
| 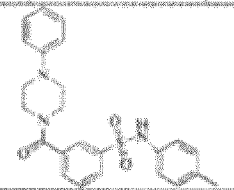 |
| 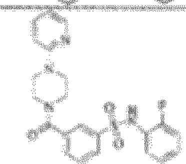 |
| 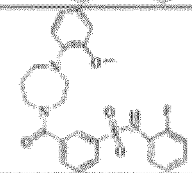 |
| 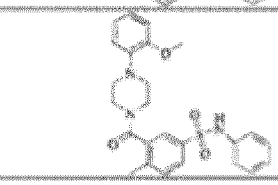 |

| Composé |
|---|
| 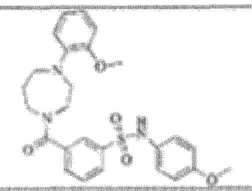 |
| 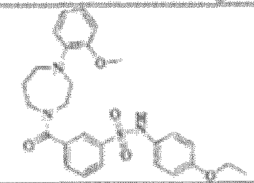 |
| 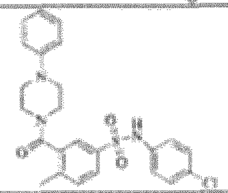 |
| 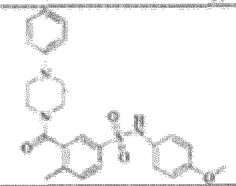 |
| 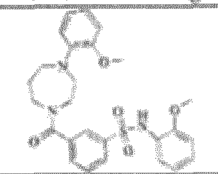 |
| 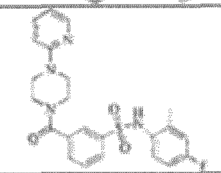 |
| 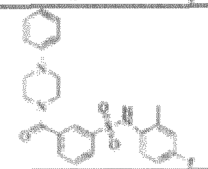 |
| 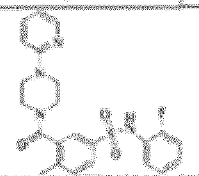 |
| 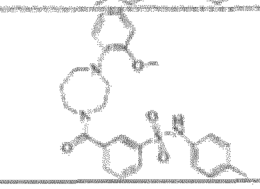 |

| Composé |
|---|
| 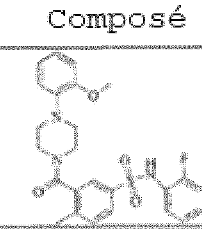 |
| 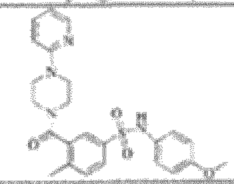 |
| 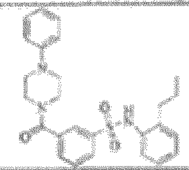 |
| 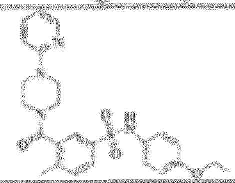 |
| 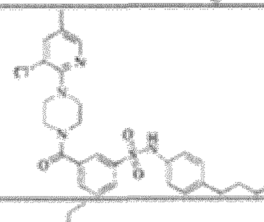 |
| 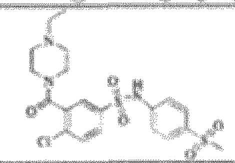 |
| 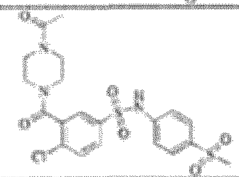 |
| 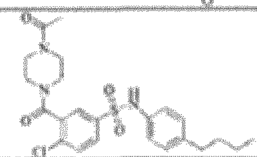 |
| 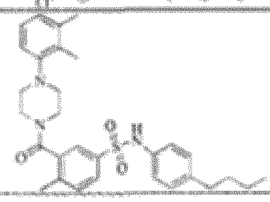 |

| Composé |
|---|
| 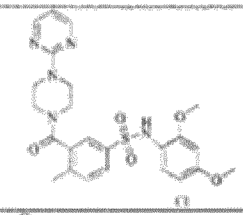 |
| 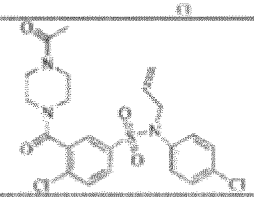 |
| 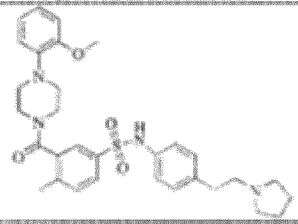 |
| 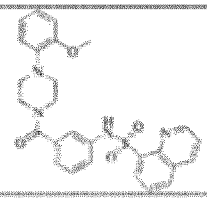 |

| Composé |
|---|
| 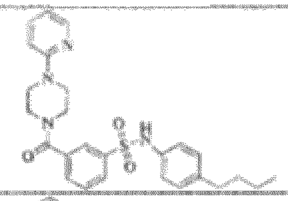 |
| 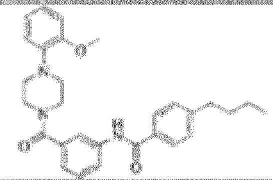 |
| 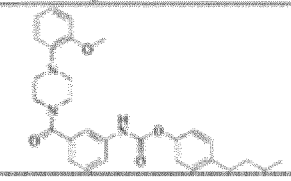 |

**3.** Utilisation selon l'une quelconque des revendications 1 et 2, le sujet étant évalué pour la présence d'un allèle mutant R132X de IDH1 avant l'administration du composé.

**4.** Utilisation selon l'une quelconque des revendications 1 et 2, le sujet étant évalué pour la présence d'un niveau élevé de 2HG avant l'administration du composé.

**5.** Utilisation selon l'une quelconque des revendications 1 et 2, l'efficacité du traitement du cancer comprenant le suivi du niveau de 2HG chez un sujet durant le traitement.

**6.** Composé de formule (I) ou un de ses sels pharmaceutiquement acceptables :

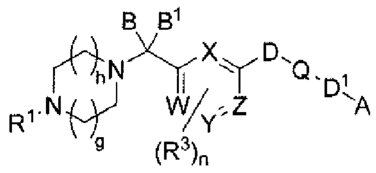

(I)

dans laquelle :

W, X, Y et Z sont choisis chacun indépendamment parmi CH ou N ;
B et $B^1$ sont choisis indépendamment parmi un hydrogène et un groupe alkyle ou, quand ils sont pris conjointement avec le carbone auquel ils sont attachés, forment un groupe carbonyle ;
Q est un groupe C=O ou $SO_2$ ;
D et $D^1$ sont choisi indépendamment parmi une liaison, un oxygène et $NR^c$ ; où au moins un de D et D1 est $NR^c$ ;
A est un groupe aryle ou hétéroaryle chacun substitué avec 0 à 3 occurrences de $R^2$ ;
$R^1$ est choisi indépendamment parmi des groupes alkyle, acyle, cycloalkyle, aryle, hétéroaryle, hétérocyclyle, hétérocyclylalkyle, cycloalkylalkyle, aralkyle et hétéroaralkyle ; dont chacun peut être éventuellement substitué avec 0 à 3 occurrences de $R^d$ ;

chaque $R^2$ est indépendamment choisi parmi des groupes halogéno, hydroxy, halogénalkyle, aryle, hétéroaryle, alkyle, $-NR^cR^{c'}$, alkyl-$NR^cR^{c'}$, $-OR^a$, -C(O)OH, $-C(O)OR^b$, $-C(O)NR^cR^{c'}$, cycloalkyle, hétérocyclyle, hétérocyclylalkyle, cycloalkylalkyle, aralkyle et hétéroaralkyle ;

chaque $R^3$ est choisi indépendamment parmi des groupes halogéno, halogénalkyle, alkyle, alcényle, alcynyle, hétérocyclyle et $-OR^a$, ou deux $R^3$ adjacents (quand n vaut 2) pris conjointement avec les atomes de carbone auxquels ils sont attachés forment un groupe hétérocyclyle éventuellement substitué ;

chaque $R^a$ est choisi indépendamment parmi des groupes alkyle, alkoxy, alkylalkoxy, alkylalkoxylalkoxy, alkyl-$C(O)OR^b$, alkyl-$C(O)OR^b$ et halogénalkyle ;

chaque $R^b$ est indépendamment un groupe alkyle ;

chaque $R^c$ et $R^{c'}$ est choisi indépendamment parmi un hydrogène, des groupes alkyle, alkyl-$C(O)OR^b$ et alcényle ;

chaque $R^d$ est choisi indépendamment parmi des groupes halogéno, halogénalkyle, alkyle, nitro, cyano et $-OR^a$, ou deux $R^d$ pris conjointement avec les atomes de carbone auxquels ils sont attachés forment un groupe hétérocyclyle éventuellement substitué ;

n vaut 0, 1 ou 2 ;

h vaut 0, 1, 2 ; et

g vaut 0, 1 ou 2 ;

destiné à être utilisé dans un procédé de traitement d'un cancer caractérisé comme ayant une mutation de IDH chez un sujet en ayant besoin.

```
  1 mskkisggsv vemqgdemtr iiwelikekl ifpyveldlh sydlgienrd atndqvtkda
 61 aeaikkhnvg vkcatitpde krveefklkq mwkspngtir nilggtvfre aiickniprl
121 vsgwvkpiii grhaygdqyr atdfvvpgpg kveitytpsd gtqkvtylvh nfeegggvam
181 gmynqdksie dfahssfqma lskgwplyls tkntilkkyd grfkdifqei ydkqyksqfe
241 aqkiwyehrl iddmvaqamk seggfiwack nydgdvqsds vaqgygslgm mtsvlvcpdg
301 ktveaeaahg tvtrhyrmyq kgqetstnpi asifawtrgl ahrakldnnk elaffanale
361 evsietieag fmtkdlaaci kglpnvqrsd ylntfefmdk lgenlkikla qakl
```

FIG. 1

```
   1 atgtccaaaa aaatcagtgg cggttctgtg gtagagatgc aaggagatga aatgacacga
  61 atcatttggg aattgattaa agagaaactc atttttccct acgtggaatt ggatctacat
 121 agctatgatt taggcataga gaatcgtgat gccaccaacg accaagtcac caaggatgct
 181 gcagaagcta taaagaagca taatgttggc gtcaaatgtg ccactatcac tcctgatgag
 241 aagagggttg aggagttcaa gttgaaacaa atgtggaaat caccaaatgg caccatacga
 301 aatattctgg gtggcacggt cttcagagaa gccattatct gcaaaaatat cccccggctt
 361 gtgagtggat gggtaaaacc tatcatcata ggtcgtcatg cctatgggga tcaatacaga
 421 gcaactgatt ttgttgttcc tgggcctgga aaagtagaga taacctacac accaagtgac
 481 ggaacccaaa aggtgacata cctggtacat aactttgaag aaggtggtgg tgttgccatg
 541 gggatgtata atcaagataa gtcaattgaa gattttgcac acagttcctt ccaaatggct
 601 ctgtctaagg gttggccttt gtatctgagc accaaaaaca ctattctgaa gaaatatgat
 661 gggcgtttta aagacatctt tcaggagata tatgacaagc agtacaagtc ccagtttgaa
 721 gctcaaaaga tctggtatga gcataggctc atcgacgaca tggtggccca agctatgaaa
 781 tcagagggag gcttcatctg ggcctgtaaa aactatgatg gtgacgtgca gtcggactct
 841 gtggcccaag ggtatggctc tctcggcatg atgaccagcg tgctggtttg tccagatggc
 901 aagacagtag aagcagaggc tgcccacggg actgtaaccc gtcactaccg catgtaccag
 961 aaaggacagg agacgtccac caatcccatt gcttccattt ttgcctggac cagagggtta
1021 gcccacagag caaagcttga taacaataaa gagcttgcct tctttgcaaa tgctttggaa
1081 gaagtctcta ttgagacaat tgaggctggc ttcatgacca aggacttggc tgcttgcatt
1141 aaaggtttac ccaatgtgca acgttctgac tacttgaata catttgagtt catggataaa
1201 cttggagaaa acttgaagat caaactagct caggccaaac tttaa
```

FIG. 1A

```
   1 cctgtggtcc cgggtttctg cagagtctac ttcagaagcg gaggcactgg gagtccggtt
  61 tgggattgcc aggctgtggt tgtgagtctg agcttgtgag cggctgtggc gccccaactc
 121 ttcgccagca tatcatcccg gcaggcgata aactacattc agttgagtct gcaagactgg
 181 gaggaactgg ggtgataaga aatctattca ctgtcaaggt ttattgaagt caaaatgtcc
 241 aaaaaaatca gtggcggttc tgtggtagag atgcaaggag atgaaatgac acgaatcatt
 301 tgggaattga ttaaagagaa actcattttt ccctacgtgg aattggatct acatagctat
 361 gatttaggca tagagaatcg tgatgccacc aacgaccaag tcaccaagga tgctgcagaa
 421 gctataaaga agcataatgt tggcgtcaaa tgtgccacta tcactcctga tgagaagagg
 481 gttgaggagt tcaagttgaa acaaatgtgg aaatcaccaa atggcaccat acgaaatatt
 541 ctgggtggca cggtcttcag agaagccatt atctgcaaaa atatcccccg gcttgtgagt
 601 ggatgggtaa aacctatcat cataggtcgt catgcttatg gggatcaata cagagcaact
 661 gattttgttg ttcctgggcc tggaaaagta gagataacct acacaccaag tgacggaacc
 721 caaaaggtga catacctggt acataacttt gaagaaggtg gtggtgttgc catggggatg
 781 tataatcaag ataagtcaat tgaagatttt gcacacagtt ccttccaaat ggctctgtct
 841 aagggttggc ctttgtatct gagcaccaaa aacactattc tgaagaaata tgatgggcgt
 901 tttaaagaca tctttcagga gatatatgac aagcagtaca agtcccagtt tgaagctcaa
 961 aagatctggt atgagcatag gctcatcgac gacatggtgg cccaagctat gaaatcagag
1021 ggaggcttca tctgggcctg taaaaactat gatggtgacg tgcagtcgga ctctgtggcc
1081 caagggtatg gctctctcgg catgatgacc agcgtgctgg tttgtccaga tggcaagaca
1141 gtagaagcag aggctgccca cgggactgta acccgtcact accgcatgta ccagaaagga
1201 caggagacgt ccaccaatcc cattgcttcc atttttgcct ggaccagagg gttagcccac
1261 agagcaaagc ttgataacaa taaagagctt gccttctttg caaatgcttt ggaagaagtc
1321 tctattgaga caattgaggc tggcttcatg accaaggact tggctgcttg cattaaaggt
1381 ttacccaatg tgcaacgttc tgactacttg aatacatttg agttcatgga taaacttgga
1441 gaaaacttga agatcaaact agctcaggcc aaactttaag ttcatacctg agctaagaag
1501 gataattgtc ttttggtaac taggtctaca ggtttacatt tttctgtgtt acactcaagg
1561 ataaaggcaa aatcaatttt gtaatttgtt tagaagccag agtttatctt ttctataagt
1621 ttacagcctt ttttttatat atacagttat tgccaccttt gtgaacatgg caagggactt
1681 ttttacaatt tttattttat ttttctagtac cagcctagga attcggttag tactcatttg
1741 tattcactgt cactttttct catgttctaa ttataaatga ccaaaatcaa gattgctcaa
1801 aagggtaaat gatagccaca gtattgctcc ctaaaatatg cataaagtag aaattcactg
1861 ccttccccte ctgtccatga ccttgggcac agggaagttc tggtgtcata gatatcccgt
1921 tttgtgaggt agagctgtgc attaaacttg cacatgactg gaacgaagta tgagtgcaac
1981 tcaaatgtgt tgaagatact gcagtcattt ttgtaaagac cttgctgaat gtttccaata
2041 gactaaatac tgtttaggcc gcaggagagt ttggaatccg gaataaatac tacctggagg
2101 tttgtcctct ccattttctt ctttctcctc ctggccctggc ctgaatatta tactactcta
2161 aatagcatat ttcatccaag tgcaataatg taagctgaat cttttttgga cttctgctgg
2221 cctgttttat ttcttttata taaatgtgat ttctcagaaa ttgatattaa acactatctt
2281 atcttctcct gaactgttga ttttaattaa aattaagtgc taattaccaa aaaaaaaa
```

FIG. 1B

MAGYLRVVRSLCRASGSRPAWAPAALTAPTSQEQPRRHYADKRIKVAKPV
VEMDGDEMTRIIWQFIKEKLILPHVDIQLKYFDLGLPNRDQTDDQVTIDS
ALATQKYSVAVKCATITPDEARVEEFKLKKMWKSPNGTIRNILGGTVFRE
PIICKNIPRLVPGWTKPITIGRHAHGDQYKATDFVADRAGTFKMVFTPKD
GSGVKEWEVYNFPAGGVGMGMYNTDESISGFAHSCFQYAIQKKWPLYMST
KNTILKAYDGRFKDIFQEIFDKHYKTDFDKNKIWYEHRLIDDMVAQVLKS
SGGFVWACKNYDGDVQSDILAQGFGSLGLMTSVLVCPDGKTIEAEAAHGT
VTRHYREHQKGRPTSTNPIASIFAWTRGLEHRGKLDGNQDLIRFAQMLEK
VCVETVESGAMTKDLAGCIHGLSNVKLNEHFLNTTDFLDTIKSNLDRALG
RQ

Amino Acid sequence of IDH2 (GenBank Accession No. NM_002168.2)

FIG. 2

```
   1 atggccggct acctgcgggt cgtgcgctcg ctctgcagag cctcaggctc gcggccggcc
  61 tgggcgccgg cggccctgac agcccccacc tcgcaagagc agccgcggcg ccactatgcc
 121 gacaaaagga tcaaggtggc gaagcccgtg gtggagatgg atggtgatga gatgacccgt
 181 attatctggc agttcatcaa ggagaagctc atcctgcccc acgtggacat ccagctaaag
 241 tattttgacc tcgggctacc aaaccgtgac cagactgatg accaggtcac cattgactct
 301 gcactggcca cccagaagta cagtgtggct gtcaagtgtg ccaccatcac ccctgatgag
 361 gcccgtgtgg aagagttcaa gctgaagaag atgtggaaaa gtcccaatgg aactatccgg
 421 aacatcctgg gggggactgt cttccgggag cccatcatct gcaaaaacat cccacgccta
 481 gtccctggct ggaccaagcc catcaccatt ggcaggcacg cccatggcga ccagtacaag
 541 gccacagact ttgtggcaga ccgggccggc actttcaaaa tggtcttcac ccaaaagat
 601 ggcagtggtg tcaaggagtg ggaagtgtac aacttccccg caggcggcgt gggcatgggc
 661 atgtacaaca ccgacgagtc catctcaggt tttgcgcaca gctgcttcca gtatgccatc
 721 cagaagaaat ggccgctgta catgagcacc aagaacacca tactgaaagc ctacgatggg
 781 cgtttcaagg acatcttcca ggagatcttt gacaagcact ataagaccga cttcgacaag
 841 aataagatct ggtatgagca ccggctcatt gatgacatgg tggctcaggt cctcaagtct
 901 tcgggtggct ttgtgtgggc ctgcaagaac tatgacggag atgtgcagtc agacatcctg
 961 gcccagggct ttggctccct tggcctgatg acgtccgtcc tggtctgccc tgatgggaag
1021 acgattgagg ctgaggccgc tcatgggacc gtcacccgcc actatcggga gcaccagaag
1081 ggccggccca ccagcaccaa ccccatcgcc agcatctttg cctggacacg tggcctggag
1141 caccggggga agctggatgg gaaccaagac ctcatcaggt ttgcccagat gctggagaag
1201 gtgtgcgtgg agacggtgga gagtggagcc atgaccaagg acctggcggg ctgcattcac
1261 ggcctcagca atgtgaagct gaacgagcac ttcctgaaca ccacggactt cctcgacacc
1321 atcaagagca acctggacag agccctgggc aggcagtag
```

cDNA sequence of IDH2 (GenBank Accession No. NM_002168.2)

FIG. 2A

```
   1 ccagcgttag cccgcggcca ggcagccggg aggagcggcg cgcgctcgga cctctcccgc
  61 cctgctcgtt cgctctccag cttgggatgg ccggctacct gcgggtcgtg cgctcgctct
 121 gcagagcctc aggctcgcgg ccggcctggg cgccggcggc cctgacagcc cccacctcgc
 181 aagagcagcc gcggcgccac tatgccgaca aaaggatcaa ggtggcgaag cccgtggtgg
 241 agatggatgg tgatgagatg acccgtatta tctggcagtt catcaaggag aagctcatcc
 301 tgccccacgt ggacatccag ctaaagtatt ttgacctcgg gctcccaaac cgtgaccaga
 361 ctgatgacca ggtcaccatt gactctgcac tggccaccca gaagtacagt gtggctgtca
 421 agtgtgccac catcacccct gatgaggccc gtgtggaaga gttcaagctg aagaagatgt
 481 ggaaaagtcc caatggaact atccggaaca tcctgggggg gactgtcttc cgggagccca
 541 tcatctgcaa aaacatccca cgcctagtcc ctggctggac caagcccatc accattggca
 601 ggcacgccca tggcgaccag tacaaggcca cagactttgt ggcagaccgg gccggcactt
 661 tcaaaatggt cttcacccca aaagatggca gtggtgtcaa ggagtgggaa gtgtacaact
 721 tccccgcagg cggcgtgggc atgggcatgt acaacaccga cgagtccatc tcaggttttg
 781 cgcacagctg cttccagtat gccatccaga agaaatggcc gctgtacatg agcaccaaga
 841 acaccatact gaaagcctac gatgggcgtt tcaaggacat cttccaggag atctttgaca
 901 agcactataa gaccgacttc gacaagaata agatctggta tgagcaccgg ctcattgatg
 961 acatggtggc tcaggtcctc aagtcttcgg gtggctttgt gtgggcctgc aagaactatg
1021 acggagatgt gcagtcagac atcctggccc agggctttgg ctcccttggc ctgatgacgt
1081 ccgtcctggt ctgccctgat gggaagacga ttgaggctga ggccgctcat gggaccgtca
1141 cccgccacta tcgggagcac cagaagggcc ggcccaccag caccaacccc atcgccagca
1201 tctttgcctg gacacgtggc ctggagcacc gggggaagct ggatgggaac caagacctca
1261 tcaggtttgc ccagatgctg gagaaggtgt gcgtggagac ggtggagagt ggagccatga
1321 ccaaggacct ggcgggctgc attcacggcc tcagcaatgt gaagctgaac gagcacttcc
1381 tgaacaccac ggacttcctc gacaccatca agagcaacct ggacagagcc ctgggcaggc
1441 agtaggggga ggcgccaccc atggctgcag tggaggggcc agggctgagc cggcgggtcc
1501 tcctgagcgc ggcagagggt gagcctcaca gcccctctct ggaggccttt ctaggggatg
1561 tttttttata agccagatgt ttttaaaagc atatgtgtgt ttcccctcat ggtgacgtga
1621 ggcaggagca gtgcgtttta cctcagccag tcagtatgtt ttgcatactg taatttatat
1681 tgcccttgga acacatggtg ccatatttag ctactaaaaa gctcttcaca aaaaaaaaaa
```

mRNA *sequence of* IDH2 (GenBank Accession No. NM_002168.2)

FIG. 2B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61285122 B
- US 61313532 B
- WO 200966072 A2


### Non-patent literature cited in the description


- **KANG et al.** *Int. J. Cancer,* 2009, vol. 125, 353-355
- **R. LAROCK.** Comprehensive Organic Transformations. VCH Publishers, 1989
- **T.W. GREENE ; P.G.M. WUTS.** Protective Groups in Organic Synthesis. John Wiley and Sons, 1991
- **L. FIESER ; M. FIESER.** Fieser and Fieser's Reagents for Organic Synthesis. John Wiley and Sons, 1994
- Encyclopedia of Reagents for Organic Synthesis. John Wiley and Sons, 1995
- **OBRECHT, D. ; VILLALGRODO, J.M.** Solid-Supported Combinatorial and Parallel Synthesis of Small-Molecular-Weight Compound Libraries. Pergamon-Elsevier Science Limited, 1998
- **CZARNIK, A.W.** *Curr. Opin. Chem. Bio.,* 1997, vol. 1, 60
- **BERGE et al.** Pharmaceutically Acceptable Salts. *J. Pharm. ScL.,* 1977, vol. 66, 1-19
- **T. GREEN ; P. WUTS.** Protective Groups in Organic Synthesis. John Wiley and Sons, 1999
- *Acta Neuropathol,* 2008, vol. 116, 597-602
- *N Engl J Med.,* 19 February 2009, vol. 360 (8), 765-73
- **NEKRUTENKO et al.** *Mol. Biol. Evol.,* 1998, vol. 15, 1674-1684
- **GEISBRECHT et al.** *J. Biol. Chem.,* 1999, vol. 274, 30527-30533
- **WIEMANN et al.** *Genome Res.,* 2001, vol. 11, 422-435
- The MGC Project Team. *Genome Res.,* 2004, vol. 14, 2121-2127
- **SJOEBLOM et al.** *Science,* 2006, vol. 314, 268-274
- **LUO et al.** *J Chromatogr A,* 2007, vol. 1147, 153-64
- **MUNGER et al.** *Nat Biotechnol,* 2008, vol. 26, 1179-86